# EUROPEAN PATENT APPLICATION

(11) **EP 4 644 396 A1**
(43) Date of publication of application: **05.11.2025**
(21) Application number: 23910769.1
(22) Date of filing: 27.12.2023
(51) Int. Cl.: C07D 519/00, C07D 471/14, C07D 471/04, A61K 31/4162, A61K 31/4985, A61P 37/00, A61P 37/02

(54) **TOLL-LIKE RECEPTOR INHIBITOR, AND PREPARATION THEREFOR AND APPLICATION THEREOF**

(30) Priority: 28.12.2022 CN 202211700845; 15.06.2023 CN 202310712078; 30.08.2023 CN 202311106941
(71) Applicant: Visionpharma (Nantong) Co., Ltd, Jiangsu 226001 (CN)
(72) Inventor: TANG, Guozhi, Nantong, Jiangsu 226001 (CN); ZHOU, Yunlong, Nantong, Jiangsu 226001 (CN); LIU, Yongfu, Nantong, Jiangsu 226001 (CN); WANG, Yingyi, Nantong, Jiangsu 226001 (CN); WANG, Junpu, Nantong, Jiangsu 226001 (CN); YANG, Jiangtao, Nantong, Jiangsu 226001 (CN); LI, Chunshui, Nantong, Jiangsu 226001 (CN); CHEN, Junli, Nantong, Jiangsu 226001 (CN); ZHANG, Guoliang, Nantong, Jiangsu 226001 (CN); MA, Dawei, Nantong, Jiangsu 226001 (CN)
(74) Representative: Lavoix
(86) International application number: PCT/CN2023/142500
(87) International publication number: WO 2024/140851

(57) **Abstract**

The present invention provides a toll-like receptor inhibitors, and preparation therefor and an application thereof. Specifically, the present invention provides a compound as shown in formula I below, a pharmaceutically acceptable salt, racemate, R-isomer, or S-isomer thereof, or a mixture thereof. The compound can be used for preparing a pharmaceutical composition for treating or preventing autoimmune diseases or chronic inflammatory diseases.

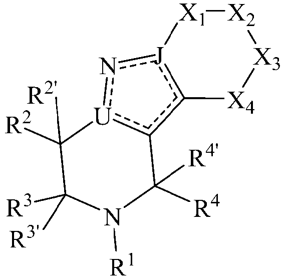

## Description

### Technical field

This invention, in particular, provides a class of toll-like receptor inhibitors, their preparation and applications thereof.

### Background Technology

Autoimmune diseases are a kind of chronic systemic inflammatory disorders characterized by dysregulation of the immune system that ultimately leads to decreased tolerance to autoantigens. Although the exact biology and pathogenesis of these disorders remain unclear, aberrant processes of the innate and adaptive immune systems have been shown to be involved in their pathogenesis. Multiple studies have shown that toll-like receptors (TLRs) play an important role in a variety of autoimmune diseases, including dry syndrome, systemic lupus erythematosus, multiple sclerosis, rheumatoid arthritis, systemic sclerosis and psoriasis.

TLRs are a class of structurally conserved proteins that form the first barrier in the innate immune response. By identifying various conservative molecular patterns associated with causative agents (pathogen-associated molecular patterns, or PAMP), TLRs recognize endogenous ligands released after invasive microbial and tissue damage or the death of non-physiologic cells. Upon those ligand binding, TLRs are activated, and signal cascades are initiated to produce pro-inflammatory cytokines. There are 10 known members of the human TLRs family, which are type I transmembrane proteins characterized by leucine-rich extracellular domains and containing a conserved toll / interleukin-1 receptor (TDR). Cytoplasmic tail of the toll / interleukin (IL) -1 receptor, TIR) domain. In this family, TLR3, TLR7, TLR8, and TLR9 are located in the endosomal compartment. Endosomal TLRs recognize viral and endogenous double-stranded RNA (dsRNA; TLR3), single-stranded RNA (ssRNA; TLR7 / 8) or an unmethylated CpG sequence (TLR9).

TLR7 and 8 have been linked to the onset and disease progression of certain autoimmune disorders that target auto-RNA and DNA / protein complexes, which are most likely released during normal cell death and clearance. There are substantial scientific studies that link inappropriate or sustained endogenous activation of the TLR7 / 8 pathway to constant responses to autoantigens in many autoimmune diseases. TLR7 has been shown to play an important role in the pathogenesis of lupus erythematosus. In addition, TLR8 polymorphism was associated with rheumatoid arthritis. Since most autoimmune diseases may benefit from treatments involving the regulation of interferon (IFN) production and B cell activity, thus, compounds modulating TLR7 and / or TLR8 activity and methods of using these compounds may provide substantial therapeutic benefits for a variety of autoimmune patients.

In summary, there is an urgent need to discover and develop compounds that can be used to modulate TLR7 and / or TLR8 activity, for the treatment of autoimmune diseases.

### SUMMARY

The present invention provides a class of novel compounds that can be used to modulate TLR7 and / or TLR8 activities for treatment of autoimmune diseases.

The first aspect of the present invention provides a compound shown in formula I, a pharmaceutically acceptable salt, a racemate, an R-isomer, an S-isomer, or mixtures thereof: 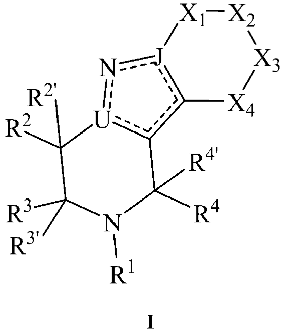 wherein,
the dashed line is a chemical bond or none; and 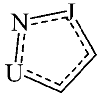 is an aromatic ring;
U is selected from the group consisting of: C and N;
J is selected from the group consisting of: C and N;
X₁, X₂, X₃, and X₄ are each independently selected from the group consisting of: bond, - O-, -S-, -S(=O)-, -S(=O)₂-, -C(=O)-, -C(R⁵R⁵')-, -C(R¹⁵)(L₁-NR¹⁶R¹⁶')-, -N(R⁸)-;
R¹ is selected from the group consisting of: substituted or unsubstituted fused 5, 6 membered heteroaryl, and substituted or unsubstituted fused 6, 6 membered heteroaryl;
R², R³, R⁴, R²', R³', and R⁴' are each independently selected from the group consisting of: H, deuterium, substituted or unsubstituted C₁₋₆ alkoxy, carboxyl, NH₂, substituted or unsubstituted C₁₋₆ alkyl, halogen, cyano, substituted or unsubstituted C₂₋₆ ester group, substituted or unsubstituted C₁₋₆ alkylene-COOH, substituted or unsubstituted C₂₋₆ acylamino, substituted or unsubstituted (CH₂)C(O)N(R¹⁷)₂, substituted or unsubstituted C₃₋₇ cycloalkyl, hydroxyl, substituted or unsubstituted C₁₋₉ heteroalkyl, substituted or unsubstituted 3-to 9- membered heterocycloalkyl; or R², R³, R⁴, R²', R³', and R⁴' located on the same carbon atom together form=O; or R², R²' and the carbon atom to which they attached, R³, R³' and the carbon atom to which they attached, or R⁴, R⁴' and the carbon atom to which they attached together form a substituted or unsubstituted 3- to 6- membered cycloalky, or a substituted or unsubstituted 4- to 6- membered heterocyclyl;
R⁵ and R⁵' are each independently selected from the group consisting of: H, deuterium, substituted or unsubstituted C₁₋₆ alkoxy, carboxyl, NH₂, halogenated C₁₋₆ alkyl, substituted or unsubstituted C₁₋₆ alkyl, halogen, cyano, substituted or unsubstituted C₂₋₆ ester group, substituted or unsubstituted C₁₋₆ alkylene-COOH, substituted or unsubstituted C₂₋₆ acylamino, substituted or unsubstituted (CH₂)C(O)N(R¹⁷)₂, substituted or unsubstituted C₃₋₇ cycloalkyl, hydroxyl, substituted or unsubstituted C₆-C₁₀ aryl-(CH₂)-, substituted or unsubstituted 5-to 12- membered heteroaryl-(CH₂)-, substituted or unsubstituted C₆-C₁₀ aryl, substituted or unsubstituted 5-to 12- membered heteroaryl, substituted or unsubstituted C₁₋₉ heteroalkyl, substituted or unsubstituted 3-to 9- membered heterocycloalkyl, substituted or unsubstituted 3-to 9- membered heterocycloalkyl-(CH₂)-; or each R⁵, and R⁵' located on the same carbon atom together form =O;
or R⁵, R⁵' and the carbon atom to which they attached together form a substituted or unsubstituted 3- to 6- membered cycloalky, or a substituted or unsubstituted 4- to 6-membered heterocyclyl;
or two of R⁵, R⁵' and R⁸ located on different atom together with the carbon or nitrogen atom to which they attached (optionally including the ring atom between the carbon atom or nitrogen atom to which they attached) together form a substituted or unsubstituted 3- to 6- membered cycloalky, or a substituted or unsubstituted 4- to 9- membered heterocyclyl;
L₁ is a chemical bond or C₁~C₆ alkylene, preferably a chemical bond;
R¹⁵ is independently selected from the group consisting of: H, deuterium, C₁₋₆ alkoxy, halogenated C₁₋₆ alkyl, C₁₋₆ alkyl, halogen, cyano, C₃₋₇ cycloalkyl, hydroxyl, hydroxyl substituted C₁₋₆ alkyl; or R¹⁵ and R¹⁶, R¹⁵ and R¹⁶' together form a substituted or unsubstituted 4- to 6- membered heterocycloalkyl; or R¹⁵, L₁ and the carbon atom to which they attached together form a substituted or unsubstituted 3- to 6- membered cycloalky, or a substituted or unsubstituted 4- to 6- membered heterocyclyl;
R¹⁶ and R¹⁶' are each independently selected from the group consisting of: H, deuterium, substituted or unsubstituted 3- to 9- membered carbocyclyl (saturated, unsaturated monocyclic ring, spiro ring, fused ring, or bridged ring), substituted or unsubstituted 4-to 9- membered heterocyclyl (saturated, unsaturated monocyclic ring, spiro ring, fused ring, or bridged ring), substituted or unsubstituted C₁₋₆ alkyl, benzyl, - (CH₂)ₙN(R⁸⁻¹R⁸⁻²), -(CH₂)ₙC(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, -C(O)(CH₂)ₙR⁸⁻³, C₁₋₆ alkyl NHS(O)₂-, C₁₋₆ alkyl NHC(O)-, -C(O)(CH₂)ₙN(R⁸⁻¹R⁸⁻²), -SO₂(CH₂)ₙN(R⁸⁻¹R⁸⁻²), (CH₂)ₙSO₂N(R⁸⁻¹R⁸⁻²), C₁-C₆ alkoxy, C₁-C₆ alkyl-amido, C₆-C₁₀ arylbenzyl, and 5 or 6 membered heteroarylbenzyl;
or R¹⁶ and R¹⁶' together form a substituted or unsubstituted 4-to 8- membered heterocycloalkyl;
R¹⁷ is selected from the group consisting of: H, substituted or unsubstituted C₁₋₆ alkyl, substituted or unsubstituted C₁₋₉ heteroalkyl, substituted or unsubstituted C₆-C₁₀ aryl, substituted or unsubstituted 5- or 6-membered heteroaryl, substituted or unsubstituted 3- to 9- membered heterocycloalkyl, and substituted or unsubstituted C₃₋₇ cycloalkyl;
R⁸ is selected from the group consisting of: H, deuterium, substituted or unsubstituted 3- to 9- membered carbocyclyl, substituted or unsubstituted 4- to 9- membered heterocyclyl, substituted or unsubstituted 3- to 9- membered carbocyclyl-(CH₂)-, substituted or unsubstituted 4- to 9- membered heterocyclyl-(CH₂)-, substituted or unsubstituted C₁₋₆ alkyl, substituted or unsubstituted benzyl, and substituted or unsubstituted group selected from the group consisting of: -(CH₂)ₙN(R⁸⁻¹R⁸⁻²), -(CH₂)ₙC(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, - C(O)(CH₂)ₙR⁸⁻³, C₁₋₆ alkyl NHS(O)₂-, C₁₋₆ alkyl NHC(O)-, -C(O)(CH₂)ₙN(R⁸⁻¹R⁸⁻²), - SO₂(CH₂)ₙN(R⁸⁻¹R⁸⁻²), (CH₂)ₙSO₂N(R⁸⁻¹R⁸⁻²), C₁-C₆ alkoxy, C₁-C₂ alkyl-amido, substituted or unsubstituted C₆-C₁₀ aryl-(CH₂)-, substituted or unsubstituted 5- to 12- membered heteroaryl-(CH₂)-; wherein, when a group is substituted, the "substituted" can be located at the CH, CH₂, or CH₃ position of the group;
wherein, the "substituted" refers to one or more hydrogen atoms on the group are substituted with substituents selected from the group consisting of: halogen, hydroxyl, carboxyl, benzyl, oxo(=O), -(CH₂)ₙN(R⁸⁻¹R⁸⁻²), -(CH₂)ₙC(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, - C(O)(CH₂)ₙR⁸⁻³, amino, C₁₋₆ alkyl NHS(O)₂-, C₁₋₆ alkyl NHC(O)-, -C(O)(CH₂)ₙN(R⁸⁻¹R⁸⁻²), -SO₂(CH₂)ₙN(R⁸⁻¹R⁸⁻²), (CH₂)ₙSO₂N(R⁸⁻¹R⁸⁻²), nitro, cyano, unsubstituted or halogenated C₁-C₆ alkyl, C₂-C₁₀ alkenyl, C₁-C₆ alkoxy, C₁-C₆ alkyl-amido, C₆-C₁₀ aryl, 5 or 6 membered heteroaryl, -O-(C₆-C₁₀ aryl), -O- (5 or 6 membered heteroaryl), and unsubstituted or halogenated C₃₋₇ heterocycloalkyl;
R⁸⁻¹ and R⁸⁻² are selected from the group consisting of: hydrogen, C₁₋₆ alkyl, C₁₋₆ haloalkyl, C₃₋₆ cycloalkyl, 4- to 6- membered heterocycloalkyl, or R⁸⁻¹, R⁸⁻² and the nitrogen atom to which they attached together form 4- to 6- membered heterocycloalkyl; the cycloalkyl and heterocycloalkyl can be substituted with one or more halogen; wherein R⁸⁻³ is selected from the group consisting of: hydrogen, C₁₋₆ alkyl, C₁₋₆ alkoxy, C₁₋₆ haloalkyl, C₃₋₆ cycloalkyl, and 4- to 6- membered heterocycloalkyl; and the cycloalkyl and heterocycloalkyl can be substituted with one or more halogen or C₁₋₆ alkyl; n is 0, 1, 2, 3, 4, 5, or 6;
unless otherwise specified, when appeared alone or as part of other groups, the heterocycloalkyl or heteroaryl has 1, 2, or 3 heteroatoms selected from N, O, and S; the heteroalkyl is an alkyl group in which 1-3 CH₂ or CH on the carbon chain are substituted by heteroatoms selected from the group consisting of: NH, N, O, and S; the cycloalkyl and heterocycloalkyl include saturated monocyclic ring, spiro ring, fused ring, and bridged ring; and the carbocyclyl and heterocyclyl include saturated or partially unsaturated non-aromatic rings, including monocyclic ring, spiro ring, fused ring, and bridged ring.

In another preferred embodiment, the compound of formula I has a structure as shown in formula I-1', formula I-2', formula I-3', or formula I-4': 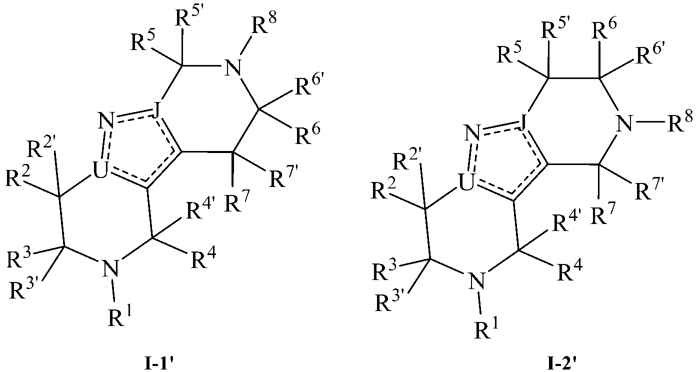 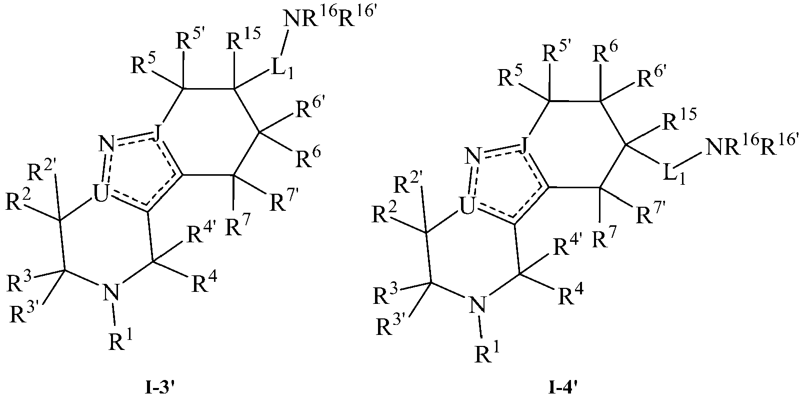
R⁵, R⁶, R⁷, R⁵', R⁶', and R⁷' are each independently selected from the group consisting of: H, deuterium, substituted or unsubstituted C₁₋₆ alkoxy, halogenated C₁₋₆ alkyl, substituted or unsubstituted C₁₋₆ alkyl, halogen, cyano, substituted or unsubstituted C₁₋₆ ester group, substituted or unsubstituted C₁₋₆ carboxylic acid, substituted or unsubstituted C₁₋₆ acylamino, substituted or unsubstituted C₃₋₇ cycloalkyl, hydroxyl, hydroxyl substituted C₁₋₆ alkyl, substituted or unsubstituted C₆-C₁₀ arylbenzyl, substituted or unsubstituted 5 or 6 membered heteroaryl benzyl, substituted or unsubstituted C₆-C₁₀ aryl, substituted or unsubstituted 5 or 6 membered heteroaryl, C₁₋₉ heteroalkyl with 1 to 3 heteroatoms, wherein the heteroatoms are selected from one or more of NH, N, O, and S; or two of R⁵, R⁶, R⁷ , R⁵', R⁶', and R⁷' located on the same carbon atom together form=O;
or R⁵, R⁵' and the carbon atom to which they attached, R⁶, R⁶' and the carbon atom to which they attached, or R⁷, R⁷' and the carbon atom to which they attachedtogether form a substituted or unsubstituted 3- to 6- membered cycloalky, or a substituted or unsubstituted 4- to 6- membered heterocyclyl;
or R⁵ and R⁶, R⁵ and R⁷, or R⁶ and R⁷ and the carbon atom to which they attached (optionally including the ring atom between the carbon atoms or nitrogen atom to which they attached) together form a substituted or unsubstituted 3- to 6- membered cycloalky, or a substituted or unsubstituted 4- to 9- membered heterocyclyl;
the definitions of the remaining groups are as described in the first aspect.

In another preferred embodiment, R¹ is selected from the group consisting of: 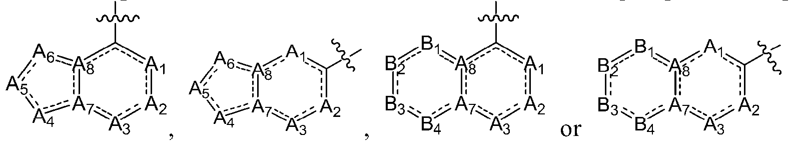
wherein, A₁, A₂, A₃, A₄, A₅, A₆, B₁, B₂, B₃, and B₄ are each independently selected from the group consisting of: CR, N, and NR¹⁴;
A₇ and A₈ are independently selected from the group consisting of: C, and N;
R¹⁴ is selected from the group consisting of: H, C₁₋₆ alkyl, C₁₋₆ alkyl substituted with halogen, and C₃₋₇ cycloalkyl; and
each R is independently selected from the group consisting of: H, halogen, cyano, C₁₋₆ alkyl, C₁₋₆ alkoxy, C₃₋₇ cycloalkyl, C₁₋₆ alkyl substituted with Ra, and C₁₋₆ alkoxy substituted with Rb; wherein, Ra and Rb are each independently selected from the group consisting of: OH, halogen, cyano, C₁₋₃ alkyl, and C₁₋₃ alkoxy.

In another preferred embodiment, R¹ is selected from the group consisting of: 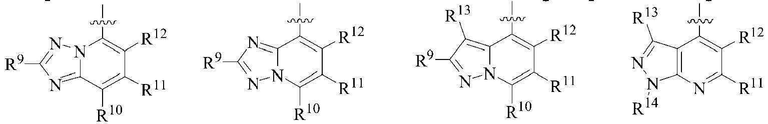 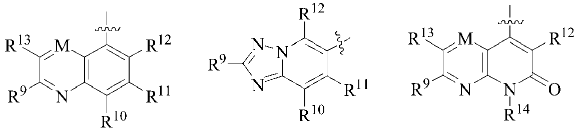
R⁹, R¹⁰, R¹¹, R¹², and R¹³ are each independently selected from the group consisting of: H, halogen, cyano, C₁₋₆ alkyl, C₁₋₆ alkoxy, C₃₋₇ cycloalkyl, C₁₋₆ alkyl substituted Ra, and C₁₋₆ alkoxy substituted with Rb; wherein, Ra and Rb are each independently selected from the group consisting of: OH, halogen, cyano, C₁₋₃ alkyl and C₁₋₃ alkoxy;
R¹⁴ is selected from the group consisting of: H, C₁₋₆ alkyl, C₁₋₆ alkyl substituted with halogen, and C₃₋₇ cycloalkyl; and
M is selected from the group consisting of: CH and N.

In another preferred embodiment, M is CH.

In another preferred embodiment, the compound of formula I has a structure shown in formula II-1, formula II-2, formula II-2', formula II-3 or formula II-4: 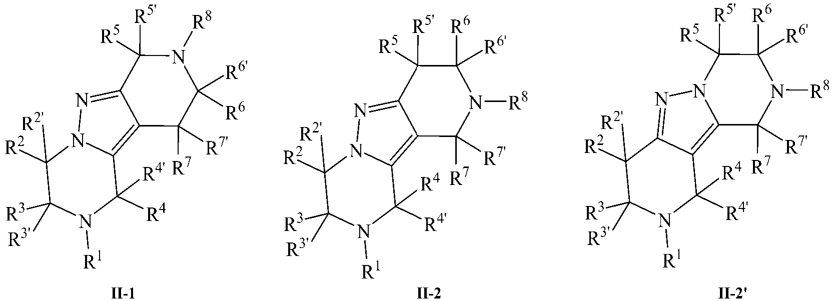 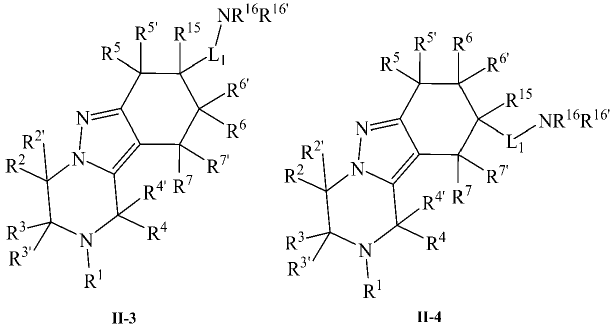 wherein, the definitions of each group are as described in the first aspect.

In another preferred embodiment, the compound of formula I has a structure as shown in formula III-1, formula III-2, formula III-2', formula III-3 or formula III-4; 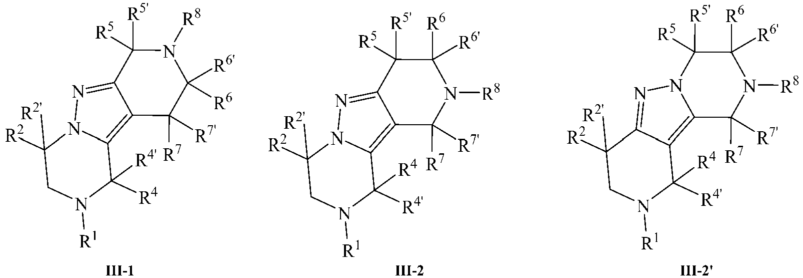 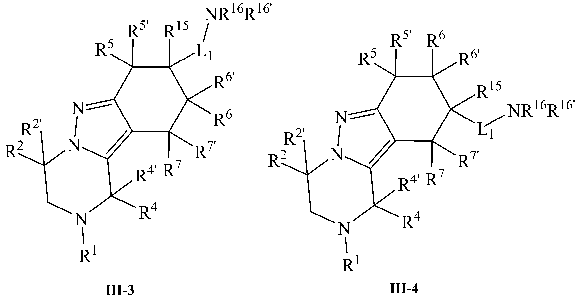 the definitions of each group are as described in the first aspect.

In another preferred embodiment, in the compounds of formula III-1, III-2, III-2', III-3, or III-4, R², R²', R⁴, R⁴', and R¹⁵ are each independently selected from the group consisting of: H, halogen, C₁₋₆ alkoxy, halogenated C₁₋₆ alkyl, C₁₋₆ alkyl, cyano, and C₃₋₇ cycloalkyl;
R⁵, R⁶, R⁷, R⁵', R⁶', and R⁷' are each independently selected from the group consisting of: H, deuterium, substituted or unsubstituted C₁₋₆ alkoxy, halogenated C₁₋₆ alkyl, substituted or unsubstituted C₁₋₆ alkyl, cyano, substituted or unsubstituted C₂₋₆ ester group, substituted or unsubstituted C₁₋₆ alkyl-COO, substituted or unsubstituted C₂₋₆ acylamino, substituted or unsubstituted (CH₂)C(O)N(R¹⁷)₂, substituted or unsubstituted C₃₋₇ cycloalkyl, hydroxyl, C₁₋₆ alkyl substituted with hydroxyl, substituted or unsubstituted benzyl, substituted or unsubstituted 5-to 9- membered heteroaryl-(CH₂)-, substituted or unsubstituted phenyl, substituted or unsubstituted 5-to 9- membered heteroaryl, substituted or unsubstituted C₁₋₉ heteroalkyl, substituted or unsubstituted 3-to 9- membered heterocycloalkyl, and substituted or unsubstituted 3-to 9- membered heterocycloalkyl-(CH₂)-, wherein the heteroatoms are selected from one or more of NH, N, O, and S; R¹⁷ is selected from the group consisting of: H, and substituted or unsubstituted C₁₋₆ alkyl;
or R³, R⁵' and the carbon atom to which they attached, R⁶ and R⁶' and the carbon atom to which they attached, or R⁷ and R⁷' and the carbon atom to which they attached together form a substituted or unsubstituted 3-to 6- membered cycloalkyl, or a substituted or unsubstituted 4-to 6- membered heterocyclyl;
or R⁵ and R⁶, R⁵ and R⁷, or R⁶ and R⁷ form a substituted or unsubstituted 3-to 6-membered cycloalkyl, or a substituted or unsubstituted 4-to 7- membered heterocyclyl;
or R⁶ and R⁶' together form=O; or R⁵ and R⁵' together form=O.

In another preferred embodiment, R¹⁶ and R¹⁶' are each independently selected from the group consisting of: H, deuterium, substituted or unsubstituted C₁₋₆ alkyl, and benzyl;
or R¹⁶ and R¹⁶' together form a substituted or unsubstituted 4-to 6- membered heterocycloalkyl.

In another preferred embodiment, R⁸ is selected from the group consisting of: H, substituted or unsubstituted 3-to 9- membered carbocyclyl (saturated, unsaturated monocyclic ring, spiro ring, fused ring, or bridged ring), substituted or unsubstituted 4-to 9- membered heterocyclyl (saturated, unsaturated monocyclic ring, spiro ring, fused ring, or bridged ring), substituted or unsubstituted C₁₋₆ alkyl, and substituted or unsubstituted group selected from the group consisting of: -(CH₂)ₙN(R⁸⁻¹R⁸⁻²), -(CH₂)ₙC(O)N(R⁸⁻¹R⁸⁻²), - (CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, - (CH₂)ₙC(O)R⁸⁻³, -C(O)(CH₂)ₙR⁸⁻³, C₁₋₆ alkyl NHS(O)₂-, C₁₋₆ alkyl NHC(O)-, - C(O)(CH₂)ₙN(R⁸⁻¹R⁸⁻²), -SO₂(CH₂)ₙN(R⁸⁻¹R⁸⁻²), -(CH₂)ₙSO₂N(R⁸⁻¹R⁸⁻²), C₁-C₆ alkoxy, C₁-C₂ alkyl-amido, C₆-C₁₀ aryl-(CH₂)-, and 5-to 9- membered heteroaryl-(CH₂)-.

In another preferred embodiment, R⁸⁻¹ and R⁸⁻² are selected from the group consisting of: hydrogen, C₁₋₆ alkyl, or R⁸⁻¹, R⁸⁻² and the N to which they attached together form a 4-to 6 -membered heterocycloalkyl, the cycloalkyl and heterocycloalkyl can be substituted with one or more halogens; R⁸⁻³ is selected from the group consisting of: hydrogen, C₁₋₆ alkyl, C₁₋₆ alkoxy, C₁₋₆ haloalkyl, C₃₋₆ cycloalkyl, and 4-to 6- membered heterocycloalkyl, the cycloalkyl and heterocycloalkyl can be substituted with one or more halogens or C₁₋₆ alkyl; n is 0, 1, 2, or 3.

In another preferred embodiment, R⁸ is selected from the group consisting of: H, C(O)NH₂, CH₂C(O)NH₂, CH₂C(O)N(CH₃)₂, ubstituted or unsubstituted 3-to 9- membered carbocyclyl, substituted or unsubstituted 4- to 9- membered nitrogen-containing heterocyclyl, substituted or unsubstituted 4- to 9- membered oxygen-containing heterocyclyl, substituted or unsubstituted C₁₋₆ alkyl, benzyl, -CH₂CH₂OH, -CH₂C(CH₃)₂OH, 5 or 6- membered heteroaryl benzyl.

In another preferred examples, R⁸ is selected from the following groups: H, C(O)NH₂, CH₂C(O)NH₂, CH₂C(O)N(CH₃)₂, substituted or unsubstituted C₁₋₆ alkyl, benzyl, - CH₂CH₂OH, -CH₂C(CH₃)₂OH, 5 or 6- membered heteroaryl benzyl, substituted or unsubstituted 3- to 9- membered carbocyclyl, substituted or unsubstituted 4- to 9-membered nitrogen-containing heterocyclyl, substituted or unsubstituted 4- to 9- membered oxygen-containing heterocyclyl, or selected from the group consisting of:

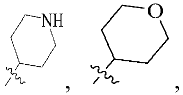

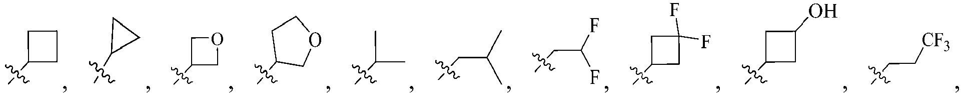

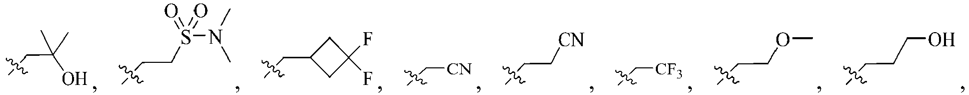

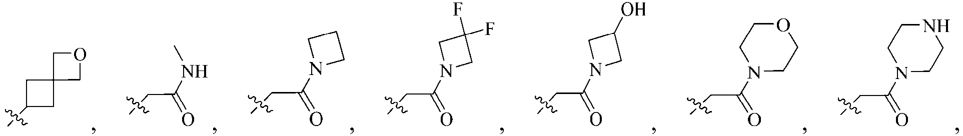

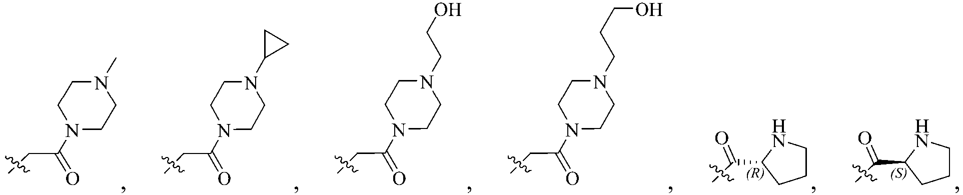

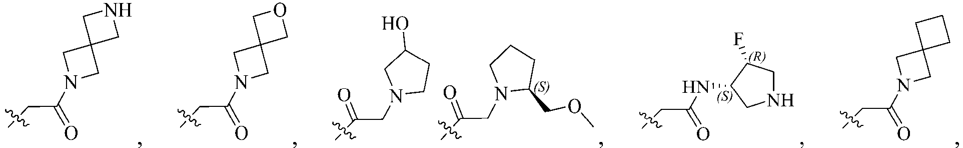

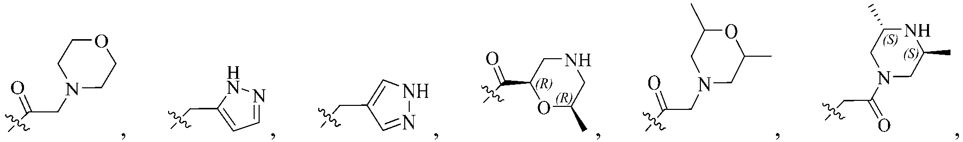

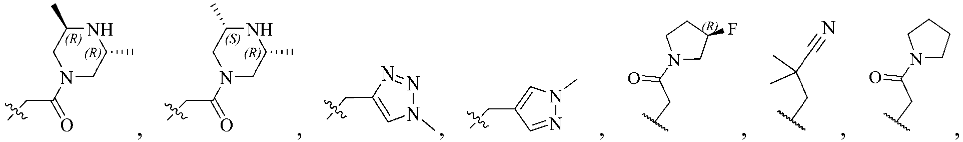

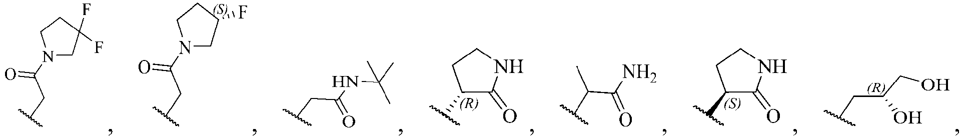

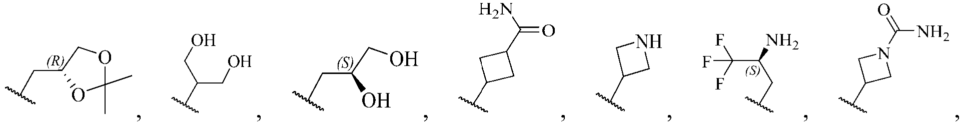

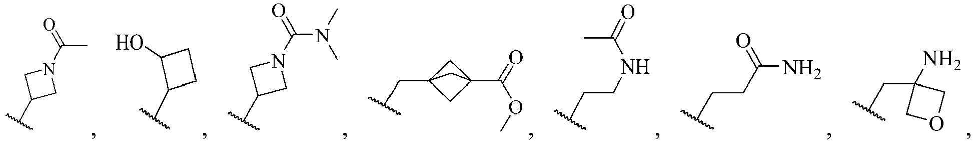

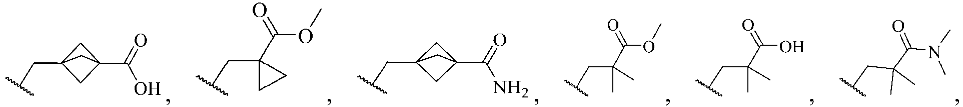

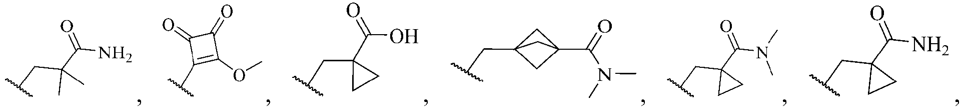

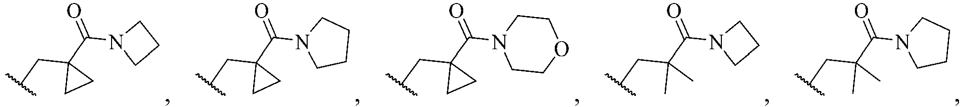

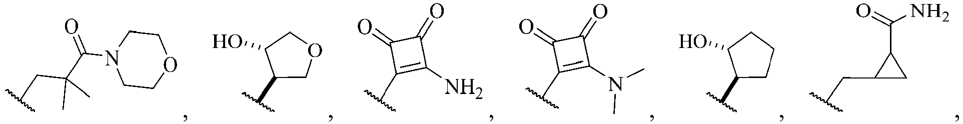

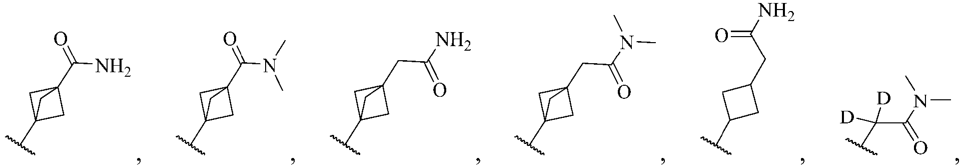

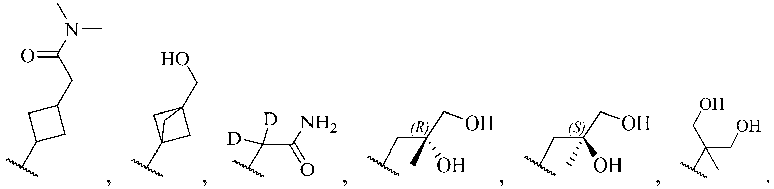

In another preferred embodiment, the compound is selected from the group consisting of:

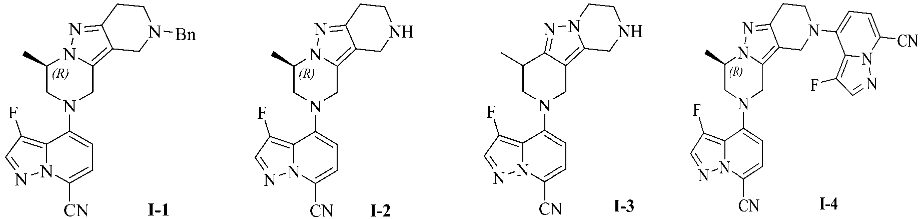

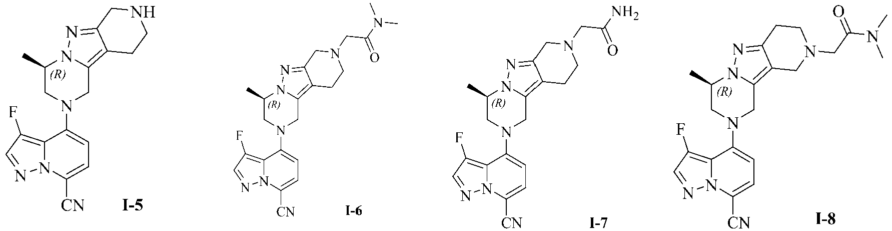

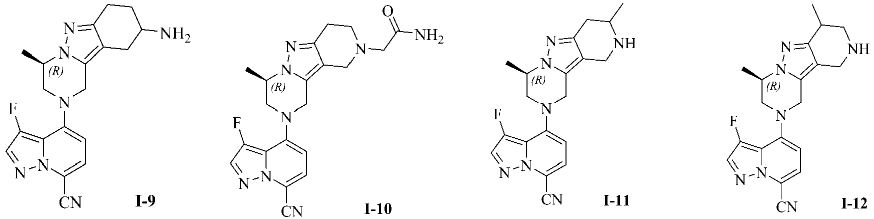

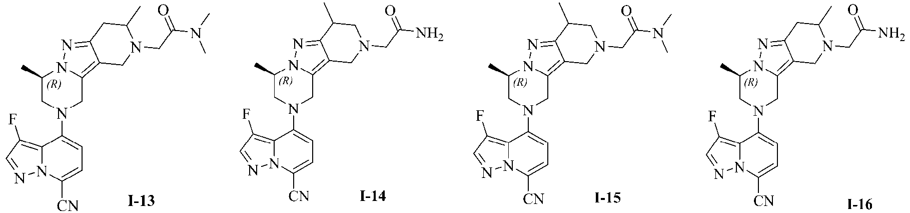

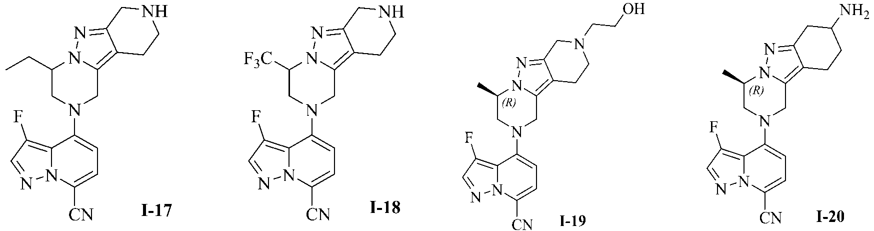

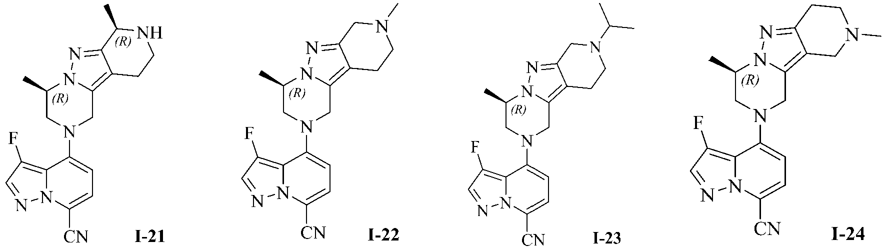

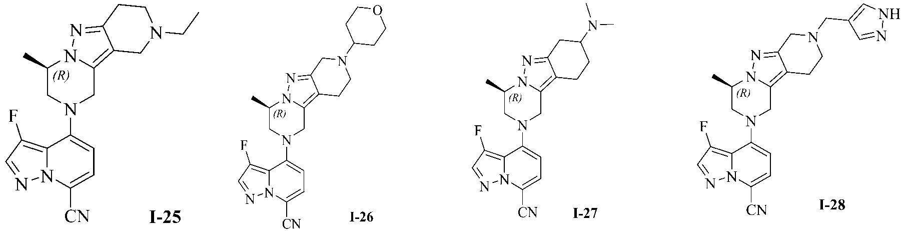

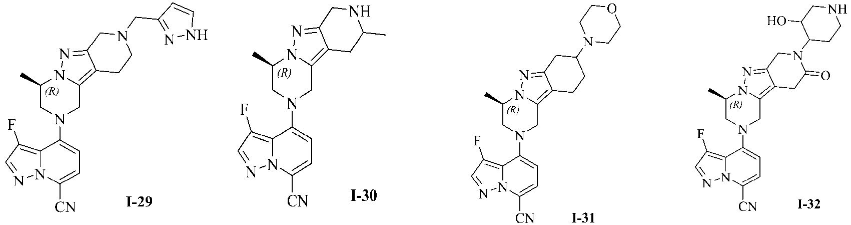

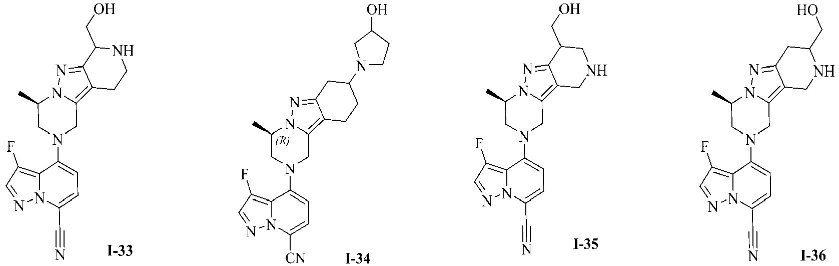

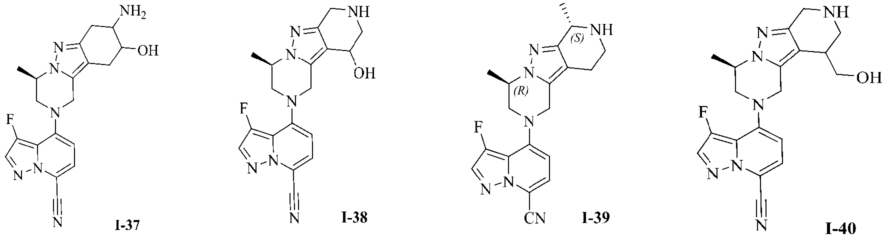

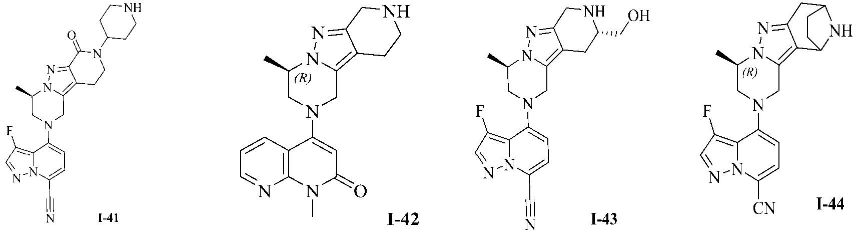

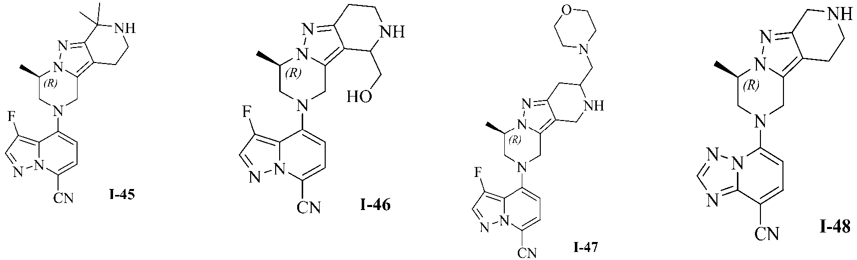

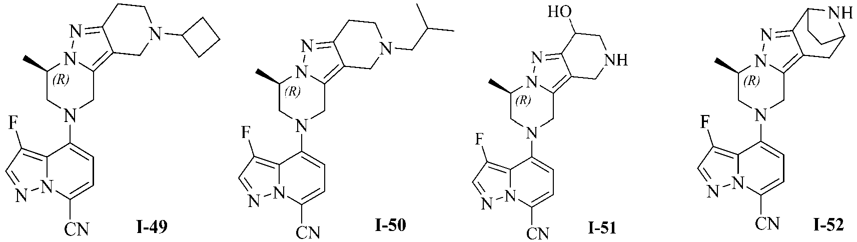

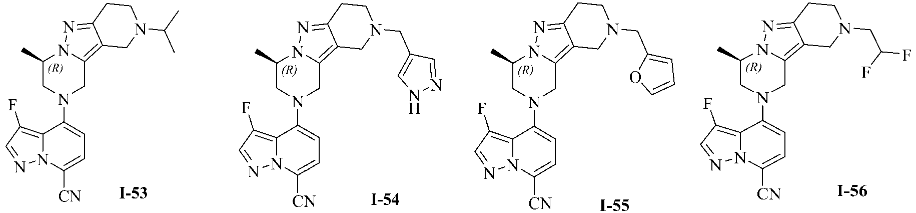

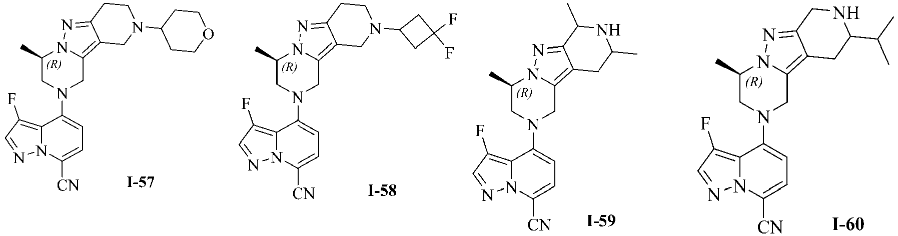

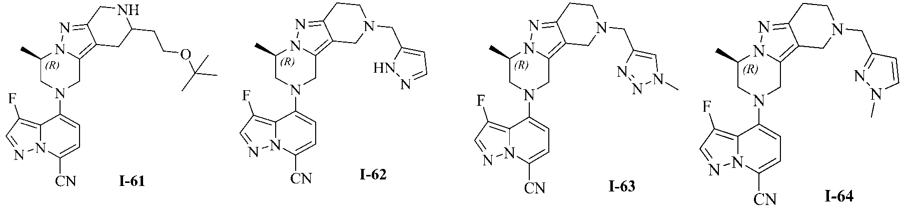

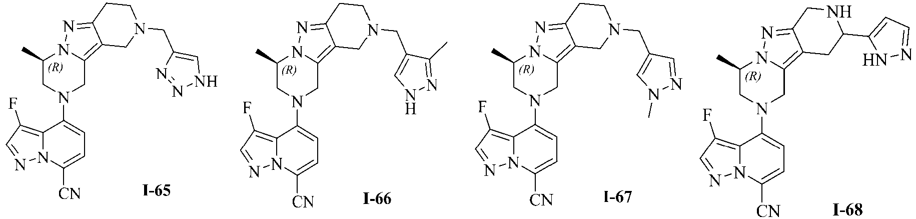

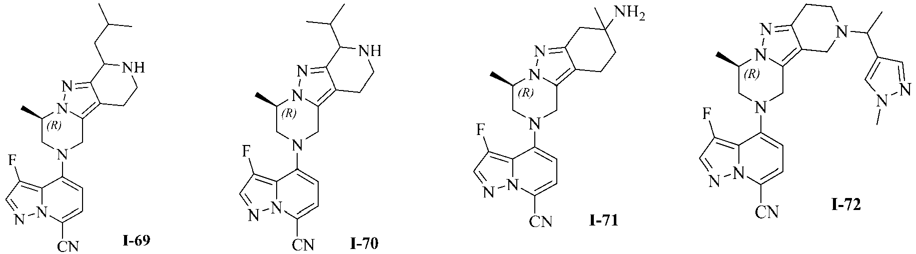

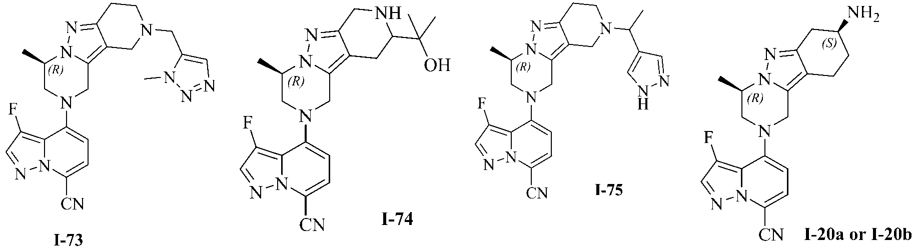

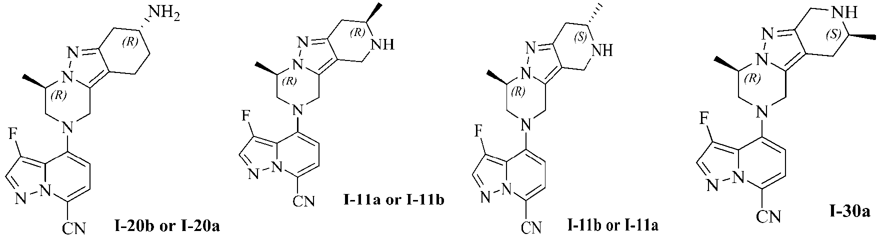

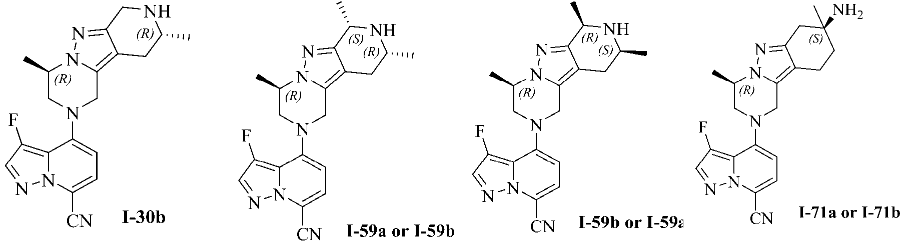

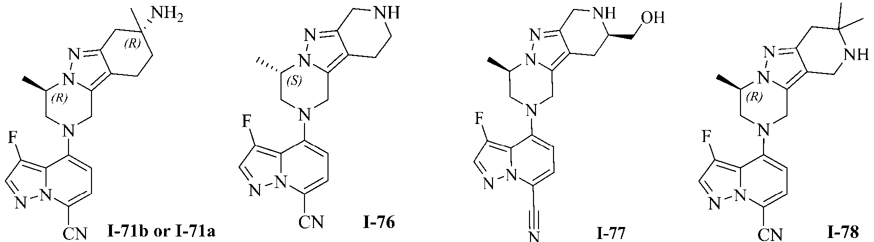

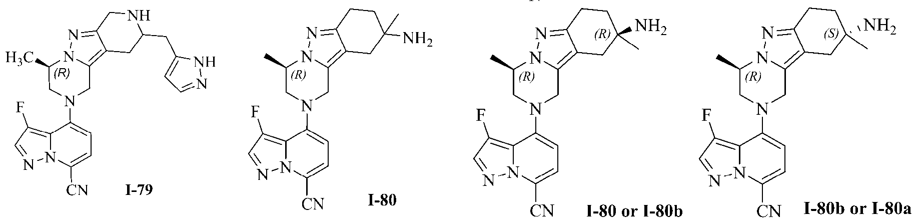

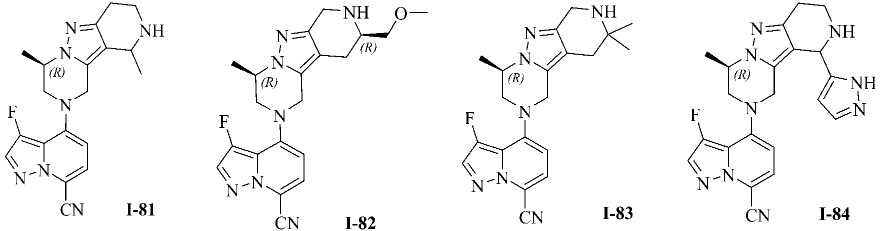

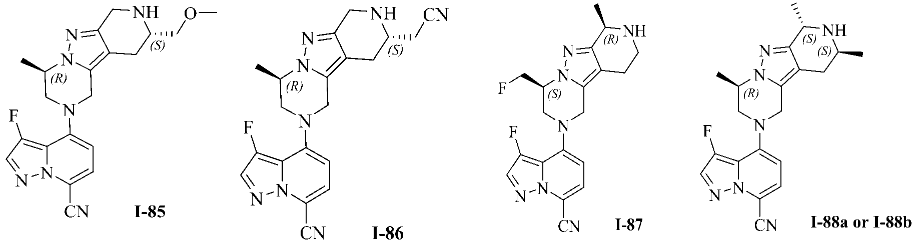

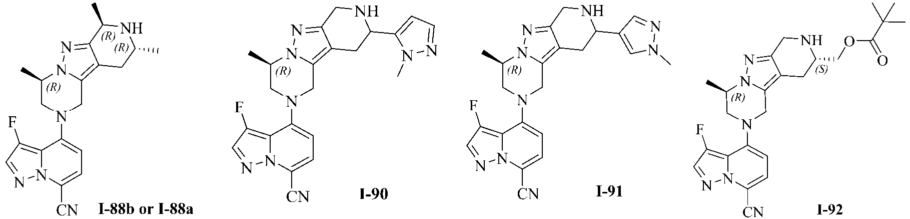

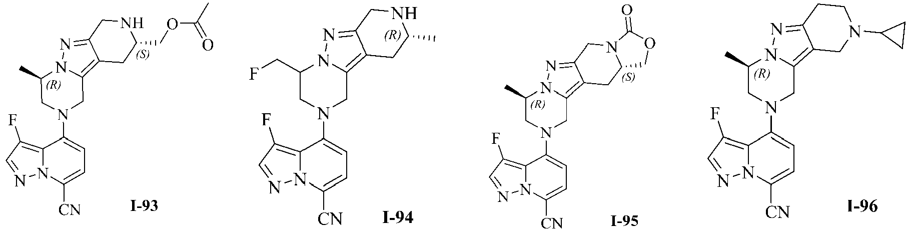

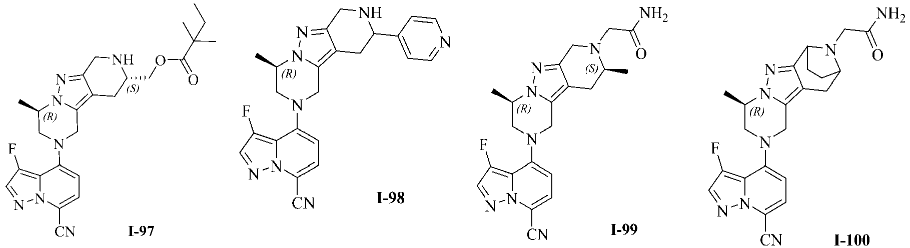

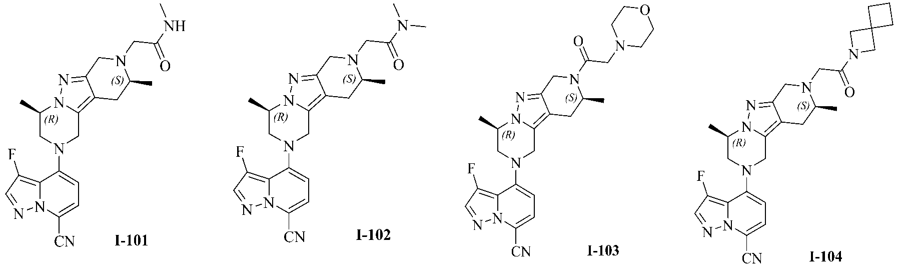

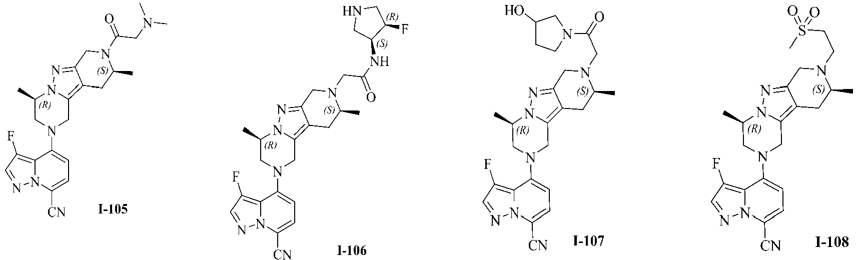

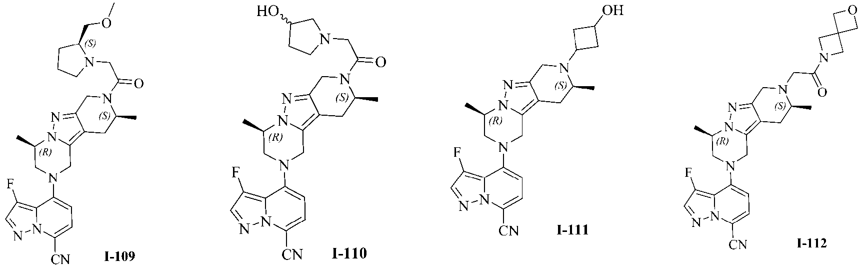

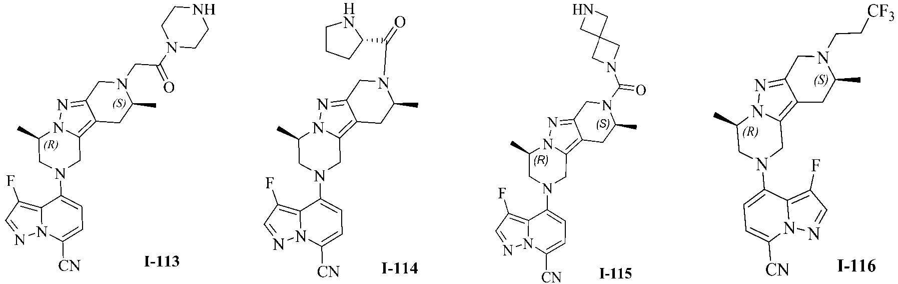

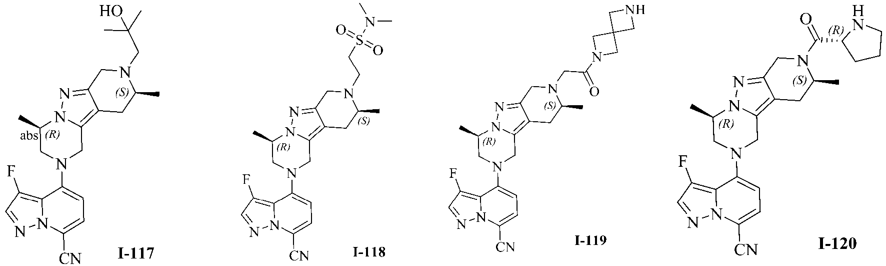

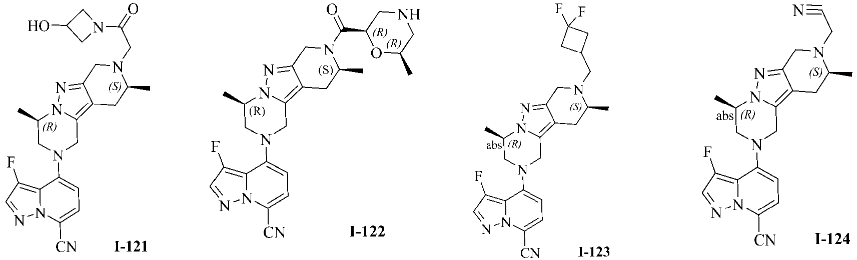

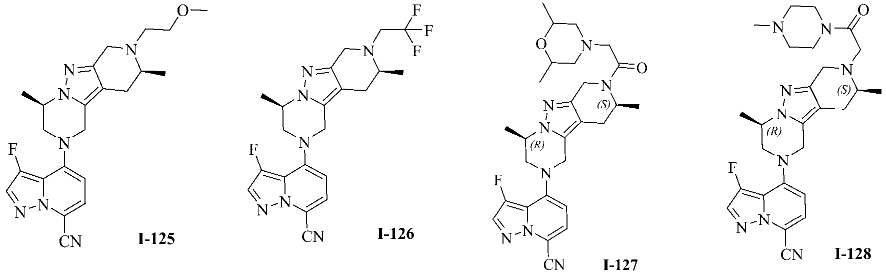

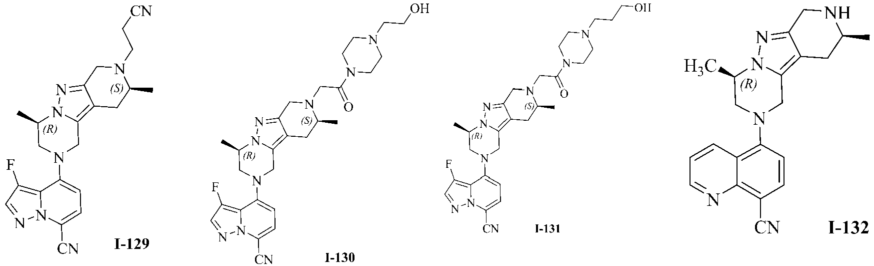

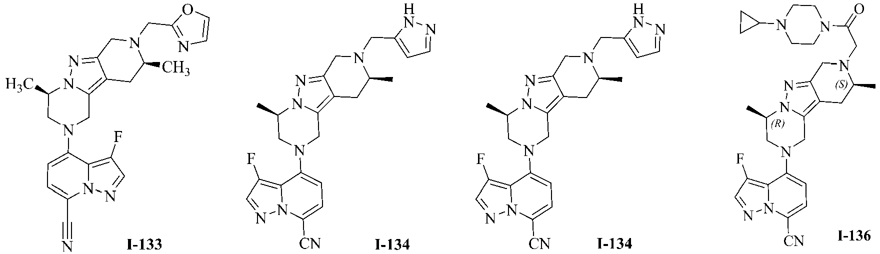

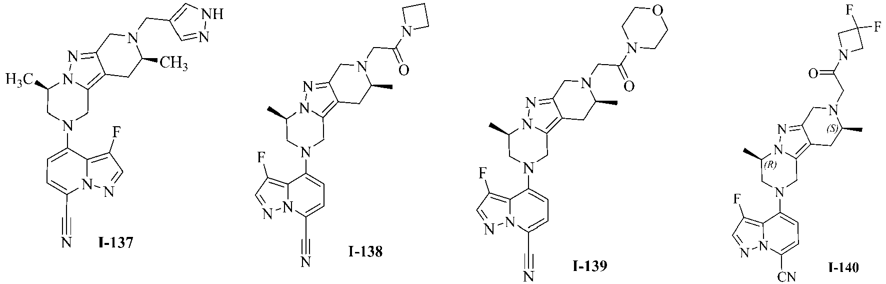

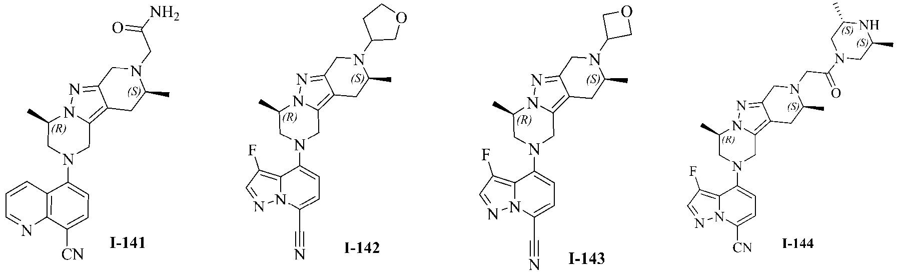

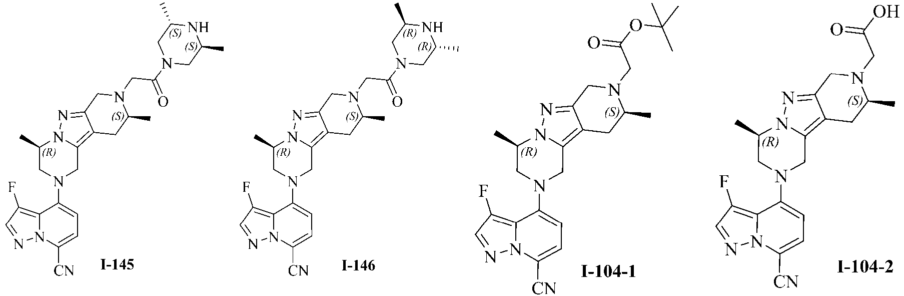

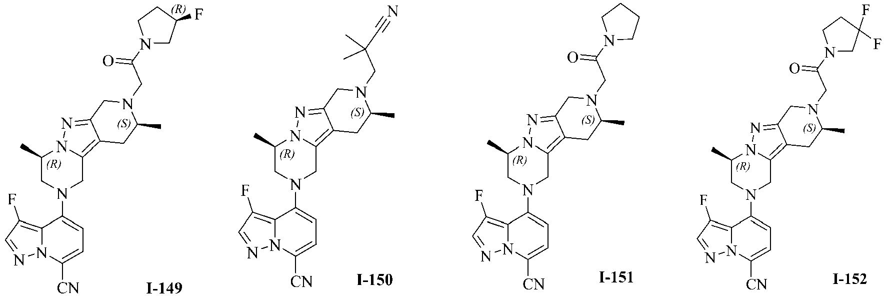

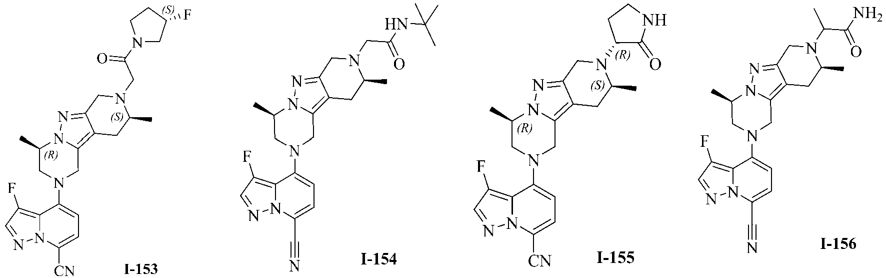

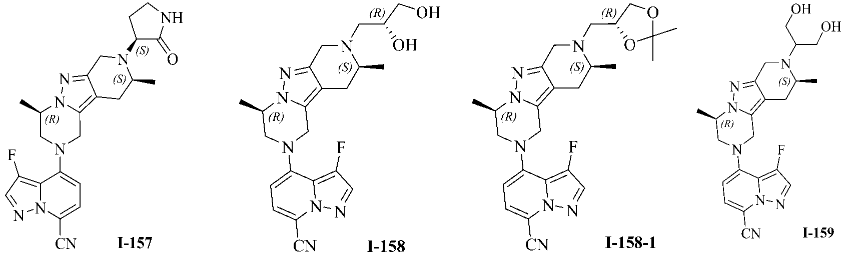

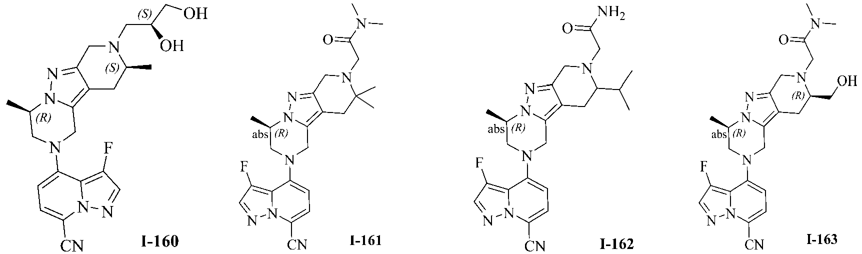

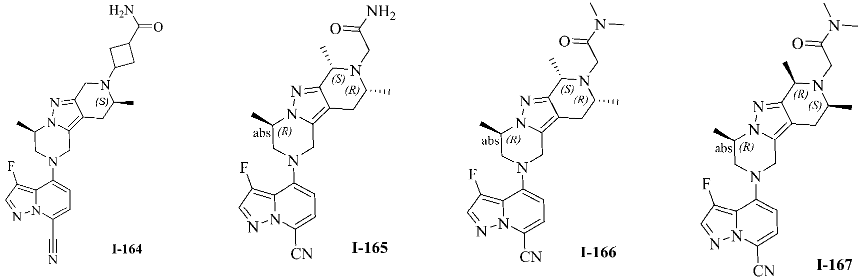

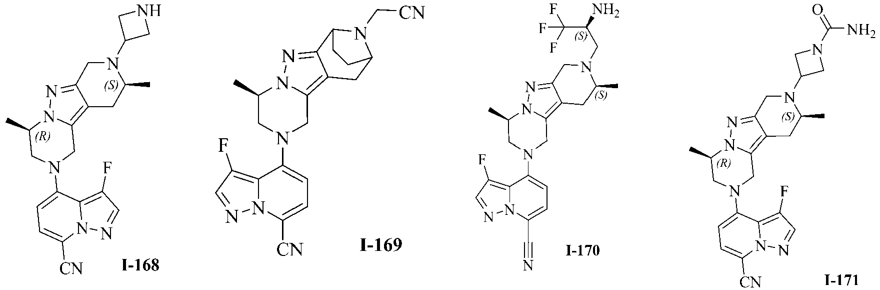

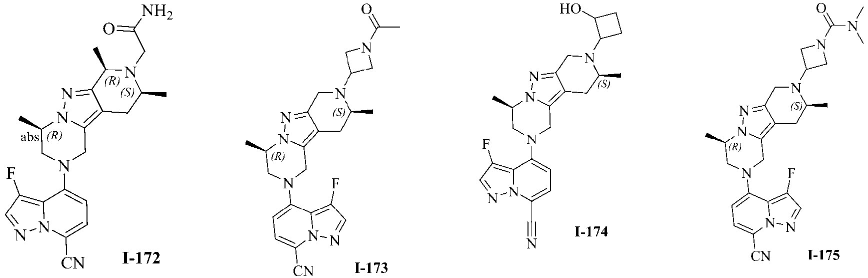

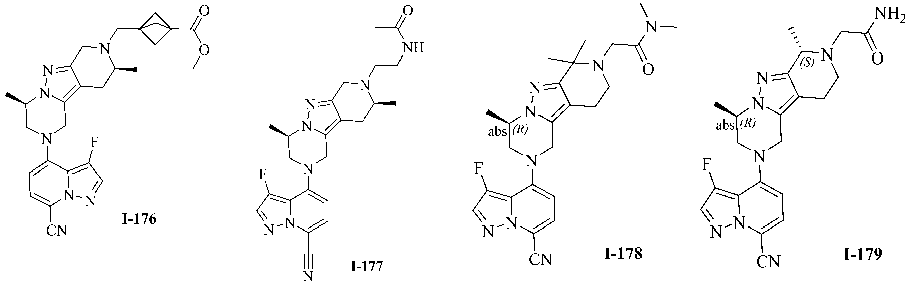

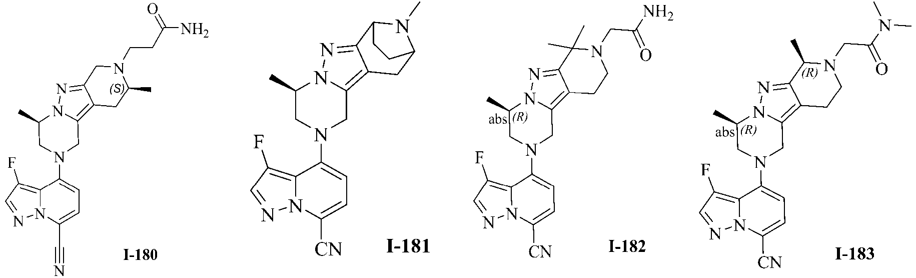

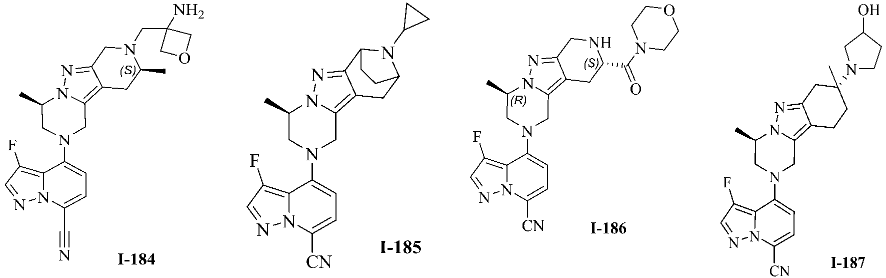

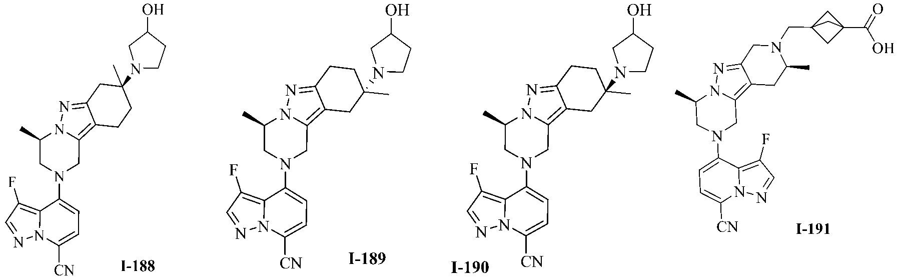

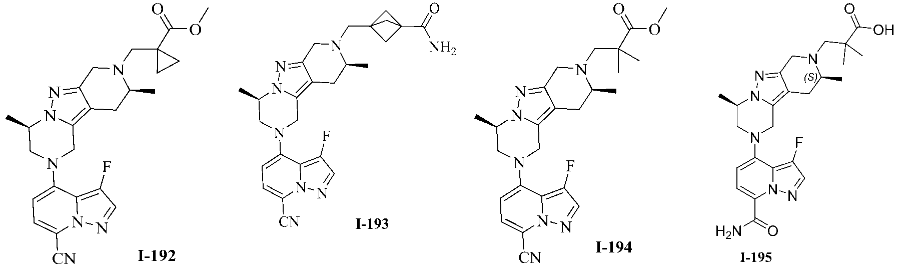

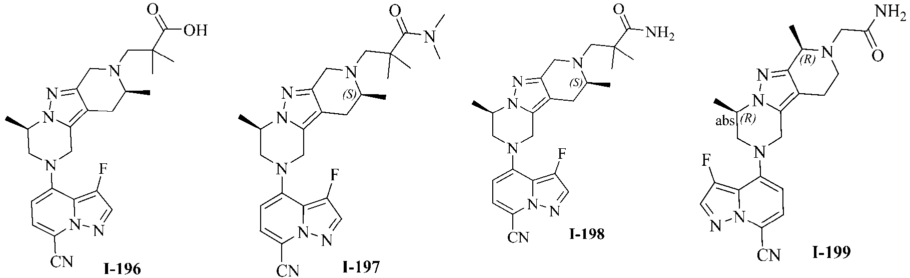

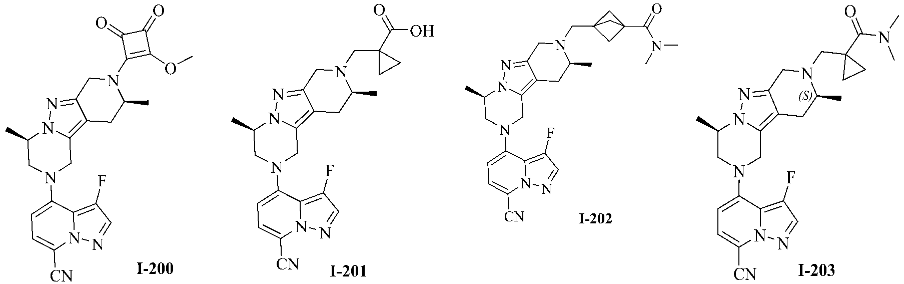

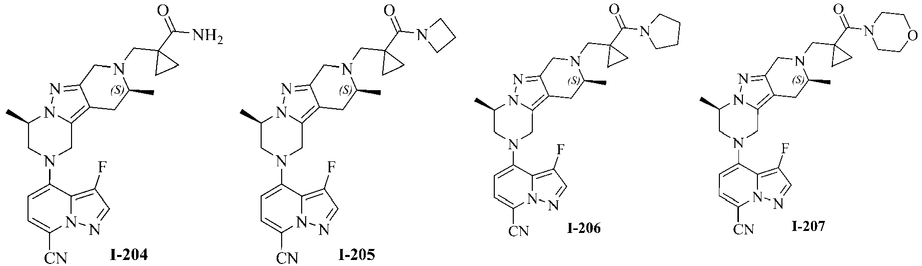

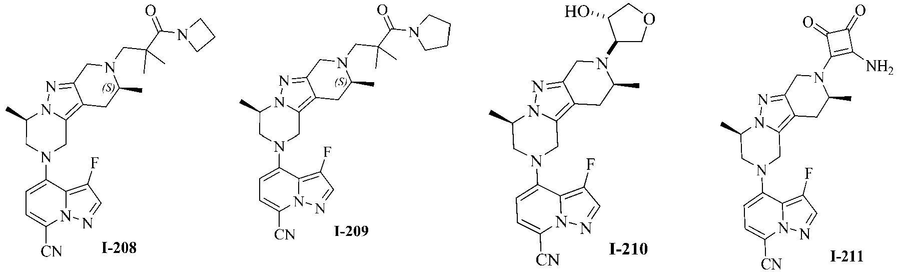

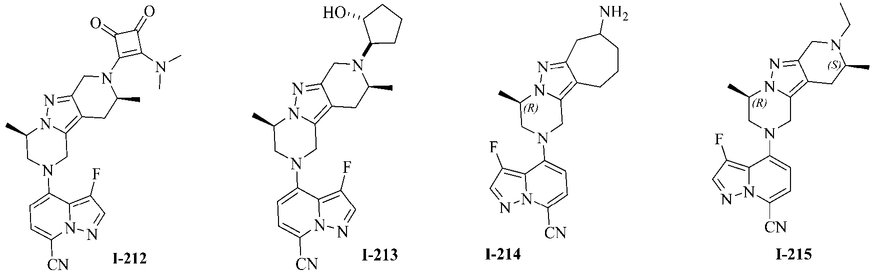

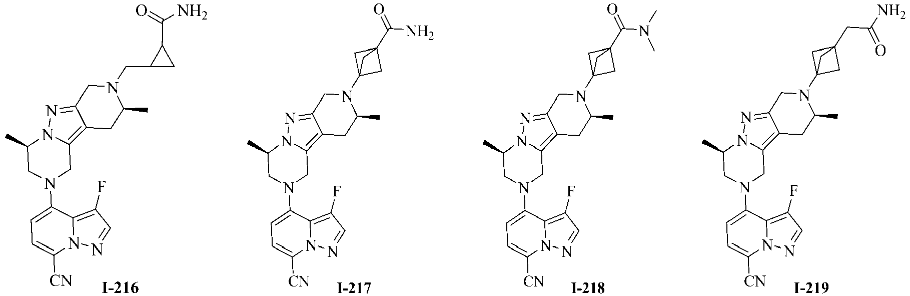

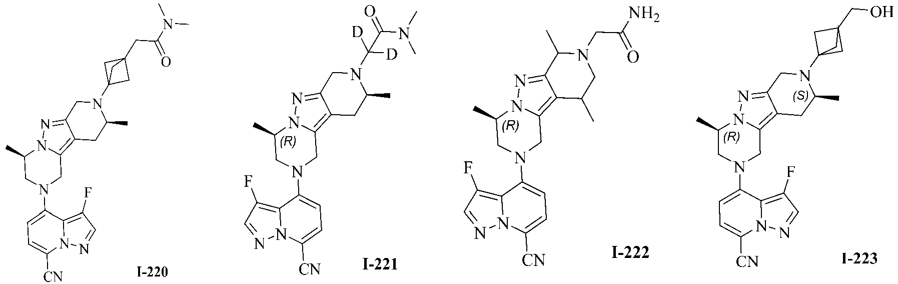

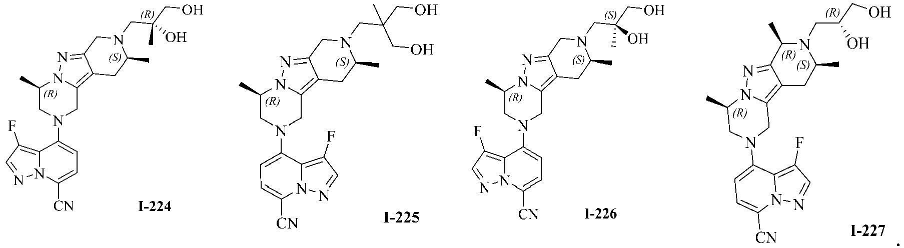

The second aspect of the present invention provides a pharmaceutical composition, wherein, the pharmaceutical composition comprises: one or more of the compound of formula I according to the first aspect of the present invention, the pharmaceutically acceptable salt, the racemate, the R-isomer, the S-isomer, ormixtures thereof, and one or more pharmaceutically acceptable carriers, excipients, adjuvants, excipients, and/or diluents.

The third aspect of the present invention provides a use of the compound of formula I according to the first aspect of the present invention, the pharmaceutically acceptable salt, the racemate, the R-isomer, the S-isomer, or mixtures thereof in the preparation of a pharmaceutical composition for the treatment or prevention of autoimmune diseases or chronic inflammatory diseases.

In another preferred embodiment, the disease is selected from the group consisting of: Sjogren's syndrome, systemic lupus erythematosus, multiple sclerosis, rheumatoid arthritis, systemic sclerosis, psoriasis, systemic lupus erythematosus, and lupus nephritis.

Another aspect of the present invention provides a conjugate, wherein the conjugate is obtained by connecting a compound or an intermediate of Formula B as described herein, to a biomolecule or monoclonal antibody (mAb) via chemical bonds.

The forth aspect of the present invention provides an intermediate shown in formula B 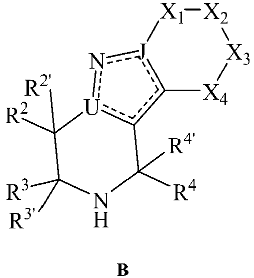
wherein, the definitions of each group are as described in the first aspect of the present invention;
preferably, the intermediate is selected from the group consisting of:

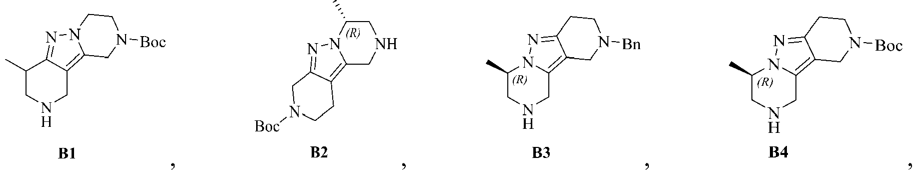

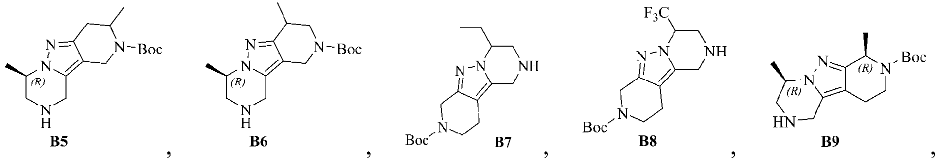

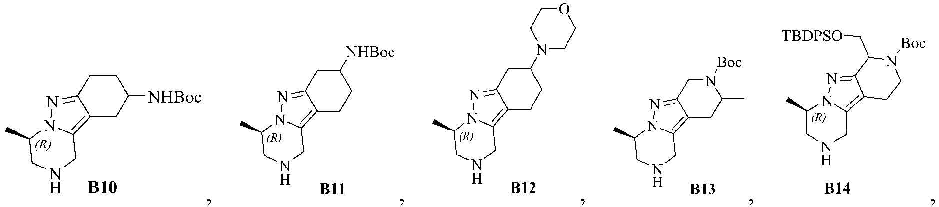

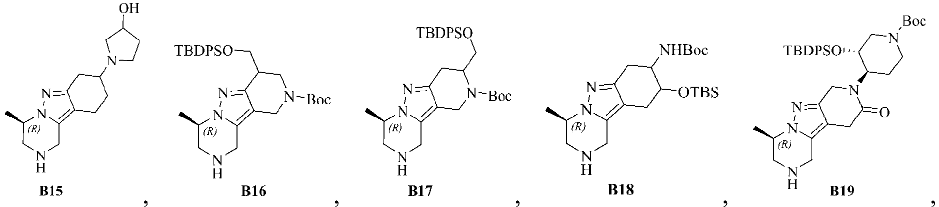

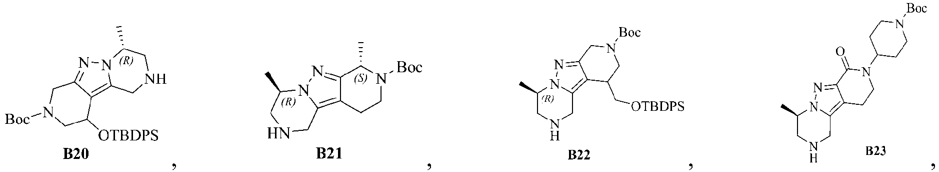

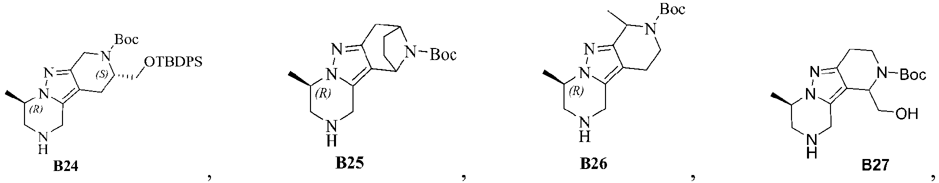

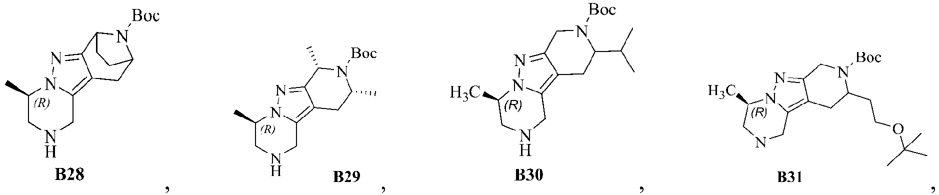

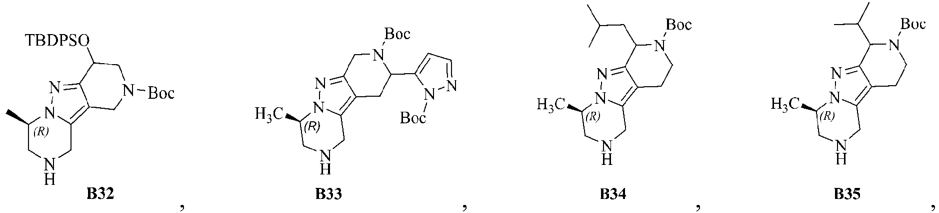

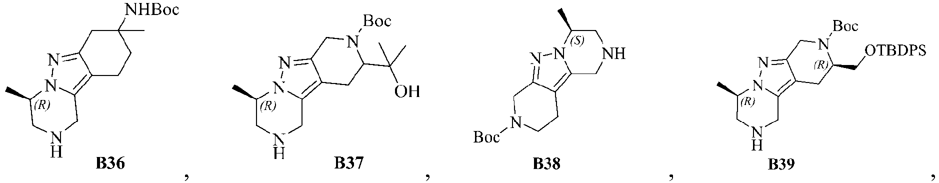

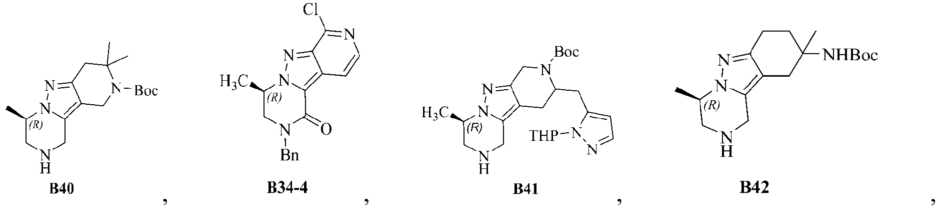

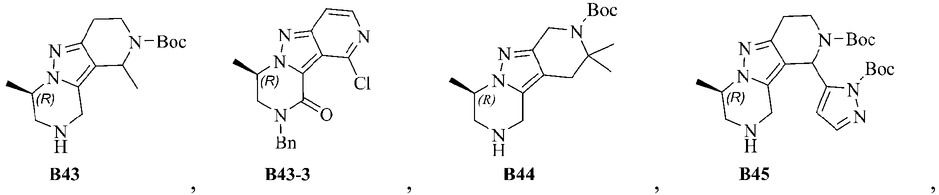

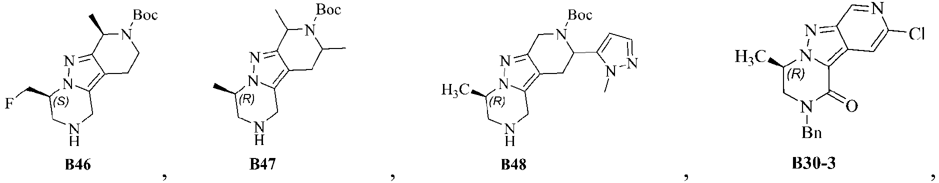

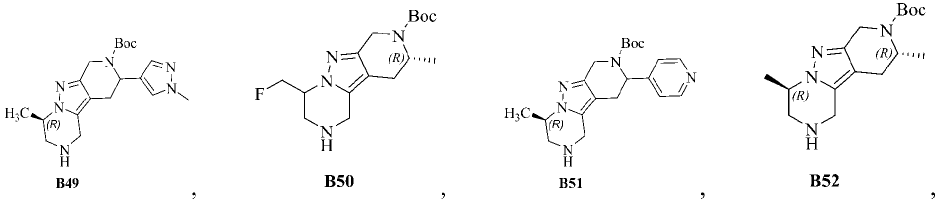

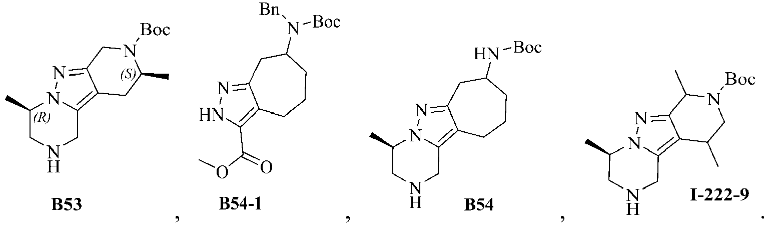

It should be understood that, within the scope of the present invention, the above technical features of the present and the technical features specified below (e.g. embodiments) may be combined to constitute a new technical solution.

### DETAILED DESCRIPTION OF THE EMBODIMENT

After long-term and in-depth research, the inventor has discovered a class of small molecule compounds with TLR7 and/or TLR8 inhibitory activity. These compounds have novel structures and exhibit inhibitory activity comparable or superior to similar compounds in the prior art. Based on the above findings, the inventor has completed the present invention.

### Terminology

In the present invention, said halogen is F, Cl, Br or I.

In the present invention, unless specifically noted, the terms used have a general meaning known to technical personnel in this field.

In the present invention, terms "C₁-C₆ alkyl" refer to linear or branched chain alkyls with 1 to 6 carbon atoms and includes, non-restrictively, methyl, ethyl, propyl, isopropyl, butyl, isobutyl, etc.

In the present invention, terms "C₁-C₆ alkoxy" refer to linear or branched chains of alkanes with 1 to 6 carbon atoms and includes, non-restrictively, methyl, ethyl, propyl, isopropyl, butyl, isobutyl, etc.

In the present invention, the term "C₃-C₇ cycloalkyl" refers to a cyclic alkyl having 3 to 7 carbon atoms on the ring, including, without limitation, cyclopropyl, cyclobutyl, cyclopentyl, cyclohexyl, cycloheptyl, cyclooctyl, and cyclodecyl. The terms "C₃-C₆ cycloalkyl", and "C₃-C₆ cycloalkyl" have similar meanings.

In the present invention, the term "aromatic ring" or "aryl" has the same meaning, and preferably "aryl" is "C₆-C₁₂ aryl" or "C₆-C₁₀ aryl". The term "C₆-C₁₂ aryl" refers to an aromatic ring group having 6 to 12 carbon atoms and does not contain heteroatoms on the ring, such as phenyl, naphthyl, etc. The term "C₆-C₁₀ aryl" has a similar meaning.

In the present invention, the term "aromatic heterocycle" or "heteroaryl" has the same meaning and refers to a heteroaromatic group containing one or more heteroatoms. The heteroatoms referred herein include oxygen, sulfur and nitrogen. For example, furyl, thienyl, pyridyl, pyrazolyl, pyrrolyl, N-alkyl pyrrolyl, pyrimidine, pyrazine, imidazolyl, tetrazolyl, etc. The heteroaryl ring may be fused to an aryl, heterocyclyl or cycloalkyl ring, wherein the ring connected to the parent structure is a heteroaryl ring. The heteroaryl may be optionally substituted or unsubstituted.

In the present invention, the term "3-9-membered carbocyclyl" refers to a 3-9-membered cyclic group that is saturated or unsaturated (non aromatic ring, including monocyclic ring, spiro ring, fused ring, or bridged ring, etc.), and whose ring skeleton structure only includes carbon atoms, such as cyclopentyl, cyclohexyl, etc.

In the present invention, the term "3-9-membered heterocyclyl" refers to a saturated or unsaturated (non aromatic ring, including monocyclic ring, spiro ring, fused ring, or bridged ring, etc.) 3-9-membered heterocyclic group containing 1-3 heteroatoms selected from oxygen, sulfur, and nitrogen on the ring, such as dioxolane. The term "3-7 membered heterocyclyl" has a similar meaning.

In the present invention, the term "substituted" means that one or more hydrogen atoms on a specific group are substituted by a specific substituent. The specific substituents are the substituents described in the foregoing, or the substituents that appear in each embodiment. Unless otherwise specified, a substituted group may have a substituent selected from a specific group at any of the substituted sites of the group, and the substituents may be the same or different in each position. Cyclic substituents, such as heterocycloalkyl, can be attached to another ring, such as cycloalkyl, to form a spiro bicyclic system, for example, two rings have a common carbon atom. Those skilled in the art will understand that the combinations of substituents contemplated by the present invention are those that are stable or chemically achievable. The substituents are, for example, but not limited to, C₁₋₈ alkyl, C₂₋₈ alkenyl, C₂₋₈ alkynyl, C₃₋₈ cycloalkyl, 3-to 12-membered heterocyclyl, aryl, heteroaryl, halogen, hydroxyl, carboxyl (-COOH), C₁₋₈ aldehyde group, C₂₋₁₀ acyl, C₂₋₁₀ ester, amino, alkoxy, C₁₋₁₀ sulfonyl, and the like.

### Compounds of formula I with TLR7 and/or TLR8 regulatory activity

The present invention provides a compound shown in formula I, which has a structure as shown in formula I-1', formula I-2', formula 1-3', or formula 1-4', a pharmaceutically acceptable salt, a racemate, an R-isomer, an S-isomer, or mixtures thereof: 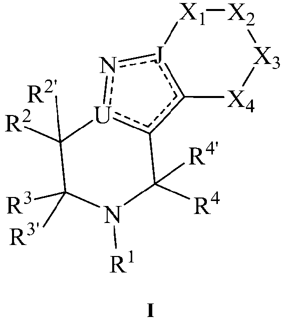 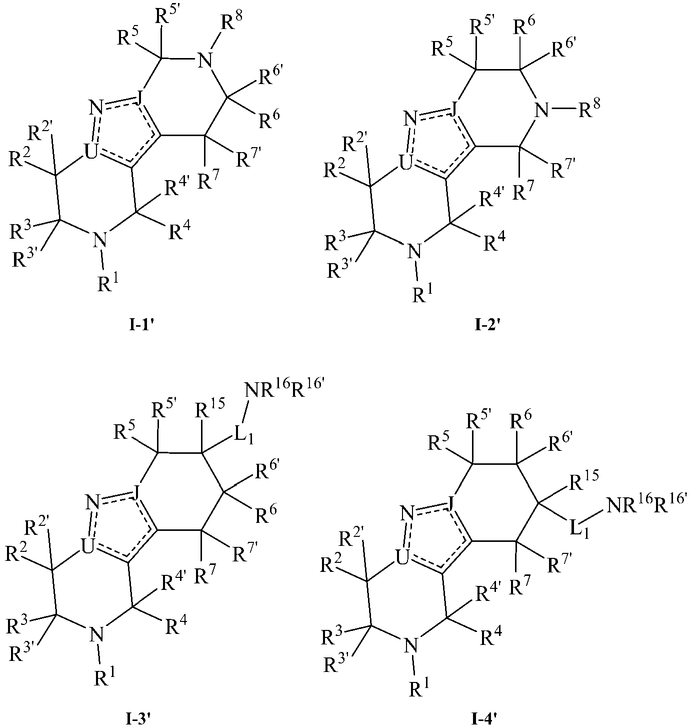 wherein,
the dashed line is a chemical bond or none; and 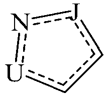 is an aromatic ring;
U is selected from the group consisting of: C and N;
J is selected from the group consisting of: C and N;
X₁, X₂, X₃, and X₄ are each independently selected from the group consisting of: bond, - O-, -S-, -S(=O)-, -S(=O)₂-, -C(=O)-, -C(R⁵R⁵')-, -C(R⁶R^{6'})-, -C(R⁷R^{7'})-, -C(R¹⁵)(L₁-NR¹⁶R¹⁶')-, -N(R⁸)-;
R¹ is selected from the group consisting of: substituted or unsubstituted fused 5, 6 membered heteroaryl, and substituted or unsubstituted fused 6, 6 membered heteroaryl;
R², R³, R⁴, R²', R³', and R⁴' are each independently selected from the group consisting of: H, deuterium, substituted or unsubstituted C₁₋₆ alkoxy, halogenated C₁₋₆ alkyl, substituted or unsubstituted C₁₋₆ alkyl, halogen, cyano, substituted or unsubstituted C₁₋₆ ester group, substituted or unsubstituted C₁₋₆ carboxylic acid, substituted or unsubstituted C₁₋₆ acylamino, substituted or unsubstituted C₃₋₇ cycloalkyl, hydroxyl, C₁₋₆ alkyl substituted with hydroxyl, C₁₋₉ heteroalkyl with 1~3 heteroatoms, wherein the heteroatoms are selected from one or more of NH, N, O, and S; or R², R³, R⁴, R²', R³', and R⁴' located on the same carbon atom together form=O;
or R², R²' and the carbon atom to which they attached, R³, R³' and the carbon atom to which they attached, or R⁴, R⁴' and the carbon atom to which they attached together form a substituted or unsubstituted 3- to 6- membered cycloalky, or a substituted or unsubstituted 4- to 6- membered heterocyclyl;
R⁵, R⁶, R⁷ , R^{5'}, R^{6'}, and R^{7'} are each independently selected from the group consisting of: H, deuterium, substituted or unsubstituted C₁₋₆ alkoxy, halogenated C₁₋₆ alkyl, substituted or unsubstituted C₁₋₆ alkyl, halogen, cyano, substituted or unsubstituted C₁₋₆ ester group, substituted or unsubstituted C₁₋₆ carboxylic acid, substituted or unsubstituted C₁₋₆ acylamino, substituted or unsubstituted C₃₋₇ cycloalkyl, hydroxyl, C₁₋₆ alkyl substituted with hydroxyl, substituted or unsubstituted C₆-C₁₀ aryl benzyl, substituted or unsubstituted 5 to 6- membered heteroaryl benzyl, substituted or unsubstituted C₆-C₁₀ aryl, substituted or unsubstituted 5 to 6- membered heteroaryl, C₁₋₉ heteroalkyl with 1~3 heteroatoms, wherein the heteroatoms are selected from one or more of NH, N, O, and S; or each R⁵, R⁶, R⁷ , R^{5'}, R^{6'}, and R^{7'} located on the same carbon atom together form =O;
or R³, R⁵' and the carbon atom to which they attached, R⁶, R⁶' and the carbon atom to which they attached, R⁷, R⁷' and the carbon atom to which they attached, together form a substituted or unsubstituted 3- to 6- membered cycloalky, or a substituted or unsubstituted 4- to 6- membered heterocyclyl;
or R⁵(R⁵') and R⁶ (R⁶') , R⁵ (R⁵') and R⁷ (R⁷') , R⁶ (R⁶') and R⁷ (R⁷') together form a substituted or unsubstituted 3- to 6- membered cycloalky, or a substituted or unsubstituted 4- to 9- membered heterocyclyl;
L₁ is a bond or C₁~C₆ alkylene, preferably L₁ is a single bond;
R¹⁵ is independently selected from the group consisting of: H, deuterium, C₁₋₆ alkoxy, halogenated C₁₋₆ alkyl, C₁₋₆ alkyl, halogen, cyano, C₃₋₇ cycloalkyl, hydroxyl, hydroxyl substituted C₁₋₆ alkyl; or R¹⁵ and R¹⁶, R¹⁵ and R¹⁶' together form a substituted or unsubstituted 4- to 6- membered heterocycloalkyl; or R¹⁵, L₁ and the carbon atom to which they attached together form a substituted or unsubstituted 3- to 6- membered cycloalky, or a substituted or unsubstituted 4- to 6- membered heterocyclyl;
R¹⁶ and R¹⁶' are each independently selected from the group consisting of: H, deuterium, substituted or unsubstituted 3- to 9- membered carbocyclyl (saturated, unsaturated monocyclic ring, spiro ring, fused ring, or bridged ring), substituted or unsubstituted 4-to 9- membered heterocyclyl (saturated, unsaturated monocyclic ring, spiro ring, fused ring, or bridged ring), substituted or unsubstituted C₁₋₆ alkyl, benzyl, - (CH₂)ₙN(R⁸⁻¹R⁸⁻²), -(CH₂)ₙC(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, -C(O)(CH₂)ₙR⁸⁻³, C₁₋₆ alkyl NHS(O)₂-, C₁₋₆ alkyl NHC(O)-, -C(O)(CH₂)ₙN(R⁸⁻¹R⁸⁻²), -SO₂(CH₂)ₙN(R⁸⁻¹R⁸⁻²), (CH₂)ₙSO₂N(R⁸⁻¹R⁸⁻²), C₁-C₆ alkoxy, C₁-C₆ alkyl-amido, C₆-C₁₀ arylbenzyl, and 5 or 6 membered heteroarylbenzyl;
or R¹⁶ and R¹⁶' together form a substituted or unsubstituted 4-to 6- membered heterocycloalkyl;
R⁸ is selected from the group consisting of: H, deuterium, substituted or unsubstituted 3- to 9- membered carbocyclyl (saturated, unsaturated monocyclic ring, spiro ring, fused ring, or bridged ring), substituted or unsubstituted 4- to 9- membered heterocyclyl (saturated, unsaturated monocyclic ring, spiro ring, fused ring, or bridged ring), substituted or unsubstituted C₁₋₆ alkyl, benzyl, -(CH₂)ₙN(R⁸⁻¹R⁸⁻²), -(CH₂)ₙC(O)N(R⁸⁻¹R⁸⁻²), - (CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, - (CH₂)ₙC(O)R⁸⁻³, -C(O)(CH₂)ₙR⁸⁻³, C₁₋₆ alkyl NHS(O)₂-, C₁₋₆ alkyl NHC(O)-, - C(O)(CH₂)ₙN(R⁸⁻¹R⁸⁻²), -SO₂(CH₂)ₙN(R⁸⁻¹R⁸⁻²), (CH₂)ₙSO₂N(R⁸⁻¹R⁸⁻²), C₁-C₆ alkoxy, C₁-C₂ alkyl-amido, C₆-C₁₀ aryl benzyl, 5 to 6- membered heteroaryl benzyl;
wherein, the "substituted" refers to one or more hydrogen atoms on the group are substituted with substituents selected from the group consisting of: halogen, hydroxyl, carboxyl, benzyl, oxo(=O), -(CH₂)ₙN(R⁸⁻¹R⁸⁻²), -(CH₂)ₙC(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, - C(O)(CH₂)ₙR⁸⁻³, amino, C₁₋₆ alkyl NHS(O)₂-, C₁₋₆ alkyl NHC(O)-, -C(O)(CH₂)ₙN(R⁸⁻¹R⁸⁻²), -SO₂(CH₂)ₙN(R⁸⁻¹R⁸⁻²), (CH₂)ₙSO₂N(R⁸⁻¹R⁸⁻²), nitro, cyano, unsubstituted or halogenated C₁-C₆ alkyl, C₂-C₁₀ alkenyl, C₁-C₆ alkoxy, C₁-C₆ alkyl-amido, C₆-C₁₀ aryl, 5 or 6 membered heteroaryl, -O-(C₆-C₁₀ aryl), -O- (5 or 6 membered heteroaryl), and substituted or unsubstituted C₃₋₇ heterocycloalkyl;
wherein, R⁸⁻¹ and R⁸⁻² are selected from the group consisting of: hydrogen, C₁₋₆ alkyl, C₁₋₆ haloalkyl, C₃₋₆ cycloalkyl, 4- to 6- membered heterocycloalkyl, or R⁸⁻¹, R⁸⁻² and the nitrogen atom to which they attached together form 4- to 6- membered heterocycloalkyl with N or N and O and a heteroatom number of 1 or 2; the cycloalkyl and heterocycloalkyl can be substituted with one or more halogen; wherein R⁸⁻³ is selected from the group consisting of: hydrogen, C₁₋₆ alkyl, C₁₋₆ alkoxy, C₁₋₆ haloalkyl, C₃₋₆ cycloalkyl, and 4- to 6-membered heterocycloalkyl; and the cycloalkyl and heterocycloalkyl can be substituted with one or more halogen or C₁₋₆ alkyl; n is 0, 1, 2, 3, 4, 5, or 6.

### Method for preparation of compounds of formula I-1 ', I-2', I-3 ', I-4'

The present invention also provides a method for preparing compounds as shown in formulas I-1', I-2', 1-3', I-4', which is any of the following methods:
Method 1, comprising the following steps: in a solvent, under the action of a base, reacting a compound shown in formula III with compounds shown in formulas IV-1, IV-2, IV-3, and IV-4 to obtain compounds shown in formulas I-1', I-2', I-3', and 1-4' 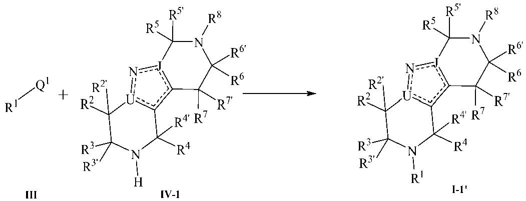 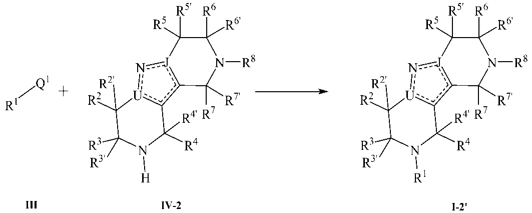 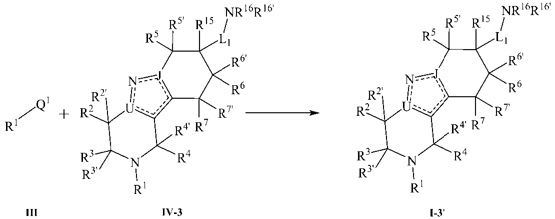 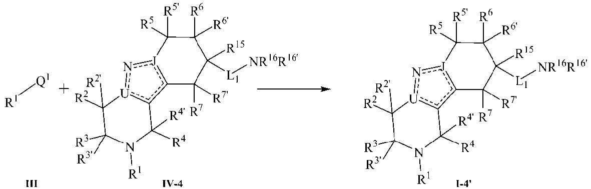
Q¹ is fluorine, chlorine or bromine, R¹, R², R³ , R⁴, R⁵, R⁶, R⁷, R²', R³', R⁴', R⁵', R⁶', R7', R⁸, R¹⁵, R¹⁶, R¹⁶', U, J and L1 as defined above;
Method 2, which comprises the following steps: in the solvent, under the action of a base, the compound shown in formula VIII and the compound shown in formula I-1'-1, I-2'-1 undergo a substitution reaction to obtain the compound shown in formula I-1', I-2'; 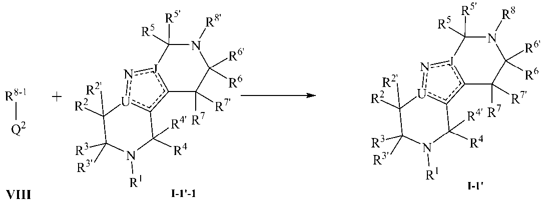 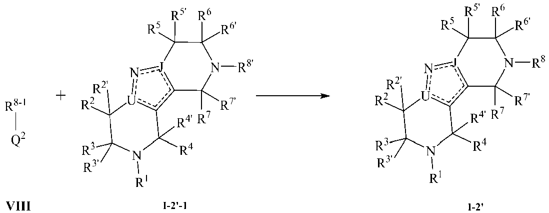
Q² is OMs, fluorine, chlorine, or bromine; R⁸' is hydrogen, "a 3- to 9-membered heterocycloalkyl with 1~3 heteroatoms and the heteroatoms are selected from one or more of N, O, and S"; the definitions of R¹, R², R³, R⁴, R⁵, R⁶, R⁷, R²', R³', R⁴', R⁵', R⁶', R⁷', R⁸, U, and J are as previously described.
R⁸⁻¹ is "3- to 7-membered heterocycloalkyl with 1 to 3 heteroatoms and the heteroatoms are selected from one or more of N, O, and S", "C₁-C₁₀ heteroaryl with 1 to 4 heteroatoms", substituted or unsubstituted C₁-C₆ alkyl with 1 to 3 heteroatoms, substituted or unsubstituted C₃-C₇ cycloalkyl with 1 to 3 heteroatoms, C₁-C₆ alkoxycarbonyl, (CH₂)ₙNH₂, (CH₂)ₙC(O)NH₂, (CH₂)ₙC(O)OH, C₁-C₆ amide, C₁-C₆ sulfonamide, C(O)(CH₂)ₙNH₂, SO₂(CH₂)ₙNH₂, (CH₂)ₙSO₂NH₂, unsubstituted or halogenated C₁-C₆ alkyl, C₂-C₁₀ alkenyl, C₁-C₆ alkoxy, C₁-C₆ alkyl-amino, C₆-C₁₀ aryl, 5- or 6-membered heteroaryl; n is 0, 1, or 2.

### Pharmaceutical compositions containing active ingredients

Because the compounds of the present invention have excellent toll-like receptor suppressive activity, the compound and its various crystal forms are therefore medicinally acceptable inorganic or organic salts, hydrates or solvent compounds, and pharmaceutical compositions containing compounds of the present invention as the main active ingredients may be used to prevent and / or treat (stabilize, mitigate or cure) diseases or conditions related to the abnormal expression or activation of toll-like receptors (especially TLR7, TLR8) such as autoimmune diseases or chronic inflammatory diseases; Ideally, the autoimmune diseases described are selected from the following groups: Dry syndrome, systemic lupus erythematosus, multiple sclerosis, rheumatoid arthritis, systemic sclerosis and psoriasis, Systemic lupus erythematosus, lupus nephritis.

The pharmaceutical composition of the present invention contains within a safe and effective quantity a compound of the present-invention and a pharmaceutically acceptable embodiment or carrier. The term "safe and effective" means that the amount of the compound is sufficient to significantly improve the condition without causing serious side effects. Typically, the drug composition contains 0.01-99.99% percentage of the compounds / agents of the present invention by weight, or better, 0.1-99.9% of the present-invention compounds or agents. Ideally, the "dose" described is a capsule or tablet.

"Pharmaceutically acceptable vector" means one or more compatible solid or liquid fillers or gel substances that are suitable for human use and must be of sufficient purity and low toxicity. There are no special limitations on the ways in which the compounds or drug compositions of the present invention can be administered as solid dosage forms or liquid formulations. The representative ways of administration include (but are not limited to): oral, gastrointestinal, intravenous, intramuscular, or subcutaneous.

Solid dosage forms for oral administration include capsules, tablets, pills, powders, and granules. In these solid dosage forms, the active compound is mixed with at least one conventional inert excipient (or carrier), such as sodium citrate or dicalcium phosphate, or with the following components: (a) fillers or compatibilizers, such as starch, lactose, sucrose, glucose, mannitol, and silica acid; (b) binders, such as hydroxymethylcellulose, alginate, gelatin, polyvinylpyrrolidone, sucrose, and arabic gum; (c) humectant , such as glycerin; (d) disinfectants, such as agar, calcium carbonate, potato starch or cassava starch, alginic acid, certain complex silicates, and sodium carbonate; (e) dissolution-retarding agents, such as paraffin wax; (f) absorption accelerators, such as quaternary amine compounds; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. In capsules, tablets, and pills, the dosage forms may also contain buffering agents.

The solid dosage forms such as tablets, sugar pills, capsules, pills, and granules can be prepared using coating and shell materials, such as enteric coatings and other materials known in the art. They may contain opaque agents, and the active compound or compound in this composition can be released in a delayed way in a given portion of the digestive tract. Examples of usable embedding components are polymers and waxs. If necessary, the active compound can also form microcapsules with one or more above-mentioned excipients.

Liquid dosage forms for oral administration include pharmaceutically acceptable emulsions, solutions, suspensions, syrups or tinctures. In addition to active compound, liquid dosages may include inert diluents commonly used in this field, such as water or other solvents, solubilizers, and emulsifiers, such as ethanol, isopropanol, ethyl carbonate, ethyl acetate, propylene glycol, 1,3-butanediol, dimethylformamide, as well as oils, particularly cottonseed oil, peanut oil, corn germ oil, olive oil, castor oil, and sesame oil, or a combination thereof.

In addition to these inert diluents, the composition may also contain adjuvants, such as wetting agents, emulsifiers and suspensions, sweeteners, flavoring agents, and spices.

In addition to active compound, suspensions may contain suspending agents, such as ethoxylated isooctadecanol, polyoxyethylene sorbitol and dehydrated sorbitol esters, microcrystalline cellulose, methanol aluminum and agar, or a combination thereof.

The compositions for parenteral injection may include physiologically acceptable sterile aqueous or anhydrous solutions, dispersions, suspensions or emulisons, and sterile powders that can be re-dissolved into sterile injectable solutions or dispersions. The suitable aqueous and non-aqueous carriers, diluents, solvents, or excipients include water, ethanol, polyols, and a suitable combination thereof.

The compounds of the invention may be administered alone or in combination with other pharmaceutically acceptable compounds (including agents to treat autoimmune diseases).

When combined, the drug composition also includes one or more (2, 3, 4, or more) other pharmacologically acceptable compounds. One or more of these other medicinally acceptable compounds (2, 3, 4, or more, of these may be used simultaneously, separately or sequentially with the compounds of the present invention to prevent and / or treat diseases or conditions associated with the abnormal expression or activation of toll-like receptors (especially TLR7, TLR8).

When a pharmaceutical composition is used, a safe and effective quantity of the compound of the present invention is applied to a mammal requiring treatment (e.g., humans) in a dose that is considered to be an effective dose of administration in pharmacy. Of course, the specific dosage should also consider the route of administration, the patient's condition, etc., which are within the range of skilled physicians.

The present invention is further elaborated below in the context of specific embodiments. It should be understood that these embodiments are used only to illustrate the invention and not to limit the scope of the invention. The experimental methods in the following embodiments do not specify specific conditions, usually according to conventional conditions or as recommended by the manufacturers.

In the following embodiments, the composition is structurally determined by nuclear magnetic resonance (NMR) or / and mass spectrometry (MS). The NMR shift (δ) is measured by 10⁻⁶(ppm) units are given. The NMR was determined using a Bruker AVANCE-400 NMR apparatus in which the solvent was deuterated dimethyl sulfoxide (DMSO-*d*₆), deuterated chloroform (CDCl₃), deuterated methanol (CD₃OD), tetramethylsilane (TMS) was used as internal standard.

SHIMADZU LC System (Spectrum Column: Xselect ^{®} CSH^{™}Prep-C18, 19 * 150mm, liquid processor LH-40, pump LC-20AP, detector SPD-20A, system controller CBM-20A, solvent system: acetonitrile and 0.05% TFA aqueous solution).

LC / MS spectra of the compound were obtained using LC / MS (Agilent Technologies 1200 Series). LC / MS conditions are as follows (run time is 10 minutes):
Acidic conditions: A: 0.05% aqueous solution of TFA; B: 0.05% acetonitrile solution of TFA;
Basic conditions: A: 0.05% NH₃•H₂O aqueous solution; B: Acetonitrile
Neutral conditions: A: 10 mM NH₄OAc aqueous solution; B: Acetonitrile
SFC separation of chiral compounds: column type, CHIRALPAK AD-H, 2cm × 25cm, 5µm chromatographic column. Mobile phase A: hexane (0.5% 2mM, NH₃-MeOH); Mobile phase B: Ethanol or defined in the experiments.

Unless specifically stated, in the following embodiments, intermediates and final compounds were purified using silica gel column chromatography, or using X select CSH^{™} Prep-C18(5µm, OBD^{™}19 * 150mm) or X Bridge TM Prep Phenyl (5µm, OBD)^{™}30 * 100 mm) or purified by preparative HPLC (prep-HPLC) on a reverse-phase chromatography column.

In general, silica gel of 200 ~ 300 mesh from Yantai Huanghai was used in flash chromatography purifications. Silica gel plates from Yantai Huanghai HSGF254 or Qingdao GF254 were used for thin-layer chromatography (TLC).

The CombiFlash Rapid Preparation Instrument uses the Combiflash Rf200 (TELEDYNE ISCO).

Starting materials or reagents of the invention may be synthesized or in accordance with methods known in the art, or purchased from ABCR GmbH & Co.KG, Acros Organics, Aldrich Chemical Company, Shaoyuan Chemical Technology (Accela ChemBio Inc), Darui Chemical and other companies.

**Acronyms:** Ac₂O: acetic anhydride; AIBN: 2,2-azobisisobutyronitrile; BINAP: 2,2'-Bis (diphenylphosphino)-1,1'-binaphthyl; Boc₂O: di-tert-butyl dicarbonate; conc. HCl: concentrated hydrochloric acid; Cs₂CO₃: cesium carbonate; DCE: 1,2-dichloroethane; DCM: dichloromethane; DIAD: diisopropyl azodicarboxylate; Dioxane:1, 4-dioxane; DIEA: N, N-diisopropyl ethylamine; DMAP: 4-dimethylaminopyridine; DMF: dimethylformamide; DMSO: dimethyl sulfoxide; DMF-DMA: N, N-dimethylformamide dimethyl acetal; EtOAc, ethyl acetate; EtOH: ethanol; HOAc: acetic acid; H₂: hydrogen; I₂: iodine; K₂CO₃: potassium carbonate; K₃PO₄: potassium phosphate; LAH or LiAlH₄: lithium aluminum hydride; LiHMDS: lithium bis (trimethylsilyl)amide; LiOH: lithium hydroxide; KF: potassium fluoride; MCPBA: m-chloroperoxybenzoic acid; MeOH: methanol; N₂: nitrogen; NaH: sodium hydride; NaHCO₃: Sodium bicarbonate; NaHMDS: sodium bis (trimethylsilyl)amide; PPA: polyphosphoric acid; PCy3: tricyclohexylphosphine; Pd(dppf)Cl₂: [1,1' -bis(diphenylphosphino)ferrocene]dichloro palladium (II); Pd(OAc)₂: palladium acetate; Pd/C: palladium carbon; Pd₂(dba)₃: tris(dibenzylideneacetone)dipalladium; Pd(PPh₃)₄: tetrakis(triphenylphosphine)palladium; POCl₃: phosphorus oxychloride; PPh₃: triphenylphosphine; rt: room temperature; tert-BuOK: potassium tert-butoxide; tert-BuLi: tert-butyllithium; TEA: triethylamine; TFA: trifluoroacetic acid; TFAA: trifluoroacetic anhydride; TfOH: trifluoromethyl sulfonic acid; THF: tetrahydrofuran; TLC: thin layer chromatography; TLR7: toll-like receptor 7; TLR8: toll-like receptor 8; XantPhos: 4,5-bis (diphenylphosphino)-9,9-dimethylxanthene; Xphos: 2-dicyclohexylphosphino-2' ,4' ,6' -triisopropylbiphenyl; Zn: zinc; ZnCl₂: zinc chloride; Zn(CN)₂: zinc cyanide.

### Preparation of intermediate A1

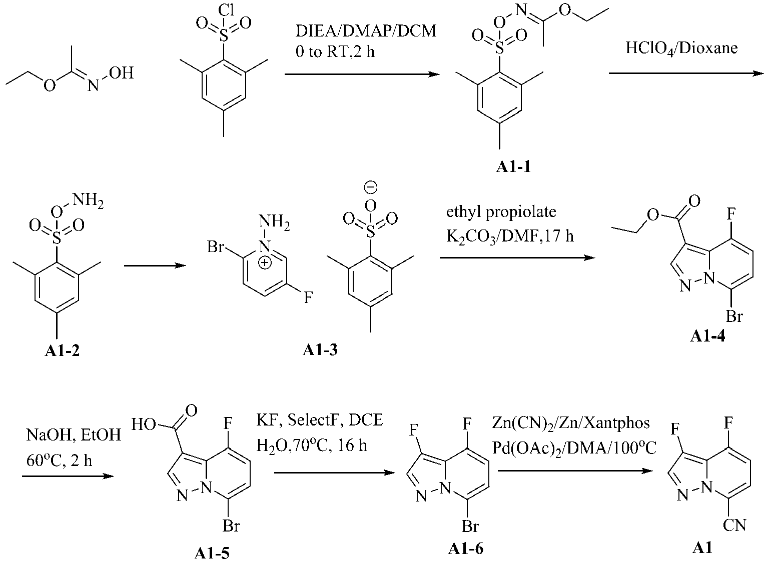

### Step 1: Preparation of intermediate A1-1

To a mixture of ethyl (E)-N-hydroxyacetimidate (100 g, 969.7 mmol), DMAP (12 g, 98.2 mmol) and DIEA (155 g, 1.2 mmol) in DCM (30 mL) was added 2,4,6-trimethylbenzenesulfonyl chloride (233 g, 1.07 mmol) at 0 °C. The reaction mixture was stirred at rt for 1 hour before quenched with ice water and extracted with DCM. The organic phase was washed with saturated saline, dried with Na₂SO₄ and concentrated. Purification by flash chromatography gave **intermediate A1-1** as white solid (220 g, 72.3% yield). MS: 286.2 (M+H)⁺.

### Step 2: Preparation of intermediate A1-2

To a mixture of **intermediate A1-1** (220 g, 771.0 mmol) in 1,4-dioxane (600 mL) was slowly added HClO₄ solution (120 mL) at 0 °C. The reaction mixture was stirred at 0 °C for 1 hour before quenched with ice water and extracted with EtOAc. The organic phase was washed with saturated saline, dried with Na₂SO₄ and concentrated. Purification by flash chromatography gave **intermediate A1-2** as white solid (80 g, 42% yield). MS: 216.2 (M+H)⁺.

### Step 3: Preparation of intermediate A1-3

A reaction mixture of **intermediate A1-2** (90 g, 418 mmol) and 2-bromo-5-fluoropyridine (73 g, 418 mmol) in anhydrous DCM (1000 mL) was stirred at 10 °C for 18 hours before it was concentrated. The crude product was recrystallized from EtOAc to give **A1-3** as white solid (80g, 99% yield). MS: 191.0 (M+H)⁺.

### Step 4: Preparation of intermediate A1-4

A reaction mixture of **intermediate A1-3** (80 g, 416 mmol), K₂CO₃ (115 g, 833 mmol) and ethyl propionate (49 mL, 500 mmol) in DMF (1300 mL) was stirred at 10 °C for 18 hours. The reaction mixture was extracted with EtOAc, and the organic phase was washed with saturated saline, dried with Na₂SO₄ and concentrated. Purification by flash chromatography gave **intermediate A1-4** as white solid (9.1 g, 7.6% yield). MS: 287.2 (M+H)⁺.

### Step 5: Preparation of intermediate A1-5

A reaction mixture of **intermediate A1-4** (10.8 g, 37.6 mmol), and NaOH (4.5 g, 112.8 mmol) in EtOH (190 mL) and water (147 mL) was stirred at rt for 1 hour then stirred at 60 °C for 2 hours. The reaction mixture was concentrated, quenched with icy water and a solution of 1N HCl was added to reach pH 5. The grey precipitation was filtered and dried to obtain **intermediate A1-5** as solid (9.0 g, 92.7% yield). MS: 261.0 (M+H)⁺.

### Step 6: Preparation of intermediate A1-6

A reaction mixture of **intermediate A1-5** (0.880 g, 3.4 mmol), SelectFluro (2.4 g, 6.77 mmol) and KF (0.792 g, 13.6 mmol) in water (10 mL) and DCE (12 mL) was stirred at rt for 1 hour then the temperature was slowly raised to 70 °C and stirred for 16 hours. The reaction mixture was extracted with EtOAc, and the organic phase was washed with saturated saline, dried with Na₂SO₄ and concentrated. Purification by flash chromatography gave **intermediate A1-6** as white solid (0.38 g, 48.0% yield). MS: 233.0 (M+H)⁺.

### Step 7: Preparation of intermediate A1

To a mixture of Zn(CN)₂ (383 mg, 3.26 mmol), zinc powder (21.2 mg, 326.2 mmol), Xantphos, Pd(OAc)₂ in DMA (10 mL) was added **intermediate A1-5** (0.38 g, 1.63 mmol) under N₂ protection. The reaction mixture was stirred at 120 °C for 2 hours. The mixture was diluted with water and extracted with EtOAc. The organic phase washed with saturated saline, dried with Na₂SO₄ and concentrated. Purification by flash chromatography gave **intermediate A1** as white solid (0.18 g, 47.4% yield). MS: 180.0 (M+H)⁺. ¹H NMR (400 MHz, CDCl₃) δ 7.98 (d, *J* = 3.6 Hz, 1H), 7.29 (dd, *J* = 8.0, 4.8 Hz, 1H), 6.84 - 6.77 (m, 1H).

### Preparation of intermediate B1

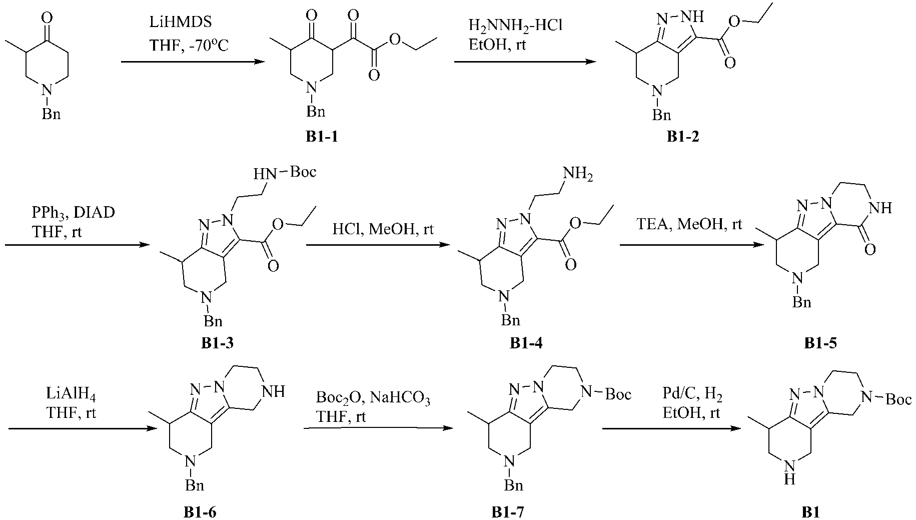

### Step 1: Preparation of intermediate B1-1

To a mixture of LiHMDS (11 mL, 11 mmol) in THF was added 1-benzyl-3-methylpiperidin-4-one (5 g, 25 mmol) under N₂ protection. The reaction mixture was stirred at -70 °C for 1 hour before diethyl oxalate (3.6 g, 25 mmol) was added and the reaction mixture was stirred at rt for 4 hours. The mixture was quenched with icy water and extracted with EtOAc. The organic phase washed with saturated saline, dried with Na₂SO₄ and concentrated. Purification by flash chromatography gave **Intermediate B1-1** (7 g, 90%) as yellow solid. MS: 304.2 (M+H)⁺.

### Step 2: Preparation of intermediate B1-2

A reaction mixture of **intermediate B1-1** (6 g, 20 mmol) and thaliana (1.5 g, 22 mmol) in EtOH (20 mL) was stirred at room temperature for 4 hours. The mixture was quenched with water and extracted with EtOAc, and the organic phase was washed with saturated saline, dried with Na₂SO₄ and concentrated. Purification by flash chromatography gave **Intermediate B1-2** (5 g, 80%) as white solid. MS: 300.0 (M+H)⁺.

### Step 3: Preparation of intermediate B1-3

A reaction mixture of **intermediate B1-2** (6 g, 20 mmol), (2-hydroxyethyl) tert-butyl carbamate (3.5 g, 18 mmol) in THF (50 mL) was added PPh₃ (7 g, 25 mmol) and DIAD (5 g, 25 mmol) at 0°C. The resulting mixture was stirred at room temperature for 4 hours. The mixture was quenched with water and extracted with EtOAc, and the organic phase was washed with saturated saline, dried with Na₂SO₄ and concentrated. Purification by flash chromatography gave **Intermediate B1-3** (8 g, 93%) as yellow solid. MS: 443.2 (M+H)⁺.

### Step 4: Preparation of intermediate B1-4

To a solution of **B1-3** (8 g, 18.1 mmol) in MeOH (30 mL) was added HCl (30 mL, 4.0 M in 1,4-dioxane). The resulting mixture was stirred at room temperature for 3 hours. The resulting mixture was concentrated under vacuum to give **Intermediate B1-4** (6 g) as yellow solid. MS: 343.0 (M+H)⁺.

### Step 5: Preparation of intermediate B1-5

To a solution of **intermediate B1-4** (2 g, 6 mmol) in MeOH(30 mL) was added TEA (10 mL). The resulting mixture was stirred at room temperature for 3 hours. The resulting mixture was concentrated under vacuum. Purification by flash chromatography gave **Intermediate B1-5** (1 g, 60%) as white solid. MS: 297.0 (M+H)⁺.

### Step 6: Preparation of intermediate B1-6

A reaction mixture of **intermediate B1-5** (1 g, 2.5 mmol) in THF (10 mL) was added LAH (2.5 mL, 2.5 mmol, 1 mol/L in THF) at 0°C. The resulting mixture was stirred at room temperature for 4 hours. The mixture was quenched with water and extracted with EtOAc, and the organic phase was washed with saturated saline, dried with Na₂SO₄ and concentrated. Purification by flash chromatography gave **Intermediate B1-6** (0.6 g, 90%) as white solid. MS: 283.2 (M+H)⁺.

### Step 7: Preparation of intermediate B1-7

A reaction mixture of **Intermediate B1-6** (0.7 g, 2.5 mmol), Boc₂O (0.8 g, 3.8 mmol) in THF (10 mL) and sat. NaHCO₃ solution (5 mL) was stirred at room temperature for 8 hours. The mixture was quenched with water and extracted with EtOAc, and the organic phase was washed with saturated saline, dried with Na₂SO₄ and concentrated. Purification by flash chromatography gave **Intermediate B1-7** (0.9 g, 95%) as yellow solid. MS: 383.2 (M+H)⁺.

### Step 8: Preparation of intermediate B1

A reaction mixture of **Intermediate B1-7** (0.9 g, 2.4 mmol) and Pd/C (0.35 g) in EtOH (20 mL) was stirred at room temperature overnight. The solid were filtered out. The resulting mixture was concentrated under vacuum to give **Intermediate B1-7** (700 mg, 92%) as white solid. MS: 293.3 (M+H)⁺.

### Preparation of intermediate B2

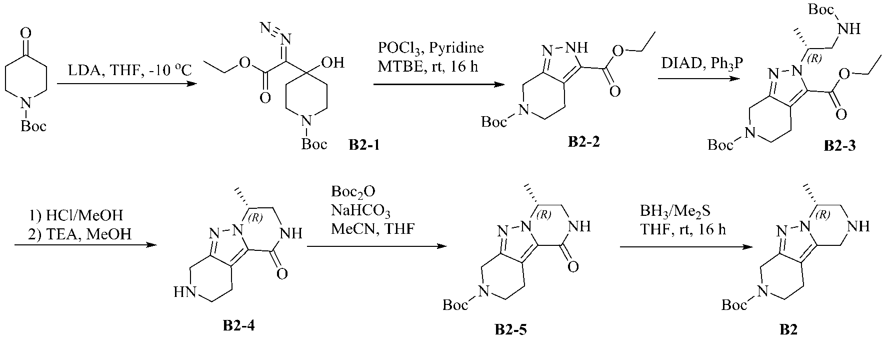

### Step 1: Preparation of intermediate B2-1

A reaction mixture of tert-butyl 4-oxypiperidine-1-carboxylate (1 g, 5.02 mmol) in THF (10 mL) was added LDA (3.7 mL, 7.53 mmol, 2 mol/L in THF). The resulting mixture was stirred at -10 °C for 1 hour. Then ethylene heavy nitrogen acetaldehyde (0.601 g, 5.27 mmol) was added to the mixture. The resulting mixture was stirred at room temperature overnight. The mixture was quenched with water and extracted with EtOAc, and the organic phase was washed with saturated saline, dried with Na₂SO₄ and concentrated. Purification by flash chromatography gave **Intermediate B2-1** (0.9 g, 95%) as brown solid. MS: 336.2 (M+Na)⁺.

### Step 2: Preparation of intermediate B2-2

A reaction mixture of triclosan phosphate (23.39 g, 153 mmol), **Intermediate B2-1** (23.9 g, 76 mmol) and pyridine (121 g, 1525 mmol) in MTBE (200 mL) was stirred at room temperature for 16 h. The mixture was quenched with water and extracted with EtOAc, and the organic phase was washed with saturated saline, dried with Na₂SO₄ and concentrated. Purification by flash chromatography gave **Intermediate B2-2** (13.8 g, 61.4%) as brown solid. MS: 318.2 (M+Na)⁺.¹H NMR (400 MHz, chloroform-*d*) δ 4.61 (s, 1H), 4.39 (q, *J =* 7.1 Hz, 2H), 4.30 - 4.08 (m, 2H), 3.67 (s, 2H), 2.86 (t, *J* = 5.9 Hz, 1H), 1.49 (d, *J* = 5.8 Hz, 9H), 1.34 - 1.23 (m, 3H).

### Step 3: Preparation of intermediate B2-3

A reaction mixture of **Intermediate B2-2** (4.2 g, 14.22 mmol), (S) - (2-hydroxypropyl) tert-butyl carbamate (2.74 g, 15.64 mmol) in THF (50 mL) was added PPh₃ (5.60 g, 21.33 mmol) and DIAD (4.31 g, 21.33 mmol) at 0°C. The resulting mixture was stirred at room temperature for 4 hours. The mixture was quenched with water and extracted with EtOAc, and the organic phase was washed with saturated saline, dried with Na₂SO₄ and concentrated. Purification by flash chromatography gave **Intermediate B2-3** (3.9 g, 60.6%) as yellow solid. MS: 443.2 (M+H)⁺.

### Step 4: Preparation of intermediate B2-4

A reaction mixture of **Intermediate B2-3** (1.2 g, 2.65 mmol) in THF (5 mL) and con. HCl (5 mL) was stirred at room temperature for 3 hours. Then the resulting mixture was concentrated under vacuum. Then TEA (2 mL) and MeOH (5 mL) was added to the mixture and the mixture was stirred at room temperature for 16 hours. The mixture was quenched with water and extracted with EtOAc, and the organic phase was washed with saturated saline, dried with Na₂SO₄ and concentrated. Purification by flash chromatography gave **Intermediate B2-4** (512 mg, 94%) as yellow solid. 207.2 (M+H)⁺.

### Step 5: Preparation of intermediate B2-5

A reaction mixture of **Intermediate B2-4** (440 mg, 2.133 mmol), Boc₂O (512 mg, 2.347 mmol) in THF (5 mL) and sat. NaHCO₃ solution (5 mL) was stirred at room temperature for 16 hours. Then the resulting mixture was concentrated under vacuum. Then TEA (2 mL) and MeOH (5 mL) was added to the mixture and the mixture was stirred at room temperature for 16 hours. The mixture was quenched with water and extracted with EtOAc, and the organic phase was washed with saturated saline, dried with Na₂SO₄ and concentrated. Purification by flash chromatography gave Intermediate **B2-5** (570 mg, 87%) as yellow solid. MS: 307.2 (M+H)⁺. ¹H NMR (400 MHz, DMSO-*d*₆) δ 8.09 (s, 1H), 4.42 (d, *J* = 21.6 Hz, 3H), 3.61 (d, *J* = 12.7 Hz, 1H), 3.53 (s, 2H), 3.25-3.27 (m, 1H), 2.69 (s, 2H), 1.46 (s, 3H), 1.41 (s, 9H).

### Step 6: Preparation of intermediate B2

A reaction mixture of **Intermediate B2-5** (390 mg, 1.273 mmol), borane dimethyl sulphide ether (967 mg, 12.73 mmol) in THF (6 mL) was stirred at room temperature for 16 hours. The mixture was quenched with water and extracted with EtOAc, and the organic phase was washed with saturated saline, dried with Na₂SO₄ and concentrated. Purification by flash chromatography gave Intermediate **Intermediate B2** (245 mg, 65.8%) as yellow oil. MS: 293.2 (M+H)⁺.

### Preparation of intermediate B3

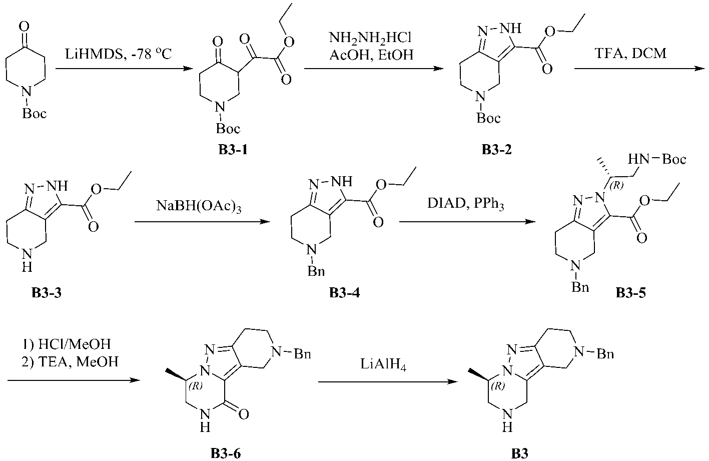

### Step 1: Preparation of intermediate B3-1

To a mixture of LiHMDS (65 mL, 65 mmol) in THF was added 4-hydroxypyrimidin-1-carboxylate (10 g, 50.2 mmol) under N₂ protection. The reaction mixture was stirred at - 70 °C for 1 hour before diethylene glycolate (9.53 g, 65.2 mmol) was added and the reaction mixture was stirred at room temperature for 1 hour. The mixture was quenched with icy water and extracted with EtOAc. The organic phase washed with saturated saline, dried with Na₂SO₄ and concentrated. Purification by flash chromatography gave **Intermediate B3-1** (8.5 g, 56.6%) as yellow oil. MS: 300.2 (M+H)⁺.

### Step 2: Preparation of intermediate B3-2

A reaction mixture of **Intermediate B3-1** (1.85 g, 30.7 mmol), trichlorate (2.379 g, 34.7 mmol) in EtOH (10 mL) and HOAc (1 mL) was stirred at room temperature for 2 hours. The mixture was quenched with water and extracted with EtOAc, and the organic phase was washed with saturated saline, dried with Na₂SO₄ and concentrated. Purification by flash chromatography gave **Intermediate B3-2** (4.18 g, 46.1%) as yellow solid. MS: 296.0 (M+H)⁺.

### Step 3: Preparation of intermediate B3-3

A reaction mixture of **Intermediate B3-2** (3.75 g, 12.70 mmol) and TFA (10 mL) in DCM (10 mL) was stirred at room temperature for 1 hour. The mixture was quenched with water and extracted with EtOAc, and the organic phase was washed with saturated saline, dried with Na₂SO₄ and concentrated. Purification by flash chromatography gave **Intermediate B3-3** (1.74 g, 70.2%) as yellow solid. MS: 196.0 (M+H)⁺.

### Step 4: Preparation of intermediate B3-4

A reaction mixture of **Intermediate B3-3** (840 mg, 4.30 mmol), formaldehyde (502 mg, 4.73 mmol) and sodium borate hydride (1.82 g, 8.61 mmol) in THF (5 mL) was stirred at room temperature for 16 hours. The mixture was quenched with water and extracted with EtOAc, and the organic phase was washed with saturated saline, dried with Na₂SO₄ and concentrated. Purification by flash chromatography gave **Intermediate B3-4** (940 mg, 77%) as yellow solid. MS: 286.2 (M+H)⁺.

### Step 5: Preparation of intermediate B3-5

**Intermediate B3-5** was synthesized by following the protocols of **Intermediate B2-3.** MS: 443.1 (M+H)⁺.

### Step 6: Preparation of intermediate B3-6

**Intermediate B3-6** was synthesized by following the protocols of **Intermediate B2-4.** MS: 297.1 (M+H)⁺.

### Step 7: Preparation of intermediate B3

**Intermediate B3** was synthesized by following the protocols of **Intermediate B1-6.** MS: 283.1 (M+H)⁺.

### Preparation of intermediate B4

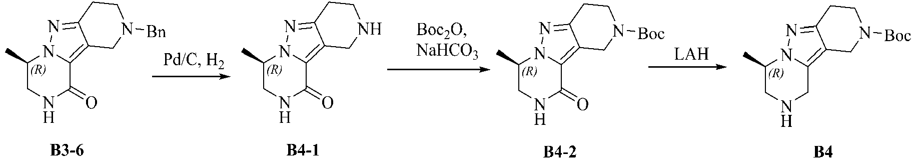

### Step 1: Preparation of intermediate B4-1

**Intermediate B4-1** was synthesized by following the protocols of **Intermediate B1** MS: 207.1 (M+H)⁺.

### Step 2: Preparation of intermediate B4-2

**Intermediate B4-2** was synthesized by following the protocols of **Intermediate B2-5.** MS: 307.1 (M+H)⁺.

### Step 3: Preparation of intermediate B4

**Intermediate B4** was synthesized by following the protocols of **Intermediate B3.** MS: 293.1 (M+H)⁺.

### Preparation of intermediate B5

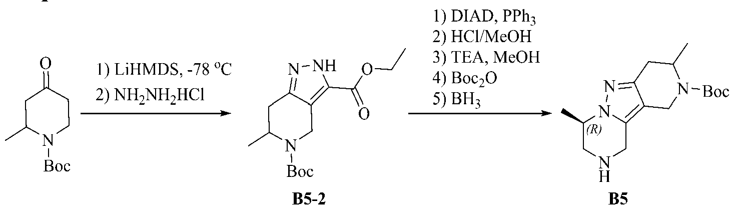

### Step 1: Preparation of intermediate B5-2

**Intermediate B5-2** was synthesized by following the protocols of **Intermediate B3-2.** MS: 310.1 (M+H)⁺.

### Step 2: Preparation of intermediate B5

**Intermediate B5** was synthesized by following the protocols of **Intermediate B2.** MS: 307.1 (M+H)⁺.

### Preparation of intermediate B6

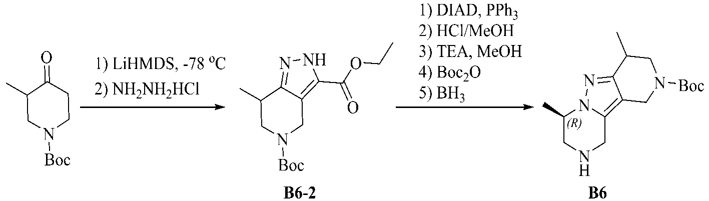

**Intermediate B6** was synthesized by following the protocols of **Intermediate B5.** MS: 307.1 (M+H)⁺.

### Preparation of intermediate B7

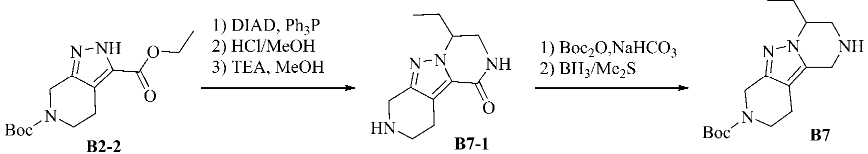

**Intermediate B7** was synthesized by following the protocols of **Intermediate B2.** MS: 307.1 (M+H)⁺.

### Preparation of intermediate B8

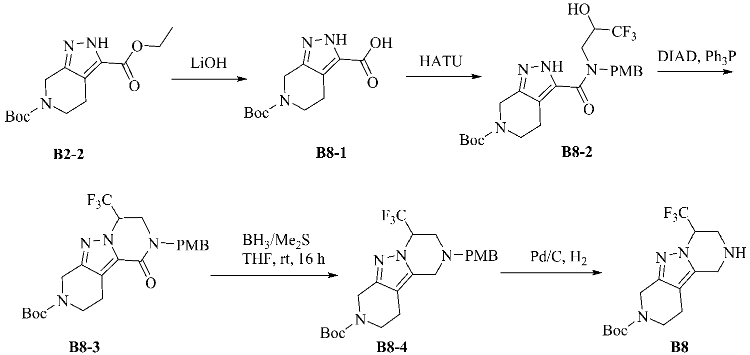

### Step 1: Preparation of intermediate B8-3

**Intermediate B8-3** was synthesized by following the protocols of **Intermediate B10-5.** MS: 481.1 (M+H)⁺.

### Step 2: Preparation of intermediate B8-4

**Intermediate B8-4** was synthesized by following the protocols of **Intermediate B2.** MS: 467.1 (M+H)⁺.

### Step 3: Preparation of intermediate B8

**Intermediate B8** was synthesized by following the protocols of **Intermediate B4-1.** MS: 347.1 (M+H)⁺.

### Preparation of intermediate B9

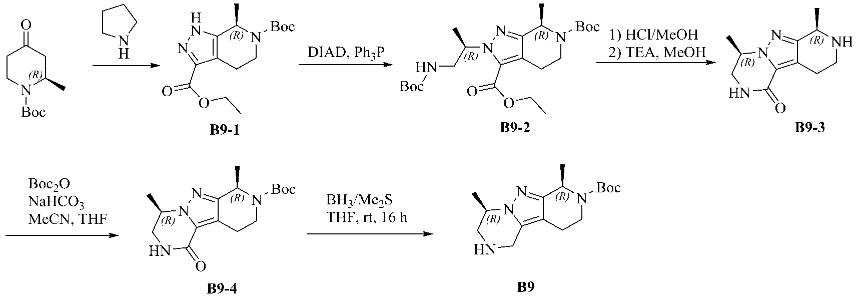

### Step 1: Preparation of intermediate B9-1

To a reaction mixture *tert-*butyl (R)-2-methyl-4-oxopiperidine-1-carboxylate (300 mg, 1.407 mmol) and ethyl diazoacetate (321 mg, 2.81 mmol) in DMSO (5 mL) was added pyrrolidine (200 mg, 2.81 mmol) dropwise, and the mixture was stirred at rt for 12 hours. The mixture was quenched with icy water and extracted with EtOAc, and the organic phase was washed with saturated saline, dried with Na₂SO₄ and concentrated. Purification by flash chromatography gave **Intermediate B9-1** (1.9 g, 65.5%) as brown solid. MS: 310.1 (M+H)⁺.

### Step 2: Preparation of intermediate B9

**Intermediate B9** was synthesized by following the protocols of **Intermediate B5.** MS: 307.1 (M+H)⁺.

### Preparation of intermediate B10

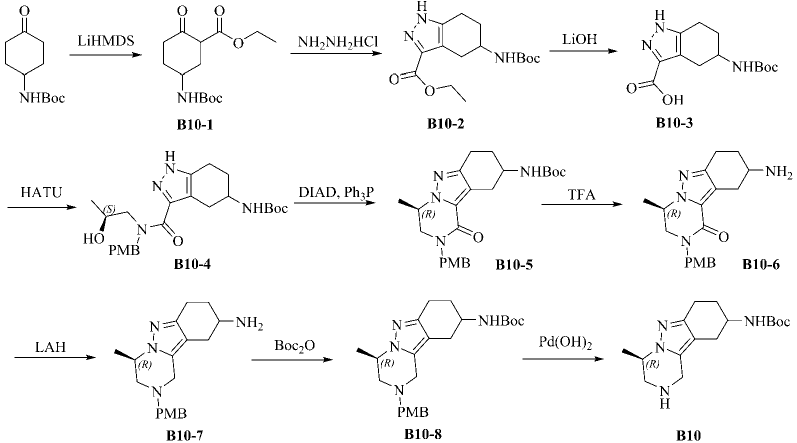

### Step 1: Preparation of intermediate B10-2

**Intermediate B10-2** was synthesized by following the protocols of **Intermediate B5-2.** MS: 310.1 (M+H)⁺.

### Step 2: Preparation of intermediate B10-3

A reaction mixture of **Intermediate B10-2** (6.4 g, 20.69 mmol) and LiOH (1.6 g, 41.38 mmol) in MeOH (10 mL), THF (10 mL) and water (10 mL) was stirred at room temperature for 16 hours. Then PH value was adjusted to 1 by the addition of HCl (6 N). The mixture was extracted with EtOAc, and the organic phase was washed with saturated saline, dried with Na₂SO₄ and concentrated. Purification by flash chromatography gave **Intermediate B10-3** (4 g, 68.7%) as white solid. MS: 282.1 (M+H)⁺.

### Step 3: Preparation of intermediate B10-4

A reaction mixture of **Intermediate B10-3** (4 g, 14.22 mmol), DIEA (9.2 g, 71.1 mmol), S) -1- ((4-methoxybenzyl) amino) propanol (3.6 g, 18.48 mmol) and HATU (6.5 g, 17.06 mmol) in DMF (50 mL) was stirred at room temperature for 16 hours. The mixture was quenched with water and extracted with EtOAc, and the organic phase was washed with saturated saline, dried with Na₂SO₄ and concentrated. Purification by flash chromatography gave **Intermediate B10-4** (5.2 g, 80%) as white solid. MS: 459.1 (M+H)⁺.

### Step 4: Preparation of intermediate B10-5

**Intermediate B10-5** was synthesized by following the protocols of **Intermediate B3-5.** MS: 441.1 (M+H)⁺.

### Step 5: Preparation of intermediate B10-6

**Intermediate B10-6** was synthesized by following the protocols of **Intermediate B3-3.** MS: 341.1 (M+H)⁺.

### Step 6: Preparation of intermediate B10-7

**Intermediate B10-7** was synthesized by following the protocols of **Intermediate B4.** MS: 327.1 (M+H)⁺.

### Step 7: Preparation of intermediate B10-8

**Intermediate B10-8** was synthesized by following the protocols of **Intermediate B2-5.** MS: 427.1 (M+H)⁺.

### Step 8: Preparation of intermediate B10

**Intermediate B10** was synthesized by following the protocols of **Intermediate B4-1.** MS: 307.1 (M+H)⁺.

### Preparation of intermediate B11

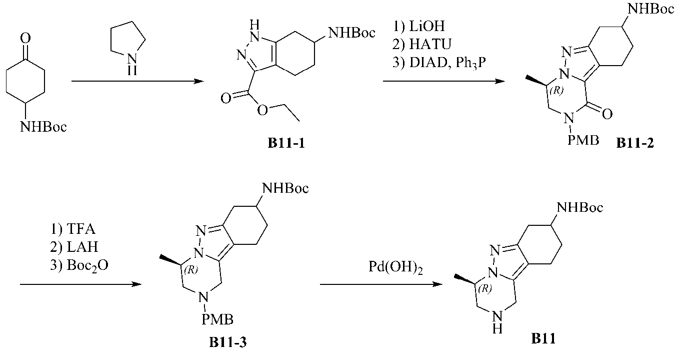

### Step 1: Preparation of intermediate B11-1

**Intermediate B11-1** was synthesized by following the protocols of **Intermediate B9-1.** MS: 310.1 (M+H)⁺.

### Step 2: Preparation of intermediate B11

**Intermediate B11** was synthesized by following the protocols of **Intermediate B10.** MS: 307.1 (M+H)⁺.

### Preparation of intermediate B12

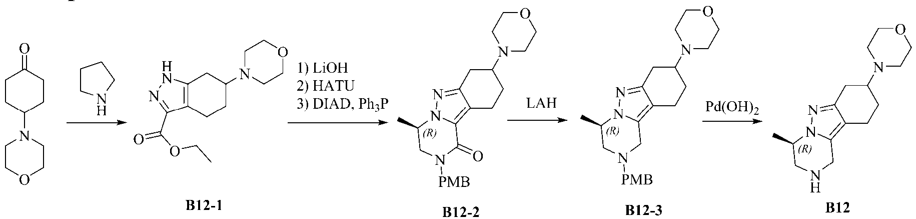

**Intermediate B12** was synthesized by following the protocols of **Intermediate B10.** MS: 277.1 (M+H)⁺.

### Preparation of intermediate B13

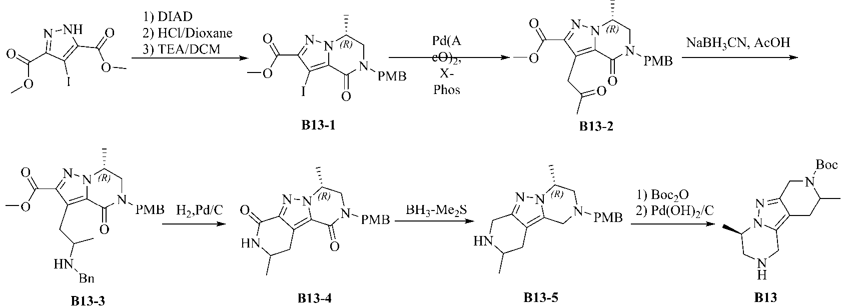

### Step 1: Preparation of intermediate B13-1

**Intermediate B13-1** was synthesized by following the protocols of Intermediate **B3-6.** MS: 456.1 (M+H)⁺.

### Step 2: Preparation of intermediate B13-2

A reaction mixture of **Intermediate B13-1**(1.7 g, 3.73 mmol), K₃PO₄ (2.38 g, 11.2 mmol), Pd(OAc)₂ (84 mg, 0373 mmol) and X-Phos (356 mg, 0.747 mmol) in acetone (10 mL) was stirred at 50 °C for 12 hours under N₂. The mixture was quenched with water and extracted with EtOAc, and the organic phase was washed with saturated saline, dried with Na₂SO₄ and concentrated. Purification by flash chromatography gave **Intermediate B13-2** (1.2 g, 83%) as yellow solid. MS: 386.2 (M+H)⁺.

### Step 3: Preparation of intermediate B13-3

A reaction mixture of benzylamine (0.53 g, 4.94 mmol), cyanide boron hydride (0.414 g, 6.59 mmol), acetic acid (0.198 g, 3.3 mmol) and **Intermediate B13-2** (1.27 g, 3.3 mmol) in MeOH (10 mL) was stirred at 25 °C for 12 hours. The mixture was quenched with water and extracted with EtOAc, and the organic phase was washed with saturated saline, dried with Na₂SO₄ and concentrated. Purification by flash chromatography gave Intermediate **B13-3** (1.4 g, 89%) as yellow solid. MS: 477.1 (M+H)⁺.

### Step 4: Preparation of intermediate B13-4

A reaction mixture of **Intermediate B13-3** (1.4 g, 2.94 mmol) and Pd/C (340 mg \) in MeOH (10 mL) was stirred at 50 °C for 8 hours. The solid were filtered out and the filtrate was concentrated under vacuum. This gave **Intermediate B13-4** (910 mg, 87%) as yellow solid. MS: 355.3 (M+H)⁺.

### Step 5: Preparation of intermediate B13-5

**Intermediate B13-5** was synthesized by following the protocols of **Intermediate B2.** MS: 327.1 (M+H)⁺.

### Step 6: Preparation of intermediate B13

**Intermediate B13** was synthesized by following the protocols of **Intermediate B10.** MS: 307.1 (M+H)⁺.

### Preparation of intermediate B14

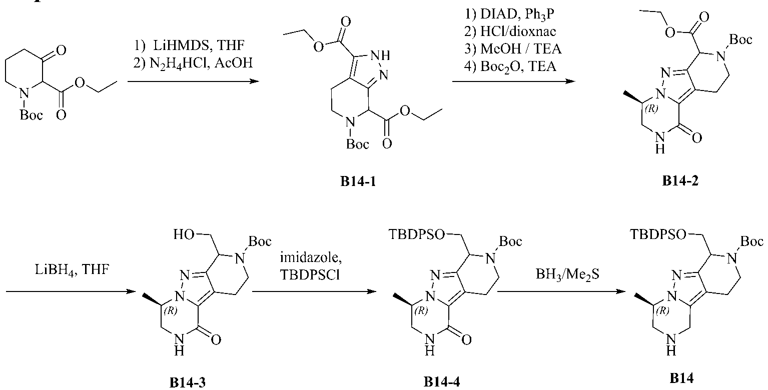

### Step 1: Preparation of intermediate B14-1

**Intermediate B14-1** was synthesized by following the protocols of **Intermediate B3-2.** MS: 368.1 (M+H)⁺. ¹H NMR (400 MHz, chloroform-*d*) δ 5.88 (s, 0.4H), 5.68 (s, 0.6H), 4.51 (dd, *J* = 13.5, 5.6 Hz, 1H), 4.43 (q, *J* = 7.1 Hz, 2H), 4.39 - 4.33 (m, 1H), 4.29 (q, *J =* 7.1 Hz, 2H), 3.33 - 3.19 (m, 1H), 2.99 (t, *J*= 15.6 Hz, 1H), 2.82 (tt, *J* = 12.1, 5.7 Hz, 1H), 1.54 (s, 9H), 1.43 (t, *J*= 7.1 Hz, 3H), 1.33 (td, *J* = 6.9, 4.0 Hz, 3H).

### Step 2: Preparation of intermediate B14-2

**Intermediate B14-2** was synthesized by following the protocols of **Intermediate B2-5.** MS: 379.1 (M+H)⁺.

### Step 3: Preparation of intermediate B14-3

A reaction mixture of **Intermediate B14-2** (1 g, 2.64 mmol) and LiBH₄ (5.29 mmol) in THF (15 mL) was stirred at 20 °C for 4 hours under N₂. The mixture was quenched with water and extracted with EtOAc, and the organic phase was washed with saturated saline, dried with Na₂SO₄ and concentrated. Purification by flash chromatography gave **Intermediate B14-3** (723 mg, 81%) as yellow solid. MS: 337.1 (M+H)⁺.

### Step 4: Preparation of intermediate B14-4

A reaction mixture of **Intermediate B14-3** (0.723 g, 2.149 mmol), imidazole (439 mg, 6.45 mmol) and TBDPSCl (884.8 mg, 3.22 mmol) in DCM (10 mL) was stirred at 20 °C for 1 hour. The mixture was quenched with water and extracted with EtOAc, and the organic phase was washed with saturated saline, dried with Na₂SO₄ and concentrated. Purification by flash chromatography gave **Intermediate B14-4** (1.1 g, 89%) as yellow oil. MS: 575.1 (M+H)⁺. ¹H NMR (400 MHz, DMSO-*d*₆) δ 8.11 (s, 1H), 7.65 - 7.53 (m, 4H), 7.52 - 7.38 (m, 6H), 5.24 (dd, *J*= 47.4, 19.2 Hz, 1H), 4.33 (dd, *J =* 23.2, 10.7 Hz, 1.5H), 4.11 (s, 0.5H), 3.93 (d, *J* = 18.8 Hz, 2H), 3.70 - 3.56 (m, 1H), 3.30 (d, *J*= 11.5 Hz, 1H), 3.09 (dd, *J*= 26.5, 12.9 Hz, 1H), 2.82 (t, *J=* 17.2 Hz, 1H), 2.64 - 2.53 (m, 1H), *J* = 18.5, 8.4 Hz, 12H), 0.93 (d, *J =* 12.4 Hz, 9H).

### Step 5: Preparation of intermediate B14

**Intermediate B14** was synthesized by following the protocols of **Intermediate B2.** MS: 561.1 (M+H)⁺.

### Preparation of intermediate B15

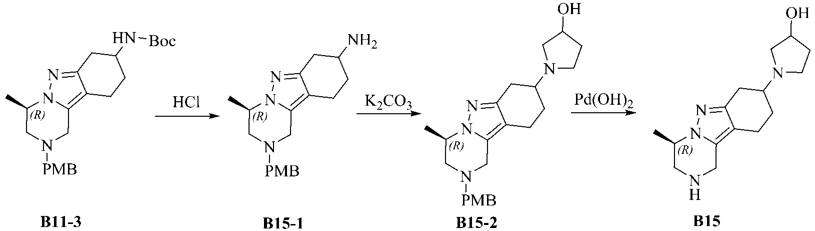

### Step 1: Preparation of intermediate B15-1

**Intermediate B15-1** was synthesized by following the protocols of Intermediate **B1-4.** MS: 327.1 (M+H)⁺.

### Step 2: Preparation of intermediate B15-2

A reaction mixture of **Intermediate B15-1** (200 mg, 0.613 mmol, K₂CO₃ (254 mg, 1838 mmol) and 1,4-dibromobutane-2-ol (213 mg, 0.613 mmol) in acetonitrile (10 mL) was stirred at 100 °C for 16 hours. The mixture was quenched with water and extracted with EtOAc, and the organic phase was washed with saturated saline, dried with Na₂SO₄ and concentrated. Purification by flash chromatography gave **Intermediate B15-2** (210 mg, 86%) as yellow oil. MS: 397.1 (M+H)⁺.

### Step 3: Preparation of intermediate B15

**Intermediate B15** was synthesized by following the protocols of Intermediate **B12.** MS: 277.5 (M+H)⁺.

### Preparation of intermediate B16

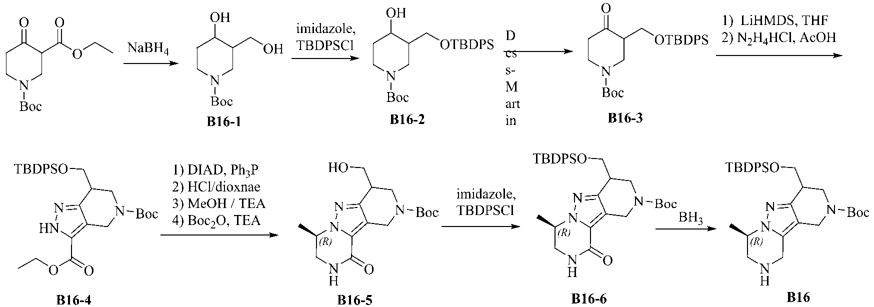

### Step 1: Preparation of intermediate B16-1

To a mixture of 1-(*tert*-butyl) 3-ethyl 4-oxopiperidine-1,3-dicarboxylate (25 g, 92 mmol) in MeOH (300 mL) was added sodium borohydride (10.46 g, 276 mmol) and the reaction mixture was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B16-1** (20 g, 94%) as colorless oil. MS: 232.1 (M+H)⁺.

### Step 2: Preparation of intermediate B16-2

**Intermediate B16-2** was synthesized by following the protocols of **Intermediate B14-4.** MS: 470.1 (M+H)⁺.

### Step 3: Preparation of intermediate B16-3

A reaction mixture of **Intermediate B16-2** (25 g, 53.2 mmol) and Dess-Martin periodinane (33.9 g, 80 mmol) in DCM (120 mL) was stirred at 25 °C for 1 hour. The mixture was quenched with water and extracted with EtOAc, and the organic phase was washed with saturated saline, dried with Na₂SO₄ and concentrated. Purification by flash chromatography gave **Intermediate B16-3** (21 g, 84%) as colorless oil. MS: 468.1 (M+H)⁺. ¹H NMR (400 MHz, chloroform-*d*) δ 7.66 (ddd, *J* = 7.9, 6.3, 1.6 Hz, 4H), 7.45 - 7.36 (m, 6H), 4.43 (d, *J* = 8.0 Hz, 1H), 4.22 - 3.71 (m, 3H), 3.22 (d, *J* = 62.8 Hz, 2H), 2.67 (dq, *J* = 10.2, 6.2 Hz, 1H), 2.50 - 2.34 (m, 2H), 1.50 (s, 9H), 1.05 (s, 9H).

### Step4: Preparation of intermediate B16-4

**Intermediate B16-4** was synthesized by following the protocols of **Intermediate B14-1.** MS: 564.1 (M+H)⁺. ¹H NMR (400 MHz, Chloroform-d) δ 7.65 - 7.60 (m, 4H), 7.43 - 7.37 (m, 6H), 4.74 (d, *J* = 17.1 Hz, 1H), 4.55 to 4.35 (m, 3H), 3.82 (d, *J* = 30.0 Hz, 3H), 3.35 (d, *J* = 106.0 Hz, 2H), 2.04 (s, 1H), 1.45 (s, 9H), 1.40 (d, *J* = 7.3 Hz, 3H), 1.08 (s, 9H).

### StepS: Preparation of intermediate B16-5

**Intermediate B16-5** was synthesized by following the protocols of **Intermediate B14-2.** MS: 337.1 (M+H)⁺.

### Step6: Preparation of intermediate B16

**Intermediate B16** was synthesized by following the protocols of **Intermediate B14.** MS: 561.1 (M+H)⁺.

### Preparation of intermediate B17

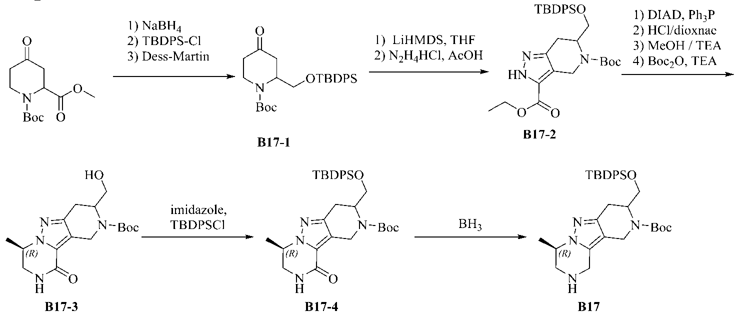

**Intermediate B17** was synthesized by following the protocols of **Intermediate B16.** MS: 561.1 (M+H)⁺.

### Preparation of intermediate B18

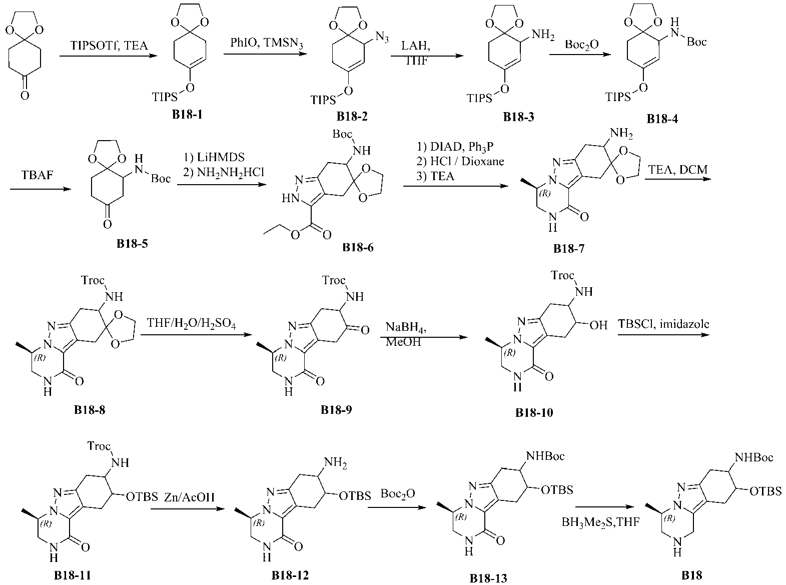

### Step1: Preparation of intermediate B18-1

A reaction mixture of TIPS-OTf (7.06 g, 23.05 mmol), 1,4-dioxaspiro[4.5]decan-8-one (3 g, 19.21 mmol) and TEA (3.5 g, 34.6 mmol) in DCM (30 mL) was stirred at 25 °C for 2 hours under N₂. The mixture was quenched with water and extracted with EtOAc, and the organic phase was washed with saturated saline, dried with Na₂SO₄ and concentrated. Purification by flash chromatography gave **Intermediate B18-1** (3.12 g, 52%) as colorless oil.

### Step2: Preparation of intermediate B18-2

A reaction mixture of TMSN₃ (3.35 g, 29.1 mmol), PhIO (32 g, 14.54 mmol) and **Intermediate B18-1**(2.84 g, 9.09 mmol) in DCM (60 mL) was stirred at 25 °C for 2 hours under N₂. The reaction mixture was concentrated to give the crude product **Intermediate B18-2** which was used directly in the next step.

### Step3: Preparation of intermediate B18-3

A reaction mixture of LAH (7.3 mL, 18.18 mmol, 2.5 mol/l in THF) and **Intermediate B18-2** (3.21 g, 9.09 mmol) in DCM (50 mL) was stirred at -10 °C for 3 hours under N₂. The mixture was quenched with water and extracted with EtOAc, and the organic phase was washed with saturated saline, dried with Na₂SO₄ and concentrated. **Intermediate B18-3** was obtained and used directly in the next reaction.

### Step4: Preparation of intermediate B18-4

**Intermediate B18-4** was synthesized by following the protocols of **Intermediate B2-5.** MS: 450.1 (M+Na)⁺.¹H NMR (400 MHz, chloroform-*d*) δ 4.71 (s, 1H), 4.59 (d, *J* = 15.7 Hz, 1H), 4.16 - 3.90 (m, 4H), 2.52 (d, *J*= 13.5 Hz, 1H), 2.25 (dd, *J*= 20.2, 8.1 Hz, 2H), 1.80 (ddd, *J* = 34.7, 13.3, 6.8 Hz, 1H), 1.63 (s, 1H), 1.46 (s, 9H), 1.10 - 1.02 (m, 18H).

### Step5: Preparation of intermediate B18-5

A reaction mixture of TBAF (6.2 mL, 6.21 mmol, 1 mol/L in THF) and **Intermediate B18-4** (1.77 g, 4.14 mmol) in THF (25 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B18-5** (645 mg, 57.4%) as yellow solid. MS: 272.1 (M+H)⁺. ¹H NMR (400 MHz, chloroform-*d*) δ 4.63 (s, 1H), 4.21 - 4.00 (m, 5H), 2.76 - 2.65 (m, 1H),2.60 - 2.37 (m, 3H), 2.04 (dt, *J* = 8.0, 5.2 Hz, 1H), 1.88 (d, *J* = 12.3 Hz, 1H), 1.46 (d, *J* = 2.1 Hz, 9H).

### Step6: Preparation of intermediate B18-6

**Intermediate B18-6** was synthesized by following the protocols of **Intermediate B3-2.** MS: 368.1 (M+H)⁺. ¹H NMR (400 MHz, chloroform-*d*) δ 4.70 (s, 1H), 4.41 (q, *J* = 7.1 Hz, 2H), 4.23 (s, 1H), 4.17 (s, 2H), 4.06 (d, *J*= 6.2 Hz, 2H), 3.24 (dd, *J* = 16.0, 5.7 Hz, 1H), 3.17 (d, *J* = 17.5 Hz, 1H), 2.98 (d, *J=* 17.6 Hz, 1H), 2.85 (dd, *J* = 16.0, 8.8 Hz, 1H), 1.50 (s, 9H), 1.42 (t, *J* = 7.1 Hz, 3H).

### Step7: Preparation of intermediate B18-7

**Intermediate B18-7** was synthesized by following the protocols of **Intermediate B3-6.** MS: 279.1 (M+H)⁺. ¹H NMR (400 MHz, DMSO-*d*₆) δ 8.37 (s, 1H), 8.18 (d, *J=* 3.1 Hz, 1H), 4.41 (dt, *J=* 13.8, 7.1 Hz, 1H), 4.14 - 3.98 (m, 4H), 3.75 - 3.60 (m, 2H), 3.36 - 3.28 (m, 2 H), 3.23 (dt, *J* = 15.5, 5.2 Hz, 1H), 3.17 - 3.10 (m, 1H), 3.06 (q, *J=* 7.3 Hz, 3H), 2.80 (td, *J* = 16.3, 6.7 Hz, 2H), 1.44 (t, *J* = 6.7 Hz, 3H), 1.23 (t, *J* = 7.3 Hz, 4H).

### Step8: Preparation of intermediate B18-8

A reaction mixture of 2,2,2-trichloroethylchloroformate (1.05 g, 4.96 mmol) and **Intermediate B18-7** (920 mg, 3.31 mmol) and TEA (1.004 g, 9.92 mmol) in DCM (5 mL) was stirred at 0 °C for 1 hour. The mixture was quenched with water and extracted with EtOAc, and the organic phase was washed with saturated saline, dried with Na₂SO₄ and concentrated. Purification by flash chromatography gave **Intermediate B18-8** (467 mg, 31.1%) as yellow oil. MS: 454.1 (M+H)⁺.

### Step9: Preparation of intermediate B18-9

A reaction mixture of **Intermediate B18-8** (190 mg, 0.419 mmol), H₂O (1 mL) and H₂SO₄ (1 mL) in THF (4 mL) was stirred at 65 °C for 3 hours. The mixture was quenched with water and extracted with EtOAc, and the organic phase was washed with saturated saline, dried with Na₂SO₄ and concentrated. Purification by flash chromatography gave **Intermediate B18-9** (87 mg, 50.6%) as yellow solid. MS: 409.1 (M+H)⁺.

### Step10: Preparation of intermediate B18-10

**Intermediate B18-10** was synthesized by following the protocols of **Intermediate B16-1.** MS: 411.1 (M+H)⁺.

### Step11: Preparation of intermediate B18-11

**Intermediate B18-11** was synthesized by following the protocols of **Intermediate B16-2.** MS: 525.1 (M+H)⁺. ¹H NMR (400 MHz, DMSO-*d*₆) δ 8.09 (s, 1H), 7.69 (dd, *J=* 8.6, 5.2 Hz, 1H), 4.83 (dd, *J* = 12.2, 3.3 Hz, 1H), 4.74 - 4.68 (m, 1H), 4.38 (s, 1H), 3.95 (s, 1H), 3.86 - 3.77 (m, 1H), 3.67 - 3.58 (m, 1H), 3.34 - 3.24 (m, 1H), 3.15 - 3.05 (m, 1H), 2.94 (dd, *J=* 15.1, 4.2 Hz, 1H), 2.72 (dd, *J=* 16.2, 7.8 Hz, 1H), 2.60 (dd, *J* = 16.6, 8.2 Hz, 1H), 1.44 (t, *J* = 5.9 Hz, 3H), 0.87 (s, 9H), 0.13 - 0.09 (m, 6H).

### Step12: Preparation of intermediate B18-12

A reaction mixture of **Intermediate B18-11**(300 mg, 0.57 mmol), zinc powder (182.4 mg, 2.85 mmol) in HOAc (3 mL) was stirred at 25 °C for 3 hours. The mixture was quenched with water. Then filter to remove the zinc powder then wash with EtOAc. The mixture was extracted with EtOAc, and the organic phase was washed with saturated saline, dried with Na₂SO₄ and concentrated. Purification by flash chromatography gave **Intermediate B18-12** (220 mg, 50.6%) which was used directly in the next reaction.

### Step13: Preparation of intermediate B18

**Intermediate B18** was synthesized by following the protocols of **Intermediate B9.** MS: 437.1 (M+H)⁺.

### Preparation of intermediate B19

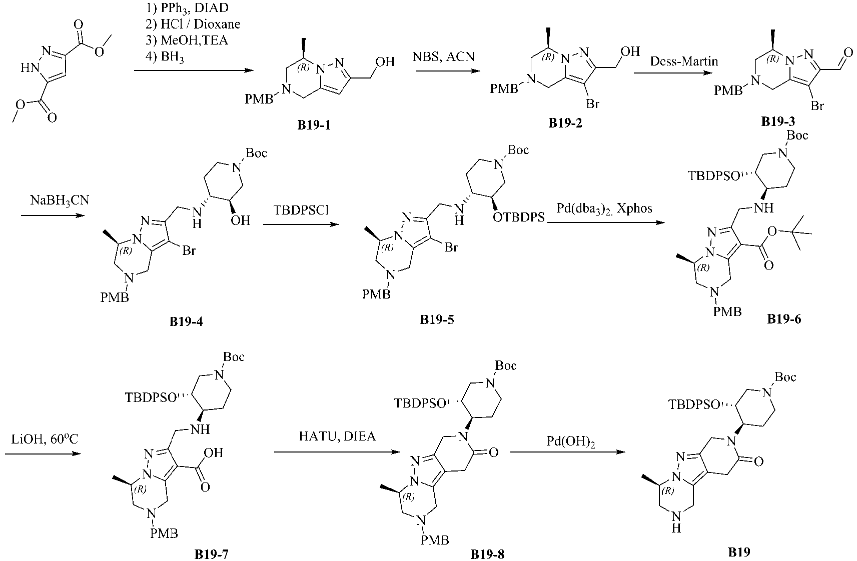

### Step 1: Preparation of intermediate B19-1

**Intermediate B19-1** was synthesized by following the protocols of **Intermediate B2.** MS: 288.2 (M+H)⁺.

### Step 2: Preparation of intermediate B19-2

A reaction mixture of **Intermediate B19-1** (1.5 g, 5.22 mmol) and NBS (1.115 g, 6.26 mmol) in DCM (20 mL) was stirred at 25 °C for 1 hour. The mixture was quenched with water and extracted with EtOAc, and the organic phase was washed with saturated saline, dried with Na₂SO₄ and concentrated. Purification by flash chromatography gave **Intermediate B19-2** (1.6 g, 84%) as yellow solid. MS: 366.1&368.1 (M+H)⁺.

### Step 3: Preparation of intermediate B19-3

**Intermediate B19-3** was synthesized by following the protocols of Intermediate **B16-3.** MS: 364.1 & 366.1(M+H)⁺.

### Step 4: Preparation of intermediate B19-4

A reaction mixture of **Intermediate B19-3** (900 mg, 2.47 mmol), HOAc (148 mg, 247 mmol) and (*trans*)-4-amino-3-hydroxypiperidine-1-carboxylate *tert*-butyl ester (588 mg, 2.72 mmol) in MeOH (10 mL) was stirred at 25 °C for 1 hour before sodium cyanoborohydride (311 mg, 4.94 mmol) was added to the mixture. The resulting mixture was stirred rt for 2 hours. The mixture was quenched with water and extracted with EtOAc, and the organic phase was washed with saturated saline, dried with Na₂SO₄ and concentrated. Purification by flash chromatography gave **Intermediate B19-4** (850 mg, 60.9%) as white solid. MS: 564.2&566.2 (M+H)⁺.

### Step 5: Preparation of intermediate B19-5

**Intermediate B19-5** was synthesized by following the protocols of Intermediate **B16-2.** MS: 802.2 & 804.2(M+H)⁺.

### Step 6: Preparation of intermediate B19-6

A reaction mixture of **Intermediate B19-5** (850 mg, 1.059 mmol), Pd₂(dba)₃ (97 mg, 0.106 mmol), Xphos (101 mg, 0.212 mmol) and bromo[2-(1,1-dimethylethoxy)-2-oxoethyl] Zinc (551 mg, 2.117 mmol) in THF (5 mL) was stirred at 70 °C for 2 hours under N₂. The mixture was quenched with water and extracted with EtOAc, and the organic phase was washed with saturated saline, dried with Na₂SO₄ and concentrated. Purification by flash chromatography gave **Intermediate B19-6** (520 mg, 59.6%) as yellow solid. MS: 824.5 (M+H)⁺.

### Step 7: Preparation of intermediate B19-8

**Intermediate B19-8** was synthesized by following the protocols of **Intermediate B10-4.** MS: 764.5 (M+H)⁺.

### Step 8: Preparation of intermediate B19

**Intermediate B19** was synthesized by following the protocols of **Intermediate B10.** MS: 644.3 (M+H)⁺.

### Preparation of intermediate B20

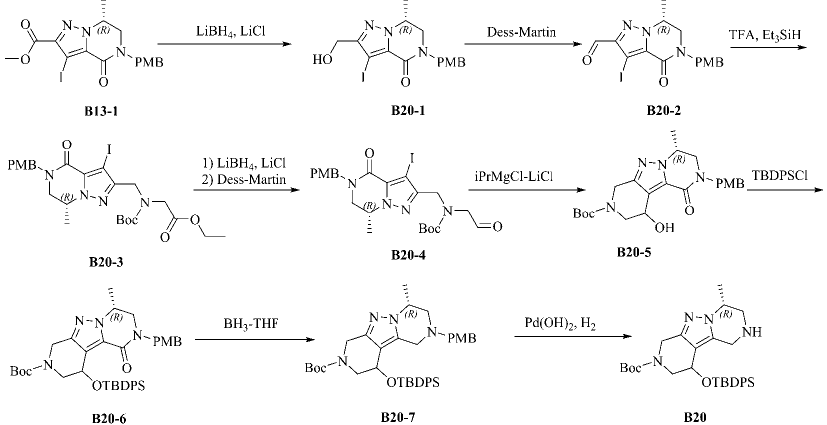

### Step 1: Preparation of intermediate B20-1

A reaction mixture of **Intermediate B13-1** (6 g, 13.18 mmol), LiCl (1.676 g, 39.5 mmol) and LiBH₄ (0.861 g, 39.5 mmol) in THF (30 mL) was stirred at 25 °C for 4 hours under H₂. The mixture was quenched with water and extracted with EtOAc, and the organic phase was washed with saturated saline, dried with Na₂SO₄ and concentrated. Purification by flash chromatography gave **Intermediate B20-1** (4.5 g, 80%) as white solid. MS: 428.1 (M+H)⁺.

### Step 2: Preparation of intermediate B20-2

**Intermediate B20-2** was synthesized by following the protocols of Intermediate **B16-3.** MS: 426.1(M+H)⁺.

### Step 3: Preparation of intermediate B20-3

A reaction mixture of **Intermediate B20-2** (5 g, 11.76 mmol), TFA (6 g, 58.8 mmol) and triethyl silane (13.67 g, 118 mmol) in DCM (100 mL) was stirred at 25 °C for 16 hours under H₂. The mixture was quenched with water and extracted with EtOAc, and the organic phase was washed with saturated saline, dried with Na₂SO₄ and concentrated. Purification by flash chromatography gave **Intermediate B20-3** (3.3 g, 45.8%) as colorless oil. MS: 613.2 (M+H)⁺.

### Step 4: Preparation of intermediate B20-4

**Intermediate B20-4** was synthesized by following the protocols of **Intermediate B20-2.** MS: 569.2 (M+H)⁺.

### Step 5: Preparation of intermediate B20-5

A reaction mixture of **Intermediate B20-4** (620 mg, 1.091 mmol) and i-PrMgCl.LiCl (1.2 mL, 1.636 mmol, 1 mol/L in THF) in THF (10 mL) was stirred at -78 °C for 2 hours under H₂. The mixture was quenched with water and extracted with EtOAc, and the organic phase was washed with saturated saline, dried with Na₂SO₄ and concentrated. Purification by flash chromatography gave **Intermediate B20-5** (150 mg, 51.8%) as colorless oil. MS: 443.2 (M+H)⁺.

### Step 6: Preparation of intermediate B20-7

**Intermediate B20-7** was synthesized by following the protocols of Intermediate **B17.** MS: 667.1 (M+H)⁺.

### Step 7: Preparation of intermediate B20

**Intermediate B20** was synthesized by following the protocols of Intermediate **B10.** MS: 547.1(M+H)⁺.

### Preparation of intermediate B21

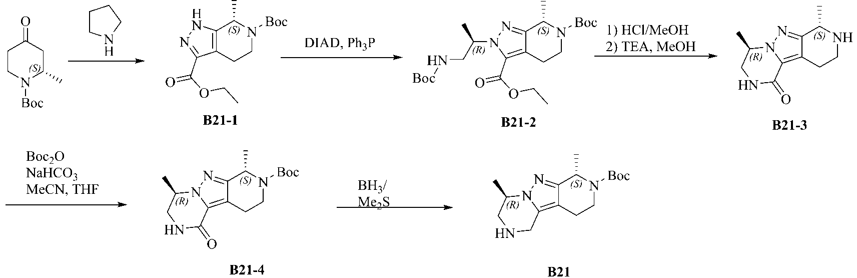

**Intermediate B21** was synthesized by following the protocols of **Intermediate B9.** MS: 307.1(M+H)⁺.

### Preparation of intermediate B22

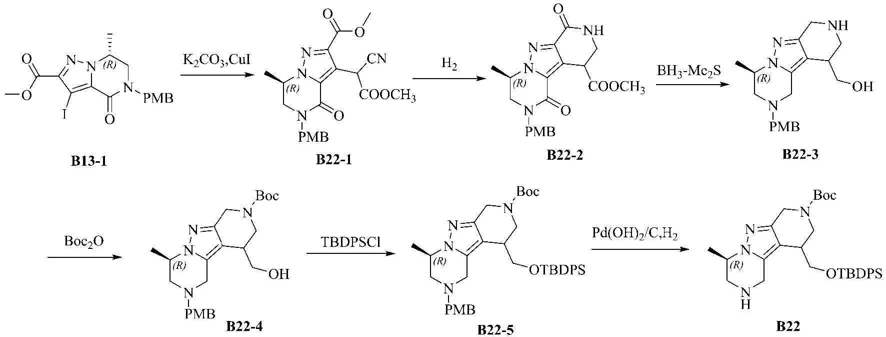

### Step 1: Preparation of intermediate B22-1

A reaction mixture of methyl cyanoacetate (1.959 g, 19.77 mmol), CuI (126 mg, 0.659 mmol), K₂CO₃ (3.64 g, 26.4 mmol) and **Intermediate B13-1** (3 g, 6.59 mmol) in DMSO (40 mL) was stirred at 90 °C for 12 hours under N₂. The mixture was quenched with water and extracted with EtOAc, and the organic phase was washed with saturated saline, dried with Na₂SO₄ and concentrated. Purification by flash chromatography gave **Intermediate B22-1** (1.5 g, 60.9%) as yellow solid. MS: 427.4 (M+H)⁺.

### Step 2: Preparation of intermediate B22-2

A reaction mixture of **Intermediate B22-1**(2.3 g, 5.39 mmol) and nickel powder (0.317 g, 5.39 mmol) in MeOH (10 mL) was stirred at 40 °C for 12 hours under H₂. The mixture was quenched with water and extracted with EtOAc, and the organic phase was washed with saturated saline, dried with Na₂SO₄ and concentrated. Purification by flash chromatography gave **Intermediate B22-2** (1.2 g, 55.8%) as yellow solid. MS: 399.2 (M+H)⁺.

### Step 3: Preparation of intermediate B22-3

**Intermediate B22-3** was synthesized by following the protocols of **Intermediate B2.** MS: 343.1 (M+H)⁺.

### Step 4: Preparation of intermediate B22-4

**Intermediate B22-4** was synthesized by following the protocols of **Intermediate B2-5.** MS: 443.1 (M+H)⁺.

### Step 5: Preparation of intermediate B22-5

**Intermediate B22-5** was synthesized by following the protocols of **Intermediate B14-4.** MS: 682.1 (M+H)⁺.

### Step 6: Preparation of intermediate B22

**Intermediate B22** was synthesized by following the protocols of **Intermediate B10.** MS: 561.1(M+H)⁺.

### Preparation of intermediate B23

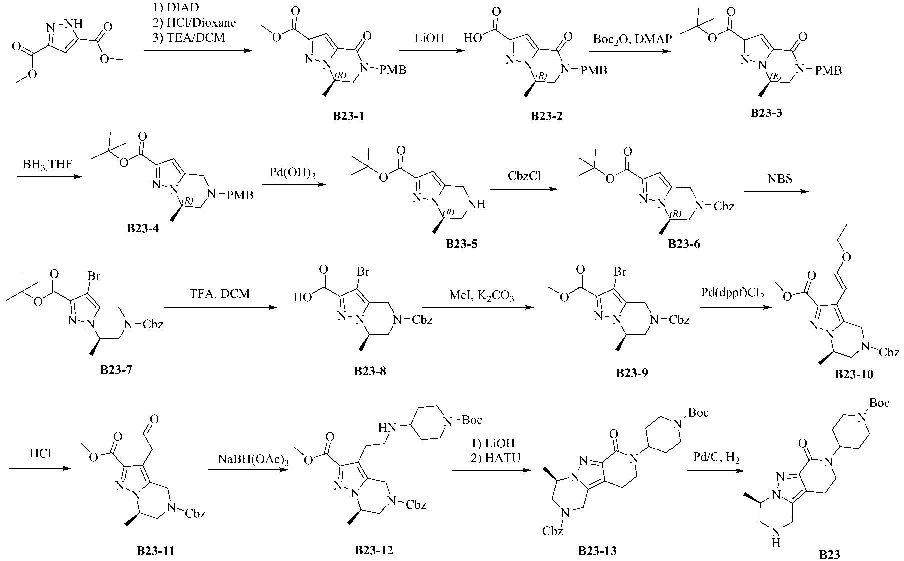

### Step 1: Preparation of intermediate B23-1

**Intermediate B23-1** was synthesized by following the protocols of **Intermediate B13-1.** MS: 330.1 (M+H)⁺.

### Step 2: Preparation of intermediate B23-2

**Intermediate B23-2** was synthesized by following the protocols of **Intermediate B10-3.** MS: 316.1 (M+H)⁺.

### Step 3: Preparation of intermediate B23-3

A reaction mixture of **Intermediate B23-2** (100 mg, 0.317 mmol), Boc₂O (104 mg, 0.476 mmol) and DMAP (3.87 mg, 0.032 mmol) in THF (1 mL) was stirred at 60 °C for 1 hour. The mixture was quenched with water and extracted with EtOAc, and the organic phase was washed with saturated saline, dried with Na₂SO₄ and concentrated. Purification by flash chromatography gave **Intermediate B23-3** (100 mg, 85%) as white solid. MS: 372.2 (M+H)⁺.

### Step 4: Preparation of intermediate B23-5

**Intermediate B23-5** was synthesized by following the protocols of **Intermediate B20.** MS: 547.1(M+H)⁺.

### Step 5: Preparation of intermediate B23-6

A reaction mixture of **Intermediate B23-5** (300 mg, 1.264 mmol), CbzCl (259 mg, 1.517 mmol) and TEA (192 mg, 1.896 mmol) in DCM (5 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B22-6** (450 mg, 96%) as colorless oil. MS: 372.2 (M+H)⁺.

### Step 6: Preparation of intermediate B23-7

A reaction mixture of **Intermediate B23-6** (2.7 g, 2.727 mmol), NBS (1.553 g, 8.72 mmol) in Ac₂O (20 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B22-7** (3 g, 92%) as yellow solid. MS: 450.1&452.1 (M+H)⁺.

### Step 7: Preparation of intermediate B23-8

A reaction mixture of **Intermediate B22-7** (3 g, 6.66 mmol) and TFA (10 mL) in DCM (10 mL) was stirred at 25 °C for 2 hours. The reaction mixture is then concentrated under vacuum to give **Intermediate B22-8** (2.5 g, 95%) as yellow solid and used in the next reaction without further purification. MS: 394.1&396.1 (M+H)⁺.

### Step 8: Preparation of intermediate B23-9

A reaction mixture of **Intermediate B23-8** (2.5 g, 6.34 mmol), MeI (1.35 g, 9.51 mmol) and K₂CO₃ (1.753 g, 12.68 mmol) in DMF (20 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B22-9** (2.5 g, 97%) as yellow solid. MS: 408.1&410.1 (M+H)⁺.

### Step 9: Preparation of intermediate B23-10

A reaction mixture of **Intermediate B23-9** (2.5 g, 6.12 mmol), 2-(2-ethoxyvinyl)-4,4,5,5-tetramethyl-1,3,2-dioxaborolane (2.426 g, 12.25 mmol), PdCl₂(dppf) (0.448 g, 0.612 mmol) and K₂CO₃ (2.54 g, 18.37 mmol) in 1,4-dioxane (30 mL) and water (6 mL) was stirred at 100 °C for 4 hours. The mixture was quenched with water and extracted with EtOAc, and the organic phase was washed with saturated saline, dried with Na₂SO₄ and concentrated. Purification by flash chromatography gave **Intermediate B22-10** (1 g, 40.9%) as yellow oil. MS: 400.2 (M+H)⁺.

### Step 10: Preparation of intermediate B23-11

A reaction mixture of **Intermediate B23-10** (1 g, 2.503 mmol) in HCl (5 mL, 4 M in dioxane) was stirred at 25 °C for 2 hours. The reaction mixture is then concentrated under vacuum. **Intermediate B22-11** (550 mg, 59.2%) was obtained as yellow oil and used directly in the next reaction. MS: 372.2 (M+H)⁺.

### Step 11: Preparation of intermediate B23-12

**Intermediate B23-12** was synthesized by following the protocols of **Intermediate B13-3.** MS: 556.3 (M+H)⁺.

### Step 12: Preparation of intermediate B23-13

**Intermediate B23-13** was synthesized by following the protocols of **Intermediate B10-4.** MS: 524.2 (M+H)⁺.

### Step 13: Preparation of intermediate B23

**Intermediate B23** was synthesized by following the protocols of **Intermediate B8.** MS: 390.2 (M+H)⁺.

### Preparation of intermediate B24

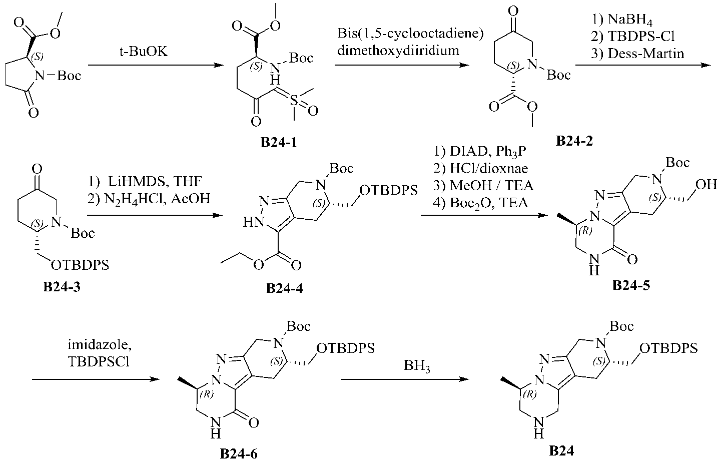

### Step 1: Preparation of Intermediate B24-1

A reaction mixture of t-BuOK (10.15 g, 90 mmol) and trimethylsulfoxonium iodide (22.6 g, 103 mmol) in THF (500 mL) was stirred at 0 °C for 0.5 hour. Then 1-(*tert*-butyl) 2-methyl (S)-5-oxopyrrolidine-1,2-dicarboxylate (20 g, 82 mmol) in THF (100 mL) was added to the mixture. The resulting mixture was stirred at 25 °C for 1 hour. The mixture was quenched with water and extracted with EtOAc, and the organic phase was washed with saturated saline, dried with Na₂SO₄ and concentrated. Purification by flash chromatography gave **Intermediate B24-1** (47 g, 137%) as white solid. MS: 400.2 (M+H)⁺.

### Step 2: Preparation of Intermediate B24-2

A reaction mixture of **Intermediate B24-1**(5 g, 14.91 mmol) and bis(1,5-cyclooctadiene)(methoxy)iridium(I) dimer (0.1 g, 0.149 mmol) in DCE (100 mL) was stirred at 85 °C for 15 hours. The mixture was quenched with water and extracted with EtOAc, and the organic phase was washed with saturated saline, dried with Na₂SO₄ and concentrated. Purification by flash chromatography gave **Intermediate B24-2** (1.6 g, 41.7%) as colorless oil.

### Step 3: Preparation of Intermediate B24

**Intermediate B24** was synthesized by following the protocols of **Intermediate B16.** MS: 561.2 (M+H)⁺.

### Preparation of intermediate B25

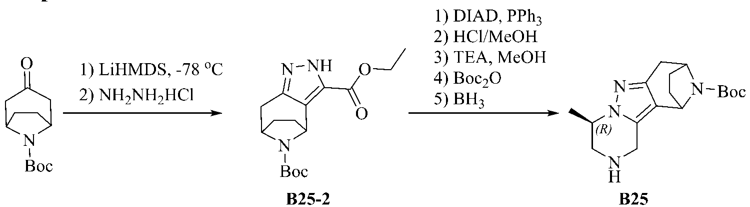

**Intermediate B25** was synthesized by following the protocols of Intermediate **B5.** MS: 319.2 (M+H)⁺.

### Preparation of intermediate B26

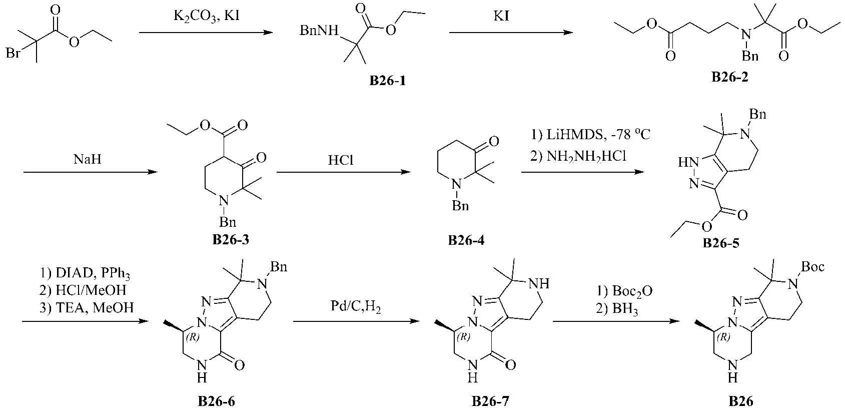

### Step 1: Preparation of intermediate B26-1

A reaction mixture of ethyl 2-bromo-2-methylpropanoate (60 g, 310.8 mmol), benzylamine (33 g, 308 mmol), K₂CO₃ (42.5 mg, 308 mmol) and KI (51.1 g, 308 mmol) in EtOH (300 mL) was stirred at 80 °C for 16 hours. The mixture was quenched with water and extracted with EtOAc, and the organic phase was washed with saturated saline, dried with Na₂SO₄ and concentrated. Purification by flash chromatography gave **Intermediate B26-1** (17.5 g, 25.8%) as colorless oil. MS: 222.1 (M+H)⁺.

### Step 2: Preparation of intermediate B26-2

A reaction **Intermediate B26-1**(17.5 g, 79 mmol), ethyl 4-bromobutyrate (16.97 g, 87 mmol) and KI (0.656 g, 3.95 mmol) in EtOH (300 mL) was stirred at 125 °C for 16 hours. The mixture was quenched with water and extracted with EtOAc, and the organic phase was washed with saturated saline, dried with Na₂SO₄ and concentrated. Purification by flash chromatography gave **Intermediate B26-2** (5.2 g, 19.6%) as colorless oil. MS: 336.2 (M+H)⁺.

### Step 3: Preparation of intermediate B26-3

A reaction mixture of **Intermediate B26-2** (5.2 g, 15.5 mmol), NaH (0.682 g, 17.05 mmol) in THF (100 mL) was stirred at 0 °C for 4 hours. The mixture was quenched with water and extracted with EtOAc, and the organic phase was washed with saturated saline, dried with Na₂SO₄ and concentrated. Purification by flash chromatography gave **Intermediate B26-3** (4 g, 89%) as yellow oil. MS: 290.2 (M+H)⁺.

### Step 4: Preparation of intermediate B26-4

A reaction mixture of **Intermediate B26-3** (4 g, 13.82 mmol) in HCl (6 N, 30 mL) was stirred at 25 °C for 3 hours. Then the pH value was adjusted to pH8 by addition of K₂CO₃ solution. The mixture was extracted with EtOAc, and the organic phase was washed with saturated saline, dried with Na₂SO₄ and concentrated. **Intermediate B26-4** (2.4 g, 80%) was obtained as yellow oil. MS: 218.4 (M+H)⁺. ¹H NMR (400 MHz, chloroform-*d*) δ 7.42 - 7.29 (m, 5H), 3.67 (s, 2H), 2.70 (t, *J =* 6.0 Hz, 2H), 2.54 (t, *J =* 7.1 Hz, 2H), 1.92 - 1.83 (m, 2H), 1.35 (s, 6H).

### Step 5: Preparation of intermediate B26-5

**Intermediate B26-5** was synthesized by following the protocols of **Intermediate B3-2.** MS: 314.4 (M+H)⁺. ¹H NMR (400 MHz, chloroform-*d*) δ 7.49 - 7.43 (m, 2H), 7.40 - 7.33 (m, 2H), 7.30 (d, *J* = 6.3 Hz, 1H), 4.39 (q, *J* = 7.1 Hz, 2H), 3.75 (s, 2H), 2.75 (d, *J* = 2.6 Hz, 4H), 1.58 (s, 6H), 1.39 (t, *J* = 7.1 Hz, 3H).

### Step 6: Preparation of intermediate B26-6

**Intermediate B26-6** was synthesized by following the protocols of Intermediate **B2-4.** MS: 325.1 (M+H)⁺.

### Step 7: Preparation of intermediate B26-7

**Intermediate B26-7** was synthesized by following the protocols of **Intermediate B8.** MS: 235.2 (M+H)⁺.

### Step 8: Preparation of intermediate B26

**Intermediate B26** was synthesized by following the protocols of **Intermediate B2.** MS: 320.2 (M+H)⁺.

### Preparation of intermediate B27

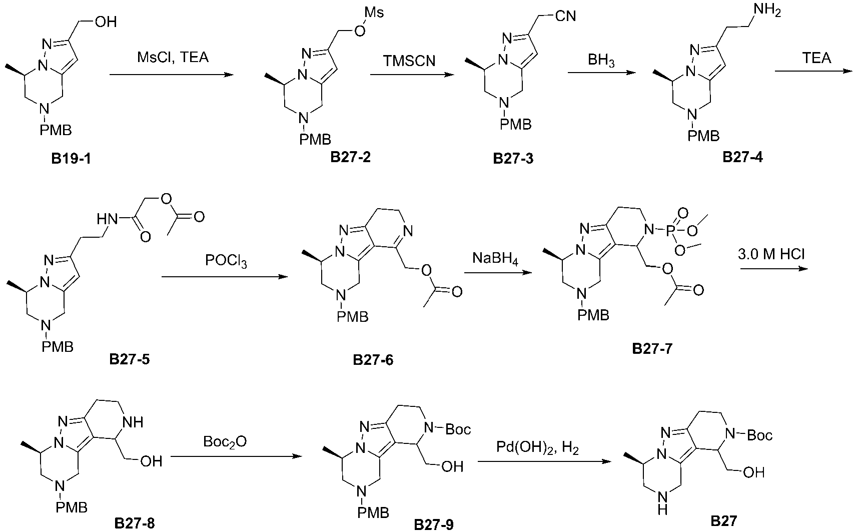

### Step 1: Preparation of intermediate B27-2

A reaction mixture of TMSCl (6.86 g, 59.9 mmol), **Intermediate B19-1** (8.6 g, 29.9 mmol) and TEA (9.09 g, 90 mmol) in DCM (60 mL) was stirred at 0 °C for 2 hours. The mixture was quenched with water and extracted with EtOAc, and the organic phase was washed with saturated saline, dried with Na₂SO₄ and concentrated. **Intermediate B27-2** was obtained as yellow oil.

### Step 2: Preparation of intermediate B27-3

A reaction mixture of **Intermediate B27-2** (4.45g, 12.2 mmol), TMSCN (1.81 g, 18.3 mmol) and TBAF (18.3 mL, 18,3 mmol, 1 mol/L in THF) in THF (20 mL) was stirred at 25 °C for 16 hours. The mixture was quenched with water and extracted with EtOAc, and the organic phase was washed with saturated saline, dried with Na₂SO₄ and concentrated. Purification by flash chromatography gave **Intermediate B27-3** (2.7 g, 77.6%) as yellow oil. MS: 297.2 (M+H)⁺.

### Step 3: Preparation of intermediate B27-4

A reaction mixture of **Intermediate B27-3** (2.7 g, 9.11 mmol) and BH3.THF (50 mL, 1 mol/L in THF) in THF (10 mL) was stirred at 60 °C for 16 hours. The mixture was quenched with water and extracted with EtOAc, and the organic phase was washed with saturated saline, dried with Na₂SO₄ and concentrated. Purification by flash chromatography gave **Intermediate B27-4** (2.74 g, 100%) as yellow oil. MS: 300.2 (M+H)⁺.

### Step 4: Preparation of intermediate B27-5

A reaction mixture of **Intermediate B27-4** (2.74 g, 9.11 mmol), acetoxyacetyl chloride (1.87 g, 13.67 mmol) and TEA (2.77 g, 27.3 mmol) in DCM (25 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B27-5** (2.3 g, 63%) as yellow oil. MS: 401.2 (M+H)⁺. ¹H NMR (400 MHz, chloroform-*d*) δ 7.32 - 7.27 (m, 2H), 6.97 (s, 1H), 6.94 - 6.89 (m, 2H), 5.78 (s, 1H), 4.58 (d, *J* = 2.3 Hz, 2H), 4.30 (d, *J=* 5.0 Hz, 1H), 3.85 (s, 3H), 3.73 - 3.54 (m, 6H), 3.07 - 2.99 (m, 1H), *J* = 12.0, 7.0 Hz, 1H), 2.17 (s, 3H), 1.55 (d, *J* = 6.4 Hz, 3H).

### Step 5: Preparation of intermediate B27-6

A reaction mixture of **Intermediate B27-5** (590 mg, 1.47 mmol) and POCl₃ (5 mL) in anhydride (25 mL) was stirred at 25 °C for 3 hours. The mixture was quenched with water and extracted with EtOAc, and the organic phase was washed with saturated saline, dried with Na₂SO₄ and concentrated. Purification by flash chromatography gave **Intermediate B27-6** (334 mg, 59.3%) as yellow oil. MS: 383.2 (M+H)⁺.

### Step 6: Preparation of intermediate B27-7

A reaction mixture of **Intermediate B27-6** (334 mg, 8.84 mmol) and NaBH4 (49.8 mg, 1.3 mmol) (5 mL) in MeOH (5 mL) was stirred at 25 °C for 16 hours. The mixture was quenched with water and extracted with EtOAc, and the organic phase was washed with saturated saline, dried with Na₂SO₄ and concentrated. **Intermediate B27-7** was obtained and used directly in the next reaction.

### Step 7: Preparation of intermediate B27-8

A reaction mixture of **Intermediate B27-7** in HCl (15 mL, 3 N) was stirred at 80 °C for 1 hour. The mixture was quenched with water and extracted with EtOAc, and the organic phase was washed with saturated saline, dried with Na₂SO₄ and concentrated. **Intermediate B27-8** was obtained and used directly in the next reaction. MS: 343.1 (M+H)⁺.

### Step 8: Preparation of intermediate B27

**Intermediate B27** was synthesized by following the protocols of **Intermediate B10.** MS: 323.1 (M+H)⁺.

### Preparation of intermediate B28

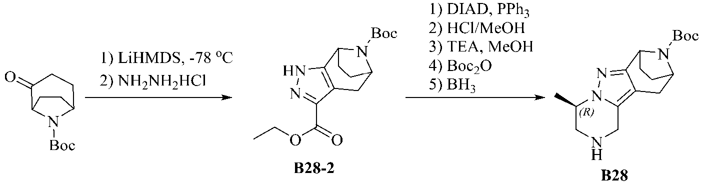

**Intermediate B28** was synthesized by following the protocols of **Intermediate B5.** MS: 319.2 (M+H)⁺.

### Preparation of intermediate B29

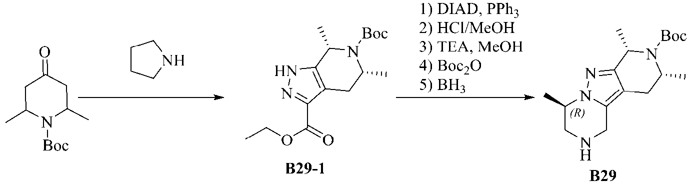

**Intermediate B29** was synthesized by following the protocols of **Intermediate B9.** MS: 321.2 (M+H)⁺.

### Preparation of intermediate B30

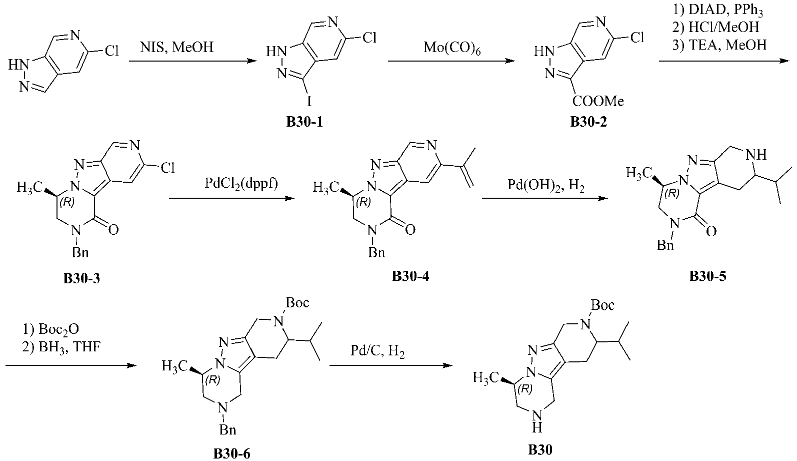

### Step 1: Preparation of intermediate B30-1

A reaction mixture of 5-chloro-1H-pyrazole [3, 4-c] pyridine (5 g, 32.6 mmol) and NIS (6.76 g, 39.1 mmol) in MeOH (80 mL) was stirred at 25 °C for 5 hours. The mixture was quenched with water and extracted with EtOAc, and the organic phase was washed with saturated saline, dried with Na₂SO₄ and concentrated. Purification by flash chromatography gave **Intermediate B30-1** (8 g, 88%) as yellow solid. MS: 280.1 (M+H)⁺.

### Step 2: Preparation of intermediate B30-2

A reaction mixture of **Intermediate B30-1** (8 g, 28.6 mmol), Mo(CO)₆ (7.56 g, 28.6 mmol), Pd(OAc)₂ (0.643 g, 2.86 mmol), Xantphos (3.31 g, 5.73 mmol) and TEA (5.79 g, 57.3 mmol) in MeOH (35 mL) and DMSO (35 mL) was stirred at 60 °C for 2 hours. The mixture was quenched with water and extracted with EtOAc, and the organic phase was washed with saturated saline, dried with Na₂SO₄ and concentrated. Purification by flash chromatography gave **Intermediate B30-2** (3.5 g, 57.8%) as yellow solid. MS: 212.1 (M+H)⁺.

### Step 3: Preparation of intermediate B30-3

**B30-3** was synthesized by following the protocols of **Intermediate B26-6.** MS: 327.1 (M+H)⁺.

### Step 4: Preparation of intermediate B30-4

A reaction mixture of **Intermediate B30-3** (600 mg, 1.836 mmol), potassium isopropenyltrifluoroborate (543 mg g, 3.67 mmol), PdCl₂(dppf) (134 mg, 0.184 mmol) and K₂CO₃ (508 mg, 3.67 mmol) in 1,4-dioxane (10 mL) and H₂O (2 mL) was stirred at 80 °C for 4 hours. The mixture was quenched with water and extracted with EtOAc, and the organic phase was washed with saturated saline, dried with Na₂SO₄ and concentrated. Purification by flash chromatography gave **Intermediate B30-4** (300 mg, 49.2%) as yellow solid. MS: 333.1 (M+H)⁺.

### Step 5: Preparation of intermediate B30-5

A reaction mixture of **Intermediate B30-4** (300 mg, 0.9 mmol) and Pd(OH)₂ (12.67 mg, 0.09 mmol) in HOAc (4 mL) was stirred at 60 °C for 12 hours. The reaction mixture is then cooled to room temperature, filtered and concentrated to get **Intermediate B30-5** (300 mg, 99%) as yellow oil. MS: 339.2 (M+H)⁺.

### Step 6: Preparation of intermediate B30-6

**Intermediate B30-6** was synthesized by following the protocols of Intermediate **B2.** MS: 425.2 (M+H)⁺.

### Step 7: Preparation of intermediate B30

**Intermediate B30** was synthesized by following the protocols of **Intermediate B8.** MS: 335.2 (M+H)⁺.

### Preparation of intermediate B31

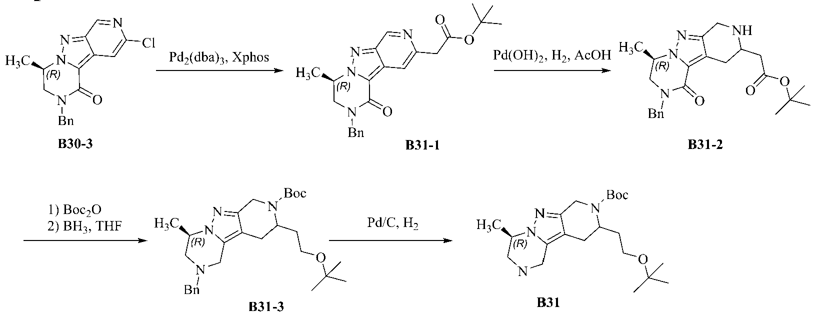

**Intermediate B31-1** was synthesized by following the protocols of **Intermediate B19-6.** MS: 407.2 (M+H)⁺.

**Intermediate B31** was synthesized by following the protocols of **Intermediate B30.** MS: 393.3 (M+H)⁺.

### Preparation of intermediate B32

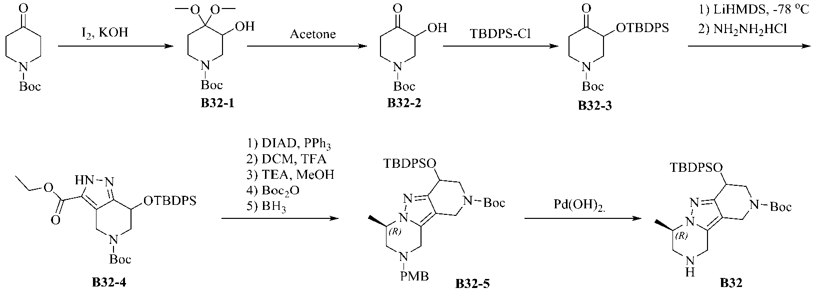

### Step 1: Preparation of intermediate B32-1

A reaction mixture of **Intermediate B32-1(7.2** g, 27.6 nmol), KOH (10.56 g, 188 mmol) and I₂ (22.93 g, 90 mmol) in MeOH (200 mL) was stirred at 25 °C for 3 hours. The mixture was quenched with water and extracted with EtOAc, and the organic phase was washed with saturated saline, dried with Na₂SO₄ and concentrated. Purification by flash chromatography gave **Intermediate B32-1** (9.8 g, 50%) as colorless oil. MS: 262.1 (M+H)⁺.

### Step 2: Preparation of intermediate B32-2

A reaction mixture of **Intermediate B32-1**(7.2 g, 27.6 nmol), acetone (0.474 g, 2.76% nmol) and methamphetamine (1.5 mL) in DCM (50 mL) was stirred at 25 °C for 48 hours. The mixture was quenched with water and extracted with EtOAc, and the organic phase was washed with saturated saline, dried with Na₂SO₄ and concentrated to give **Intermediate B32-2** (9.8 g, 50%) as colorless oil. MS: 160.1 (M-56)⁺.

### Step 3: Preparation of intermediate B32-3

**Intermediate B32-3** was synthesized by following the protocols of **Intermediate B16-2.** MS: 454.1 (M+H)⁺.

### Step 4: Preparation of intermediate B32-5

**Intermediate B32-5** was synthesized by following the protocols of **Intermediate B16.** MS: 667.1 (M+H)⁺.

### Step 5: Preparation of intermediate B32

**Intermediate B32** was synthesized by following the protocols of **Intermediate B27.** MS: 547.1 (M+H)⁺.

### Preparation of intermediate B33

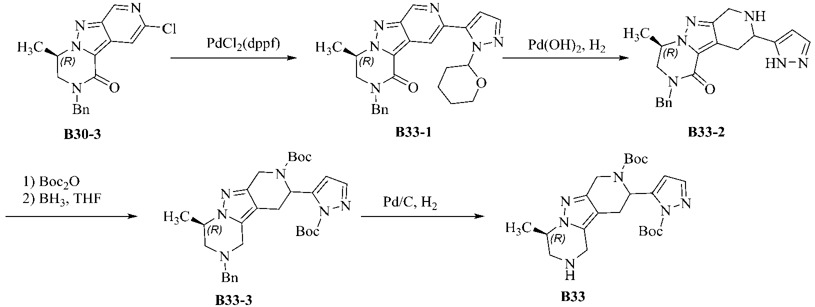

**Intermediate B33** was synthesized by following the protocols of **Intermediate B30.** MS: 459.1 (M+H)⁺.

### Preparation of Intermediate B34

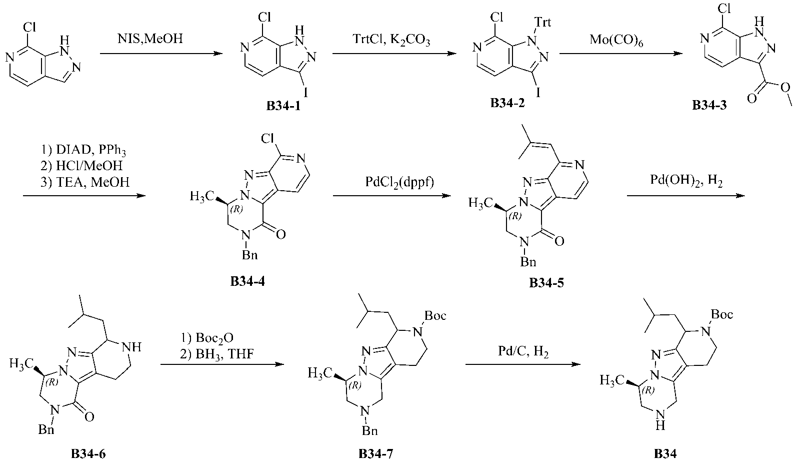

### Step 1: Preparation of intermediate B34-1

**B34-1** was synthesized by following the protocols of **Intermediate B30-1.** MS: 280.2 (M+H)⁺.

### Step 2: Preparation of intermediate B34-2

A reaction mixture of TrtCl (5.71 g, 20.47 mmol), **Intermediate B34-1** (5.2 g, 18.61 mmol) and K₂CO₃ (6.43 g, 46.5 mmol) in DMF (100 mL) was stirred at 25 °C for 12 hours. The mixture was quenched with water and extracted with EtOAc, and the organic phase was washed with saturated saline, dried with Na₂SO₄ and concentrated. Purification by flash chromatography gave **Intermediate B34-2** (8.2 g, 84%) as yellow solid. MS: 522.4 (M+H)⁺.

### Step 3: Preparation of intermediate B34

**Intermediate B34** was synthesized by following the protocols of **Intermediate B30.** MS: 349.1 (M+H)⁺.

### Preparation of Intermediate B35

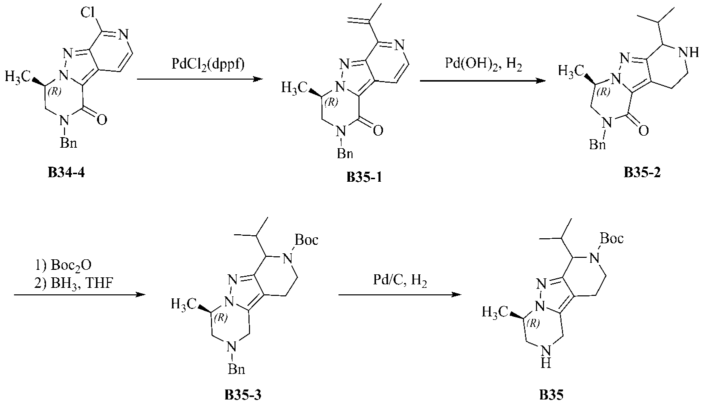

**Intermediate B35** was synthesized by following the protocols of **Intermediate B30.** MS: 335.1 (M+H)⁺.

### Preparation of intermediate B36

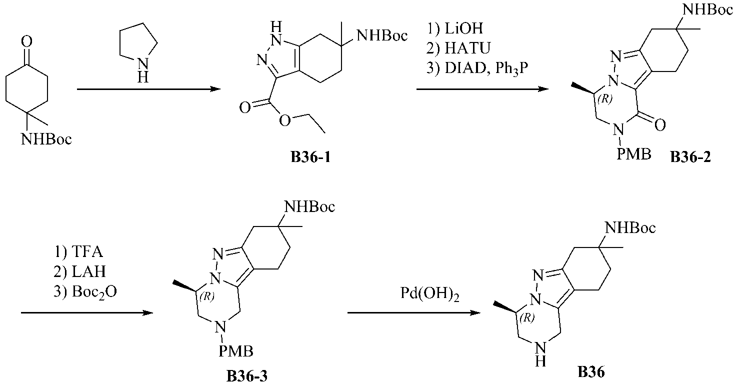

**Intermediate B36** was synthesized by following the protocols of **Intermediate B11.** MS: 321.1 (M+H)⁺.

### Preparation of intermediate B37

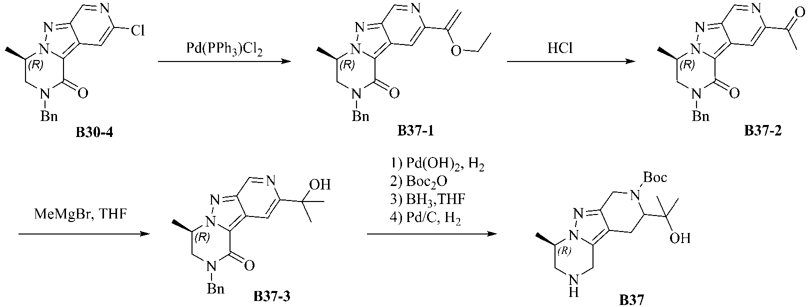

### Step 1: Preparation of intermediate B37-1

**Intermediate B37-1** was synthesized by following the protocols of **Intermediate B30-4.** MS: 363.2 (M+H)⁺.

### Step 2: Preparation of intermediate B37-2

A reaction mixture of **Intermediate B37-1** (300 mg, 0.828 mmol) and con. HCl (2 mL in MeOH (2 mL) was stirred at 25 °C for 2 hours. The reaction mixture is then concentrated under vacuum. **Intermediate B37-2** (0.2 g, 72.3%) was obtained as yellow oil and used directly in the next reaction. MS: 335.2 (M+H)⁺. ¹H NMR (400 MHz, CDCl₃) δ 9.37 (d, *J* = 1.4 Hz, 1H), 8.94 (d, *J=* 1.4 Hz, 1H), 7.45 - 7.36 (m, 5H), 4.96 - 4.81 (m, 3H), 3.86 (dd, *J=* 13.2, 4.6 Hz, 1H), 3.59 (dd, *J =* 13.3, 7.7 Hz, 1H), 2.84 (s, 3H), 1.71 (d, *J =* 6.6 Hz, 3H).

### Step 3: Preparation of intermediate B37-3

A reaction mixture of MeMgBr (1.5 mL g, 1.5 mmol, 1 mol/L in THF) and **Intermediate B37-2** (0.35 g, 1.04 mmol) in THF (5 mL) was stirred at 0 °C for 2 hours. The mixture was quenched with water and extracted with EtOAc, and the organic phase was washed with saturated saline, dried with Na₂SO₄ and concentrated. Purification by flash chromatography gave **Intermediate B37-3** (0.14 g, 38.2%) as yellow solid. MS: 351.2 (M+H)⁺. ¹H NMR (400 MHz, CDCl₃) δ 9.29 (d, *J* = 1.5 Hz, 1H), 8.13 (d, *J* = 1.5 Hz, 1H), 7.42 (d, *J=* 3.7 Hz, 5H), 4.95 - 4.80 (m, 3H), 3.86 (dd, *J=* 13.2, 4.6 Hz, 1H), 3.57 (dd, *J* = 13.2, 7.6 Hz, 1H), 1.71-1.68 (m, 9H).

### Step 4: Preparation of intermediate B37

**Intermediate B37** was synthesized by following the protocols of **Intermediate B30.** MS: 351.2 (M+H)⁺.

### Preparation of intermediate B38

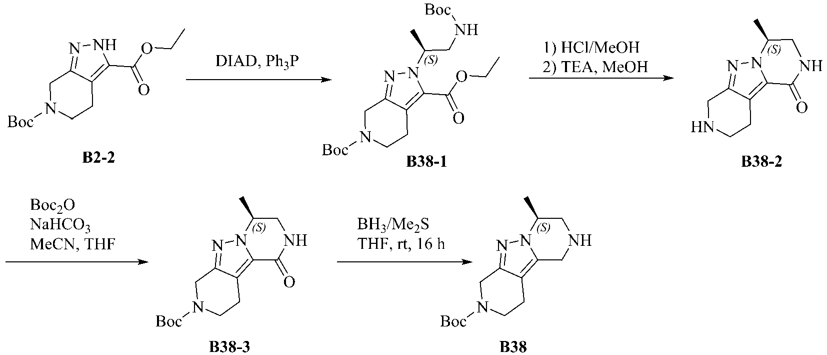

**Intermediate B38** was synthesized by following the protocols of **Intermediate B2.** MS: 293.2 (M+H)⁺.

### Preparation of intermediate B39

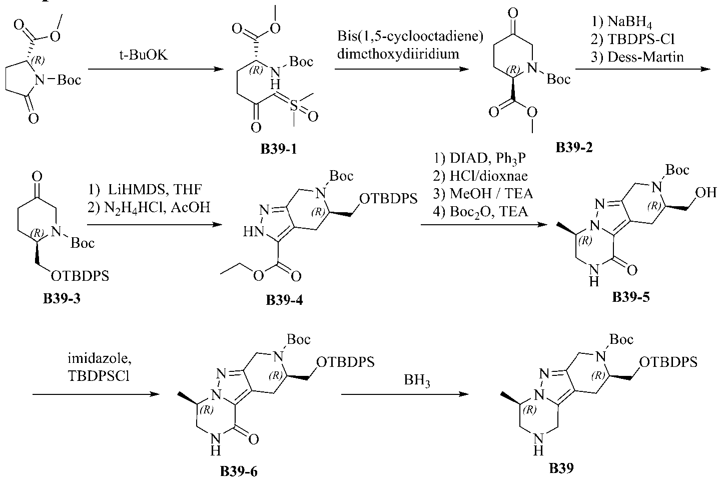

**Intermediate B39** was synthesized by following the protocols of **Intermediate B24.** MS: 561.2 (M+H)⁺. ¹H NMR (400 MHz, DMSO-*d*₆) δ 7.64 - 7.55 (m, 4H), 7.54 - 7.40 (m, 6H), 4.83 - 4.58 (m, 2H), 4.00 - 3.85 (m, 1H), 3.80 - 3.68 (m, 2H), 3.60 - 3.50 (m, 2H), 3.20 - 3.12 (m, 1H), 2.80 - 2.69 (m, 2H), 2.63 - 2.56 (m, 1H), 2.50 - 2.34 (m, 1H), 0.99 (s, 9H).

### Preparation of intermediate B40

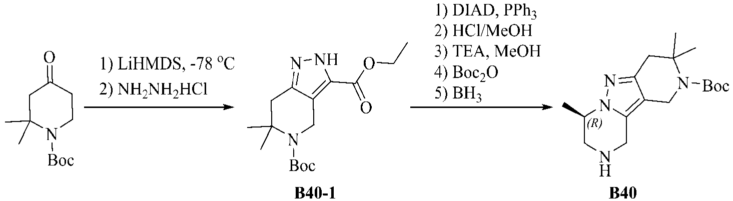

**Intermediate B40** was synthesized by following the protocols of **Intermediate B5.** MS: 321.1 (M+H)⁺.

### Preparation of intermediate B41

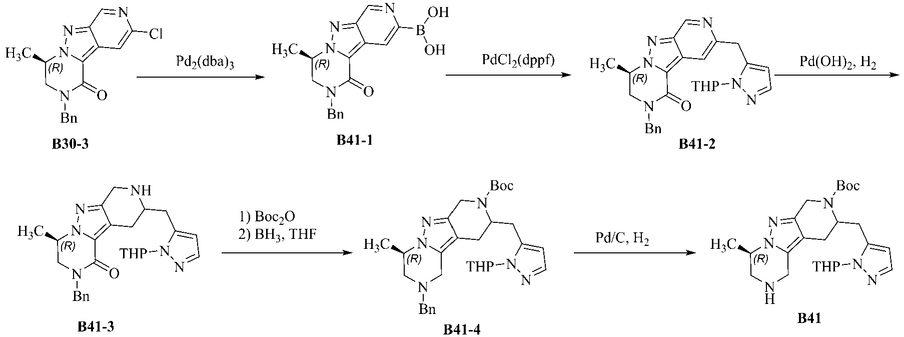

### Step 1: Preparation of intermediate B41-1

A reaction mixture of **Intermediate B30-3** (100 mg, 0.269 mmol), B₂Pin₂ (117 mg, 0.46 mmol), Pd₂(dba)₃(24.67 mg, 0.027 mmol)), tricyclic acid (15.11 mg, 0.054 mmol) and KOAc (52.9 mg, 0.539 mmol) in 1,4-dioxane (5 mL) was stirred at 0 °C for 2 hours. The mixture was quenched with water and extracted with EtOAc, and the organic phase was washed with saturated saline, dried with Na₂SO₄ and concentrated. Purification by flash chromatography gave **Intermediate B41-1** (60 mg, 66.3%) as yellow solid. MS: 337.1 (M+H)⁺.

### Step 2: Preparation of intermediate B41

**Intermediate B41** was synthesized by following the protocols of **Intermediate B30.** MS: 457.1 (M+H)⁺.

### Preparation of intermediate B42

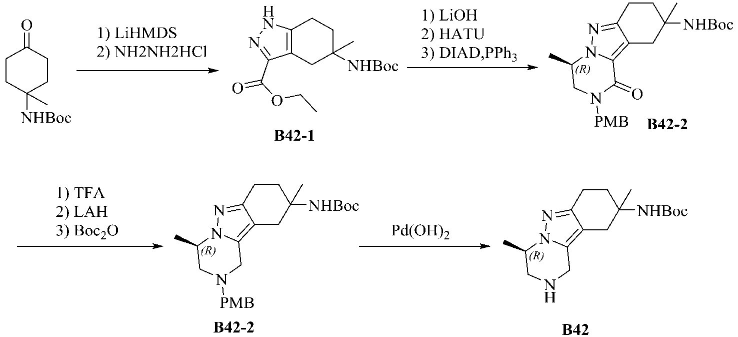

**Intermediate B42** was synthesized by following the protocols of **Intermediate B10**. MS: 321.1 (M+H)⁺.

### Preparation of intermediate B43

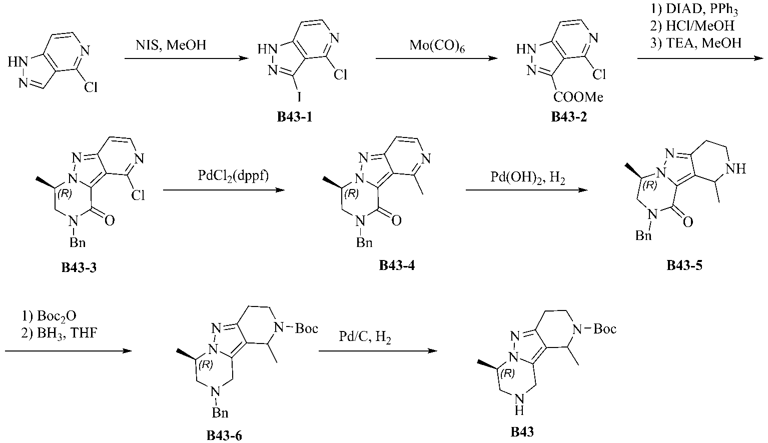

**Intermediate B43** was synthesized by following the protocols of **Intermediate B34.** MS: 307.2 (M+H)⁺.

### Preparation of intermediate B44

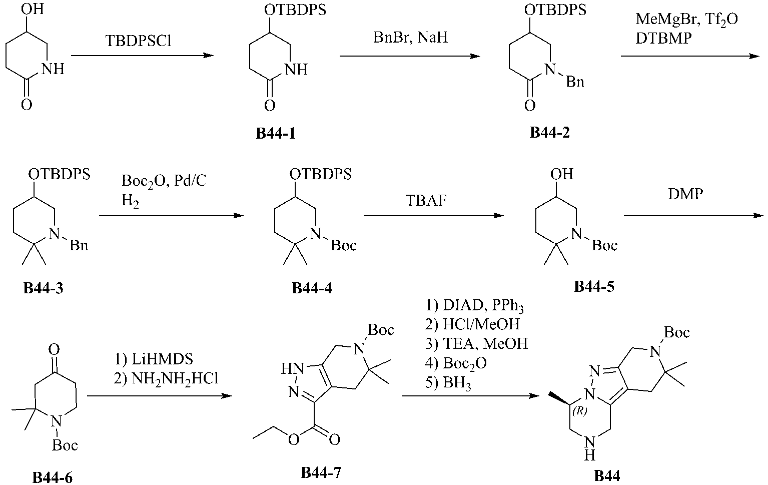

### Step 1: Preparation of intermediate B44-1

**Intermediate B44-1** was synthesized by following the protocols of **Intermediate B14-4.** MS: 354.1 (M+H)⁺.

### Step 2: Preparation of intermediate B44-2

To a reaction mixture of **Intermediate B44-1**(13.9 g, 39.3 mmol) in DMF (100 mL) was added NaH (1.88 g, 47 mmol) and the mixture was stirred at 20 °C for 30 minutes before BnBr (10.09 g, 59.0 mmol) was added. The reaction mixture was stirred for 16 hours before quenched with water and extracted with EtOAc, and the organic phase was washed with saturated saline, dried with Na₂SO₄ and concentrated. Purification by flash chromatography gave **Intermediate B44-2** (15.3 g, 88%) as yellow oil. MS: 444.2 (M+H)⁺. ¹H NMR (400 MHz, chloroform-*d*) δ 7.66 - 7.61 m, 2H, 7.56 - 7.52 m, 2H, 7.50 - 7.43 m, 2H, 7.40 dd, *J* = 7.9, 6.6 Hz, 2H, 7.35 t, *J* = 7.4 Hz, 2H, 7.30 dd, *J* = 4.8, 2.3 Hz, 3H, 7.24 dd, *J =* 6.9, 2.8 Hz, 2H, 4.76 d, *J =* 14.7 Hz, 1H, 4.35 d, *J =* 14.7 Hz, 1H, 4.13-4.09 m, 1H), 3.19 - 3.07 (m, 2H), 2.91-2.83 (m, 1H), 2.48 - 2.39 (m, 1H), 1.96 - 1.78 (m, 2H), 1.06 (s, 9H).

### Step 3: Preparation of intermediate B44-3

A reaction mixture of **Intermediate B44-2** (8.5 g, 19.16 mmol), Tf₂O (8.11 g, 28.7 mmol, and 2, 6-dibutyl-4-methylpyridine (5.90 g, 28.7 mmol) in DCM (100 mL) was stirred at -70 °C for 0.5 hours. MeMgBr solution (134mmol) was added into the reaction mixture at -70 °C and the reaction was stirred at 25 °C for 16 hours. The mixture was quenched with NH₄Cl solution and extracted with EtOAc, and the organic phase was washed with saturated saline, dried with Na₂SO₄ and concentrated. Purification by flash chromatography gave **Intermediate B44-3** (6.95 g, 79.3%) as yellow oil. MS: 458.2 (M+H)⁺. ¹H NMR (400 MHz, chloroform-*d*) δ 7.64-7.62 (m, 2H), 7.55 - 7.51 (m, 2H), 7.47 - 7.31 (m, 5H), 7.28 (d, *J =* 1.5 Hz, 5H), 7.24 (s, 1H), 3.82 (d, *J* = 14.0 Hz, 1H), 3.72-3.65 (m, 1H), 3.15 (d, *J=* 14.0 Hz, 1H), 2.62-2.58 (m, 1H), *J=* 11.4, 9.1 Hz, 1H), 1.74 - 1.67 (m, 1H),1.61 - 1.48 (m, 2H), 1.41 (dd, *J* = 13.0, 4.1 Hz, 1H), 1.14 (s, 3H), 1.10 (s, 3H), 1.04 (d, *J* = 1.3 Hz, 9H).

### Step 4: Preparation of intermediate B44-4

A reaction mixture of **Intermediate B44-3** (13.5 g, 29.5 mmol) and Pd/C (4 g) in EtOH (100 mL) was stirred at 50 °C for 22 hours under H₂. Then the solid were filtered out. TEA (8mL) and Boc₂O (9.64 g, 44.25 mmol) was added to the mixture. The resulting mixture was stirred at 65 °C for 16 hours. The mixture was quenched with water and extracted with EtOAc, and the organic phase was washed with saturated saline, dried with Na₂SO₄ and concentrated. Purification by flash chromatography gave **Intermediate B44-4** (11.9 g, 86%) as colorless oil. MS: 468.3 (M+H)⁺. ¹H NMR (400 MHz, chloroform-*d*) δ 7.74 - 7.70 (m, 4H), 7.50 - 7.39 (m, 6H), 3.94-3.88 (m, 1H), 3.67 (dd, *J=* 13.4, 4.5 Hz, 1H), 3.27 (dd, *J* = 13.5, 7.5 Hz, 1H), 1.80 - 1.59 (m, 4H), 1.46 (s, 9H), 1.44 (s, 3H), 1.40 (s, 3H), 1.11 (s, 9H).

### Step 5: Preparation of intermediate B44-5

A reaction mixture of **Intermediate B44-4** (11.9 g, 25.4 mmol) and TBAF (50.9 mL, 1 mol/L in THF) in THF (25 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B44-5** (4.4 g, 75 %) as white solid. MS: 230.2 (M+H)⁺.

### Step 6: Preparation of intermediate B44-6

**Intermediate B44-6** was synthesized by following the protocols of **Intermediate B16-3.** MS: 228.1(M+H)⁺.

### Step 7: Preparation of intermediate B44

**Intermediate B44** was synthesized by following the protocols of **Intermediate B5.** MS: 321.2 (M+H)⁺.

### Preparation of intermediate B45

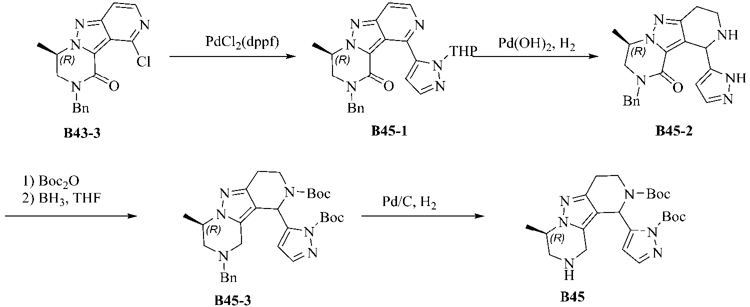

**Intermediate B45** was synthesized by following the protocols of **Intermediate B33.** MS: 459.2 (M+H)⁺.

### Preparation of intermediate B46

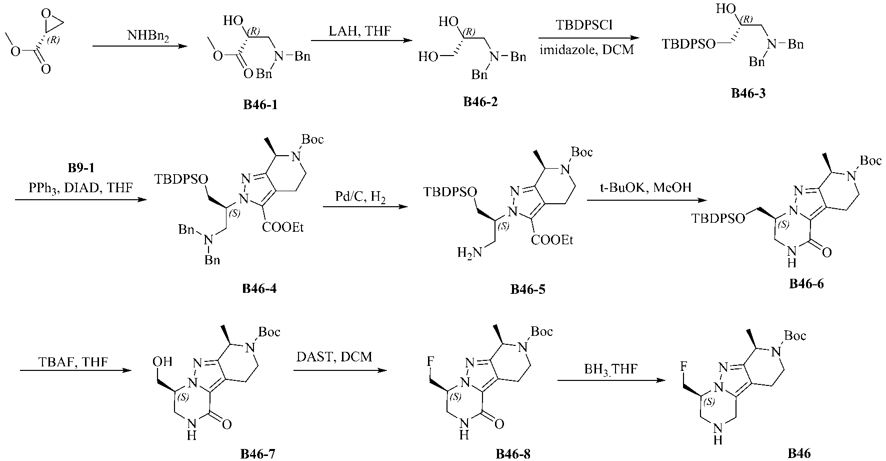

### Step 1: Preparation of intermediate B46-1

A reaction mixture of methyl (R)-oxirane-2-carboxylate (5.0 g, 49 mmol) and dibenzylamine (10.63 g, 53.9 mmol) was stirred at 85 °C for 12 hours. The mixture was quenched with icy water and extracted with EtOAc, and the organic phase was washed with saturated saline, dried with Na₂SO₄ and concentrated. Purification by flash chromatography gave **Intermediate B46-1(11.3** g, 77%) as yellow oil. MS: 300.3 (M+H)⁺.

### Step 2: Preparation of intermediate B46-2

**Intermediate B46-2** was synthesized by following the protocols of Intermediate **B1-6.** MS: 272.2 (M+H)⁺.

### Step 3: Preparation of intermediate B46-3

**Intermediate B46-3** was synthesized by following the protocols of **Intermediate B14-4.** MS: 510.2 (M+H)⁺.

### Step 4: Preparation of intermediate B46-4

**Intermediate B46-4** was synthesized by following the protocols of Intermediate **B1-3.** MS: 801.4 (M+H)⁺.

### Step 5: Preparation of intermediate B46-5

**Intermediate B46-5** was synthesized by following the protocols of **Intermediate B1.** MS: 621.7 (M+H)⁺.

### Step 6: Preparation of intermediate B46-6

A reaction mixture of **Intermediate B46-5** (830 mg, 1.337 mmol) and t-BuOK (450 mg, 4.01 mmol) in MeOH (5 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B46-6** (30 mg, 63.3%) as white solid. MS: 575.3 (M+H)⁺.

### Step 7: Preparation of intermediate B46-7

**Intermediate B46-7** was synthesized by following the protocols of **Intermediate B44-5.** MS: 337.2 (M+H)⁺.

### Step 8: Preparation of intermediate B46-8

A reaction mixture of **Intermediate B46-7** (220 mg, 0.654 mmol) and DAST (158 mg, 0.981 mmol) in DCM (5 mL) was stirred at 0 °C for 2 hours. The mixture was quenched with water and extracted with EtOAc, and the organic phase was washed with saturated saline, dried with Na₂SO₄ and concentrated. Purification by flash chromatography gave **Intermediate B46-8** (55 mg, 24.8%) as white solid. MS: 339.2 (M+H)⁺.

### Step 9: Preparation of intermediate B46

**Intermediate B46** was synthesized by following the protocols of Intermediate **B2.** MS: 325.2 (M+H)⁺.

### Preparation of intermediate B47

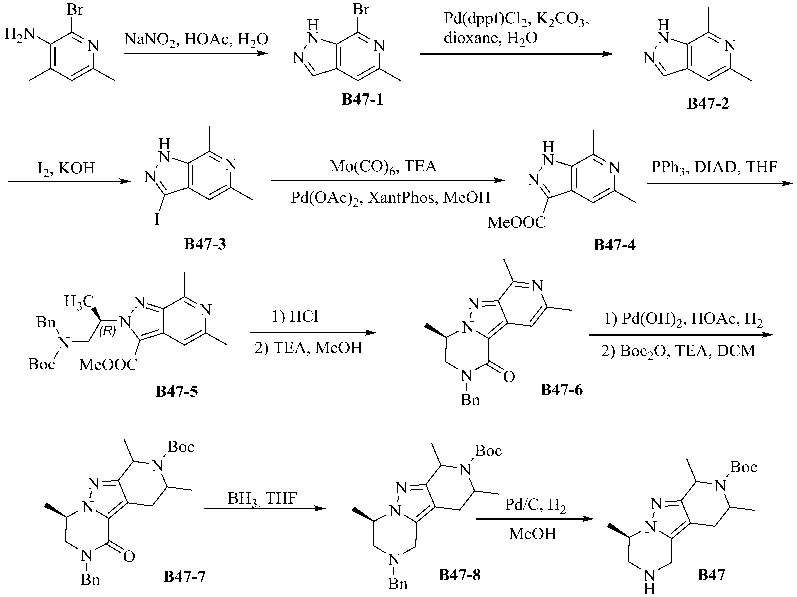

### Step 1: Preparation of intermediate B47-1

A reaction mixture of 2-bromine-4,6-dimethylpyridine-3-amine (10 g, 49.7 mmol) and sodium nitrite (5.15 g, 74.6 mmol) in H₂O (50 mL) was stirred at 25 °C for 12 hours. The mixture was quenched with water and extracted with EtOAc, and the organic phase was washed with saturated saline, dried with Na₂SO₄ and concentrated. Purification by flash chromatography gave **Intermediate B47-1(4** g, 37.9%) as yellow solid. MS: 212.0&214.0 (M+H)⁺.

### Step 2: Preparation of intermediate B47-2

**Intermediate B47-2** was synthesized by following the protocols of **Intermediate B30-4.** MS: 148.2 (M+H)⁺.

### Step 3: Preparation of intermediate B47-4

**Intermediate B47-4** was synthesized by following the protocols of **Intermediate B30-2.** MS: 206.2 (M+H)⁺.

### Step 4: Preparation of intermediate B47

**Intermediate B47** was synthesized by following the protocols of **Intermediate B30.** MS: 321.2 (M+H)⁺.

### Preparation of intermediate B48

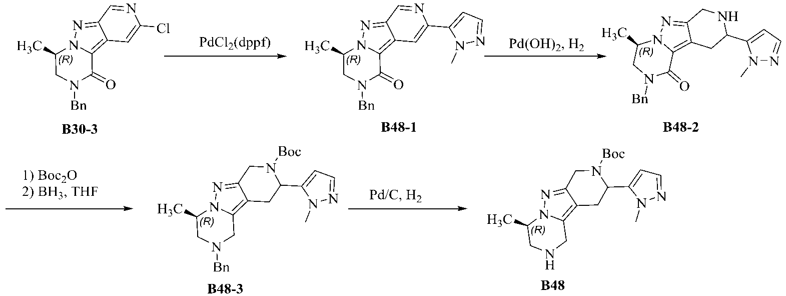

**Intermediate B48** was synthesized by following the protocols of **Intermediate B33,** MS: 373.1 (M+H)⁺.

### Preparation of intermediate B49

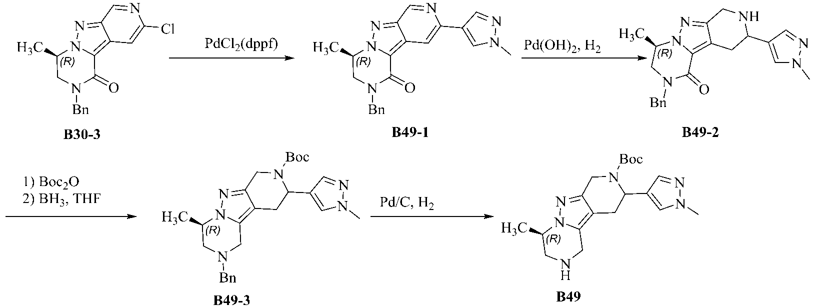

**Intermediate B49** was synthesized by following the protocols of **Intermediate B33.** MS: 373.1 (M+H)⁺.

### Preparation of intermediate B50

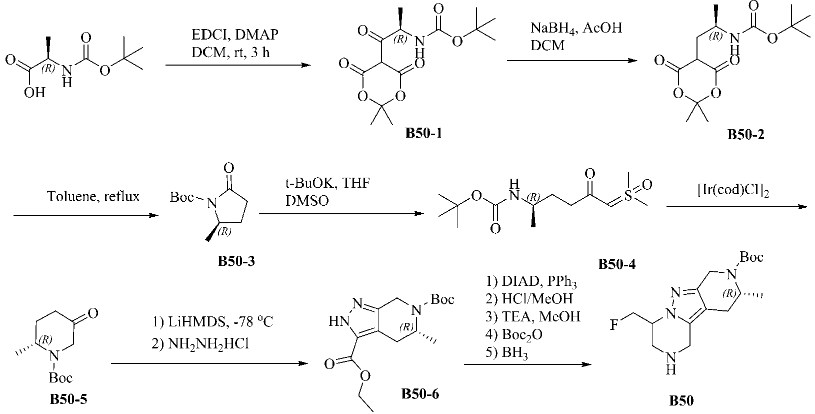

### Step 1: Preparation of intermediate B50-1

A reaction mixture of (*tert*-butoxycarbonyl)-D-alanine (10g, 52.9 mmol), 2,2-dimethyl-1,3-dioxane-4,6-dione (8.38 g, 58.1 mmol), EDCI (12.16 g, 63.4 mmol) and DMAP (9.69 g, 79 mmol) in DCM (500 mL) was stirred at 25 °C for 2 hours. The mixture was quenched with water and extracted with EtOAc, and the organic phase was washed with saturated saline, dried with Na₂SO₄ and concentrated. Purification by flash chromatography gave **Intermediate B50-1** (16.0 g, 96%) as yellow solid. MS: 316.1 (M+H)⁺.

### Step 2: Preparation of intermediate B50-2

A reaction mixture of **Intermediate B50-1**(16.7 g, 50 mmol), sodium borohydride (3.78 g, 100 mmol) and HOAc (20 mL) in DCM (200 mL) was stirred at 0 °C for 18 hours. The mixture was quenched with water and extracted with EtOAc, and the organic phase was washed with saturated saline, dried with Na₂SO₄ and concentrated to give **Intermediate B50-2** which was used directly in the next reaction.

### Step 3: Preparation of intermediate B50-3

A reaction mixture of **Intermediate B50-2** (14 g, 46.5 mmol) in toluene (500 mL) was stirred and refluxed at 100 °C for 3 hours. The mixture was quenched with water and extracted with EtOAc, and the organic phase was washed with saturated saline, dried with Na₂SO₄ and concentrated. Purification by flash chromatography gave **Intermediate B50-3** (7.87 g, 85.3%) as yellow oil.

### Step 4: Preparation of intermediate B50-5

**Intermediate B50-5** was synthesized by following the protocols of **Intermediate B24-2.** MS: 236.2 (M+Na)⁺. ¹H NMR (400 MHz, chloroform-*d*) δ 4.55 - 4.22 (m, 2H), 3.60 (d, *J =* 18.8 Hz, 1H), 2.46-2.43 (m, 2H), 2.28-2.21 (m, *J =* 14.0, 6.1 Hz, 1H), 1.68-1.58 (m, 1H), 1.49 (s, 9H), 1.26 (d, *J =* 6.4 Hz, 3H).

### Step 5: Preparation of intermediate B50

**Intermediate B50** was synthesized by following the protocols of **Intermediate B44.** MS: 325.2 (M+H)⁺.

### Preparation of intermediate B51

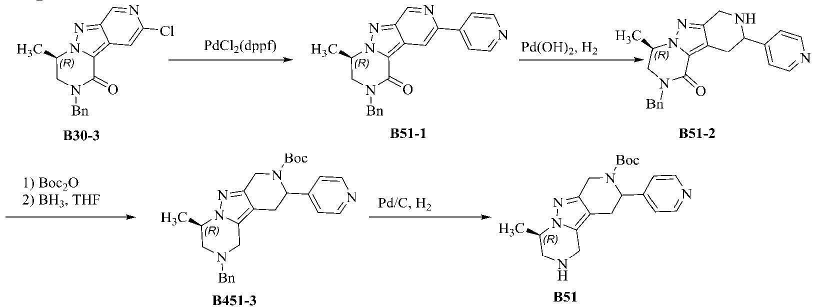

**Intermediate B51** was synthesized by following the protocols of **Intermediate B33.** MS: 370.1 (M+H)⁺.

### Preparation of intermediate B52

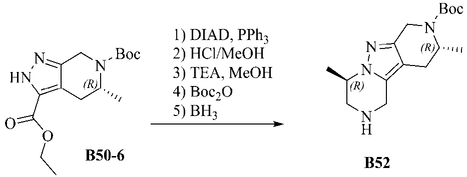

**Intermediate B52** was synthesized by following the protocols of **Intermediate B2.** MS: 307.1 (M+H)⁺.

### Preparation of intermediate B53

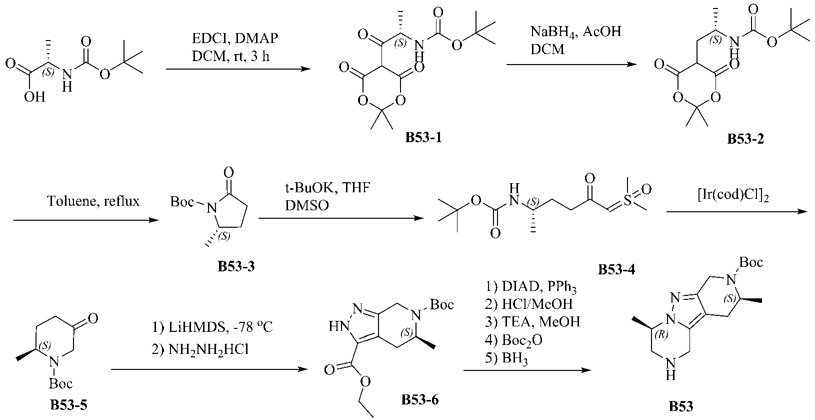

**Intermediate B53-6** was synthesized by following the protocols of **Intermediate B50-6.** MS: 310.1 (M+H)⁺.

**Intermediate B53** was synthesized by following the protocols of **Intermediate B2.** MS: 307.1 (M+H)⁺.

### Preparation of intermediate B54

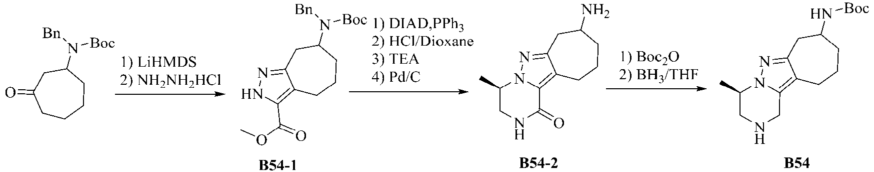

**Intermediate B54** was synthesized by following the protocols of **Intermediate B10.** MS: 321.1 (M+H)⁺.

**Examples** in this invention were synthesized according to procedures as demonstrated below.

### Example I-1

### (R)-4-(2-benzyl-7-methyl-1,3,4,7,8,10-hexahydropyrido[4',3':3,4]pyrazolo[1,5-a]pyrazin-9(2H)-yl)-3-fluoropyrazolo[1,5-a]pyridine-7-carbonitrile

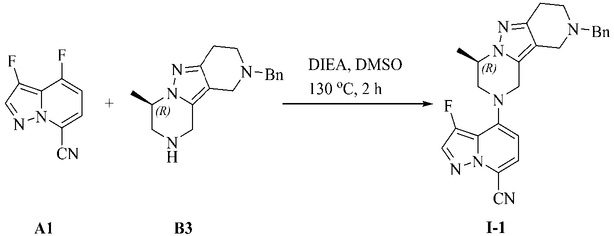

A reaction mixture of **Intermediate A1** (89 mg, 0.499 nmol), **Intermediate B3** (94 mg, 0.333 mmol) and DIEA (129 mg, 0.999 mmol) in DMF (1 mL) was stirred at 130 °C for 2 hours. The mixture was quenched with water and extracted with EtOAc, and the organic phase was washed with saturated saline, dried with Na₂SO₄ and concentrated. Purification by prep-HPLC gave **Example I-1** (101 mg, 68.5%) as yellow solid. MS: 442.1 (M+H)⁺. ¹H NMR (400 MHz, DMSO-*d*₆) δ 10.18 (s, 1H, TFA), 8.32 (s, 1H), 7.74 (t, *J =* 6.8 Hz, 1H), 7.58 - 7.53 (m, 2H), 7.52 - 7.49 (m, 3H), 6.71 - 6.64 (m, 1H), 4.53 (d, *J =* 10.5 Hz, 2H), 4.42 (d, *J* = 12.6 Hz, 3H), 4.12 (s, 2H), 4.00 (d, *J* = 13.5 Hz, 1H), 3.73 (s, 1H), 2.99 (d, *J* = 6.2 Hz, 2H), 1.47 (t, *J* = 6.6 Hz, 3H).

### Example I-2

### (R)-3-fluoro-4-(7-methyl-1,3,4,7,8,10-hexahydropyrido[4',3':3,4]pyrazolo[1,5-a]pyrazin-9(2H)-yl)pyrazolo[1,5-a]pyridine-7-carbonitrile

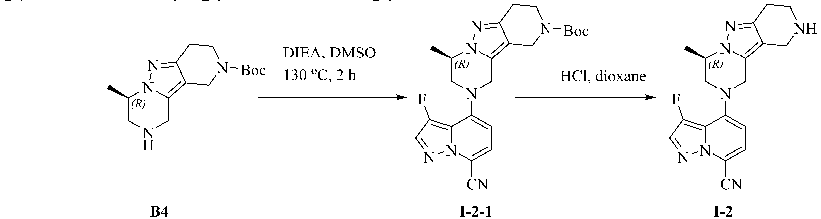

### Step 1: Preparation of intermediate I-2-1

**Intermediate I-2-1** was synthesized by following the protocols of **Example I-1.** MS: 452.1 (M+H)⁺.

### Step 2: Preparation of Example I-2

A reaction mixture of **Intermediate I-2-1** (15 mg, 0.033 mmol) in HCl (5 mL, 4M in dioxane) was stirred at 25 °C for 3 hours. The mixture was concentrated under vacuum. Purification by prep-HPLC gave **Example I-2** (2.75 mg, 23.5%) as yellow solid. MS: 352.2 (M+H)⁺. ¹H NMR (400 MHz, DMSO-*d*₆) δ 8.40 (s, 3H, TFA & NH), 8.29 (s, 1H), 7.71 (d, *J* = 7.8 Hz, 1H), 6.67 (d, *J* = 8.5 Hz, 1H), 4.36 (d, *J* = 15.9 Hz, 2H), 4.00-3.95 (m, 1H), 3.70 (d, *J =* 14.5 Hz, 3H), 2.96 (s, 2H), 2.56 (s, 2H), 2.03 (s, 1H), 1.46 (d, *J =* 6.2 Hz, 3H).

### Example I-3

### 3-Fluoro-4-(4-methyl-3,4,7,8,9,10-hexahydropyrido[4',3':3,4]pyrazolo[1,5-a]pyrazin-2(1H)-yl)pyrazolo[1,5-a]pyridine-7-carbonitrile

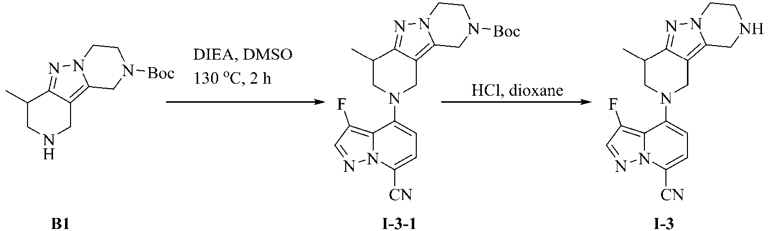

**Example I-3** was synthesized by following the protocols of **Example I-2**. MS: 352.1 (M+H)⁺. ¹H NMR (400 MHz, DMSO-*d₆*) δ 9.52 (s, 1H), 8.27 (d, *J* = 3.6 Hz, 1H), 7.69 (d, *J* = 8.4 Hz, 1H), 6.58 (d, *J* = 8.0 Hz, 1H), 4.50 - 4.17 (m, 6H), 3.94 - 3.86 (m, 1H), 3.67 *(t, J* = 6.0 Hz, 2H), 3.20 - 3.05 (m, 1H), 3.00 - 2.89 (m, 1H), 1.21 (d, *J* = 6.8 Hz, 3H).

### Example I-4

### (R)-4,4'-(7-methyl-1,3,4,7,8,10-hexahydropyrido[4',3':3,4]pyrazolo[1,5-a]pyrazine-2,9-diyl)bis(3-fluoropyrazolo[1,5-a]pyridine-7-carbonitrile)

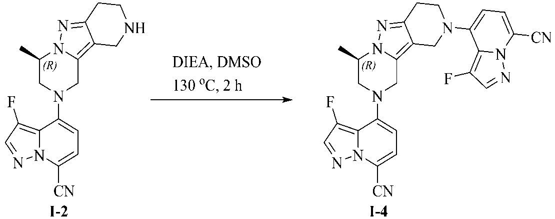

**Example I-4** was synthesized by following the protocols of **Example I-1.** MS: 511.1 (M+H)⁺. ¹H NMR (400 MHz, DMSO-*d₆*) δ 8.28 (d, J = 16.9 Hz, 2H), 7.71 (dd, J = 18.6, 7.9 Hz, 2H), 6.67 (d, J = 8.0 Hz, 1H), 6.59 (d, J = 8.2 Hz, 1H), 4.61 (d, J = 15.4 Hz, 1H), 4.46 (d, J = 15.6 Hz, 1H), 4.37 (d, J = 14.1 Hz, 2H), 4.26 (d, J = 14.2 Hz, 1H), 4.02 (d, J = 12.3 Hz, 1H), 3.70-3.60 (m, 2H), )3.38 - 3.35 (m, 1H), 2.86 (s, 2H), 1.49 (d, J = 6.4 Hz, 3H).

### Example I-5

### (R)-3-fluoro-4-(4-methyl-3,4,7,8,9,10-hexahydropyrido[3',4':3,4]pyrazolo[1,5-a] pyrazin-2(1H)-yl)pyrazolo[1,5-a]pyridine-7-carbonitrile

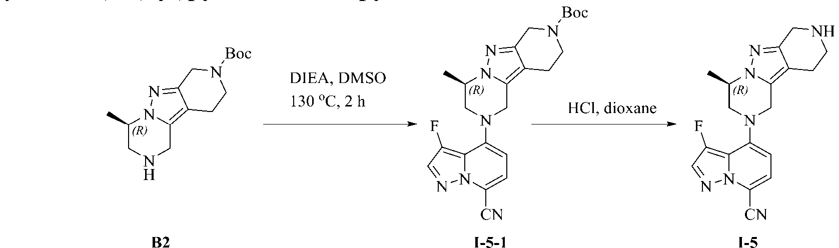

**Example I-5** was synthesized by following the protocols of **Example I-2**. MS: 352.1 (M+H)⁺. ¹H NMR (400 MHz, DMSO-*d₆*) δ 8.30 (d, *J* = 3.3 Hz, 1H), 7.73 (d, *J* = 7.9 Hz, 1H), 7.48 (d, *J* = 115.7 Hz, 3H), 6.71 (d, *J* = 8.0 Hz, 1H), 4.60 - 4.35 (m, 3H), 4.17 (s, 2H), 4.01 (d, *J* = 13.7 Hz, 1H), 3.43-3.35 (m, 1H), 3.27 (d, *J* = 6.3 Hz, 2H), 2.80 - 2.66 (m, 2H), 1.47 (d, *J* = 6.3 Hz, 3H).

### Example I-6

### (R)-2-(2-(7-cyano-3-fluoropyrazolo[1,5-a]pyridin-4-yl)-4-methyl-1,2,3,4,9,10-hexahydropyrido[3',4':3,4]pyrazolo[1,5-a]pyrazin-8(7H)-yl)-N,N-dimethylacetamide

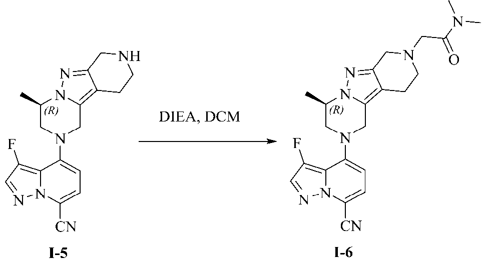

A reaction mixture of **Example I-5** (40 mg, 0.094 mmol), 2-bromo-N,N-dimethylacetamide (18.37 mg, 0.133 mmol) and TEA (28.7 mg, 0.283 mmol) in acetonitrile (3 mL) was stirred at 25 °C for 1 hour. The mixture was quenched with water and extracted with EtOAc, and the organic phase was washed with saturated saline, dried with Na₂SO₄ and concentrated. Purification by prep-HPLC gave **Example I-1** (101 mg, 68.5%) as white solid. MS: 437.1 (M+H)⁺.

### Example I-7

### (R)-2-(2-(7-cyano-3-fluoropyrazolo[1,5-a]pyridin-4-yl)-4-methyl-1,2,3,4,9,10-hexahydropyrido[3',4':3,4]pyrazolo[1,5-a]pyrazin-8(7H)-yl)acetamide

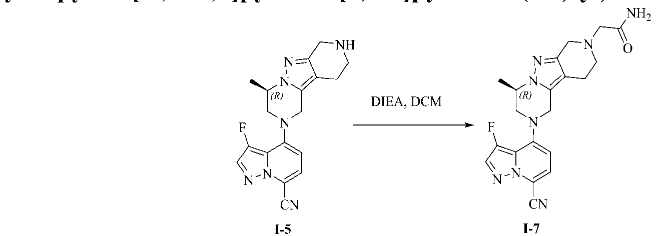

**Example I-7** was synthesized by following the protocols of **Example 1-6.** MS: 409.1 (M+H)⁺. ¹H NMR (400 MHz, DMSO-*d₆*) δ 8.28 (d, *J =* 3.2 Hz, 1H), 7.70 (d, *J =* 8.0 Hz, 1H), 7.15 (d, *J =* 27.2 Hz, 2H), 6.69 (d, *J =* 8.0 Hz, 1H), 4.48 (d, *J =* 15.6 Hz, 1H), 4.42 - 4.31 (m, 2H), 3.98 (d, *J =* 12.8 Hz, 1H), 3.57 - 3.47 (m, 2H), 3.05 (s, 2H), 2.68 (dq, *J =* 11.2, 5.6, 5.2 Hz, 2H), 2.53 (s, 2H), 1.44 (d, *J =* 6.4 Hz, 3H), 1.22 (s, 1H).

### Example I-8

### (R)-2-(9-(7-cyano-3-fluoropyrazolo[1,5-a]pyridin-4-yl)-7-methyl-3,4,7,8,9,10-hexahydropyrido[4',3':3,4]pyrazolo[1,5-a]pyrazin-2(1H)-yl)-N,N-dimethylacetamide

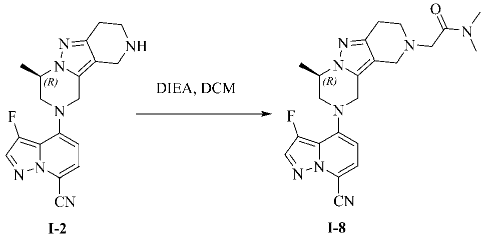

**Example I-8** was synthesized by following the protocols of **Example 1-6.** MS: 437.1 (M+H)⁺. ¹H NMR (400 MHz, DMSO-*d₆*) δ 8.29 (d, *J* = 3.6 Hz, 1H), 7.71 (d, *J* = 8.0 Hz, 1H), 6.69 (d, *J =* 8.0 Hz, 1H), 4.45 (d, *J =* 15.2 Hz, 1H), 4.37 (d, *J =* 15.2 Hz, 2H), 3.98 (d, *J = 13.6* Hz, 1H), 3.52 - 3.37 (m, 3H), 3.28 (s, 2H), 3.02 (s, 3H), 2.82 (s, 3H), 2.76 (s, 2H), 2.62 *(t, J* = 6.0 Hz, 2H), 1.46 (d, *J =* 6.4 Hz, 3H).

### Example I-9

### 4-((4R)-9-amino-4-methyl-3,4,7,8,9,10-hexahydropyrazino[1,2-b]indazol-2(1H)-yl)-3-fluoropyrazolo[1,5-a]pyridine-7-carbonitrile

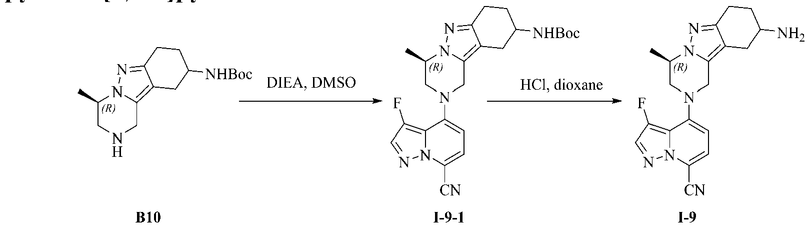

**Example I-9** was synthesized by following the protocols of **Example 1-5.** MS: 366.1 (M+H)⁺. ¹H NMR (400 MHz, DMSO-*d₆*) δ 8.29 (d, *J* = 3.4 Hz, 1H), 8.06 (s, 3H), 7.72 (d, *J* = 8.0 Hz, 1H), 6.79 - 6.65 (m, 1H), 4.48 (d, *J* = 15.4 Hz, 1H), 4.42 - 4.29 (m, 2H), 4.04 - 3.92 (m, 1H), 3.34 - 3.23 (m, 1H), 2.92 - 2.56 (m, 3H), 2.47 - 2.33 (m, 1H), 2.18 - 2.06 (m, 1H), 1.92 - 1.68 (m, 1H), 1.45 (d, *J =* 6.5 Hz, 3H).

### Example I-10

### (R)-2-(9-(7-cyano-3-fluoropyrazolo[1,5-a]pyridin-4-yl)-7-methyl-3,4,7,8,9,10-hexahydropyrido[4',3':3,4]pyrazolo[1,5-a]pyrazin-2(1H)-yl)acetamide

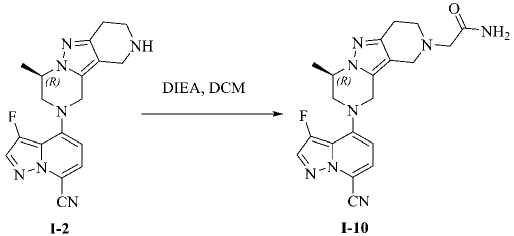

**Example I-10** was synthesized by following the protocols of **Example I-7.** MS: 409.1 (M+H)⁺. ¹H NMR (400 MHz, DMSO-*d₆*) δ 8.28 (d, *J* = 3.2 Hz, 1H), 7.70 (d, *J* = 8.0 Hz, 1H), 7.15 (d, *J =* 27.2 Hz, 2H), 6.69 (d, *J =* 8.0 Hz, 1H), 4.48 (d, *J =* 15.6 Hz, 1H), 4.42 - 4.31 (m, 2H), 3.98 (d, *J* = 12.8 Hz, 1H), 3.57 - 3.47 (m, 2H), 3.05 (s, 2H), 2.68 (dq, *J =* 11.2, 5.6, 5.2 Hz, 2H), 2.53 (s, 2H), 1.44 (d, *J =* 6.4 Hz, 3H), 1.22 (s, 1H).

### Example I-11

### 4-((7R)-3,7-dimethyl-1,3,4,7,8,10-hexahydropyrido[4',3':3,4]pyrazolo[1,5-a]pyrazin-9(2H)-yl)-3-fluoropyrazolo[1,5-a]pyridine-7-carbonitrile

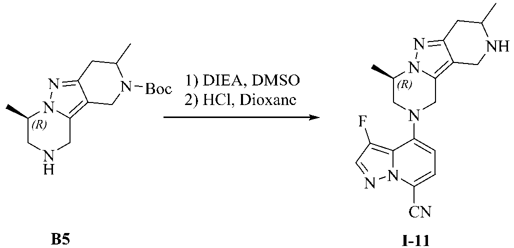

**Example I-11** was synthesized by following the protocols of **Example 1-2.** MS: 366.1 (M+H)⁺.¹H NMR (400 MHz, DMSO-*d₆*) δ 8.31 (d, *J* = 3.6 Hz, 1H), 7.75 (d, *J* = 8.0 Hz, 1H), 6.67 (d, *J =* 8.0 Hz, 1H), 4.69 - 4.33 (m, 3H), 4.25 - 3.89 (m, 3H), 3.42 (s, 3H), 2.91 (d, *J* = 16.4 Hz, 1H), 2.63 - 2.53 (m, 1H), 1.47 (d, *J* = 6.4 Hz, 3H), 1.35 (d, *J* = 6.4 Hz, 3H).

### Example I-11a and Example I-11b

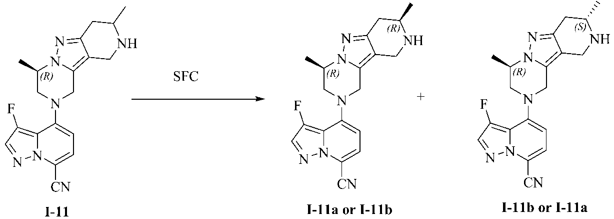

**Examples I-11a** and **I-11a** were separated through chiral resolution by SFC (column type: CHIRALPAK IE, 2cm × 25cm, 5µm chromatographic column, mobile phase A: Hex/DCM (0.5% 2mM NH₃-MeOH); B: IPA. **Example I-11a** was obtained as the first peak component, (white solid, retention time: 2.691 minute). While the second peak component was separated as **Example I-11b** (retention time: 3.307 minute). MS: 366.1 (M+H)⁺.

### Example I-12

### 4-((7R)-4,7-dimethyl-1,3,4,7,8,10-hexahydropyrido[4',3':3,4]pyrazolo[1,5-a]pyrazin-9(2H)-yl)-3-fluoropyrazolo[1,5-a]pyridine-7-carbonitrile

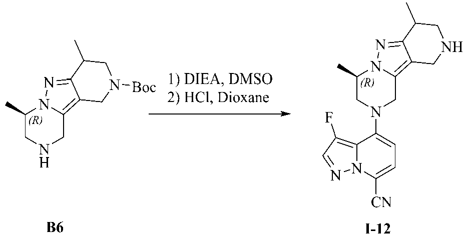

**Example I-12** was synthesized by following the protocols of **Example I-2**. MS: 366.1 (M+H)⁺. ¹H NMR (400 MHz, DMSO-*d₆*) δ 8.30 (d, *J =* 3.2 Hz, 1H), 7.74 (dd, *J =* 8.0, 2.8 Hz, 1H), 6.67 (dd, *J =* 8.0, 4.3 Hz, 1H), 4.48 (ddd, *J =* 53.6, 24.8, 15.2 Hz, 3H), 4.23 - 3.94 (m, 3H), 3.48 (d, *J =* 5.2 Hz, 1H), 3.45 (s, 1H), 3.16 - 3.06 (m, 2H), 2.83 (t, *J =* 11.2 Hz, 1H), 1.47 (t, *J =* 5.6 Hz, 3H), 1.24 (d, *J =* 7.2 Hz, 3H).

### Example I-13

### 2-((7R)-9-(7-cyano-3-fluoropyrazolo[1,5-a]pyridin-4-yl)-3,7-dimethyl-3,4,7,8,9,10-hexahydropyrido[4',3':3,4]pyrazolo[1,5-a]pyrazin-2(1H)-yl)-N,N-dimethylacetamide

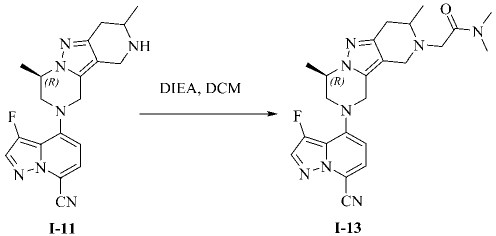

**Example I-13** was synthesized by following the protocols of **Example 1-6.** MS: 451.1 (M+H)⁺. ¹H NMR (400 MHz, DMSO-*d₆*) δ 8.29 (d, J = 3.6 Hz, 1H), 7.71 (d, J = 8.0 Hz, 1H), 6.70 (d, J = 8.0 Hz, 1H), 4.47 - 4.32 (m, 3H), 3.98 (d, J = 13.6 Hz, 1H), 3.57 (s, 1H), 3.42 (s, 2H), 3.29 (s, 2H), 3.18 (s, 1H), 3.02 (s, 3H), 2.81 (d, J = 2.4 Hz, 3H), 2.74 (d, J = 17.2 Hz, 1H), 2.35 (d, J = 16.4 Hz, 1H), 1.46 (dd, J = 6.4, 4.4 Hz, 3H), 1.01 (s, 3H).

### Example I-14

### 2-((7R)-9-(7-cyano-3-fluoropyrazolo[1,5-a]pyridin-4-yl)-4,7-dimethyl-3,4,7,8,9,10-hexahydropyrido[4',3':3,4]pyrazolo[1,5-a]pyrazin-2(1H)-yl)acetamide

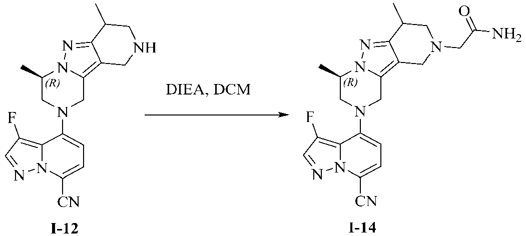

**Example I-14** was synthesized by following the protocols of **Example I-7.** MS: 423.1 (M+H)⁺. ¹H NMR (400 MHz, DMSO-*d₆*) δ 8.29 (d, *J* = 3.6 Hz, 1H), 7.71 (dd, *J* = 8.0, 2.0 Hz, 1H), 7.17 (d, *J* = 8.4 Hz, 2H), 6.73 - 6.64 (m, 1H), 4.52 - 4.28 (m, 3H), 3.99 (d, *J =* 18.0 Hz, 1H), 3.55 (dd, *J* = 23.6, 13.2 Hz, 1H), 3.28 (s, 2H), 3.04 (d, *J* = 2.8 Hz, 2H), 2.98 - 2.83 (m, 2H), 2.28 (d, *J* = 13.2 Hz, 1H), 1.46 (t, *J* = 6.4 Hz, 3H), 1.21 - 1.09 (m, 3H).

### Example I-15

### 2-((7R)-9-(7-cyano-3-fluoropyrazolo[1,5-a]pyridin-4-yl)-4,7-dimethyl-3,4,7,8,9,10-hexahydropyrido[4',3':3,4]pyrazolo[1,5-a]pyrazin-2(1H)-yl)-N,N-dimethylacetamide

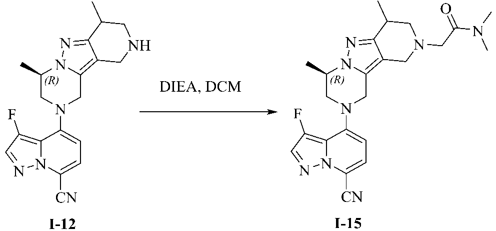

**Example I-15** was synthesized by following the protocols of **Example 1-6.** MS: 451.1 (M+H)⁺. ¹H NMR (400 MHz, DMSO-*d₆*) δ 8.29 (d, J = 3.6 Hz, 1H), 7.71 (dd, J = 8.0, 1.6 Hz, 1H), 6.69 (dd, J = 8.0, 5.2 Hz, 1H), 4.38 (td, J = 16.4, 15.2, 8.8 Hz, 3H), 3.97 (d, J *=* 13.6 Hz, 1H), 3.55 - 3.44 (m, 1H), 3.33 - 3.24 (m, 4H), 3.03 (d, J = 1.8 Hz, 3H), 2.90 - 2.83 (m, 2H), 2.83 - 2.80 (m, 3H), 2.32 (s, 1H), 1.46 (dd, J = 6.4, 4.4 Hz, 3H), 1.16 (dd, J = 6.4, 1.6 Hz, 3H).

### Example I-16

### 2-((7R)-9-(7-cyano-3-fluoropyrazolo[1,5-a]pyridin-4-yl)-3,7-dimethyl-3,4,7,8,9,10-hexahydropyrido[4',3':3,4]pyrazolo[1,5-a]pyrazin-2(1H)-yl)acetamide

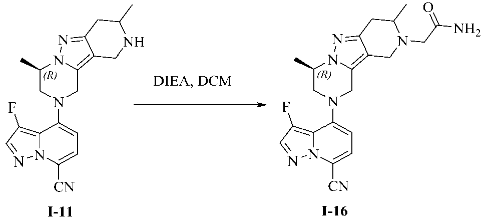

**Example I-16** was synthesized by following the protocols of **Example I-7.** MS: 423.1 (M+H)⁺. ¹H NMR (400 MHz, DMSO-*d₆*) δ 8.29 (d, *J* = 3.6 Hz, 1H), 7.71 (d, *J* = 8.0 Hz, 1H), 7.20 (d, *J =* 38.0 Hz, 2H), 6.68 (d, *J =* 8.0 Hz, 1H), 4.48 (d, *J =* 17.2 Hz, 1H), 4.37 (d, *J* = 15.2 Hz, 2H), 4.01 (s, 1H), 3.66 - 3.40 (m, 2H), 3.27 (s, 1H), 3.08 (d, *J =* 18.8 Hz, 2H), 2.94 (s, 1H), 2.77 (d, *J =* 15.6 Hz, 1H), 2.36 (d, *J =* 18.4 Hz, 1H), 1.47 (t, *J =* 5.6 Hz, 3H), 1.03 (d, *J =* 6.4 Hz, 3H).

### Example I-17

### 4-(4-Ethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

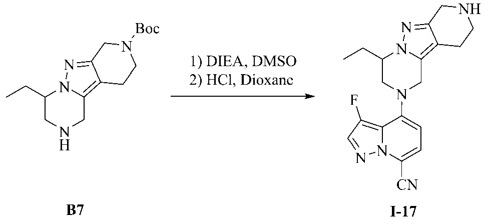

**Example I-17** was synthesized by following the protocols of **Example I-2.** MS: 366.1 (M+H)⁺. ¹H NMR (400 MHz, DMSO-*d₆*) δ 8.29 (d, J = 3.5 Hz, 1H), 8.24 (s, 1H), 7.72 (d, J = 7.9 Hz, 1H), 6.71 (d, J = 8.1 Hz, 1H), 4.49 (d, J = 15.5 Hz, 1H), 4.38 (d, J = 15.3 Hz, 1H), 4.21 (s, 1H), 4.10 - 4.01 (m, 1H), 3.96 (s, 2H), 3.39 - 3.25 (m, 1H), 3.18 - 2.99 (m, 2H), 2.66 - 2.52 (m, 2H), 2.31 - 2.15 (m, 1H), 1.70 (dt, J = 14.6, 7.6 Hz, 1H), 0.92 (t, J = 7.5 Hz, 3H).

### Example I-18

### 3-Fluoro-4-(4-(trifluoromethyl)-3,4,7,8,9,10-hexahydropyrido[3',4':3,4]pyrazolo[1,5-a]pyrazin-2(1H)-yl)pyrazolo[1,5-a]pyridine-7-carbonitrile

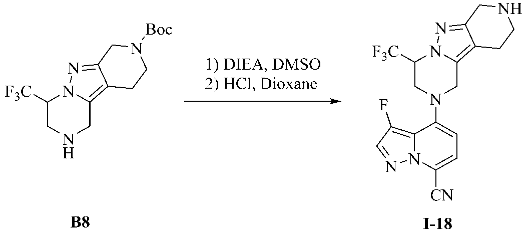

**Example I-18** was synthesized by following the protocols of **Example I-2**. MS: 406.1 (M+H)⁺. ¹H NMR (400 MHz, DMSO-*d₆*) δ 8.31 (d, *J* = 3.1 Hz, 1H), 8.23 - 8.17 (m, 1H), 7.80 - 7.64 (m, 1H), 6.79 (d, *J* = 8.0 Hz, 1H), 5.37 (d, *J* = 7.0 Hz, 1H), 4.65 (d, *J* = 15.9 Hz, 1H), 4.41 (d, *J* = 15.9 Hz, 1H), 4.08 - 3.88 (m, 4H), 3.18 - 2.99 (m, 2H), 2.55 (t, *J =* 6.4 Hz, 2H).

### Example I-19

### (R)-3-Fluoro-4-(8-(2-hydroxyethyl)-4-methyl-3,4,7,8,9,10-hexahydropyrido[3',4':3,4] pyrazolo[1,5-a]pyrazin-2(1H)-yl)pyrazolo[1,5-a]pyridine-7-carbonitrile

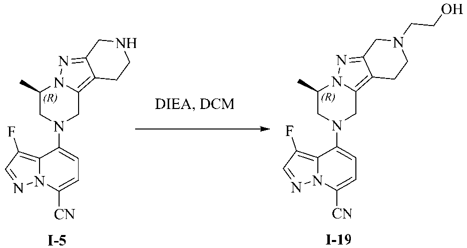

**Example I-19** was synthesized by following the protocols of **Example I-6.** MS: 396.1 (M+H)⁺. ¹H NMR (400 MHz, DMSO-*d₆*) δ 8.35 - 8.21 (m, 1H), 7.77 - 7.59 (m, 1H), 6.68 (d, *J =* 8.0 Hz, 1H), 4.52 - 4.39 (m, 2H), 4.37 - 4.28 (m, 1H), 4.05 - 3.94 (m, 1H), 3.60 - 3.49 (m, 4H), 3.31 (dd, *J =* 13.7, 9.2 Hz, 4H), 2.77 - 2.65 (m, 2H), 2.62 *(t, J* = 6.2 Hz, 2H), 1.44 (d, *J =* 6.4 Hz, 3H).

### Example I-20

### 4-((4R)-8-amino-4-methyl-3,4,7,8,9,10-hexahydropyrazino[1,2-b]indazol-2(1H)-yl)-3-fluoropyrazolo[1,5-a]pyridine-7-carbonitrile

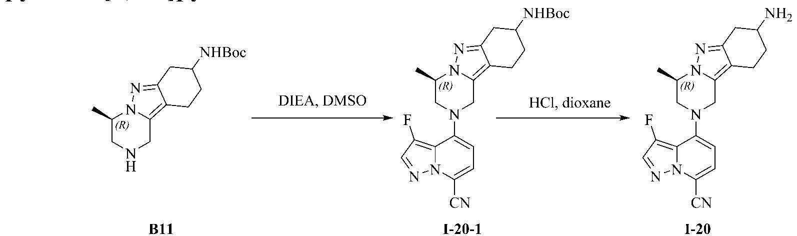

**Example I-20** was synthesized by following the protocols of **Example 1-9.** MS: 366.1 (M+H)⁺. ¹H NMR (400 MHz, DMSO-*d₆*) δ 8.36 - 8.22 (m, 1H), 7.73 (dd, J = 8.0, 1.5 Hz, 3H), 6.71 (d, J = 8.1 Hz, 1H), 4.54 - 4.30 (m, 3H), 4.05 - 3.94 (m, 1H), 3.36 - 3.28 (m, 2H), 2.98 (dt, J = 15.7, 4.6 Hz, 1H), 2.68 - 2.52 (m, 2H), 2.51 - 2.36 (m, 1H), 2.04 (s, 1H), 1.69 (s, 1H), 1.46 (d, J = 6.3 Hz, 3H).

### Examples I-20a and I-20b

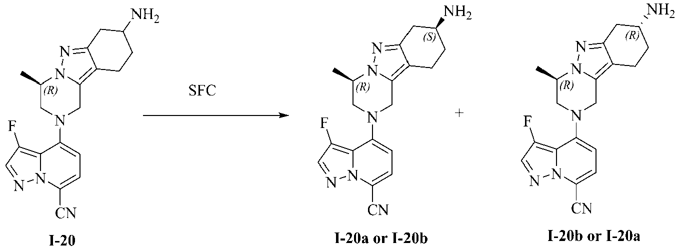

**Examples I-20a** and **I-20b** were separated through chiral resolution by SFC, mobile phase A: *tert*-butyl methyl ether, mobile phase B: EtOH (0.5% 2mM NH₃-MeOH). **Example I-20a** as the first peak component was obtained as white solid (retention time: 2.787 minute) and the second component was **Example I-20b** (retention time: 3.787 minute). MS: 366.1 (M+H)⁺.

### Example I-21

### 4-((4R,7R)-4,7-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

### 4-((4R,7R)-4,7-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

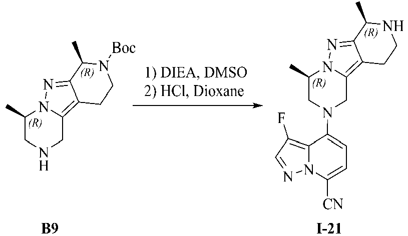

**Example I-21** was synthesized by following the protocols of **Example I-2.** MS: 366.1 (M+H)⁺. ¹H NMR (400 MHz, DMSO-*d₆*) δ 8.29 (d, *J* = 3.6 Hz, 1H), 7.72 (d, *J* = 8.0 Hz, 1H), 6.68 (d, *J* = 8.0 Hz, 1H), 4.53 - 4.29 (m, 3H), 3.98 (d, *J* = 13.6 Hz, 1H), 3.92 - 3.81 (m, 2H), 3.37 - 3.31 (m, 2H), 2.95 (s, 1H), 2.56 (s, 1H), 2.22 - 2.10 (m, 1H), 1.46 (t, *J =* 6.4 Hz, 3H), 1.26 - 1.14 (m, 3H).

### Example I-22

### (R)-4-(4,8-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

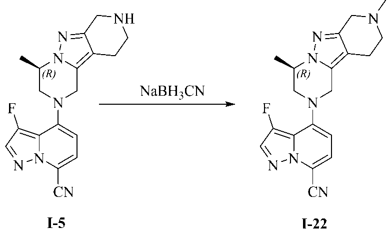

**Example I-22** was synthesized by following the protocols of **Example I-24.** MS: 366.1 (M+H)⁺. ¹H NMR (400 MHz, DMSO-*d₆*) δ 8.29 (d, *J* = 3.6 Hz, 1H), 7.71 (d, *J* = 8.0 Hz, 1H), 6.69 (d, *J* = 8.0 Hz, 1H), 4.52 - 4.30 (m, 3H), 3.99 (d, *J* = 13.6 Hz, 1H), 3.33 (s, 2H), 3.32 (s, 1H), 3.29 (s, 2H), 2.56 (t, *J* = 9.2 Hz, 2H), 2.36 (s, 3H), 1.46 (d, *J* = 6.4 Hz, 3H).

### Example I-23

### (R)-3-Fluoro-4-(8-isopropyl-4-methyl-3,4,7,8,9,10-hexahydropyrido[3',4':3,4] pyrazolo[1,5-a]pyrazin-2(1H)-yl)pyrazolo[1,5-a]pyridine-7-carbonitrile

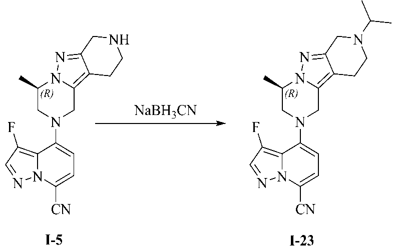

**Example I-23** was synthesized by following the protocols of **Example I-22.** MS: 394.1 (M+H)⁺. ¹H NMR (400 MHz, DMSO-*d₆*) δ 8.29 (d, J = 3.6 Hz, 1H), 7.71 (d, J = 8.0 Hz, 1H), 6.68 (d, J = 8.0 Hz, 1H), 4.51 - 4.29 (m, 3H), 3.98 (d, J = 13.6 Hz, 1H), 3.49 (d, J *=* 4.8 Hz, 2H), 3.28 (s, 1H), 2.89 (p, J = 6.4 Hz, 1H), 2.64 (dq, J = 16.8, 6.0 Hz, 2H), 2.45 (dt, J = 12.0, 5.6 Hz, 2H), 1.45 (d, J = 6.4 Hz, 3H), 1.04 (dd, J = 6.4, 1.6 Hz, 6H).

### Example 1-24

### (R)-4-(2,7-dimethyl-1,3,4,7,8,10-hexahydropyrido[4',3':3,4]pyrazolo[1,5-a]pyrazin-9(2H)-yl)-3-fluoropyrazolo[1,5-a]pyridine-7-carbonitrile

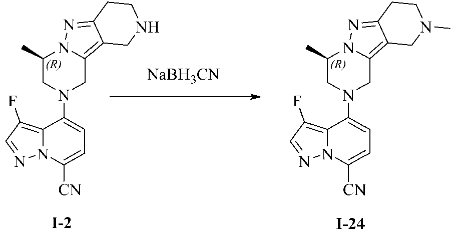

A reaction mixture of **Example I-2** (25 mg, 0.071 mmol), polyformaldehyde (14.24 mg, 0.142 mmol) and NaBH₃CN (8.94 mg, 0.122 mmol) in MeOH (5 mL) was stirred at 25 °C for 2 hours. The mixture was quenched with water and extracted with EtOAc, and the organic phase was washed with saturated saline, dried with Na₂SO₄ and concentrated. Purification by pre-HPLC gave **Example I-24** (12.6 mg, 46.8%) as white solid. MS: 316.1 (M+H)⁺. ¹H NMR (400 MHz, DMSO-*d₆*) δ 8.30 (d, *J* = 3.6 Hz, 1H), 7.72 (d, *J* = 8.0 Hz, 1H), 6.67 (d, *J* = 8.1 Hz, 1H), 4.50-4.46 (m, 1H), 4.41-4.30 (m, 2H), 4.02-3.95 (m, 1H), 3.32-3.30 (m, 1H), 3.26-3.22 (m, 2H), 2.67-2.62 (m, 4H), 2.59-2.57 (m, 1H), 2.35 (s, 3H), 1.46 (d, *J =* 6.4 Hz, 3H).

### Example I-25

### (R)-4-(2-ethyl-7-methyl-1,3,4,7,8,10-hexahydropyrido[4',3':3,4]pyrazolo[1,5-a] pyrazin-9(2H)-yl)-3-fluoropyrazolo[1,5-a]pyridine-7-carbonitrile

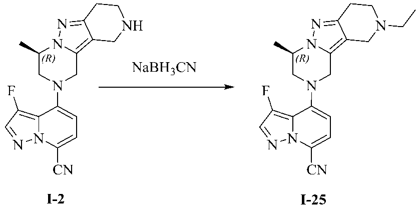

**Example I-25** was synthesized by following the protocols of **Example I-24.** MS: 380.1 (M+H)⁺. ¹H NMR (400 MHz, DMSO-*d₆*) δ 8.30 (d, *J* = 3.5 Hz, 1H), 7.73 (d, *J* = 8.0 Hz, 1H), 6.68 (d, *J* = 8.2 Hz, 1H), 4.51-4.48 (m, 1H), 4.40-4.34 (m, 2H), 3.98 (d, *J* = 12.5 Hz, 1H), 3.49-3.45(m, 1H), 3.33-3.29 (m, 2H), 2.78-2.73 (m, 1H), 2.69-2.64 (m, 3H), 2.57-2.53 (m, 2H), 1.46 (d, *J* = 6.3 Hz, 3H), 1.09 (t, *J* = 7.1 Hz, 3H).

### Example 1-26

### (R)-3-Fluoro-4-(4-methyl-8-(tetrahydro-2H-pyran-4-yl)-3,4,7,8,9,10-hexahydropyrido [3',4':3,4]pyrazolo[1,5-a]pyrazin-2(1H)-yl)pyrazolo[1,5-a]pyridine-7-carbonitrile

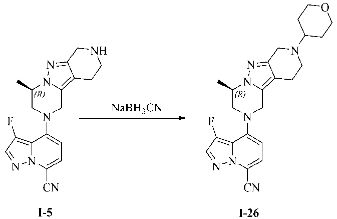

**Example I-26** was synthesized by following the protocols of **Example I-22.** MS: 436.1 (M+H)⁺. ¹H NMR (400 MHz, DMSO-*d₆*) δ 8.31 (d, *J* = 3.5 Hz, 1H), 7.73 (d, *J* = 8.0 Hz, 1H), 6.71 (d, *J* = 8.1 Hz, 1H), 4.54 - 4.39 (m, 2H), 4.39 - 4.30 (m, 2H), 4.06 - 3.97 (m, 1H), 3.93 (dd, *J* = 10.7, 4.0 Hz, 2H), 3.66 - 3.55 (m, 2H), 3.40 - 3.26 (m, 3H), 2.84 - 2.63 (m, 3H), 2.53 - 2.41 (m, 2H), 1.76 (d, *J* = 11.4 Hz, 2H), 1.61 - 1.49 (m, 4H), 1.48 (d, *J =* 6.4 Hz, 3H).

### Example I-27

### 4-((4R)-8-(dimethylamino)-4-methyl-3,4,7,8,9,10-hexahydropyrazino[1,2-b]indazol-2(1H)-yl)-3-fluoropyrazolo[1,5-a]pyridine-7-carbonitrile

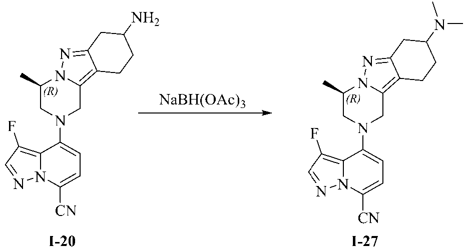

**Example I-27** was synthesized by following the protocols of **Example I-24.** MS: 394.1 (M+H)⁺. ¹H NMR (400 MHz, DMSO-*d₆*) δ 8.32 (d, *J* = 3.5 Hz, 1H), 7.75 (d, *J* = 8.0 Hz, 1H), 6.72 (d, *J* = 8.1 Hz, 1H), 4.55 - 4.34 (m, 3H), 4.08 - 3.95 (m, 1H), 3.35 (dd, *J* = 13.4, 9.5 Hz, 2H), 3.09 (s, 1H), 2.97 - 2.84 (m, 1H), 2.72 - 2.65 (m, 1H), 2.56 (s, 6H), 2.50 - 2.35 (m, 1H), 2.12 (d, *J =* 10.8 Hz, 1H),1.70 - 1.56 (m, 1 H), 1.49 (dd, *J =* 6.4, 2.2 Hz, 3H).

### Example 1-28

### (R)-4-(8-((1H-pyrazol-4-yl)methyl)-4-methyl-3,4,7,8,9,10-hexahydropyrido[3',4':3,4] pyrazolo[1,5-a]pyrazin-2(1H)-yl)-3-fluoropyrazolo[1,5-a]pyridine-7-carbonitrile

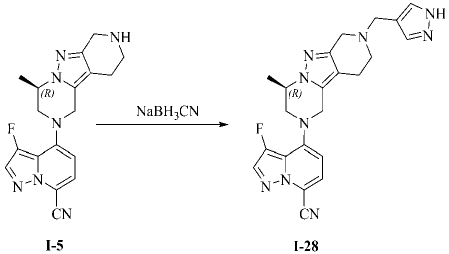

**Example I-28** was synthesized by following the protocols of **Example I-22.** MS: 432.1 (M+H)⁺. ¹H NMR (400 MHz, DMSO-*d₆*) δ 8.28 (d, J = 3.6 Hz, 1H), 8.14 (s, 1H), 7.70 (d, J = 8.0 Hz, 1H), 7.54 (s, 2H), 6.68 (d, J = 8.0 Hz, 1H), 4.41 (dt, J = 31.2, 15.6 Hz, 3H), 3.97 (d, J = 13.2 Hz, 1H), 3.60 (s, 2H), 3.43 (d, J = 9.6 Hz, 2H), 3.31 (d, J = 4.4 Hz, 1H), 2.65 (dq, J = 19.6, 6.1 Hz, 2H), 2.48 (s, 2H), 1.44 (d, J = 6.4 Hz, 3H).

### Example I-29

### (R)-4-(8-(1H-Pyrazole-3-yl) methyl)-4-methyl-3,4,7,8,9,10-hexahydropyridine [3 ', 4 ': 3, 4] pyrazino [1, 5-a] pyrazino 2 (1 H) -yl) -3-fluoropyrazolo [1, 5-a] pyridine-7-nitrile

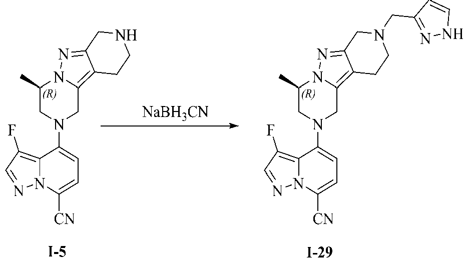

**Example I-29** was synthesized by following the protocols of **Example I-22.** MS: 432.1 (M+H)⁺. ¹H NMR (400 MHz, DMSO-*d₆*) δ 8.31 (d, *J* = 3.5 Hz, 1H), 7.73 (d, *J* = 8.0 Hz, 1H), 7.58 (d, *J* = 2.0 Hz, 1H), 6.70 (d, *J* = 8.1 Hz, 1H), 6.20 (d, *J* = 2.0 Hz, 1H), 4.55 - 4.30 (m, 3H), 4.00 (d, *J* = 10.7 Hz, 1H), 3.71 (s, 2H), 3.51 - 3.40 (m, 3H), 3.37 - 3.31 (m, 1H), 2.76 - 2.61 (m, 2H), 2.51 - 2.43 (m, 1H), 1.46 (d, *J* = 6.3 Hz, 3H).

### Example I-30

### 4-((4R)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

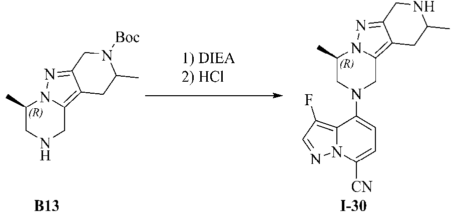

**Example I-30** was synthesized by following the protocols of **Example I-2**. MS: 366.1 (M+H)⁺. ¹H NMR (400 MHz, DMSO-*d₆*) δ 8.33 (d, J = 3.5 Hz, 1H), , 7.77 - 7.74 (m, 1H), 6.72 (d, J = 8.1 Hz, 1H), 4.57 - 4.52 (m, 1H), 4.47-4.42 (m, 2H), 4.21 - 3.97 (m, 3H), 3.42 - 3.29 (m, 3H), 2.85 - 2.69 (m, 1H), 2.45 - 2.29 (m, 1H), 1.49 (dd, J = 6.4, 2.0 Hz, 3H), 1.32 (dd, J = 6.5, 2.4 Hz, 3H).

### Examples I-30a and I-30b

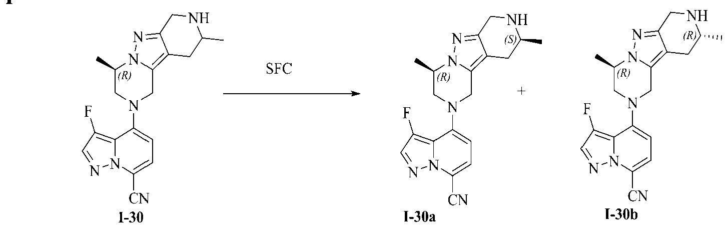

**Examples I-30a** and **I-30a** were separated through chiral resolution by SFC, mobile phase A: HEX, mobile phase B: DCM (0.5% 2mM NH₃-MeOH)). **Example I-30a** was obtained as the first peak component as white solid (retention time: 1.630 minute) and the second component peak **Example I-30b** (retention time is 1.991 minute). MS: 366.1 (M+H)⁺.

### Example I-30a and Example I-30b

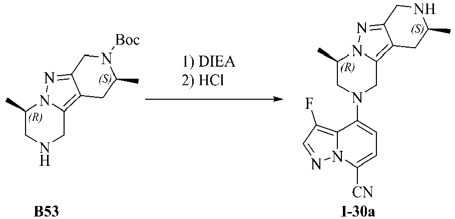

**Example I-30a** was synthesized by following the protocols of **Example I-2**. MS: 366.1 (M+H)⁺.

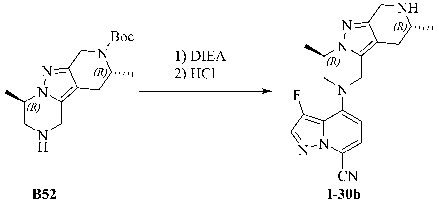

**Example I-30b** was synthesized by following the protocols of **Example I-2**. MS: 366.1 (M+H)⁺.

### Example I-31

### 3-Fluoro-4-((4R)-4-methyl-8-morpholino-3,4,7,8,9,10-hexahydropyrazino[1,2-b]indazol-2(1H)-yl)pyrazolo[1,5-a]pyridine-7-carbonitrile

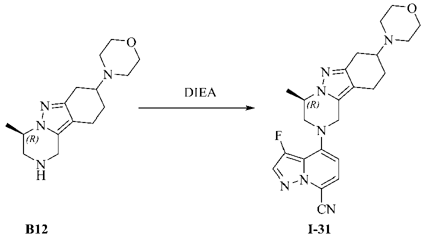

**Example I-31** was synthesized by following the protocols of **Example I-1.** MS: 436.1 (M+H)⁺. ¹H NMR (400 MHz, DMSO-*d₆*) δ 10.22 (s, 1H), 8.33 (d, *J* = 3.5 Hz, 1H), 7.76 (d, *J* = 8.0 Hz, 1H), 6.73 (dd, *J* = 8.1, 1.9 Hz, 1H), 4.58 - 4.34 (m, 4H), 4.14 - 3.96 (m, 4H), 3.76 (t, *J* = 12.0 Hz, 2H), 3.62 - 3.49 (m, 5H), 3.42 - 3.28 (m, 2H), 3.22 - 3.09 (m, 1H), 2.94 - 2.66 (m, 2H), 2.52 - 2.29 (m, 2H), 1.84 - 1.69 (m, 1H), 1.49 (d, *J* = 6.3 Hz, 3H).

### Example I-32

### 3-Fluoro-4-((4R)-8-(3-hydroxypiperidin-4-yl)-4-methyl-9-oxo-3,4,7,8,9,10-hexahydro pyrido[3',4':3,4]pyrazolo[1,5-a]pyrazin-2(1H)-yl)pyrazolo[1,5-a]pyridine-7-carbonitrile

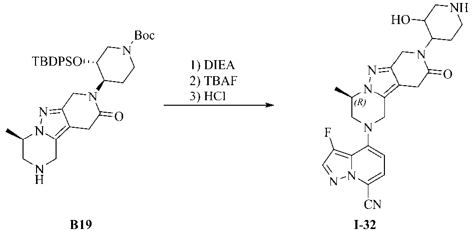

**Example I-32** was synthesized by following the protocols of **Example I-33.** MS: 465.1 (M+H)⁺. ¹H NMR (400 MHz, DMSO-*d*₆) δ 8.34-8.30 (m, 2H), 7.75 (d, *J* = 8.0 Hz, 1H), 6.71 (d, *J =* 8.1 Hz, 1H), 4.64-4.56 (m, 1H), 4.45-4.40 (m, 4H), 4.31 (s, 1H), 4.35-4.25 (m, 1H), 4.07-4.02 (m, 1H), 3.75-3.67 (m, 2H), 3.50-3.34 (m, 4H), 3.23-3.09 (m, 3H), 3.01-2.94 (m, 1H), 2.60-2.55 (m, 1H), 1.52 (d, *J =* 6.5 Hz, 3H).

### Example I-33

### 3-Fluoro-4-((4R)-7-(hydroxymethyl)-4-methyl-3,4,7,8,9,10-hexahydropyrido[3',4':3,4] pyrazolo[1,5-a]pyrazin-2(1H)-yl)pyrazolo[1,5-a]pyridine-7-carbonitrile

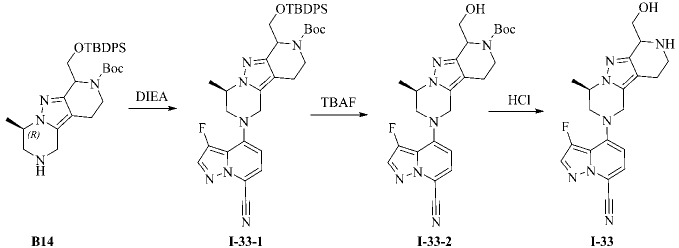

### Step 1: Preparation of intermediate I-33-1

**Intermediate I-33-1** was synthesized by following the protocols of **Example I-1.** MS: 720.1 (M+H)⁺.

### Step 2: Preparation of intermediate 1-33-2

A reaction mixture of **Intermediate I-33-1** (300 mg, 0.417 mmol) and TBAF (0.5 mL, 0.5 mmol, 1 mol/l in THF) in THF (3 mL) was stirred at 25 °C for 2 hours. The mixture was quenched with water and extracted with EtOAc, and the organic phase was washed with saturated saline, dried with Na₂SO₄ and concentrated. Purification by pre-HPLC gave **Intermediate 1-33-2** (180 mg, 90%) as yellow solid. MS: 482.2 (M+H)⁺.

### Step 3: Preparation of intermediate I-33

**Example I-33** was synthesized by following the protocols of **Example 1-2.** MS: 382.1 (M+H)⁺. ¹H NMR (400 MHz, DMSO-*d*₆) δ 8.30 (d, *J=* 3.6 Hz, 1H), 7.72 (dd, *J=* 8.1, 1.9 Hz, 1H), 6.70 (dd, *J* = 8.1, 4.3 Hz, 1H), 4.53 - 4.32 (m, 3H), 4.02 - 3.93 (m, 2H), 3.83 (td, *J=* 11.0, 3.5 Hz, 2H), 3.57 - 3.48 (m, 2H), *J* = 14.3, 9.2, 5.6 Hz, 1H), 3.17 (s, 1H), 2.90 - 2.78 (m, 1H), 2.46 (d, *J* = 6.0 Hz, 1H), 1.46 (d, *J* = 6.4 Hz, 3H).

### Example 1-34

### 3-Fluoro-4-((4R)-8-(3-hydroxypyrrolidin-1-yl)-4-methyl-3,4,7,8,9,10-hexahydro pyrazino[1,2-b]indazol-2(1H)-yl)pyrazolo[1,5-a]pyridine-7-carbonitrile

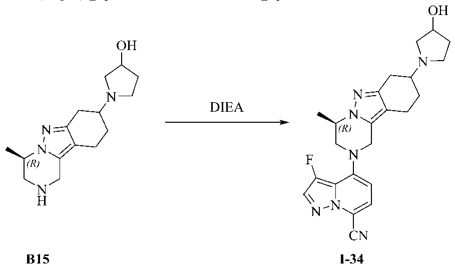

**Example I-34** was synthesized by following the protocols of **Example 1-1.** MS: 436.1 (M+H)⁺. ¹H NMR (400 MHz, DMSO-*d*₆) δ 8.32 (d, *J* = 3.6 Hz, 1H), 8.25 (s, 1H), 7.74 (d, *J= 8.0* Hz, 1H), 6.71 (dd, *J* = 8.1, 3.4 Hz, 1H), 4.54 - 4.30 (m, 3H), 4.22 (s, 1H), 4.00 (d, *J* = 13.6 Hz, 1H), 3.38 - 3.26 (m, 1H), 2.96 - 2.73 (m, 3H), 2.70 - 2.56 (m, 2H), 2.55 - 2.52 (m, 2H),2.36 (s, 1H), 2.11 - 1.93 (m, 3H), 1.67 - 1.51 (m, 2H), 1.48 (d, *J =* 6.3 Hz, 3H).

### Example I-35

### 3-Fluoro-4-((7R)-4-(hydroxymethyl)-7-methyl-1,3,4,7,8,10-hexahydropyrido[4',3':3,4] pyrazolo[1,5-a]pyrazin-9(2H)-yl)pyrazolo[1,5-a]pyridine-7-carbonitrile

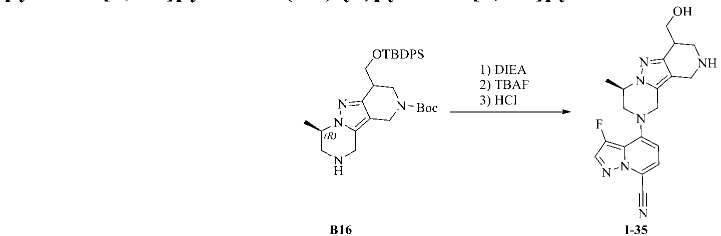

**Example I-35** was synthesized by following the protocols of **Example I-33.** MS: 382.1 (M+H)⁺. ¹H NMR (400 MHz, Methanol-*d₄*δ 8.08 d, *J =* 3.6 Hz, 1H, 7.50 d, *J =* 8.0 Hz, 1H, 6.69 d, *J =* 8.0 Hz, 1H, 4.64 s, 1H, 4.57 - 4.43 m, 3H, 4.11 - 3.99 m, 4H, 3.81 td, *J =* 10.8, 7.6 Hz, 1H, 3.47 - 3.39 m, 2H, 3.30 - 3.11 m, 1H, 1.61 d, *J =* 6.4, 2.0 Hz, 3H.

### Example 1-36

### 3-Fluoro-4-((7R)-3-(hydroxymethyl)-7-methyl-1,3,4,7,8,10-hexahydropyrido[4',3':3,4] pyrazolo[1,5-a]pyrazin-9(2H)-yl)pyrazolo[1,5-a]pyridine-7-carbonitrile

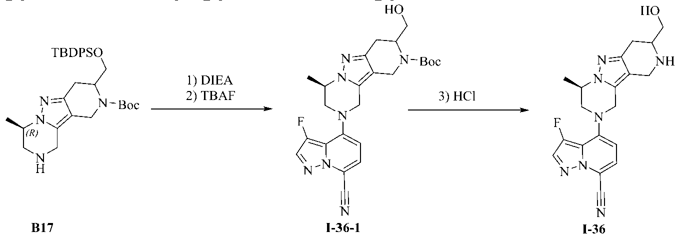

**Example I-36** was synthesized by following the protocols of **Example I-33.** MS: 382.1 (M+H)⁺. ¹H NMR (400 MHz, DMSO-*d₆*) δ 8.33 (d, *J* = 3.6 Hz, 1H), 7.76 (dd, *J=* 8.0, 2.2 Hz, 1H), 6.71 (dd, *J* = 8.1, 4.2 Hz, 1H), 4.62 - 4.35 (m, 3H), 4.13 - 3.74 (m, 4H), 3.68 - 3.63 (m, 1H), 3.54-3.50 (m, 1H), 3.40 - 3.34 (m, 2H), 3.18-3.15 (m, 1H), 2.77-2.71 (m, 1H), 2.52 - 2.41 (m, 1H), 1.50 (dd, *J* = 6.4, 3.0 Hz, 3H).

### Example I-37

### 4-((4R)-8-amino-9-hydroxy-4-methyl-3,4,7,8,9,10-hexahydropyrazino [1,2-b] indazol-2(1H)-yl)-3-fluoropyrazolo[1,5-a]pyridine-7-carbonitrile

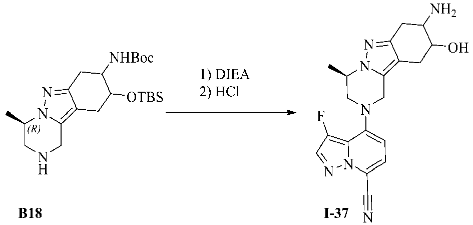

**I-37 Example** was synthesized by following the protocols of **Example I-2**. MS: 382.1 (M+H)⁺. ¹H NMR (400 MHz, DMSO-*d*₆) δ 8.39 (s, 1H), 8.32 (d, *J* = 3.5 Hz, 1H), 7.75 (d, *J* = 8.0 Hz, 1H), 6.73 (d, *J* = 8.1 Hz, 1H), 4.57 - 4.42 (m, 2H), 4.38 (d, *J=* 14.9 Hz, 2H), 4.05 - 3.96 (m, 2H),3.69 - 3.60 (m, 1H), 3.38 - 3.27 (m, 2 H), 3.07 - 2.95 (m, 2 h), 2.84 (ddd, *J=* 20.0, 15.0, 5.5 Hz, 1H), 2.31 (ddd, *J=* 23.1, 15.1, 9.4 Hz, 1H), 1.48 (dd, *J* = 6.4, 1.8 Hz, 3H).

### Example 1-38

### 3-Fluoro-4-((4R)-10-hydroxy-4-methyl-3,4,7,8,9,10-hexahydropyrido[3',4':3,4] pyrazolo[1,5-a]pyrazin-2(1H)-yl)pyrazolo[1,5-a]pyridine-7-carbonitrile

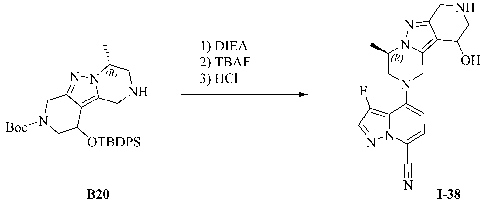

**Example I-38** was synthesized by following the protocols of **Example I-33.** MS: 368.2 (M+H)⁺. ¹H NMR (400 MHz, DMSO-*d*₆) δ 8.32 (d, *J* = 3.5 Hz, 1H), 8.22 (s, 1H), 7.74 (d, *J* = 8.0 Hz, 1H), 6.69 (d, *J* = 8.1 Hz, 1H), 4.68-4.62 (m, 2H), 4.53-4.48 (m, 1H), 4.44-4.36 (m, 1H), 4.07-4.02 (m, 1H), 3.92-3.78 (m, 2H), 3.42-3.32 (m, 1H), 3.11-3.02 (m, 1H), 2.90-2.82 (m, 1H), 1.49 (d, *J* = 6.3 Hz, 3H).

### Example I-39

### 4-((4R,7S)-4,7-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

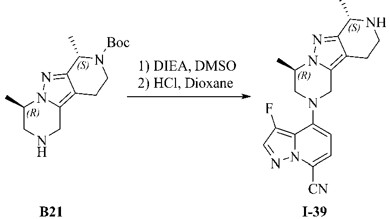

**Example I-39** was synthesized by following the protocols of **Example I-2**. MS: 366.1 (M+H)⁺. ¹H NMR (400 MHz, DMSO-*d*₆) δ 8.29 (dd, *J* = 3.6, 1.2 Hz, 1H), 7.72 (dd, *J =* 8.0, 2.4 Hz, 1H), 6.69 *(t, J* = 7.6 Hz, 1H), 4.51 - 4.34 (m, 3H), 4.09 - 3.95 (m, 2H), 3.93 - 3.82 (m, 1H), 3.37 - 3.31 (m, 1H), 3.21 (dt, *J =* 12.4, 4.8 Hz, 1H), 2.96 - 2.83 (m, 1H), 2.71 - 2.52 (m, 1H), 2.07 (ddd, *J =* 53.2, 15.0, 8.8 Hz, 1H), 1.46 (d, *J =* 6.4 Hz, 3H), 1.36 (d, *J =* 6.4 Hz, 2H), 1.20 (d, *J =* 6.4 Hz, 1H).

### Example 1-40

### 3-Fluoro-4-((4R)-10-(hydroxymethyl)-4-methyl-3,4,7,8,9,10-hexahydropyrido [3',4':3,4]pyrazolo[1,5-a]pyrazin-2(1H)-yl)pyrazolo[1,5-a]pyridine-7-carbonitrile

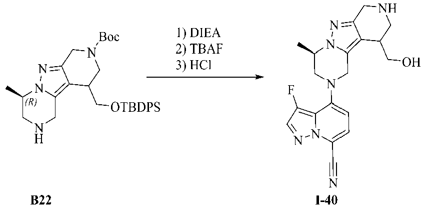

**Example I-40** was synthesized by following the protocols of **Example I-33.** MS: 382.1 (M+H)⁺. ¹H NMR (400 MHz, DMSO-*d*₆) δ 8.33 (m, 1H), 7.75 (dd, *J* = 8.0, 3.9 Hz, 1H), 6.74 *(t, J* = 8.0 Hz, 1H), 4.67 - 4.46 (m, 2H), 4.43-4.40 (m, 1H), 4.07 - 3.95 (m, 3H), 3.67 -3.60(m, 2H), 3.57 - 3.52(m, 1H), 3.48-3.41(m, 1H), 3.36 - 3.30 (m, 1H), 3.15 - 3.10 (m, 2H), 2.96 - 2, 92 (m, 1H), 1.49 (t, *J* = 7.9 3H).

### Example I-41

### (R)-3-Fluoro-4-(4-methyl-7-oxo-8-(piperidin-4-yl)-3,4,7,8,9,10-hexahydropyrido [3',4':3,4]pyrazolo[1,5-a]pyrazin-2(1H)-yl)pyrazolo[1,5-a]pyridine-7-carbonitrile

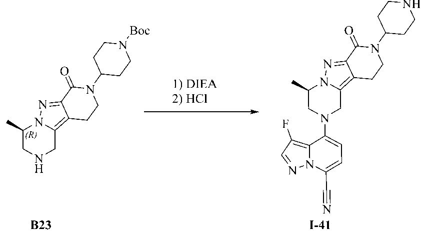

**Example I-41** was synthesized by following the protocols of **Example I-2**. MS: 449.2 (M+H)⁺. ¹H NMR (400 MHz, DMSO-*d₆*) δ 8.35 (br, 1H), 8.33 (d, *J =* 3.6 Hz, 2H), 7.76 (d, *J =* 8.0 Hz, 1H), 6.74 (d, *J =* 8.1 Hz, 1H), 4.66-4.57 (m, 2H), 4.55-4.45 (m, 2H), 4.09- 4.03 (m, 1H), 3.48-3.44 (m, 2H), 3.43-3.41 (m, 1H), 3.23-3.17 (m, 2H), 2.86-2.76 (m, 2H), 2.75-2.65 (m, 2H), 1.86-1.75 (m, 2H), 1.63-1.57 (m, 2H), 1.54 (d, *J =* 6.4 Hz, 3H).

### Example I-42

### (R)-1-methyl-4-(4-methyl-3,4,7,8,9,10-hexahydropyrido[3',4':3,4]pyrazolo[1,5-a]pyrazin-2(1H)-yl)-1,8-naphthyridin-2(1H)-one

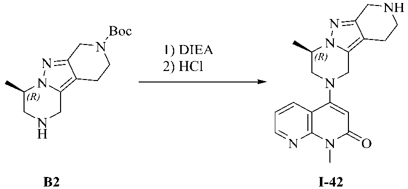

**Example I-42** was synthesized by following the protocols of **Example I-5.** MS: 351.1 (M+H)⁺. ¹H NMR (400 MHz, DMSO-*d₆*) δ 8.33 (d, *J* = 3.5 Hz, 1H), 8.19 (s, 1H), 7.75 (d, *J = 8.0* Hz, 1H), 6.72 (d, *J =* 8.1 Hz, 1H), 4.53 (d, *J* = 15.2 Hz, 1H), 4.46 - 4.33 (m, 2H), 4.08 - 3.98 (m, 1H), 3.47 - 3.27 (m, 4H), 3.10 - 2.98 (m, 1H), 2.73 - 2.58 (m, 4H), 2.15 - 2.06 (m, 2H), 1.98 - 1.86 (m, 2H), 1.77 - 1.65 (m, 2H), 1.50 (d, *J* = 6.3 Hz, 3H).

### Example 1-43

### 3-fFuoro-4-((4R,9S)-9-(hydroxymethyl)-4-methyl-3,4,7,8,9,10-hexahydropyrido [3',4':3,4]pyrazolo[1,5-a]pyrazin-2(1H)-yl)pyrazolo[1,5-a]pyridine-7-carbonitrile

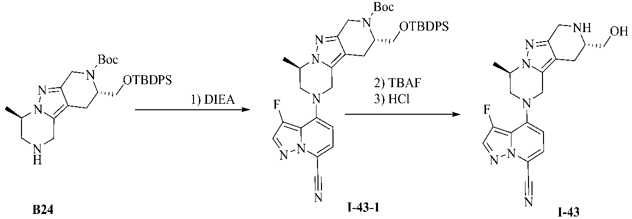

**Example I-43** was synthesized by following the protocols of **Example I-33.** MS: 382.1 (M+H)⁺. ¹H NMR (400 MHz, DMSO-*d*₆) δ 8.87 (s, 1H), 8.33 (d, *J* = 3.6 Hz, 1H), 7.76 (d, *J* = 8.0 Hz, 1H), 6.73 (d, *J* = 8.0 Hz, 1H), 5.47 (s, 1H), 4.60 - 4.52 (m, 1H), 4.51 - 4.39 (m, 2H), 4.27 - 4.13 (m, 2H), 4.08 - 3.96 (m, 1H), 3.80 - 3.70 (m, 1H), 3.65 - 3.55 (m, 1H), 3.49 - 3.42 (m, 2H), 2.80 - 2.65 (m, 1H), 2.64 - 2.57 (m, 1H), 1.50 (d, *J* = 6.4 Hz, 3H).

### Example I-44

### 3-Fluoro-4-((4R)-4-methyl-1,3,4,7,8,9,10,11-octahydro-2H-8,11-epiminocyclohepta [3,4]pyrazolo[1,5-a]pyrazin-2-yl)pyrazolo[1,5-a]pyridine-7-carbonitrile

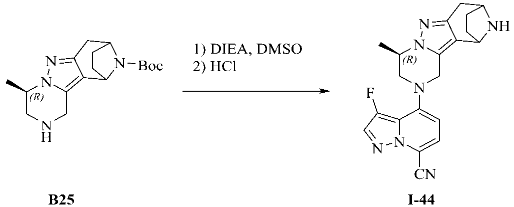

**Example I-44** was synthesized by following the protocols of **Example I-2**. MS: 378.2 (M+H)⁺. ¹H NMR (400 MHz, CDCl₃) δ 7.97 (t, *J* = 4.0 Hz, 1H), 7.30 - 7.26 (m, 1H), 6.47 - 6.46 (m, 1H), 4.87 - 4.82(m, 1H), 4.63 - 4.32 (m, 4H), 4.05- 4.01 (m, 1H), 3.46 - 3.43(m, 1H), 3.36- 3.21 (m, 1H), 2.89- 2.85 (m, 1H), 2.45- 2.31(m, 2H), 2.11- 2.08 (m, 1H), 1.90 (s, 1H), 1.62 (t, *J =* 8.0 Hz, 3H).

### Example I-45

### (R)-3-Fluoro-4-(4,7,7-trimethyl-3,4,7,8,9,10-hexahydropyrido[3',4':3,4]pyrazolo[1,5-a]pyrazin-2(1H)-yl)pyrazolo[1,5-a]pyridine-7-carbonitrile

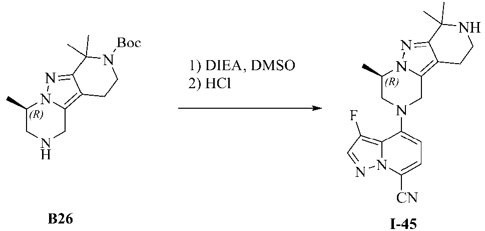

**Example I-45** was synthesized by following the protocols of **Example I-2**. MS: 380.1 (M+H)⁺. ¹H NMR (400 MHz, DMSO-*d*₆) δ 8.33 (d, *J* = 3.5 Hz, 1H), 7.76 (d, *J* = 8.0 Hz, 1H), 6.74 (d, *J* = 8.1 Hz, 1H), 4.55-4.51 (m, 1H), 4.46-4.42 (m, 2H), 4.07 - 3.97 (m, 1H), 3.41-3.35 (m, 2H), 3.31-3.29 (m 2H), 2.68-2.62 (m, 2H), 1.54 (d, *J* = 5.0 Hz, 6H), 1.50 (d, *J* = 6.4 Hz, 3H).

### Example I-46

### 3-fluoro-4-((7R)-1-(hydroxymethyl)-7-methyl-1,3,4,7,8,10-hexahydropyrido[4',3':3,4] pyrazolo[1,5-a]pyrazin-9(2H)-yl)pyrazolo[1,5-a]pyridine-7-carbonitrile

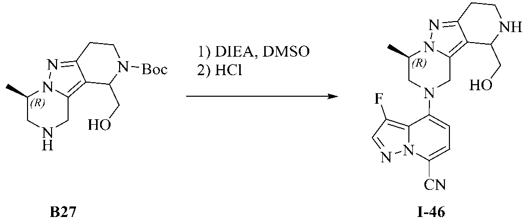

**Example I-46** was synthesized by following the protocols of **Example I-2.** MS: 382.1 (M+H)⁺. ¹H NMR (400 MHz, DMSO-*d*₆) δ 9.37 (s, 1H), 8.77 (s, 1H), 8.31 (t, *J=* 3.6 Hz, 1H), 7.74 (dd, *J* = 8.0, 2.7 Hz, 1H), 6.75 (d, *J=* 8.1 Hz, 1H), 5.65 (s, 1H),4.71 - 4.35 (m, 4H), 4.09 - 3.84 (m, 2H), 3.72 (dt, *J=* 20.2, 10.9 Hz, 1H), 3.54 - 3.41 (m, 2H), 3.29 (dd, *J* = 13.7, 9.8 Hz, 2H), 2.86 (q, *J* = 6.1, 5.5 Hz, 2H), 1.47 (t, *J* = 6.0 Hz, 3H).

### Example I-47

### 3-Fluoro-4-((7R)-7-methyl-3-(morpholinomethyl)-1,3,4,7,8,10-hexahydropyrido [4',3':3,4]pyrazolo[1,5-a]pyrazin-9(2H)-yl)pyrazolo[1,5-a]pyridine-7-carbonitrile

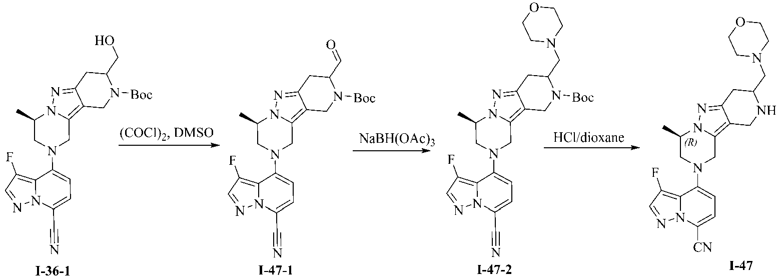

### Step 1: Preparation of intermediate I-47-1

A reaction mixture of DMSO (0.374 mmol) and oxalyl chloride (0.249 mmol) in DCM (4 mL) was stirred at -78 °C for 15 min before **Intermediate I-36-1** (100 mg, 0.208 mmol) in DCM (1 mL) was added to the flask. The resulting mixture was stirred at -78 °C for 1 hour. The mixture was quenched with TEA (1.038 mmol) and water, then extracted with EtOAc, and the organic phase was washed with saturated saline, dried with Na₂SO₄ and concentrated. Purification by flash chromatography gave **Intermediate 1-47-1** (80 mg, 80%) as white solid. MS: 480.2 (M+H)⁺.

### Step 2: Preparation of intermediate I-47-2

**Intermediate I-47-2** was synthesized by following the protocols of **Example I-24.** MS: 551.1 (M+H)⁺.

### Step 3: Preparation of Example I-47

**Example I-47** was synthesized by following the protocols of **Example I-2**. MS: 451.1 (M+H)⁺. ¹H NMR (400 MHz, DMSO-*d*₆) δ 8.33 (d, *J* = 3.2 Hz, 1H), 7.75 (d, *J* = 8.0 Hz, 1H), 6.75 -6.65 (m, 1H), 4.60 - 4.43 (m, 1H), 4.42 - 4.32 (m, 2H), 4.10 - 3.90 (m, 1H), 3.88 - 3.82 (m, 1H), 3.79 - 3.67 (m, 2H), 3.64 - 3.60 (m, 5H), 3.36 - 3.30 (m, 2H), 3.02 (s, 1H), 2.68 - 2.60 (m, 1H), 2.46 -2.43 (m, 1H), 2.42- 2.34 (m, 3H), 2.28 - 2.18 (m, 1H), 1.49 (d, *J =* 6.0 Hz, 3H).

### Example I-48

### (R)-5-(4-methyl-3,4,7,8,9,10-hexahydropyrido[3',4':3,4]pyrazolo[1,5-a]pyrazin-2(1H)-yl)-[1,2,4]triazolo[1,5-a]pyridine-8-carbonitrile

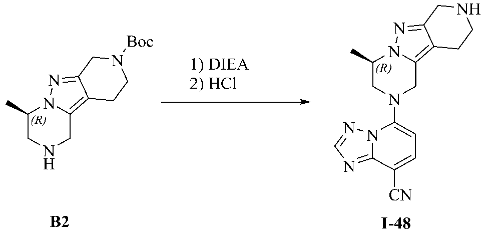

**Example I-48** was synthesized by following the protocols of **Example I-5.** MS: 335.1 (M+H)⁺. ¹H NMR (400 MHz, DMSO-*d₆*) δ 8.67 (s, 1H), 8.27 - 8.23 (m, 1H), 6.81 (d, *J =* 8.4 Hz, 1H), 4.97 (d, *J =* 15.6 Hz, 1H), 4.76 (d, *J =* 15.6 Hz, 1H), 4.61 - 4.34 (m, 3H), 3.96 - 3.91 (m, 1H), 3.84 (d, *J* = 2.0 Hz, 3H), 2.99 (q, *J =* 6.0 Hz, 2H), 1.51 (d, *J =* 6.0 Hz, 3H).

### Example I-49

### (R)-4-(2-cyclobutyl-7-methyl-1,3,4,7,8,10-hexahydropyrido[4',3':3,4]pyrazolo[1,5-a]pyrazin-9(2H)-yl)-3-fluoropyrazolo[1,5-a]pyridine-7-carbonitrile

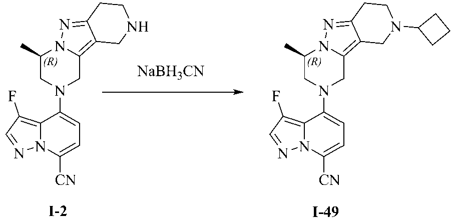

**Example I-49** was synthesized by following the protocols of **Example I-24.** MS: 406.1 (M+H)⁺. ¹H NMR (400 MHz, DMSO-*d₆*) δ 8.33 (d, *J* = 3.5 Hz, 1H), 8.19 (s, 1H), 7.75 (d, *J* = 8.0 Hz, 1H), 6.72 (d, *J* = 8.1 Hz, 1H), 4.53 (d, *J* = 15.2 Hz, 1H), 4.46 - 4.33 (m, 2H), 4.08 - 3.98 (m, 1H), 3.47 - 3.27 (m, 4H), 3.10 - 2.98 (m, 1H), 2.73 - 2.58 (m, 4H), 2.15 - 2.06 (m, 2H), 1.98 - 1.86 (m, 2H), 1.77 - 1.65 (m, 2H), 1.50 (d, *J* = 6.3 Hz, 3H).

### Example I-50

### (R)-3-Fluoro-4-(2-isobutyl-7-methyl-1,3,4,7,8,10-hexahydropyrido[4',3':3,4]pyrazolo [1,5-a]pyrazin-9(2H)-yl)pyrazolo[1,5-a]pyridine-7-carbonitrile

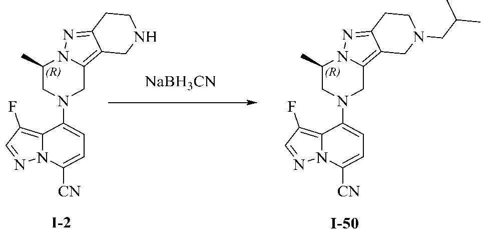

**Example I-50** was synthesized by following the protocols of **Example I-24.** MS: 408.1 (M+H)⁺. ¹H NMR (400 MHz, DMSO-*d*₆) δ 8.31 (d, *J* = 3.5 Hz, 1H), 8.17 (s, 1H), 7.74 (d, *J* = 8.0 Hz, 1H), 6.69 (d, *J* = 8.1 Hz, 1H), 4.52 (d, *J=* 15.4 Hz, 1H), 4.47 - 4.30 (m, 2H), 4.01 (dd, *J* = 13.6, 4.1 Hz, 1H), 3.55 (d, *J* = 13.6 Hz, 1H), 3.44 (d, *J=* 13.7 Hz, 1H), 3.34 (dd, *J* = 13.7, 9.2 Hz, 1H), 2.80 (s, 2H), 2.70 (d, *J* = 5.6 Hz, 2H), 2.37 (d, *J=* 7.2 Hz, 2H), 1.91 (dt, *J* = 13.5, 6.8 Hz, 1H), 1.49 (d, *J* = 6.3 Hz, 3H), 0.92 (d, *J* = 6.5 Hz, 6H).

### Example I-51

### 3-Fluoro-4-((7R)-4-hydroxy-7-methyl-1,3,4,7,8,10-hexahydropyrido[4',3':3,4] pyrazolo[1,5-a]pyrazin-9(2H)-yl)pyrazolo[1,5-a]pyridine-7-carbonitrile

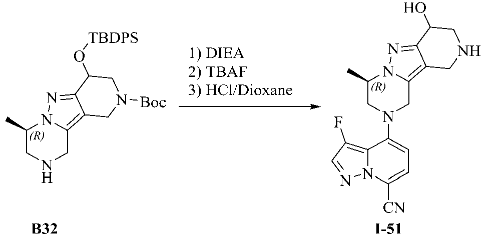

**Example I-51** was synthesized by following the protocols of **Example I-33.** MS: 368.1 (M+H)⁺. ¹H NMR (400 MHz, DMSO-*d₆*) δ 8.32 (d, *J* = 3.5 Hz, 1H), 8.24 (d, *J* = 1.7 Hz, 1H), 7.75 (d, *J* = 8.0 Hz, 1H), 6.71 (d, *J* = 8.1 Hz, 1H), 4.57 - 4.39 (m, 4H), 4.09 - 3.98 (m, 1H), 3.91 - 3.82 (m, 1H), 3.79 - 3.74 (m, 1H), 3.73 - 3.64 (m, 1H), 3.44 - 3.31 (m, 1H), 3.06 - 2.94 (m, 2H), 1.52 (d, *J =* 6.3 Hz, 3H).

### Example I-52

### 3-Fluoro-4-((4R)-4-methyl-1,3,4,7,8,9,10,11-octahydro-2H-7,10-epiminocyclohepta [3,4]pyrazolo[1,5-a]pyrazin-2-yl)pyrazolo[1,5-a]pyridine-7-carbonitrile

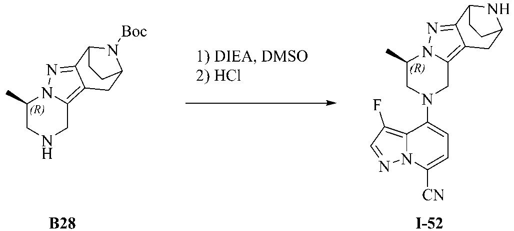

**Example I-52** was synthesized by following the protocols of **Example I-2**. MS: 378.2 (M+H)⁺. ¹H NMR (400 MHz, DMSO-*d*₆) δ 8.32 (dd, *J=* 3.6, 2.2 Hz, 1H), 7.75 (dd, *J=* 8.0, 4.8 Hz, 1H), 6.73 (dd, *J=* 11.9, 8.1 Hz, 1H), 4.63 (dd, *J =* 18.8, 50 Hz, 1H), 4.54-4.49 (m, 1H), 4.43 (s, 1H), 4.41 - 4.33 (m, 1H), 4.18-4.14 (m, 1H), 4.06 - 3.99 (m, 1H), 3.39-3.30 (m, 1H), 2.96-2.87 (m, 1H), 2.52 - 2.40 (m, 1H), 2.22 - 2.04 (m, 2H), 1.95-1.88 (m, 1H), 1.61-1.59 (m, 1H), 1.50 (t, *J =* 6.8 Hz, 3H).

### Example I-53

### (R)-3-Fluoro-4-(2-isopropyl-7-methyl-1,3,4,7,8,10-hexahydropyrido[4',3':3,4] pyrazolo[1,5-a]pyrazin-9(2H)-yl)pyrazolo[1,5-a]pyridine-7-carbonitrile

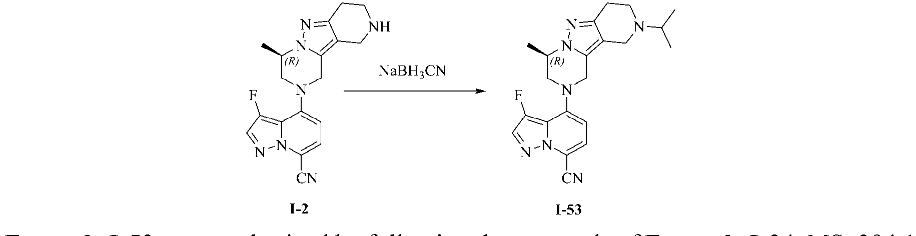

**Example I-53** was synthesized by following the protocols of **Example I-24.** MS: 394.1 (M+H)⁺. ¹H NMR (400 MHz, DMSO-*d₆*) δ 8.29 (d, *J* = 3.6 Hz, 1H), 8.16 (s, 1H), 7.72 (d, *J* = 8.0 Hz, 1H), 6.67 (d, *J* = 8.0 Hz, 1H), 4.50 (d, *J* = 15.6 Hz, 1H), 4.42 - 4.32 (m, 2H), 4.00 - 3.95 (m, 1H), 3.60 (d, *J* = 13.2 Hz, 1H), 3.51 (d, *J* = 13.2 Hz, 1H), 3.35 - 3.29 (m, 1H), 3.01 (q, *J* = 6.4 Hz, 1H), 2.87 - 2.77 (m, 2H), 2.65 (t, *J* = 5.6 Hz, 2H), 1.46 (d, *J =* 6.4 Hz, 3H), 1.09 (d, *J* = 6.4 Hz, 6H).

### Example I-54

### (R)-4-(2-((1H-Pyrazole-4-yl) methyl) -7-methyl-1,3,4,7,8,10-hexahydropyridine[4 ', 3 ': 3, 4] pyrazole [1, 5-a] pyrazine-9 (2H) -yl) -3-fluoropyrazole [1, 5-a] pyridine-7-nitrile

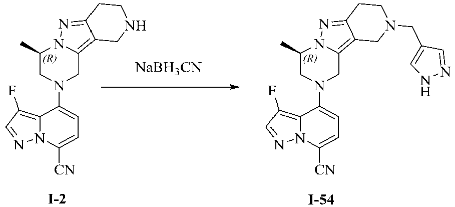

**Example I-54** was synthesized by following the protocols of **Example I-24.** MS: 432.1 (M+H)⁺. ¹H NMR (400 MHz, DMSO-*d₆*) δ 8.28 (d, *J* = 3.6 Hz, 1H), 7.69 (d, *J* = 8.0 Hz, 1H), 7.52 (s, 2H), 6.67 (d, *J =* 8.0 Hz, 1H), 4.46 - 4.30 (m, 3H), 3.96 (dd, *J =* 13.6, 4.0 Hz, 1H), 3.57 (d, *J* = 3.6 Hz, 2H), 3.38 - 3.34 (m, 2H), 3.31 (s, 1H), 3.28 (d, *J* = 4.0 Hz, 1H), 2.71 - 2.59 (m, 4H), 1.45 (d, *J* = 6.4 Hz, 3H).

### Example I-55

### (R)-3-Fluoro-4- (2- (furan-2-methyl) -7-methyl-1, 3, 4, 7, 8, 10-hexahydropyridine [4 ', 3 ': 3, 4] pyrazole [1, 5-a] pyrazine-9 (2 H) -yl) pyrazole [1, 5-a] pyridine-7-nitrile

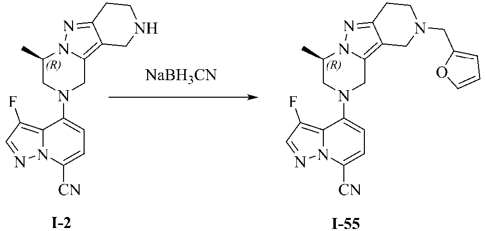

**Example I-55** was synthesized by following the protocols of **Example I-24.** MS: 432.1 (M+H)⁺. ¹H NMR (400 MHz, DMSO-*d₆*) δ 8.28 (d, *J* = 3.6 Hz, 1H), 7.70 (d, *J* = 8.0 Hz, 1H), 7.60 (d, *J* = 1.6 Hz, 1H), 6.68 (d, *J* = 8.0 Hz, 1H), 6.42 (dd, *J* = 3.2, 1.6 Hz, 1H), 6.32 (d, *J* = 3.2 Hz, 1H), 4.46 - 4.30 (m, 3H), 3.97 (dd, *J* = 13.6, 4.0 Hz, 1H), 3.69 (d, *J* = 2.4 Hz, 2H), 3.45 - 3.34 (m, 2H), 3.28 (d, *J* = 9.2 Hz, 1H), 2.72 (ddt, *J* = 16.8, 11.2, 5.2 Hz, 2H), 2.63 (d, *J* = 5.6 Hz, 2H), 1.45 (d, *J* = 6.4 Hz, 3H).

### Example I-56

### (R)-4-(2-(2, 2-difluoroethyl) -7-methyl-1, 3, 4, 7, 8, 10-hexahydropyridine [4 ', 3 ': 3, 4] pyrazole [1, 5-a] pyrazine-9 (2H) -yl) -3-fluoropyrazole [1, 5-a] pyridine-7-nitrile

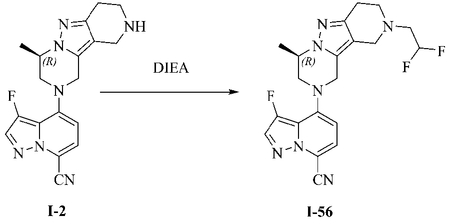

A reaction mixture of **Example I-2** (100 mg, 0.285 mmol), DIEA (184 mg, 1.423 mmol) and 2,2-difluoroethyl trifluoromethanesulfonate (91 mg, 0.427 mmol) in THF (3 mL) was stirred at 25 °C for 12 hours. The mixture was quenched with water then extracted with EtOAc, and the organic phase was washed with saturated saline, dried with Na₂SO₄ and concentrated. Purification by flash chromatography gave **Example I-56** (7.7 mg) as white solid. MS: 416.1 (M+H)⁺. ¹H NMR (400 MHz, DMSO-*d₆*) δ 8.29 (d, *J =* 3.6 Hz, 1H), 7.72 (d, *J* = 8.0 Hz, 1H), 6.67 (d, *J* = 8.0 Hz, 1H), 6.17 (tt, *J* = 55.6, 4.4 Hz, 1H), 4.51 - 4.30 (m, 3H), 3.98 (dd, *J =* 13.6, 4.0 Hz, 1H), 3.63 - 3.46 (m, 2H), 3.35 (s, 1H), 2.97 - 2.77 (m, 4H), 2.68 - 2.59 (m, 2H), 1.46 (d, *J =* 6.4 Hz, 3H).

### Example I-57

### (R)-3-Fluoro-4-(7-methyl-2-(tetrahydro-2H-Pyramum-4-yl)-1,3,4,7,8,10-hexahydro pyridine[4',3':3,4]pyrazole[1,5-a]pyrazine-9(2H)-yl)pyrazole[1,5-a]pyridine-7-nitrile

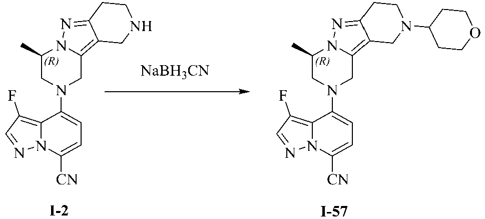

**Example I-57** was synthesized by following the protocols of **Example I-24.** MS: 436.1 (M+H)⁺. ¹H NMR (400 MHz, DMSO-*d₆*) δ 8.32 (d, J = 3.6 Hz, 1H), 7.75 (d, J = 8.0 Hz, 1H), 6.69 (d, J = 8.0 Hz, 1H), 4.52 (d, J = 15.2 Hz, 1H), 4.40 (d, J = 15.2 Hz, 2H), 3.97 (ddd, J = 28.0, 12.4, 4.0 Hz, 3H), 3.60 (d, J = 13.2 Hz, 2H), 3.51 (s, 3H), 2.92 - 2.75 (m, 2H), 2.72 - 2.60 (m, 3H), 1.77 (dd, J = 12.8, 3.6 Hz, 2H), 1.60 - 1.50 (m, 2H), 1.48 (d, J = 6.4 Hz, 3H).

### Example I-58

### (R)-4-(2-(3, 3-difluorocyclobutyl)-7-methyl-1,3, 4, 7, 8, 10-hexahydropyridine [4 ', 3 ': 3, 4] pyrazole [1, 5-a] pyrazine-9 (2H) -yl) -3-fluoropyrazole [1, 5-a] pyridine-7-nitrile

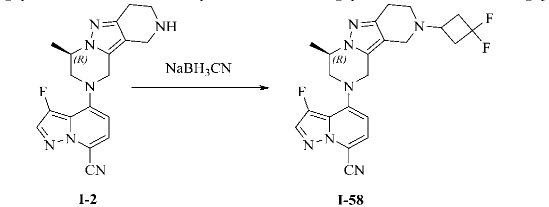

**Example I-58** was synthesized by following the protocols of **Example I-24.** MS: 442.1 (M+H)⁺. ¹H NMR (400 MHz, DMSO-*d₆*) δ 8.32 (d, J = 3.6 Hz, 1H), 7.74 (d, J = 8.0 Hz, 1H), 6.70 (d, J = 8.0 Hz, 1H), 4.52 (d, J = 15.2 Hz, 1H), 4.46 - 4.34 (m, 2H), 4.02 (dd, J *=* 14.0, 4.1 Hz, 1H), 3.46 (s, 2H), 3.34 - 3.23 (m, 2H), 2.91 (dd, J = 7.2, 3.2 Hz, 1H), 2.80 (dd, J = 13.2, 8.0 Hz, 2H), 2.72 - 2.57 (m, 4H), 2.47 (t, J = 6.4 Hz, 1H), 1.49 (d, J = 6.4 Hz, 3H).

### Example I-59

### 3-Fluoro-4-((4R,7S,9R)-4,7,9-trimethyl-3,4,7,8,9,10-hexahydropyrido[3',4':3,4] pyrazolo[1,5-a]pyrazin-2(1H)-yl)pyrazolo[1,5-a]pyridine-7-carbonitrile

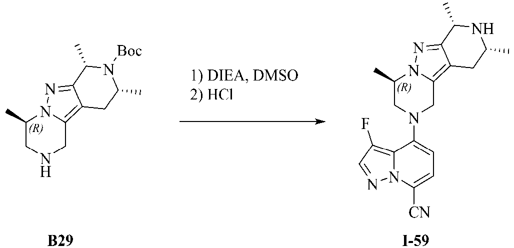

**Example I-59** was synthesized by following the protocols of **Example I-2**. MS: 380.2 (M+H)⁺. ¹H NMR (400 MHz, DMSO-*d*₆) δ 8.33 (dd, *J=* 3.6, 1.3 Hz, 1H), 7.76 (dd, *J* = 8.0, 3.0 Hz, 1H), 6.75 - 6.70 (m, 1H), 4.61 - 4.40 (m, 4H), 4.01 (d, *J* = 10.3 Hz, 1H), 3.56 (d, *J=* 5.4 Hz, 2H), 2.94 - 2.79 (m, 1H), 2.56 -2.48 (m, 1 H), 1.57 (dd, *J* = 6.7, 2.5 Hz, 3H), 1.50 (t, *J* = 6.9 Hz, 3H), 1.41 (d, *J* = 6.3 Hz, 3H).

### Examples I-59a and I-59b

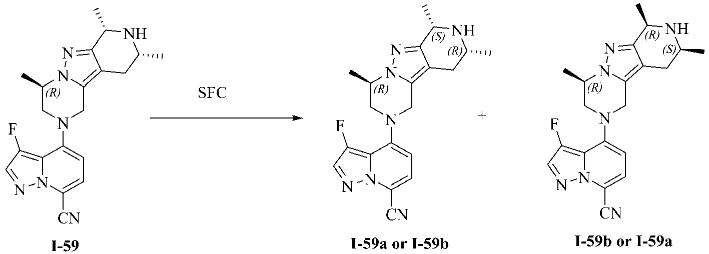

**Examples I-59a** and **I-59a** were separated through chiral resolution by SFC. The first component peak is obtained which is all white solid **Example I-59a** (retention time, 1.375 minute) and the second component peak **Example I-59b** (retention time: 1.878 minute). MS: 380.1 (M+H)⁺.

### Example I-60

### 3-Fluoro-4-((4R)-9-isopropyl-4-methyl-3,4,7,8,9,10-hexahydropyrido[3',4':3,4] pyrazolo[1,5-a]pyrazin-2(1H)-yl)pyrazolo[1,5-a]pyridine-7-carbonitrile

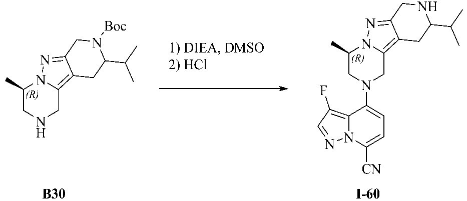

**Example I-60** was synthesized by following the protocols of **Example I-2**. MS: 394.2 (M+H)⁺. ¹H NMR (400 MHz, DMSO-*d*₆) δ 9.09 (s, 2H), 8.34 (d, *J* = 3.5 Hz, 1H), 7.77 (d, *J* = 8.0 Hz, 1H), 6.73 (d, *J* = 8.1 Hz, 1H), 4.67 - 4.57 (m, 1H), 4.53 - 4.41 (m, 2H), 4.36 - 4.25 (m, 2H), 4.10 - 4.00 (m, 1H), 3.42 - 3.36 (m, 2H), 2.92 - 2.75 (m, 1H), 2.70 - 2.55 (m, 1H), 2.20 - 2.10 (m, 1H), 1.51 (d, *J* = 6.3 Hz, 3H), 1.06 (d, *J* = 6.8 Hz, 3H), 1.02 (d, *J* = 6.8 Hz, 3H).

### Example I-61

### 4-((4R)-9-(2-(tert-butoxy)ethyl)-4-methyl-3,4,7,8,9,10-hexahydropyridine[3',4':3,4] pyrazole[1,5-a]pyrazine-2(1H)-yl)-3-fluoropyrazole[1,5-a]pyridine-7-caronitrile

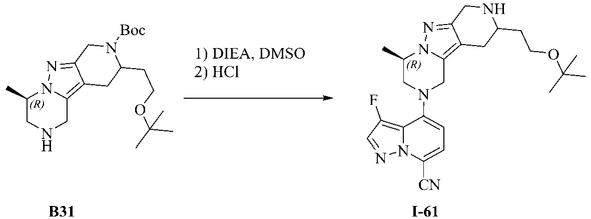

**Example I-61** was synthesized by following the protocols of **Example I-2**. MS: 452.2 (M+H)⁺. ¹H NMR (400 MHz, DMSO-*d*₆) δ 8.37 - 8.28 (m, 2H), 7.74 (d, *J* = 8.0 Hz, 1H), 6.72 (d, *J* = 8.0, 1H), 4.55 - 4.43 (m, 1H), 4.41 - 4.32 (m, 2H), 4.06 - 3.97 (m, 1H), 3.91 - 3.83 (m, 1H), 3.78 - 3.72 (m, 1H), 3.49 - 3.45 (m, 2H), 3.36 - 3.31 (m, 1H), 2.86 - 2.78 (m, 1H), 2.63 - 2.56 (m, 1H), 2.22-2.05 (m, 1H), 1.74 - 1.55 (m, 2H), 1.48 (d, *J* = 6.4 Hz, 3H), 1.16 (d, *J* = 2.0 Hz, 9H).

### Example I-62

### (R)-4-(2-(1H-Pyrazole-5-yl)methyl)-7-methyl-1,3,4,7,8,10-hexahydropyridine [4 ', 3 ': 3, 4] pyrazole [1, 5-a] pyrazine-9 (2H) -yl) -3-fluoropyrazole [1, 5-a] pyridine-7-nitrile

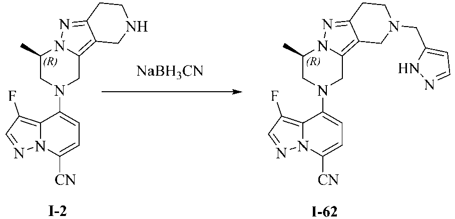

**Example I-62** was synthesized by following the protocols of **Example I-24.** MS: 432.1 (M+H)⁺. ¹H NMR (400 MHz, DMSO-*d₆*) δ 8.31 (d, *J* = 3.6 Hz, 1H), 7.72 (d, *J* = 8.0 Hz, 1H), 7.58 (s, 1H), 6.70 (d, *J* = 8.0 Hz, 1H), 6.19 (d, *J* = 2.0 Hz, 1H), 4.49 - 4.37 (m, 2H), 4.36 - 4.30 (m, 1H), 4.05 - 3.95 (m, 1H), 3.80 - 3.65 (m, 2H), 3.45 - 3.40 (m, 3H), 2.78 - 2.68 (m, 2H), 2.67 - 2.60 (m, 2H), 1.47 (d, *J* = 6.4 Hz, 3H).

### Example I-63

### (R) 3-Fluoro-4- (7-methyl-2- ((1-methyl-1H-1, 2, 3-triazol-4-yl) methyl) -1, 3, 4, 7, 8, 10-hydropyridine and [4', 3 ': 3, 4] pyrazole [1, 5-a] pyrazine-9 (2 H) -yl) pyrazole [1, 5-a] pyridine-7-nitrile

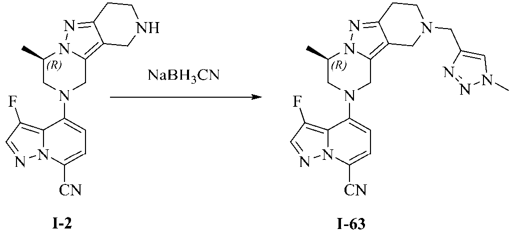

**Example I-63** was synthesized by following the protocols of **Example I-24.** MS: 447.1 (M+H)⁺. ¹H NMR (400 MHz, DMSO-*d₆*) δ 8.28 (d, *J* = 3.6 Hz, 1H), 7.96 (s, 1H), 7.70 (d, *J* = 8.0 Hz, 1H), 6.67 (d, *J* = 8.0 Hz, 1H), 4.47 - 4.28 (m, 3H), 4.02 (s, 3H), 3.97 (dd, *J =* 13.6, 4.1 Hz, 1H), 3.75 (s, 2H), 3.44 (d, *J* = 13.6 Hz, 1H), 3.34 (d, *J* = 13.6 Hz, 1H), 3.30 - 3.25 (m, 1H), 2.80 - 2.58 (m, 4H), 1.45 (d, *J* = 6.4 Hz, 3H).

### Example I-64

### (R)-3-Fluoro-4-(7-methyl-2-((1-methyl-1H-pyrazolyl-3-yl)methyl)-1,3,4,7,8,10-hexahydropyridine[4',3':3,4]pyrazole[1,5-a]pyrazine-9(2H)-yl)pyrazole[1,5-a] pyridine-7-nitrile

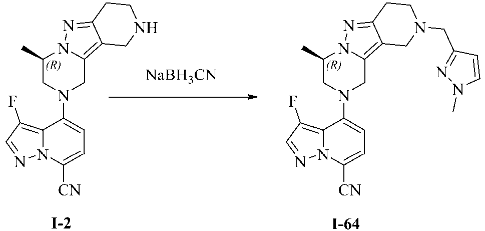

**Example I-64** was synthesized by following the protocols of **Example I-24.** MS: 446.1 (M+H)⁺. ¹H NMR (400 MHz, DMSO-*d₆*) δ 8.28 (d, *J* = 3.6 Hz, 1H), 7.70 (d, *J* = 8.0 Hz, 1H), 7.60 (d, *J* = 2.0 Hz, 1H), 6.67 (d, *J* = 8.0 Hz, 1H), 6.14 (d, *J* = 2.0 Hz, 1H), 4.46 - 4.31 (m, 3H), 4.00 - 3.93 (m, 1H), 3.79 (s, 3H), 3.61 (d, *J =* 3.2 Hz, 2H), 3.40 (d, *J* = 13.6 Hz, 1H), 3.33 (d, *J* = 8.4 Hz, 1H), 3.29 (d, *J* = 4.4 Hz, 1H), 2.75 - 2.59 (m, 4H), 1.45 (d, *J* = 6.4 Hz, 3H).

### Example I-65

### (R)-4-(2-((1H-1,2,3-triazolyl-4-yl)methyl)-7-methyl-1,3,4,7,8,10-hexahydropyridine [4',3':3,4]pyrazole[1,5-a]pyrazine-9(2H)-yl)-3-fluoropyrazole[1,5-a]pyridine-7-nitrile

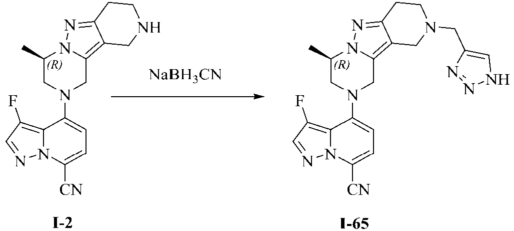

**Example I-65** was synthesized by following the protocols of **Example I-24.** MS: 433.1 (M+H)⁺. ¹H NMR (400 MHz, DMSO-*d₆*) δ 14.84 (s, 1H), 8.29 (d, *J* = 3.6 Hz, 1H), 7.70 (d, *J* = 8.0 Hz, 1H), 6.67 (d, *J* = 8.0 Hz, 1H), 4.48 - 4.28 (m, 3H), 4.01 - 3.93 (m, 1H), 3.79 (s, 2H), 3.42 (d, *J* = 13.6 Hz, 1H), 3.35 (s, 1H), 3.28 (d, *J* = 12.4 Hz, 1H), 2.78 - 2.60 (m, 4H), 1.45 (d, *J* = 6.4 Hz, 3H).

### Example I-66

### (R)-3-Fluoro-4- (7-methyl-2- ((3-methyl-1H-pyrazolyl-4-yl) methyl) -1, 3, 4, 7, 8, 10-hexahydropyridine [4 ', 3 ': 3, 4] pyrazole [1, 5-a] pyrazine-9 (2 H) -yl) pyrazole [1, 5-a] pyridine-7-nitrile

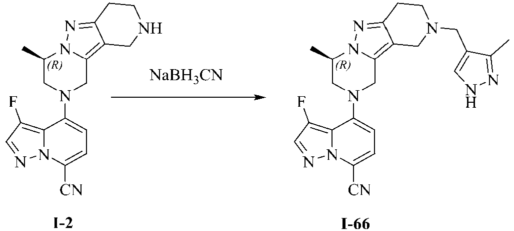

**Example I-66** was synthesized by following the protocols of **Example I-24.** MS: 446.1 (M+H)⁺. ¹H NMR (400 MHz, DMSO-*d₆*) δ 8.31 (d, *J* = 3.6 Hz, 1H), 7.72 (d, *J* = 8.0 Hz, 1H), 7.42 (s, 1H), 6.70 (d, *J* = 8.0 Hz, 1H), 4.55 - 4.43 (m, 1H), 4.42 - 4.33 (m, 2H), 4.06 - 3.94 (m, 1H), 3.60 - 3.50 (m, 5H), 2.76 - 2.68 (m, 2H), 2.66 - 2.60 (m, 2H), 2.20 (s, 3H), 1.48 (d, *J* = 6.0 Hz, 3H).

### Example I-67

### (R)-3-Fluoro-4-(7-methyl-2-((1-methyl-1H-pyrazol-4-yl)methyl)-1,3,4,7,8,10-hexahydropyrido[4',3':3,4]pyrazolo[1,5-a]pyrazin-9(2H)-yl)pyrazolo[1,5-a]pyridine-7-carbonitrile

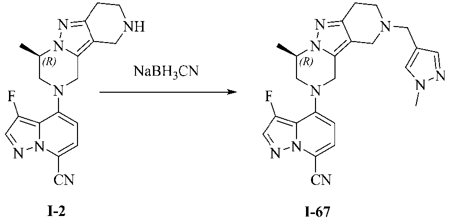

**Example I-67** was synthesized by following the protocols of **Example 1-24.** MS: 446.1 (M+H)⁺. ¹H NMR (400 MHz, DMSO-*d₆*) δ 8.31 (d, *J* = 3.6 Hz, 1H), 7.73 (d, *J* = 8.0 Hz, 1H), 7.62 (s, 1H), 7.36 (s, 1H), 6.70 (d, *J* = 8.0 Hz, 1H), 4.50 - 4.33 (m, 3H), 4.03 - 3.92 (m, 1H), 3.82 (s, 3H), 3.61 - 3.49 (m, 5H), 2.76 - 2.67 (m, 2H), 2.66 - 2.61 (m, 2H), 1.48 (d, *J* = 6.0 Hz, 3H).

### Example I-68

### 3-Fluoro-4-((4R)-4-methyl-9-(1H-pyrazol-5-yl)-3,4,7,8,9,10-hexahydropyrido[3',4':3,4]pyrazolo[1,5-a]pyrazin-2(1H)-yl)pyrazolo[1,5-a]pyridine-7-carbonitrile

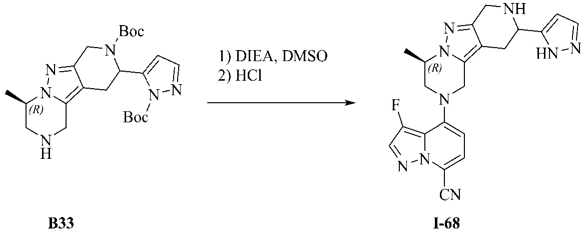

**Example I-68** was synthesized by following the protocols of **Example I-2**. MS: 418.2 (M+H)⁺. ¹H NMR (400 MHz, DMSO-*d*₆) δ 8.32 (d, *J* = 3.5 Hz, 1H), 8.21 (s, 1H), 7.75 (d, *J=* 8.0 Hz, 1H), 7.62 (s, 1H), *J=* 8.1, 2.7 Hz, 1H), 6.28 (dd, *J* = 3.6, 2.0 Hz, 1H), 4.56 - 4.52 (m, 1H), 4.50 - 4.35 (m, 2H), 4.15 - 4.01 (m, 2H), 4.00 - 3.92 (m, 2H), 3.40 - 3.34 (m, 2H), 2.95 - 2.83 (m, 1H), 2.77 - 2.60 (m, 1H), 1.50 (d, *J* = 6.3 Hz, 3H).

### Example I-69

### 3-Fluoro-4-((4R)-7-isobutyl-4-methyl-3,4,7,8,9,10-hexahydropyrido[3',4':3,4]pyrazolo [1,5-a]pyrazin-2(1H)-yl)pyrazolo[1,5-a]pyridine-7-carbonitrile

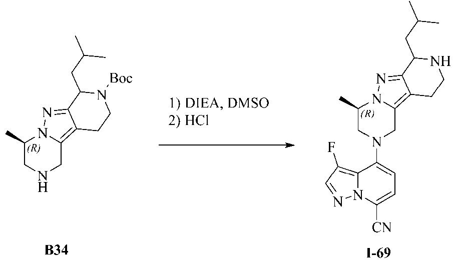

**Example I-69** was synthesized by following the protocols of **Example I-2**. MS: 408.2 (M+H)⁺. ¹H NMR (400 MHz, DMSO-*d₆*) δ 8.32 (d, *J* = 3.5 Hz, 1H), 7.75 (dd, *J* = 8.0, 2.3 Hz, 1H), 6.74 - 6.71 (m, 1H), 4.57 - 4.39 (m, 3H), 4.09-4.04(m, 2H), 4.01 -3.99 (m 1H), 3.39 - 3.23 (m, 2H), 2.97 - 2.87 (m, 1H), 2.58 - 2.54 (m, 2H), 2.08 -2.03 (m, 1H), 1.80 - 1.74 (m, 1H), 1.58 -1.55 (m, 1H), 1.50 - 1.48 (m, 3H), 0.97 (d, *J* = 6.5 Hz, 6H).

### Example I-70

### 3-Fluoro-4-((4R)-7-isopropyl-4-methyl-3,4,7,8,9,10-hexahydropyrido[3',4':3,4] pyrazolo[1,5-a]pyrazin-2(1H)-yl)pyrazolo[1,5-a]pyridine-7-carbonitrile

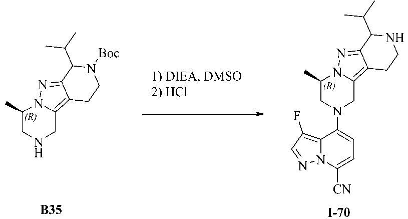

**Example I-70** was synthesized by following the protocols of **Example I-2**. MS: 394.2 (M+H)⁺. ¹H NMR (400 MHz, DMSO-*d₆*) δ 8.33-8.28 (m, 1H), 7.76-7.74 (m, 1H), 6.74-6.70 (m, 1H), 4.53 - 4.35 (m, 3H), 4.06 - 4.00 (m, 1H), 3.78 (d, *J* = 4.1 Hz, 2H), 3.39-3.33 (m, 1H), 3.26 - 3.15 (m, 1H), 2.85 - 2.75 (m, 1H), 2.52-2.49 (m, 2H), 2.28 - 2.11 (m, 1H), 1.50 -1.48 (m, 3H), 1.05 (t, *J* = 7.4 Hz, 3H), 0.97 - 0.90 (m, 3H).

### Example I-71

### 4-((4R)-8-amino-4,8-dimethyl-3,4,7,8,9,10-hexahydropyrazino[1,2-b]indazol-2(1H)-yl)-3-fluoropyrazolo[1,5-a]pyridine-7-carbonitrile

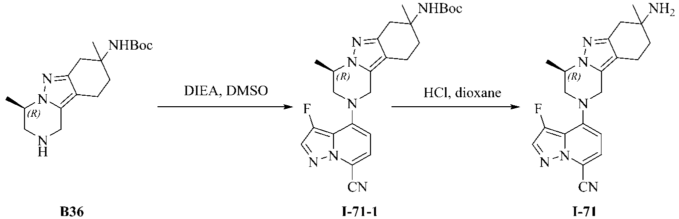

**Example I-71** was synthesized by following the protocols of **Example I-9.** MS: 380.1 (M+H)⁺. ¹H NMR (400 MHz, DMSO-*d₆*) δ 8.45 (s, 1H), 8.33 (d, *J* = 3.5 Hz, 1H), 7.75 (dd, *J* = 8.0, 1.1 Hz, 1H), 6.73 (d, *J* = 8.1 Hz, 1H), 4.54 - 4.32 (m, 3H), 4.02 (d, J = 11.4 Hz, 1H), 3.41 - 3.26 (m, 1H), 2.70 (d, *J* = 5.4 Hz, 2H), 2.66 - 2.55 (m, 1H), 2.52 - 2.34 (m, 1H), 1.79 (s, 2H), 1.49 (dd, *J* = 6.4, 2.0 Hz, 3H), 1.24 (d, *J* = 4.2 Hz, 3H).

### Examples I-71a and I-71b

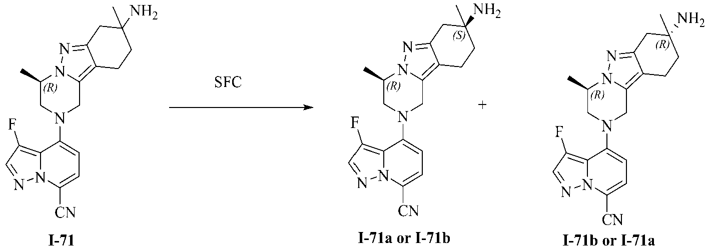

**Examples I-71a** and **I-71b** were obtained by SFC chiral separation, mobile phase A: MTBE (0.5% 2mM NH₃-MeOH), B: MeOH: DCM. **Example I-71a** was obtained as the first peak component (white solid, retention time: 1.152 minute) and the second component was obtained as **Example I-71b** (retention time: 1.78 minute). MS: 380.1 (M+H)⁺.

### Example I-72

### 3-Fluoro-4- ((7R) 7-methyl-2-(1-(1-methyl-1H-pyridox-4-yl) ethyl) -1, 3, 4, 7, 8, 10-hydropyridox and [4', 3 ': 3, 4] pyrazole [1, 5-a] pyrazine-9 (2 H) -yl) pyrazole [1, 5-a] pyridine-7-nitrile

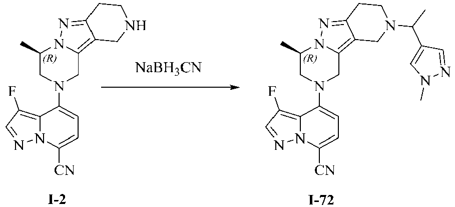

**Example I-72** was synthesized by following the protocols of **Example I-24.** MS: 460.3 (M+H)⁺. ¹H NMR (400 MHz, DMSO-*d₆*) δ 8.29 (d, J = 3.6 Hz, 1H), 7.72 (d, J = 8.0 Hz, 1H), 7.62 (s, 1H), 7.37 (s, 1H), 6.66 (dd, J = 8.0, 1.6 Hz, 1H), 4.49 - 4.31 (m, 3H), 3.97 (d, J = 12.4 Hz, 2H), 3.80 (d, J = 2.0 Hz, 3H), 3.51 (d, J = 13.2 Hz, 2H), 3.28 (s, 1H), 2.69 (d, J = 45.2 Hz, 4H), 1.44 (d, J = 6.4 Hz, 3H), 1.38 (d, J = 6.8 Hz, 3H).

### Example I-73

### (R)-3-fluoro-4-(7-methyl-2-((1-methyl-1H-1,2,3-triazol-5-yl)methyl)-1,3,4,7,8,10-hexahydropyrido[4',3':3,4]pyrazolo[1,5-a]pyrazin-9(2H)-yl)pyrazolo[1,5-a]pyridine-7-carbonitrile

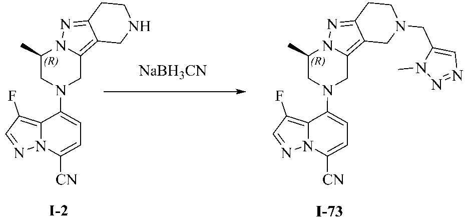

**Example I-73** was synthesized by following the protocols of **Example I-24.** MS: 447.4 (M+H)⁺. ¹H NMR (400 MHz, DMSO-*d₆*) δ 8.29 (d, *J* = 3.6 Hz, 1H), 7.70 (d, *J* = 8.0 Hz, 1H), 7.65 (s, 1H), 6.67 (d, *J* = 8.0 Hz, 1H), 4.47 - 4.31 (m, 3H), 3.99 (s, 3H), 3.95 (d, *J =* 4.0 Hz, 1H), 3.80 (s, 2H), 3.42 (d, *J =* 13.2 Hz, 1H), 3.36 (s, 1H), 3.31 - 3.25 (m, 1H), 2.71 (d, *J* = 5.6 Hz, 2H), 2.64 (d, *J* = 5.6 Hz, 2H), 1.45 (d, *J* = 6.4 Hz, 3H).

### Example I-74

### 3-Fluoro-4-((4R)-9-(2-hydroxypropan-2-yl)-4-methyl-3,4,7,8,9,10-hexahydropyrido [3',4':3,4]pyrazolo[1,5-a]pyrazin-2(1H)-yl)pyrazolo[1,5-a]pyridine-7-carbonitrile

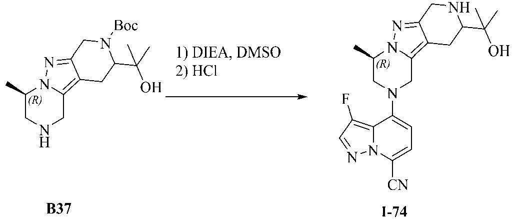

**Example I-74** was synthesized by following the protocols of **Example I-2**. MS: 410.2 (M+H)⁺. ¹H NMR (400 MHz, DMSO-*d₆*) δ 8.33 (d, *J* = 3.6 Hz, 1H), 7.76 (d, *J* = 8.0 Hz, 1H), 6.73 (d, *J* = 8.1 Hz, 1H), 4.61 - 4.36 (m, 3H), 4.04 - 3.93 (m, 2H), 3.87 - 3.82 (m, 1H), 3.40 - 3.34 (m, 2H), 2.73 - 2.60 (m, 2H), 2.39 - 2.22 (m, 1H), 1.49 (d, *J* = 6.3 Hz, 3H), 1.20 (d, *J =* 14.7 Hz, 6H).

### Example I-75

### 4-((7R)-2-(1-(1H-pyrazol-4-yl)ethyl)-7-methyl-1,3,4,7,8,10-hexahydropyrido[4',3':3,4] pyrazolo[1,5-a]pyrazin-9(2H)-yl)-3-fluoropyrazolo[1,5-a]pyridine-7-carbonitrile

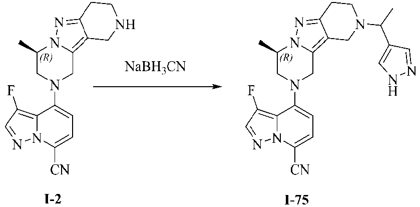

**Example I-75** was synthesized by following the protocols of **Example I-24.** MS: 446.4 (M+H)⁺. ¹H NMR (400 MHz, DMSO-*d₆*) δ 8.32 (d, *J* = 3.5 Hz, 1H), 7.74 (d, *J* = 8.0 Hz, 1H), 7.53 (s, 2H), 6.70 (dd, *J* = 8.1, 2.8 Hz, 1H), 4.52 - 4.31 (m, 3H), 4.03 - 3.89 (m, 2H), 3.46 (d, *J* = 9.7 Hz, 2H), 3.29 (d, *J =* 2.7 Hz, 1H), 2.77 - 2.56 (m, 4H), 1.47 (d, *J* = 6.3 Hz, 3H), 1.39 (d, *J* = 6.7 Hz, 3H).

### Example I-76

### (S)-3-Fluoro-4-(4-methyl-3,4,7,8,9,10-hexahydropyrido[3',4':3,4]pyrazolo[1,5-a]pyrazin-2(1H)-yl)pyrazolo[1,5-a]pyridine-7-carbonitrile

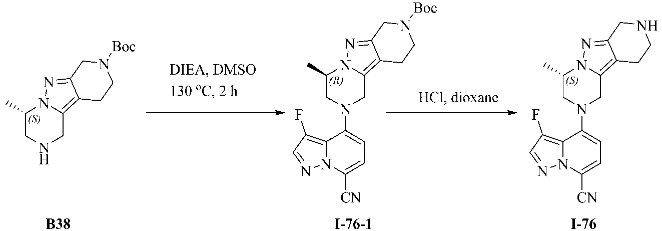

**Example I-76** was synthesized by following the protocols of **Example I-2**. MS: 352.1 (M+H)⁺. ¹H NMR (400 MHz, DMSO-*d₆*) δ 9.20 (s, 2H), 8.33 (d, *J* = 3.6 Hz, 1H), 7.78 (d, *J* = 8.0 Hz, 1H), 6.72 (d, *J* = 8.1 Hz, 1H), 4.62 - 4.41 (m, 3H), 4.32 (dd, *J* = 12.7, 4.6 Hz, 1H), 4.26 (s, 2H), 4.16 (d, *J =* 12.5 Hz, 1H), 3.40 (d, *J =* 21.5 Hz, 2H), 2.88 - 2.69 (m, 2H), 1.16 (d, *J* = 6.6 Hz, 3H).

### Example I-77

### 3-Fluoro-4-((4R,9R)-9-(hydroxymethyl)-4-methyl-3,4,7,8,9,10-hexahydropyrido [3',4':3,4]pyrazolo[1,5-a]pyrazin-2(1H)-yl)pyrazolo[1,5-a]pyridine-7-carbonitrile

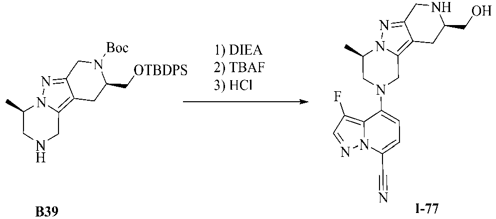

**Example I-77** was synthesized by following the protocols of **Example I-33.** MS: 382.1 (M+H)⁺. ¹H NMR (400 MHz, DMSO-*d*₆) δ 9.10 (s, 1H), 8.34 (d, *J* = 3.6 Hz, 1H), 7.77 (d, *J* = 8.0 Hz, 1H), 6.73 (d, *J* = 8.0 Hz, 1H), 5.58 (t, *J* = 4.8 Hz, 1H), 4.65 - 4.55 (m, 1H), 4.51 - 4.39 (m, 2H), 4.26 (s, 2H), 4.10 - 4.00 (m, 1H), 3.85 - 3.75 (m, 1H), 3.68 - 3.58 (m, 1H), 3.55 - 3.45 (m, 1H), 3.44 - 3.39 (m, 1H), 2.88 - 2.78 (m, 1H), 2.64 - 2.57 (m, 1H), 1.51 (d, *J* = 6.4 Hz, 3H).

### Example I-78

### (R)-3-Fluoro-4-(3,3,7-trimethyl-1,3,4,7,8,10-hexahydropyrido[4',3':3,4]pyrazolo [1,5-a]pyrazin-9(2H)-yl)pyrazolo[1,5-a]pyridine-7-carbonitrile

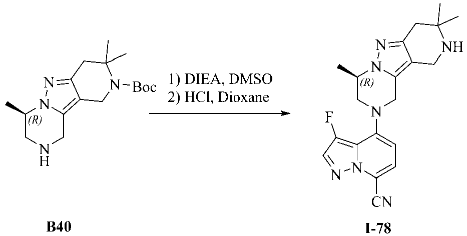

**Example I-78** was synthesized by following the protocols of **Example I-2.** MS: 380.1 (M+H)⁺. ¹H NMR (400 MHz, DMSO-*d₆*) δ 8.34 (s, 1H), 8.29 (d, *J* = 3.6 Hz, 1H), 7.72 (d, *J* = 8.0 Hz, 1H), 6.67 (d, *J =* 8.0 Hz, 1H), 4.47 (d, *J =* 31.6 Hz, 2H), 4.38 - 4.34 (m, 1H), 3.99 (dd, *J =* 13.6, 4.0 Hz, 1H), 3.85 (d, *J =* 16.0 Hz, 2H), 3.34 (dd, *J =* 13.67, 9.2 Hz, 1H), 2.56 (s, 2H), 1.47 (d, *J =* 6.4 Hz, 3H), 1.20 (d, *J =* 3.2 Hz, 6H).

### Example I-79

### 4-((4R)-9-((1H-pyrazol-5-yl)methyl)-4-methyl-3,4,7,8,9,10-hexahydropyrido[3',4':3,4] pyrazolo[1,5-a]pyrazin-2(1H)-yl)-3-fluoropyrazolo[1,5-a]pyridine-7-carbonitrile

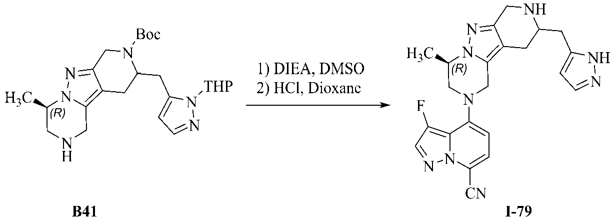

**Example I-79** was synthesized by following the protocols of **Example I-2.** MS: 432.1 (M+H)⁺. ¹H NMR (400 MHz, DMSO-*d₆*) δ 8.33 (dd, *J =* 3.6, 1.4 Hz, 1H), 8.19 (s, 1H), 7.74 (d, *J* = 8.0 Hz, 1H), 7.59 (s, 1H), 6.73 (dd, *J* = 8.1, 2.9 Hz, 1H), 6.18 (d, *J* = 2.0 Hz, 1H), 4.55 - 4.45 (m, 1H), 4.44 - 4.35 (m, 2H), 4.07 - 3.97 (m, 2H), 3.95 - 3.90 (m, 1H), 3.40 - 3.35 (m, 1H), 3.30 - 3.25 (m, 1H), 3.00 - 2.92 (m, 1H), 2.87 - 2.78 (m, 1H), 2.65 - 2.58 (m, 1H), 2.35 - 2.22 (m, 1H), 1.49 (d, *J* = 6.3 Hz, 3H).

### Example I-80

### 4-((4R)-9-Amino-4,9-dimethyl-3,4,7,8,9,10-hexahydropyrazino[1,2-b]indazol-2(1H)-yl)-3-fluoropyrazolo[1,5-a]pyridine-7-carbonitrile

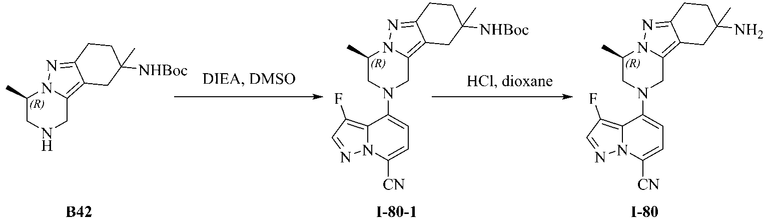

**Example I-80** was synthesized by following the protocols of **Example I-5.** MS: 380.1 (M+H)⁺. ¹H NMR (400 MHz, DMSO-*d₆*) δ 8.33 (d, *J* = 3.5 Hz, 1H), 8.11 (d, *J* = 21.5 Hz, 3H), 7.76 (dd, *J* = 8.0, 1.6 Hz, 1H), 6.75 (d, *J* = 8.1 Hz, 1H), 4.59 - 4.48 (m, 1H), 4.47 - 4.33 (m, 2H), 4.09 - 3.97 (m, 1H), 3.34 (d, *J* = 9.4 Hz, 1H), 2.78 - 2.58 (m, 4H), 2.03 - 1.85 (m, 2H), 1.50 (dd, *J* = 6.3, 1.9 Hz, 3H), 1.32 (d, *J* = 12.4 Hz, 3H).

### Examples I-80a and I-80b

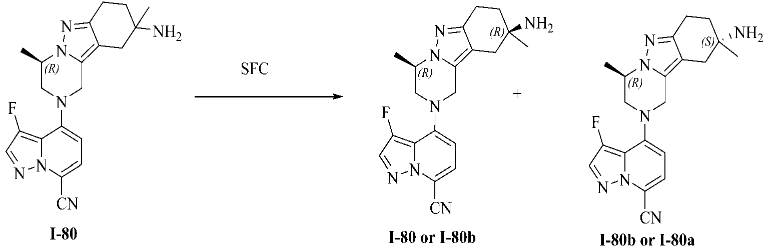

**Examples I-80a** and **I-80b** were separated through chiral resolution by SFC mobile phase A: MTBE (0.5% 2mM NH₃-MeOH), B: MeOH: DCM. **Example I-80a** was obtained as the first component (retention time: 1.736 minute) and the second component was **Example I-80b** (retention time: 2.372 minute). MS: 380.1 (M+H)⁺.

### Example I-81

### 4-((7R)-1,7-dimethyl-1,3,4,7,8,10-hexahydropyrido[4',3':3,4]pyrazolo[1,5-a]pyrazin-9(2H)-yl)-3-fluoropyrazolo[1,5-a]pyridine-7-carbonitrile

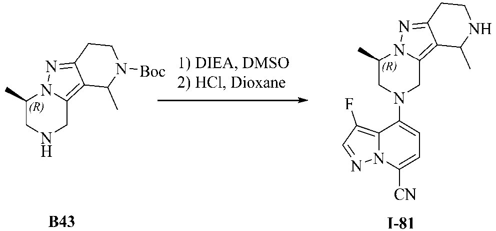

**Example I-81** was synthesized by following the protocols of **Example I-2.** MS: 366.1 (M+H)⁺. ¹H NMR (400 MHz, DMSO-*d₆*) δ 8.33 (d, *J* = 3.6 Hz, 1H), 7.77 (d, *J* = 8.0 Hz, 1H), 6.80 (d, *J =* 8.1 Hz, 1H), 4.71 - 4.67 (m, 1H), 4.57 - 4.48 (m, 2H), 4.44 - 4.38(m, 1H), 4.11 - 4.03 (m, 1H), 3.62 - 3.54 (m, 1H), 3.34 - 3.29 (m, 2H), 2.93 - 2.82 (m, 2H), 1.54 - 1.49 (m, 6H).

### Example I-82

### 3-Fluoro-4-((4R,9R)-9-(methoxymethyl)-4-methyl-3,4,7,8,9,10-hexahydropyrido [3',4':3,4]pyrazolo[1,5-a]pyrazin-2(1H)-yl)pyrazolo[1,5-a]pyridine-7-carbonitrile

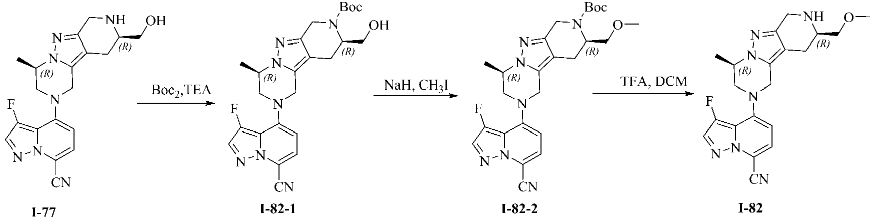

### Step 1: Preparation of intermediate I-82-1

A reaction mixture of **Example I-77** (100 mg, 0.262 mmol), TEA (133 mg) and Boc₂O (114 mg, 0.524 mmol) in MeOH (2 mL) was stirred at 25 °C for 3 hours. The mixture was quenched with water then extracted with EtOAc, and the organic phase was washed with saturated saline, dried with Na₂SO₄ and concentrated. Purification by flash chromatography gave **Intermediate I-82-1** (90 mg, 71.3%) as white solid. MS: 482.2 (M+H)⁺.

### Step 2: Preparation of intermediate I-82-2

A reaction mixture of **Intermediate I-82-1** (50 mg, 0.104 mmol) and NaH (3.74 mg, 0.156 mmol) in THF (5 mL) was stirred at 25 °C for 0.5 hour before MeI (29.5 mg, 0.208 mmol) added to the mixture. The resulting mixture was stirred at 25 °C for 12 hours. The mixture was quenched with water then extracted with EtOAc, and the organic phase was washed with saturated saline, dried with Na₂SO₄ and concentrated. Purification by flash chromatography gave **Intermediate I-82-2** (38.9 mg, 38.9%) as white solid. MS: 496.2 (M+H)⁺.

### Step 3: Preparation of Example I-82

A reaction mixture of **Intermediate I-82-2** (20 mg, 0.04 mmol) and TFA (2 mL) in DCM (2 mL) was stirred at 25 °C for 2 hours. The mixture was quenched with water and extracted with EtOAc, and the organic phase was washed with saturated saline, dried with Na₂SO₄ and concentrated. Purification by pre-HPLC gave **Example I-82** (11.2 mg, 70.2%) as white solid. MS: 395.9 (M+H)⁺. ¹H NMR (400 MHz, DMSO-*d₆*) δ 8.32 (d, *J* = 3.5 Hz, 1H), 7.74 (d, *J* = 8.0 Hz, 1H), 6.71 (d, *J=* 8.1 Hz, 1H), 4.55-4.51 (m, 1H),4.43-4.32 (m, 2H), 4.02 (dd, *J* = 13.7, 4.2 Hz, 1H), 3.91-3.87(m 1H), 3.81-3.77(m, 1H), 3.43-3.40(m, 2H),3.38-3.36 (m, 1H), 3.35-3.34 (m, 1H), 3.33 (s, 3H), 3.03-2.96 (m, , 1H), 2.60 - 2.54 (m, 1H), 2.18-2.12 (m, 1H), 1.48 (d, *J =* 6.3 Hz, 3H).

### Example I-83

### (R)-3-Fluoro-4-(4,9,9-trimethyl-3,4,7,8,9,10-hexahydropyrido[3',4':3,4]pyrazolo [1,5-a]pyrazin-2(1H)-yl)pyrazolo[1,5-a]pyridine-7-carbonitrile

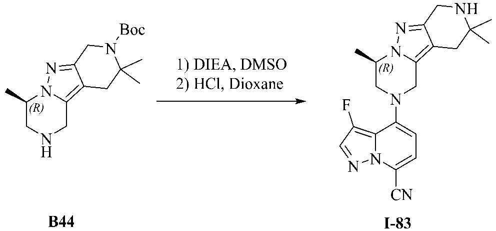

**Example I-83** was synthesized by following the protocols of **Example I-2.** MS: 380.2 (M+H)⁺. ¹H NMR (400 MHz, DMSO-*d₆*) δ 8.35 - 8.29 (m, 1H), 7.75 (d, *J* = 8.0 Hz, 1H), 6.70 (d, *J* = 8.1 Hz, 1H), 4.49 - 4.45(m, 1H), 4.43 - 4.34 (m, 2H), 4.07 - 3.98 (m, 1H), 3.83 (s, 2H), 3.39 - 3.33 (m, 1H), 2.38 (q, *J =* 15.1 Hz, 2H), 1.49 (d, *J =* 6.3 Hz, 3H), 1.16 (s, 3H), 1.13 (s, 3H).

### Example I-84

### 3-Fluoro-4-((7R)-7-methyl-1-(1H-pyrazol-5-yl)-1,3,4,7,8,10-hexahydropyrido [4',3':3,4]pyrazolo[1,5-a]pyrazin-9(2H)-yl)pyrazolo[1,5-a]pyridine-7-carbonitrile

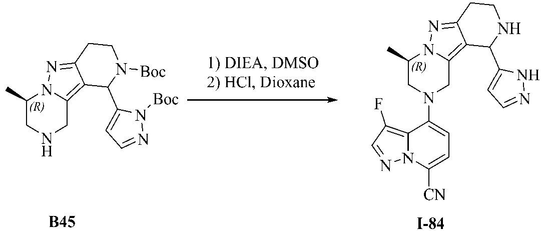

**Example I-84** was synthesized by following the protocols of **Example I-2.** MS: 418.2 (M+H)⁺. ¹H NMR (400 MHz, DMSO-*d₆*) δ 8.32 - 8.26 (m, 1H), 8.22 (s, 1H), 7.72 - 7.56 (m, 2H), 6.50 - 6.35 (m, 1H), 6.22 - 6.15 (m, 1H), 5.15 - 5.05 (m, 1H), 4.40 - 4.25 (m, 2H), 4.02 - 3.94 (m, 1H), 3.91 - 3.84 (m, 1H), 3.82 - 3.74 (m, 1H), 3.45 - 3.30 (m, 2H), 3.17 - 3.11 (m, 1H), 3.02 - 2.92 (m, 1H), 2.70 - 2.60 (m, 2H), 1.47 (d, *J =* 6.4 Hz, 3H).

### Example I-85

### 3-Fluoro-4-((4R,9S)-9-(methoxymethyl)-4-methyl-3,4,7,8,9,10-hexahydropyrido [3',4':3,4]pyrazolo[1,5-a]pyrazin-2(1H)-yl)pyrazolo[1,5-a]pyridine-7-carbonitrile

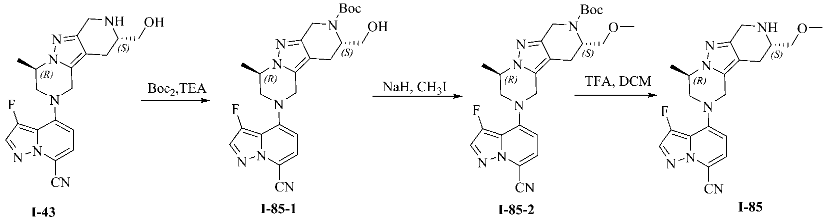

**Example I-85** was synthesized by following the protocols of **Example I-82.** MS: 396.1 (M+H)⁺. ¹H NMR (400 MHz, DMSO-*d₆*) δ 8.32 (d, *J* = 3.5 Hz, 1H), 7.75 (d, *J* = 8.0 Hz, 1H), 6.71 (d, *J* = 8.1 Hz, 1H), 4.52 - 4.40 (m, 2H), 4.37 (s, 1H), 4.01 (d, *J* = 12.3 Hz, 1H), 3.86 (d, *J* = 15.1 Hz, 1H), 3.76 (d, *J* = 15.1 Hz, 1H), 3.41 (d, *J* = 6.0 Hz, 2H), 3.36 (d, *J=* 4.6 Hz, 1H), 3.32 (s, 3H), 2.98 (dt, *J =* 10.5, 5.1 Hz, 1H), 2.48 (d, *J=* 4.1 Hz, 1H), 2.19 (dd, *J =* 15.0, 10.4 Hz, 1H), 1.48 (d, *J =* 6.4 Hz, 3H).

### Example I-86

### 4-((4R,9S)-9-(cyanomethyl)-4-methyl-3,4,7,8,9,10-hexahydropyrido[3',4':3,4] pyrazolo[1,5-a]pyrazin-2(1H)-yl)-3-fluoropyrazolo[1,5-a]pyridine-7-carbonitrile

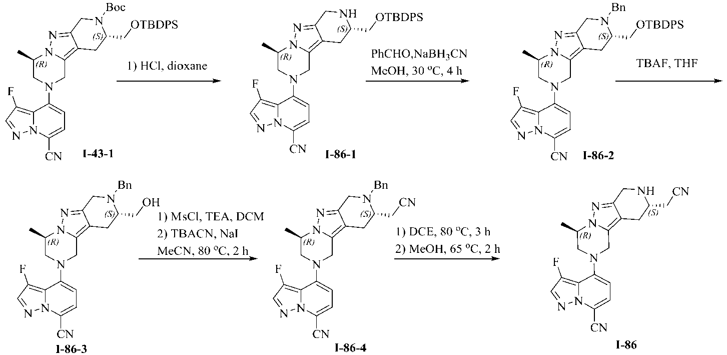

### Steps 1-3: Preparation of intermediates I-86-1, I-86-2, I-86-3

**Intermediate I-86-1** was synthesized by following the protocols of **Example I-80.** MS: 620.1 (M+H)⁺. **Intermediate I-86-2** was synthesized by following the protocols of **Example I-24.** MS: 710.1 (M+H)⁺. **Intermediate I-86-3** was synthesized by following the protocols of **Intermediate I-33-2.** MS: 472.1 (M+H)⁺.

### Step 4: Preparation of intermediate I-86-4

A reaction mixture of **Intermediate I-86-3** (100 mg, 0.212 mmol), TEA (48 mg, 0.474 mmol) and MsCl (36.4 mg, 0.318 mmol) in DCM (5 mL) was stirred at 0 °C for 2 hours. The mixture was quenched with water then extracted with EtOAc, and the organic phase was washed with saturated saline, dried with Na₂SO₄ and concentrated. Then NaI (35.6 mg, 0.238 mmol), tetrabutylammonium cyanide (213 mg, 0.792 mmol) and acetonitrile (7 mL) was added to the mixture. The resulting mixture was stirred at 80 °C for 2 hours. The mixture was quenched with water then extracted with EtOAc, and the organic phase was washed with saturated saline, dried with Na₂SO₄ and concentrated. Purification by flash chromatography gave **Intermediate I-86-4** (78 mg, 82%) as white solid. MS: 481.2 (M+H)⁺.

### Step 5: Preparation of Example I-86

A reaction mixture of **Intermediate I-86-4** (78 mg, 0.162 mmol), 1-chloroethyl chloroformate (186 mg, 1.3 mmol) in DCE (5 mL) was stirred at 85 °C for 3 hours. Then MeOH (5 mL) was added to the mixture and stirred at 70 °C for 1 hour. The mixture was quenched with water then extracted with EtOAc, and the organic phase was washed with saturated saline, dried with Na₂SO₄ and concentrated. Purification by flash chromatography gave **Example I-86** (28 mg, 44.2%) as white solid. MS: 391.1. ¹H NMR (400 MHz, DMSO-*d*₆) δ 8.33 (d, *J* = 3.5 Hz, 1H), 7.75 (d, *J* = 8.0 Hz, 1H), 6.74 (d, *J* = 8.1 Hz, 1H), 4.54-4.37 (m, 3H), 4.02 (d, *J* = 13.4 Hz, 1H), 3.89 - 3.71 (m, 2H), 3.17 (d, *J=* 12.6 Hz, 1H), 3.09 - 3.00 (m, 1H),2.77 - 2.69 (m, 2H), 2.62 (dd, *J=* 14.9, 4.1 Hz, 1H), 2.27 (dd, *J* = 14.8, 10.1 Hz, 1H), 1.49 (d, *J =* 6.5 Hz, 3H).

### Example I-87

### 3-Fluoro-4-((4S,7R)-4-(fluoromethyl)-7-methyl-3,4,7,8,9,10-hexahydropyrido [3',4':3,4]pyrazolo[1,5-a]pyrazin-2(1H)-yl)pyrazolo[1,5-a]pyridine-7-carbonitrile

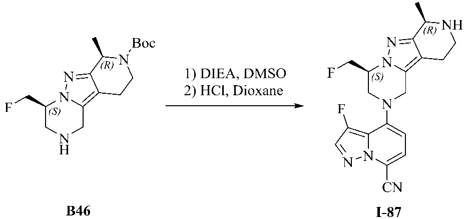

**Example I-87** was synthesized by following the protocols of **I-2.** MS: 384.2 (M+H)⁺.

### Examples I-88a and I-88b

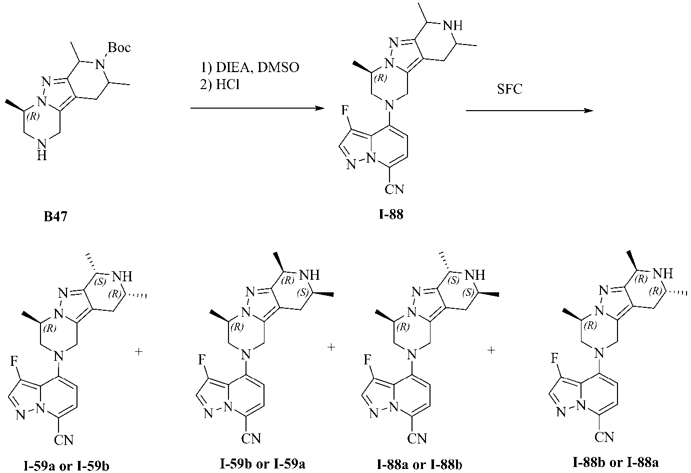

**Examples I-88** was synthesized by following the protocols of **Example I-2.** MS: 380.2 (M+H)⁺.

**Examples I-88a-b** were separated as all white solid through SFC purification with phase A: hexane (0.5% 2mM NH₃-MeOH) and phase B: ethanol. The first peak component was obtained and defined as **Example I-59a** (retention time: 1.375 minute) and the second component peak defined as **Example I-88b** (retention time: 1.493 minute) and third component peak defined as **Example I-59b** (retention time: 1.878 minute) and the fourth component peak defined as **Example I-88a** (retention time: 2.434 minute). MS: 380.1 (M+H)⁺.

**Example 1-88a:** ¹H NMR (400 MHz, DMSO-*d₆*) δ 8.33 (d, *J* = 3.5 Hz, 1H), 7.75 (d, *J =* 8.0 Hz, 1H), 6.71 (d, *J =* 8.1 Hz, 1H), 4.52 - 4.33 (m, 3H), 4.18 - 4.08 (m, 1H), 4.06 - 3.98 (m, 1H), 3.48-3.41 (m, 1H), 3.31 - 3.24 (m, 1H), 2.63 - 2.55 (m, 1H), 2.20 - 2.10 (m, 1H), 1.49 (d, *J =* 6.4 Hz, 3H), 1.36 (d, *J =* 6.7 Hz, 3H), 1.17 (d, *J* = 6.3 Hz, 3H).

**Example I-88b:** ¹H NMR (400 MHz, DMSO-*d₆*) δ 8.33 (d, *J* = 3.5 Hz, 1H), 7.75 (d, *J* = 8.0 Hz, 1H), 6.71 (d, *J* = 8.1 Hz, 1H), 4.55 - 4.37 (m, 3H), 4.23 - 4.15 (m, 1H), 4.06 - 3.98 (m, 1H), 3.48-3.41 (m, 1H), 3.28 - 3.20 (m, 1H), 2.67 - 2.58 (m, 1H), 2.17 - 2.17 (m, 1H), 1.49 (d, *J* = 6.4 Hz, 3H), 1.36 (d, *J* = 6.7 Hz, 3H), 1.17 (d, *J* = 6.3 Hz, 3H).

### Example I-90

### 3-Fluoro-4-((4R)-4-methyl-9-(1-methyl-1H-pyrazol-5-yl)-3,4,7,8,9,10-hexahydropyrido[3',4':3,4]pyrazolo[1,5-a]pyrazin-2(1H)-yl)pyrazolo[1,5-a]pyridine-7-carbonitrile

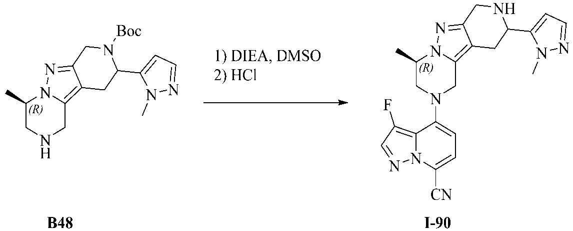

**Example I-90** was synthesized by following the protocols of **Example I-2.** MS: 432.2 (M+H)⁺. ¹H NMR (400 MHz, DMSO-*d₆*) δ 10.86 (s, 1H), 8.42 (s, 2H), 8.36 (d, *J* = 10.4 Hz, 3H), 8.04 (t, *J* = 7.7 Hz, 1H), 8.00 - 7.94 (m, 2H), 7.39 (d, *J* = 7.6 Hz, 2H), 7.33 (dd, *J* = 8.3, 6.6 Hz, 2H), 7.28 - 7.21 (m, 2H), 7.16 - 7.02 (m, 1H), 3.24 - 3.17 (m, 2H), 3.03 (d, *J* = 11.2 Hz, 3H), 2.99 - 2.92 (m, 1H), 2.69 (d, *J* = 12.1 Hz, 2H), 2.64 (d, *J* = 6.8 Hz, 2H), 2.29 (dt, *J* = 13.2, 7.1 Hz, 1H), 2.00 - 1.92 (m, 1H), 1.91 - 1.82 (m, 3H), 1.71 (dt, *J* = 14.0, 9.9 Hz, 3H), 1.54 - 1.42 (m, 3H).

### Example I-91

### 3-Fluoro-4-((4R)-4-methyl-9-(1-methyl-1H-pyrazol-4-yl)-3,4,7,8,9,10-hexahydropyrido[3',4':3,4]pyrazolo[1,5-a]pyrazin-2(1H)-yl)pyrazolo[1,5-a]pyridine-7-carbonitrile

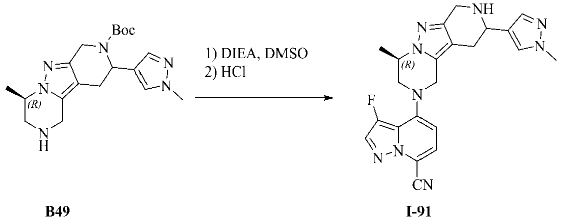

**Example I-91** was synthesized by following the protocols of **Example I-2.** MS: 432.2 (M+H)⁺. ¹H NMR (400 MHz, DMSO-*d₆*) δ 8.29 (d, J = 3.6 Hz, 1H), 7.72 (d, J = 8.0 Hz, 1H), 7.62 (d, J = 6.4 Hz, 1H), 7.40 (d, J = 2.8 Hz, 1H), 6.68 (d, J = 8.0 Hz, 1H), 4.55 - 4.32 (m, 3H), 4.00 (dd, J = 14.0, 4.4 Hz, 1H), 3.92 - 3.84 (m, 3H), 3.80 (d, J = 4.0 Hz, 3H), 3.37 - 3.31 (m, 1H), 2.79 (ddd, J = 22.8, 14.8, 4.4 Hz, 1H), 2.46 (s, 1H), 1.46 (d, J = 6.4 Hz, 3H).

### Example I-92

### ((4R,9S)-2-(7-cyano-3-fluoropyrazolo[1,5-a]pyridin-4-yl)-4-methyl-1,2,3,4,7,8,9,10-octahydropyrido[3',4':3,4]pyrazolo[1,5-a]pyrazin-9-yl)methyl pivalate

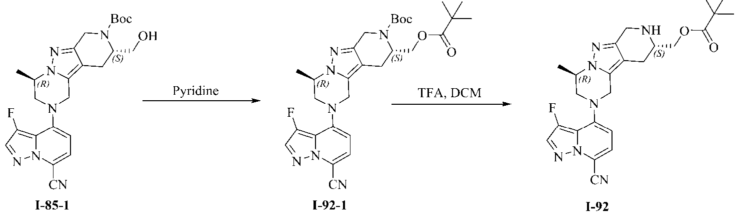

### Step 1: Preparation of intermediate I-92-1

A reaction mixture of **Intermediate I-85-1** (90 mg, 0.187 mmol), pyridine (73.0 mg, 0.935 mmol) and pivaloyl chloride (45.1 mg, 0.374 nmol) in DCM (5 mL) was stirred at 25 °C for 2 hours. The mixture was quenched with water then extracted with EtOAc, and the organic phase was washed with saturated saline, dried with Na₂SO₄ and concentrated. Purification by flash chromatography gave **Intermediate I-92-1** (70 mg, 66.2%) as white solid. MS: 566.4 (M+H)⁺.

### Step 2: Preparation of Example I-92

**Example I-92** was synthesized by following the protocols of **Example I-2.** MS: 466.2 (M+H)⁺. ¹H NMR (400 MHz, DMSO-*d₆*) δ 8.32 (d, *J* = 3.5 Hz, 1H), 7.74 (d, *J* = 8.0 Hz, 1H), 6.74 (d, *J* = 8.1 Hz, 1H), 4.56 - 4.32 (m, 3H), 4.14-4.12 (m, 1H), 4.03-4.00 (m, 2H), 3.90 - 3.86 (m, 1H), 3.79-3.75 (m, 1H), 3.35-3.32 (m, 2H), 3.06-3.02 (m, 1H), 2.56 -2.54(m, 1H), 2.30-2.24 (m, 1H), 1.49 (d, *J* = 6.4 Hz, 3H), 1.20 (s, 9H).

### Example I-93

### ((4R,9S)-2-(7-cyano-3-fluoropyrazolo[1,5-a]pyridin-4-yl)-4-methyl-1,2,3,4,7,8,9,10-octahydropyrido[3',4':3,4]pyrazolo[1,5-a]pyrazin-9-yl)methyl acetate

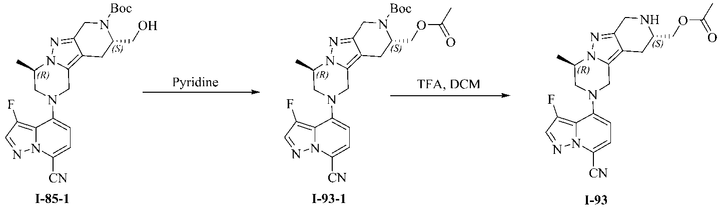

**Example I-93** was synthesized by following the protocols of **Example I-92.** MS: 424.2 (M+H)⁺. ¹H NMR (400 MHz, DMSO-*d₆*) δ 8.32 (d, *J* = 3.5 Hz, 1H), 7.75 (d, *J* = 8.0 Hz, 1H), 6.72 (d, *J=* 8.1 Hz, 1H), 4.43 (dd, *J* = 29.8, 14.5 Hz, 3H), 4.17 - 3.97 (m, 3H), 3.88 (d, *J* = 15.3 Hz, 1H), 3.79 (d, *J =* 15.4 Hz, 1H), 3.30-3.28 (m, 1H), 3.06 (t, *J=* 6.3 Hz, 1H), 2.57-2.55 (m, 1H), *J* = 14.9, 10.3 Hz, 1H), 2.08 (s, 3H), 1.48 (d, *J* = 6.4 Hz, 3H).

### Example I-94

### 3-Fluoro-4-((9R)-4-(fluoromethyl)-9-methyl-3,4,7,8,9,10-hexahydropyrido [3',4':3,4]pyrazolo[1,5-a]pyrazin-2(1H)-yl)pyrazolo[1,5-a]pyridine-7-carbonitrile

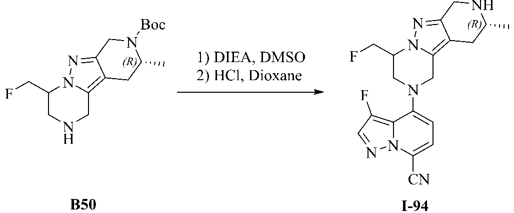

**Example I-94** was synthesized by following the protocols of **Example I-2.** MS: 384.2 (M+H)⁺. ¹H NMR (400 MHz, DMSO-*d₆*) δ 8.34 (d, *J=* 3.5 Hz, 1H), 7.77 (dd, *J* = 7.9, 1.4 Hz, 1H), 6.74 (d, *J* = 8.1 Hz, 1H), 5.19 - 5.09 (m, 0.5H), 5.07 - 4.98 (m, 0.5H), 4.95 - 4.87 (m, 0.5H), 4.80 (d, *J* = 9.4 Hz, 0.5H), 4.60-4.54 (m, 2H), 4.52 - 4.42 (m, 1H), 4.19-4.05 (m, 3H), 3.71 - 3.61 (m, 2H), 2.85 - 2.68 (m, 1H), 2.30 (d, *J* = 13.9 Hz, 1H), 1.31-1.26 (m, 3H).

### Example I-95

### 3-Fluoro-4-((8R,12aS)-8-methyl-3-oxo-1,8,9,11,12,12a-hexahydro-3H,5H,10H-oxazolyl [3 ‴,4 ':1'6']pyridine and [3',4':3,4] pyrazole [1,5-a] pyrazine-10-yl) pyrazole [1,5-a]pyridine-7-nitrile

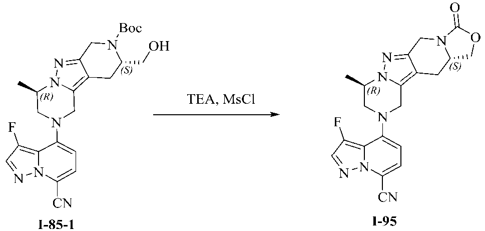

A reaction mixture of **Intermediate I-85-1** (144 mg, 0.3mmol), TEA (90 mg, 0.892 mmol) and MsCl (68.1 mg, 0.595 mmol) in MeOH (5 mL) was stirred at 25 °C for 2 hours. The mixture was quenched with water and extracted with EtOAc, and the organic phase was washed with saturated saline, dried with Na₂SO₄ and concentrated. Purification by pre-HPLC gave **Example I-95** as white solid. MS: 408.4 (M+H)⁺. ¹H NMR (400 MHz, DMSO-*d₆*) δ 8.33 (d, *J* = 3.5 Hz, 1H), 7.75 (d, *J* = 8.0 Hz, 1H), 6.73 (d, *J* = 8.1 Hz, 1H), 4.60 - 4.54 (m, 2H), 4.54 - 4.49 (m, 2H), 4.43 (s, 1H), 4.24 (d, *J* = 15.8 Hz, 1H), 4.15 (dd, *J* = 8.6, 5.4 Hz, 1H), 4.08 - 3.98 (m, 2H), 3.44 - 3.40 (m, 1H), 2.82 (dd, *J* = 15.0, 4.6 Hz, 1H), 2.52 - 2.46 (m, 1H), 1.50 (d, *J* = 6.4 Hz, 3H).

### Example I-96

### (R)-4-(2-Cyclopropyl-7-methyl-1,3,4,7,8,10-hexahydropyrido[4',3':3,4]pyrazolo[1,5-a]pyrazin-9(2H)-yl)-3-fluoropyrazolo[1,5-a]pyridine-7-carbonitrile

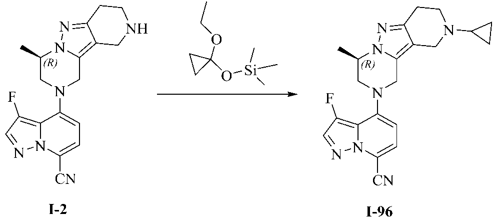

A reaction mixture of **Example I-2** (50 mg, 0.142 mmol), 1-ethoxy-1-trimethylsiloxylcyclopropane (49.6 mg, 0.285 mmol), acetic acid (8.54 mg, 0.142 mmol) and NaBH3CN (14.31 mg, 0.228 mmol) in MeOH (2 mL) was stirred at 60 °C for 16 hours. The mixture was quenched with water and extracted with EtOAc, and the organic phase was washed with saturated saline, dried with Na₂SO₄ and concentrated. Purification by pre-HPLC gave **Example I-96** as white solid. MS: 392.1 (M+H)⁺. ¹H NMR (400 MHz, DMSO-*d₆*) δ 8.32 (d, *J* = 3.5 Hz, 1H), 7.74 (d, *J* = 8.0 Hz, 1H), 6.70 (d, *J* = 8.1 Hz, 1H), 4.57 - 4.30 (m, 3H), 4.00 (d, *J* = 11.7 Hz, 1H), 3.56 (d, *J* = 25.4 Hz, 2H), 3.29 (d, *J* = 13.8 Hz, 1H), 2.89 (s, 2H), 2.63 (d, *J* = 6.3 Hz, 2H), 1.88 (s, 1H), 1.47 (d, *J* = 6.3 Hz, 3H), 0.56 - 0.35 (m, 4H).

### Example I-97

### ((4R,9S)-2-(7-cyano-3-fluoropyrazolo[1,5-a]pyridin-4-yl)-4-methyl-1,2,3,4,7,8,9,10-octahydropyrido[3',4':3,4]pyrazolo[1,5-a]pyrazin-9-yl)methyl 2,2-dimethylbutanoate

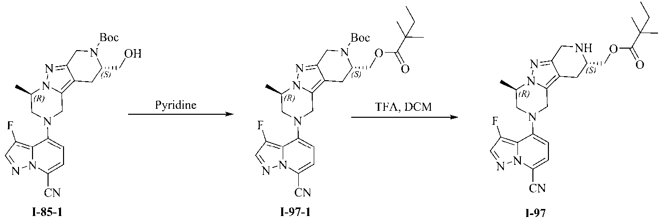

**Example I-97** was synthesized by following the protocols of **Example I-92.** MS: 480.2 (M+H)⁺. ¹H NMR (400 MHz, DMSO-*d₆*) δ 8.29 (d, *J* = 3.5 Hz, 1H), 7.72 (d, *J* = 8.0 Hz, 1H), 6.71 (d, *J* = 8.0 Hz, 1H), 4.50 - 4.32 (m, 3H), 4.11 (dd, *J* = 10.9, 5.9 Hz, 1H), 4.04 - 3.95 (m, 2H), 3.88 - 3.71 (m, 2H), 3.00 (dd, *J* = 10.1, 4.9 Hz, 1H), 2.34 - 2.18 (m, 1H), 1.53 (q, *J* = 7.5 Hz, 2H), 1.46 (d, *J* = 6.3 Hz, 3H), 1.12 (s, 7H), 0.79 (t, *J* = 7.5 Hz, 3H).

### Example I-98

### 3-Fluoro-4-((4R)-4-methyl-9-(pyridin-4-yl)-3,4,7,8,9,10-hexahydropyrido[3',4':3,4] pyrazolo[1,5-a]pyrazin-2(1H)-yl)pyrazolo[1,5-a]pyridine-7-carbonitrile

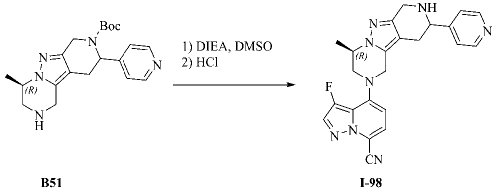

**Example I-98** was synthesized by following the protocols of **I-2.** MS: 429.2 (M+H)⁺. ¹H NMR (400 MHz, DMSO-*d₆*) δ 8.70 - 8.64 (m, 2H), 8.33 (d, *J* = 3.5 Hz, 1H), 7.76 (d, *J* = 8.0 Hz, 1H), 7.53 (d, *J* = 5.2 Hz, 2H), 6.70 (dd, *J* = 8.1, 2.3 Hz, 1H), 4.60 - 4.56 (m, 1H), 4.48-4.42(m, 2H), 4.36 - 4.14 (m, 2H), 4.10 - 3.99 (m, 1H), 3.43-3.37 (m 3H), 3.09 - 2.94 (m, 1H), 2.76 - 2.63 (m, 1H), 1.51 (d, *J =* 6.3 Hz, 3H).

### Example I-99

### 2-((4R,9S)-2-(7-cyanide-3-fluoropyridox and [1,5-a]pyridox-4-base)-4,9-dimethyl-1, 2, 3, 4, 9, 10-hydropyridox [3', 4': 3, 4]pyridoz and [1, 5]pyridoxine-8 (7H)- Base) acetylamine

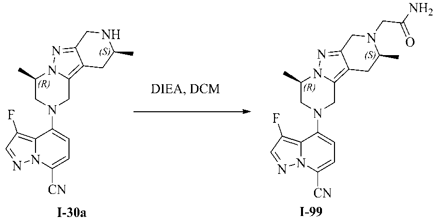

**I-99 Example** was synthesized by following the protocols of **Example 1-7.** MS: 423.1 (M+H)⁺. ¹H NMR (400 MHz, DMSO-*d₆*) δ 8.32 (d, *J* = 3.5 Hz, 1H), 7.74 (d, *J* = 8.0 Hz, 1H), 7.29 - 7.22 (m, 1H), 7.17 (s, 1H), 6.71 (d, *J* = 8.1 Hz, 1H), 4.53-4.36 (m, 3H), 4.06 - 3.97 (m, 1H), 3.76 - 3.55 (m, 3H), 3.13-3.04 (m, 2H), 2.94 (d, *J* = 16.2 Hz, 1H), 2.66 (dd, *J =* 15.7, 5.3 Hz, 1H),2.26-2.20(m, 1H), 1.49 (d, *J =* 6.3 Hz, 3H), 1.08 (d, *J =* 6.5 Hz, 3H).

### Example I-100

### 2-((4R)-2-(7-cyanide-3-fluoropyridox and [1, 5-a]pyridox-4-yl)-4-methyl-1, 3, 4, 7, 8, 9, 10, 11-hydro-2H-7, 10-pyriminocyclohexane [3,4]pyridoxine and [1, 5-a] pyrazine-12yl) acetamide

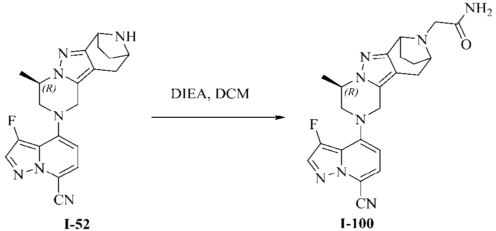

**Example I-100** was synthesized by following the protocols of **Example I-7.** MS: 435.1 (M+H)⁺. ¹H NMR (400 MHz, DMSO-*d₆*) δ 8.32 (d, *J =* 3.5 Hz, 1H), 7.74 (dd, *J =* 8.0, 1.6 Hz, 1H), 7.25-7.19 (m, 2H), 6.72 (dd, *J =* 8.1, 6.2 Hz, 1H), 4.53 - 4.33 (m, 3H), 4.08 - 3.91 (m, 2H), 3.54 (s, 1H), 3.34 - 3.29 (m, 1H), 3.14-3.07 (m, 1H), 2.89 - 2.67 (m, 2H), 2.24 - 2.10 (m, 3H), 1.71-1.68 (d, *J =* 11.1 Hz, 1H), 1.53 - 1.48 (m, 3H), 1.48-1.36 (m, 1H).

### Example I-101

### 2-((4R,9S)-2-(7-cyanide-3-fluoropyridox and [1,5-a]pyridox-4-base)-4,9-dimethyl-1, 2, 3, 4, 9, 10-hydropyridox [3 ', 4 ': 3, 4] pyrazolo [1, 5-a] pyrazine-8 (7 H) -yl)-N-methyl acetamide

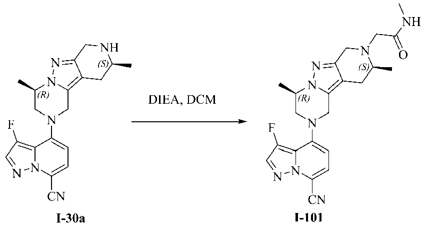

**Example I-101** was synthesized by following the protocols of **I-99 Exampl.** MS: 437.1 (M+H)⁺. ¹H NMR (400 MHz, DMSO-*d₆*) δ 8.29 (d, J = 3.6 Hz, 1H), 7.72 (d, J = 8.0 Hz, 1H), 6.72 - 6.65 (m, 1H), 4.42 (dt, J = 32.0, 16.0 Hz, 3H), 3.99 (d, J = 13.2 Hz, 1H), 3.61 (d, J = 9.2 Hz, 3H), 3.10 (s, 1H), 2.98 (s, 2H), 2.62 (d, J = 4.4 Hz, 3H), 2.22 (d, J = 18.0 Hz, 1H), 2.00 (d, J = 7.6 Hz, 2H), 1.47 (d, J = 6.4 Hz, 3H), 1.05 (d, J = 6.4 Hz, 3H).

### Example I-102

### 2-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-N,N-dimethylacetamide

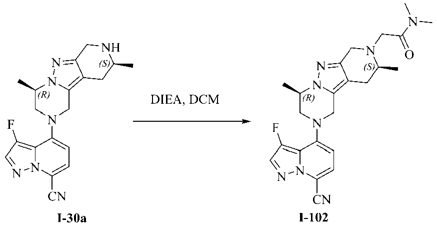

**Example I-102** was synthesized by following the protocols of **I-99 Example.** MS: 451.1 (M+H)⁺. ¹H NMR (400 MHz, DMSO-*d₆*) δ 8.32 (d, *J =* 3.6 Hz, 1H), 7.74 (d, *J =* 8.0 Hz, 1H), 6.71 (d, *J* = 8.1 Hz, 1H), 4.56 - 4.32 (m, 3H), 4.02 (dd, *J* = 13.7, 4.1 Hz, 1H), 3.70-3.62 (m, 2H), 3.19 (d, *J =* 22.0 Hz, 1H), 3.05 (s, 3H), 2.85 (s, 3H), 2.69-2.65 (m, 1H), 2.29 - 2.18 (m, 1H), 1.49 (d, *J =* 6.4 Hz, 3H), 1.09 (d, *J =* 6.5 Hz, 3H).

### Example I-103

### 4-((4R,9S)-4,9-dimethyl-8-(2-morpholinoacetyl)-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

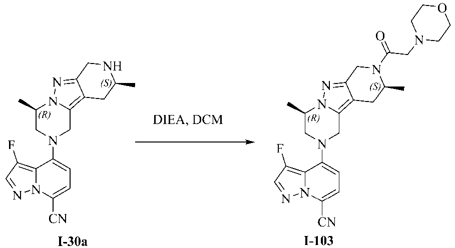

**Example I-103** A composite reference**I-99 Example** it was prepared **Example I-103.** MS: 493.1 (M+H)⁺.¹H NMR (500 MHz, DMSO-*d₆*) δ 8.20 (d, *J* = 3.6 Hz, 1H), 7.61 (d, *J =* 8.0 Hz, 1H), 6.66 (d, *J* = 8.0 Hz, 1H), 5.06 (s, 1H), 4.46 (s, 2H), 4.42 - 4.33 (m, 1H), 3.97 (dd, *J* = 13.7, 4.3 Hz, 1H), 3.59 (t, *J* = 4.7 Hz, 4H), 3.42 (dd, *J* = 13.6, 8.6 Hz, 1H), 3.04 (s, 7H), 2.78 (s, 1H), 2.50 (s, 1H), 2.41-2.35 (m, 1H), 1.50 (d, *J =* 6.5 Hz, 3H), 1.13 (s, 3H).

### Example I-104

### 4-((4R,9S)-4,9-dimethyl-8-(2-oxo-2-(2-azaspiro[3.3]heptan-2-yl)ethyl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

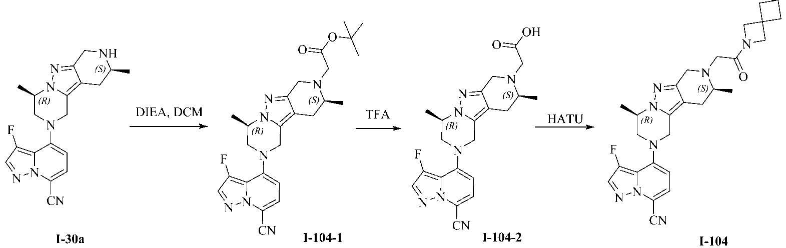

**Example 1-104-1** was synthesized by following the protocols of **Example I-6.** MS: 480.1 (M+H)⁺. ¹H NMR (400 MHz, DMSO-*d₆*) δ 8.31 (d, *J =* 3.5 Hz, 1H), 7.73 (d, *J =* 8.0 Hz, 1H), 6.70 (d, *J* = 8.1 Hz, 1H), 4.52 - 4.30 (m, 3H), 4.05 - 3.95 (m, 1H), 3.80 - 3.63 (m, 2H), 3.44 (d, *J* = 16.8 Hz, 1H), 3.36-3.32(m, 1H), 3.32-3.26 (m, 1H), 3.10-3.09 (m, 1H), 2.61 (dd, *J* = 15.0, 4.5 Hz, 1H), 2.17 (dd, *J* = 15.1, 7.3 Hz, 1H), 1.48 (d, *J* = 6.4 Hz, 3H), 1.44 (s, 9H), 1.08 (d, *J* = 6.5 Hz, 3H).

**Example I-104-2** was synthesized by following the protocols of **Example I-82.** MS: 424.6 (M+H)⁺. ¹H NMR (400 MHz, DMSO-*d₆*) δ 8.31 (d, J = 3.5 Hz, 1H), 7.73 (d, J = 8.0 Hz, 1H), 6.70 (d, J = 8.1 Hz, 1H), 4.53 - 4.37 (m, 3H), 4.01 (d, J = 11.0 Hz, 1H), 3.90-3.87 (m, 1H), 3.80-3.76 (m, 1H), 3.47-3.42 (m, 1H), 3.40 - 3.34 (m, 1H), 3.27-3.21 (m, 2H), 2.68 (dd, J = 15.4, 4.7 Hz, 1H), 2.25 (dd, J = 15.4, 7.2 Hz, 1H), 1.48 (d, J = 6.3 Hz, 3H), 1.12 (d, J = 6.5 Hz, 3H).

**Example I-104** was synthesized by following the protocols of **Intermediate B10-4.** MS: 503.6 (M+H)⁺. ¹H NMR (400 MHz, DMSO-*d₆*) δ 8.32 (d, *J* = 3.6 Hz, 1H), 7.74 (d, *J* = 8.0 Hz, 1H), 6.71 (d, *J* = 8.0 Hz, 1H), 4.51 (d, *J* = 15.2 Hz, 1H), 4.42 (d, *J* = 15.6 Hz, 1H), 4.15 (s, 2H), 4.02 (d, *J* = 13.6 Hz, 1H), 3.85 (s, 2H), 3.63 (d, *J* = 3.2 Hz, 1H), 3.24 - 3.09 (m, 2H), 2.71 - 2.59 (m, 1H), 2.20 (dd, *J* = 15.2, 6.4 Hz, 1H), 2.13 (t, *J* = 7.6 Hz, 4H), 1.79 (d, *J* = 7.2 Hz, 1H), 1.49 (d, *J* = 6.4 Hz, 3H), 1.26 (s, 4H), 1.06 (d, *J* = 6.4 Hz, 3H).

### Example I-105

### 4-((4R,9S)-8-(dimethylglycyl)-4,9-dimethyl-3,4,7,8,9,10-hexahydropyrido[3',4':3,4] pyrazolo[1,5-a]pyrazin-2(1H)-yl)-3-fluoropyrazolo[1,5-a]pyridine-7-carbonitrile

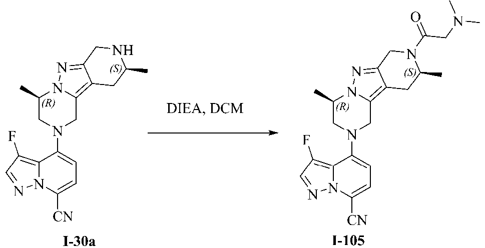

**Example I-105** was synthesized by following the protocols of **I-99 Example.** MS: 451.6 (M+H)⁺. ¹H NMR (400 MHz, DMSO-*d₆*) δ 8.34 (s, 1H), 8.32 (d, *J* = 3.6 Hz, 1H), 7.74 (d, *J* = 8.0 Hz, 1H), 6.71 (d, *J* = 8.0 Hz, 1H), 5.20 - 4.71 (m, 2H), 4.49 (s, 2H), 4.45 - 4.34 (m, 1H), 4.02 (d, *J* = 13.6 Hz, 1H), 3.89 (d, *J* = 16.8 Hz, 1H), 3.42 (dd, *J* = 13.6, 9.2 Hz, 1H), 3.29 - 3.04 (m, 2H), 2.88 - 2.61 (m, 1H), 2.40 (dd, *J* = 15.2, 8.0 Hz, 1H), 2.20 (d, *J* = 6.8 Hz, 6H), 1.52 (d, *J* = 6.4 Hz, 3H), 1.12 (dd, *J* = 36.8, 6.8 Hz, 3H).

### Example I-106

### 2-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-N-((3S,4R)-4-fluoropyrrolidin-3-yl)acetamide

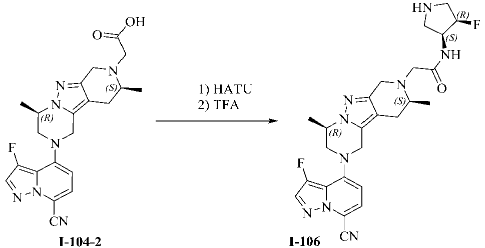

**Example I-106** was synthesized by following the protocols of **Example I-104.** MS: 510.1 (M+H)⁺. ¹H NMR (400 MHz, DMSO-*d₆*) δ 8.32 (d, *J =* 3.6 Hz, 1H), 7.84 (d, *J* = 8.3 Hz, 1H), 7.75 (d, *J* = 8.0 Hz, 1H), 6.72 (d, *J* = 8.1 Hz, 1H), 5.18-5.02 (m, 1H), 4.55 - 4.40 (m, 2H), 4.37 (dd, *J* = 9.7, 5.0 Hz, 2H), 3.74 (d, *J* = 15.3 Hz, 1H), 3.66 (d, *J* = 15.2 Hz, 1H), 3.37 (dd, *J* = 13.6, 9.4 Hz, 2H), 3.32 - 3.24 (m, 2H), 3.22-3.18 (m, 1H), 3.16 - 3.04 (m, 3H), 2.76 (t, *J* = 10.5 Hz, 1H), 2.66 (dd, *J* = 15.3, 4.9 Hz, 1H), 2.24 (dd, *J* = 15.4, 6.5 Hz, 1H), 1.50 (d, *J* = 6.4 Hz, 3H), 1.09 (d, *J* = 6.6 Hz, 3H).

### Example I-107

### 3-Fluoro-4-((4R,9S)-8-(2-(3-hydroxypyrrolidin-1-yl)-2-oxoethyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

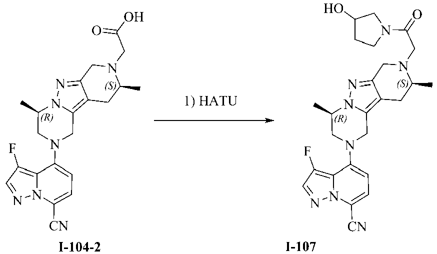

**Example I-107** was synthesized by following the protocols of **Example I-104.** MS: 493.1 (M+H)⁺. ¹H NMR (400 MHz, DMSO-*d₆*) δ 8.32 (d, J = 3.6 Hz, 1H), 7.74 (d, J = 8.0 Hz, 1H), 6.71 (d, J = 8.1 Hz, 1H), 4.91 (s, 1H), 4.54 - 4.22 (m, 4H), 4.02 (d, J = 12.1 Hz, 1H), 3.76 - 3.53 (m, 4H), 3.41-3.39 (m, 2H), 3.32 - 3.22 (m, 3H), 3.18 - 3.09 (m, 1H), 2.64 (dd, J = 15.1, 4.8 Hz, 1H), 2.21 (dd, J = 15.2, 6.2 Hz, 1H), 1.93 (d, J = 9.3 Hz, 2H), 1.49 (d, J = 6.3 Hz, 3H), 1.08 (dd, J = 6.6, 2.3 Hz, 3H).

### Example I-108

### 4-((4R,9S)-4,9-dimethyl-8-(2-(methylsulfonyl)ethyl)-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

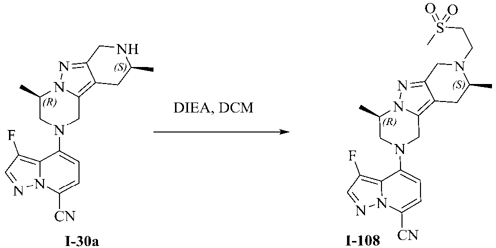

**Example I-108** was synthesized by following the protocols of **I-99 Example.** MS: 472.1 (M+H)⁺. ¹H NMR (400 MHz, DMSO-*d₆*) δ 8.32 (d, *J* = 3.6 Hz, 1H), 7.74 (d, *J* = 8.0 Hz, 1H), 6.70 (d, *J* = 8.1 Hz, 1H), 4.55 - 4.33 (m, 3H), 4.02 (d, *J* = 11.6 Hz, 1H), 3.68 (s, 2H), 3.41 - 3.35 (m, 3H), 3.12-3.08 (m, 1H), 3.03 (s, 3H), 3.00-2.95 (m, 1H), 2.92 - 2.84 (m, 1H), 2.68-2.63 (m, 1H), 2.24 (dd, *J* = 15.3, 5.7 Hz, 1H), 1.50 (d, *J* = 6.4 Hz, 3H), 1.08 (d, *J* = 6.6 Hz, 3H).

### Example I-109

### 3-Fluoro-4-((4R,9S)-8-(2-((S)-2-(methoxymethyl)pyrrolidin-1-yl)acetyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

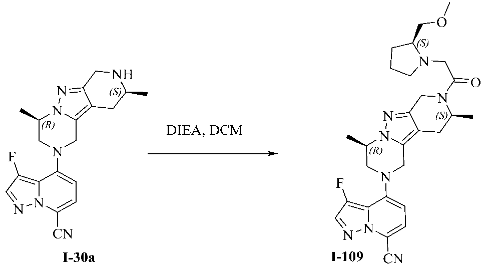

**Example I-109** was synthesized by following the protocols of **I-99 Example.** MS: 521.1 (M+H)⁺. ¹H NMR (400 MHz, DMSO-*d₆*) δ 8.32 (d, *J =* 3.5 Hz, 1H), 7.74 (d, *J =* 8.0 Hz, 1H), 6.71 (d, *J =* 8.1 Hz, 1H), 5.20-5.11(m, 1H), 4.84-4.81(m, 0.5 H), 4.49 (s, 2H), 4.40 (s, 1H), 4.01 (dd, *J =* 13.7, 4.4 Hz, 1H), 3.92-3.81 (m, 2H), 3.46 - 3.41 (m, 1H), 3.40-3.38 (m, 1H), 3.36-3.34 (m 1H), 3.32 (s, 1H), 3.25 (s, 3H), 3.17-3.12 (m, 1H), 3.01 - 2.60 (m, 3H), 2.44-2.36 (m, 2H), 1.94 - 1.81 (m, 1H), 1.79 - 1.56 (m, 2H), 1.52 (d, *J =* 6.4 Hz, 3H), 1.48 (d, *J* = 7.2 Hz, 1H), 1.16 (d, *J =* 6.6 Hz, 2H), 1.07 (d, *J =* 6.9 Hz, 1H).

### Example I-110

### 3-Fluoro-4-((4R,9S)-8-(2-(3-hydroxypyrrolidin-1-yl)acetyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

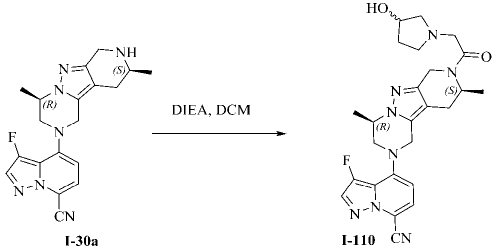

**Example I-110** was synthesized by following the protocols of **I-99 Exampl.** MS: 493.1 (M+H)⁺. ¹H NMR (400 MHz, DMSO-*d₆*) δ 8.32 (d, *J* = 3.5 Hz, 1H), 7.74 (d, *J* = 8.0 Hz, 1H), 6.70 (d, *J* = 8.1 Hz, 1H), 5.19 - 5.07 (m, 1H), 4.98 (dd, *J* = 16.2, 5.8 Hz, 0.5 H), 4.82 - 4.64 (m, 1H), 4.48 (d, *J* = 3.6 Hz, 2H), 4.39-4.30 (m, 1.5H), 4.20 (s, 1H), 4.02 (d, *J =* 13.5 Hz, 1H), 3.88 (d, *J* = 16.7 Hz, 1H), 3.48 - 3.40 (m, 2H), 3.32 - 2.99 (m, 2H), 2.84 - 2.61 (m, 3H), 2.39 -2.35(m, 2H), 2.01-1.96 (m, 1H), 1.56 (s, 1H), 1.51 (d, *J* = 6.4 Hz, 3H), 1.11 (dd, *J* = 37.8, 6.8 Hz, 3H).

### Example I-111

### 3-Fluoro-4-((4R, 9S)-8-(3-hydroxycyclobutyl)-4, 9-dimethyl-3, 4, 7, 8, 9, 10-hexahydropyridine [3 ', 4 ': 3, 4] pyrazole [1, 5-a] pyrazine-2 (1 H) -yl) pyrazole [1, 5-a] pyridine-7-nitrile

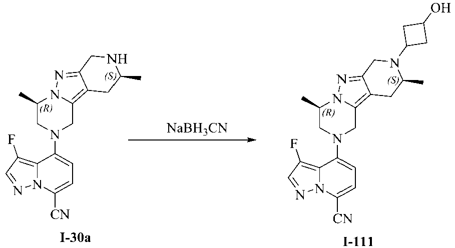

**Example I-111** was synthesized by following the protocols of **Example I-24.** MS: 436.1 (M+H)⁺. ¹H NMR (400 MHz, DMSO-*d₆*) δ 8.28 (d, *J* = 3.5 Hz, 1H), 8.14 (s, 0H), 7.70 (d, *J* = 8.0 Hz, 1H), 6.66 (d, *J* = 8.1 Hz, 1H), 4.95 (s, 1H), 4.46 - 4.36 (m, 2H), 4.35 - 4.27 (m, 1H), 3.97 (dd, *J* = 13.8, 4.2 Hz, 1H), 3.75 (t, *J* = 7.3 Hz, 1H), 3.50 (d, *J* = 4.5 Hz, 1H), 3.36 - 3.33 (m, 2H), 3.12 - 3.06 (m, 1H), 2.62 (dt, *J* = 13.5, 6.6 Hz, 2H), 2.38 (dt, *J* = 11.8, 6.0 Hz, 2H), 2.18 (dd, *J* = 15.1, 2.8 Hz, 1H), 1.62 (q, *J* = 8.8 Hz, 2H), 1.46 (d, *J* = 6.4 Hz, 3H), 0.91 (d, *J* = 6.6 Hz, 3H).

### Example I-112

### 4-((4R, 9S) -4, 9-dimethyl-8-(2-oxydioxy-2-(2-oxytrioxy-6-nitrous [3.3] helium-6-ethyl) ethyl) -3, 4, 7, 8, 9, 10-hydropyridine [3 ', 4 ': 3, 4] pyrazole [1, 5-a] pyrazine-2 (1 H) - yl) -3-fluoropyrazole [1, 5-a] pyridine-7-nitrile

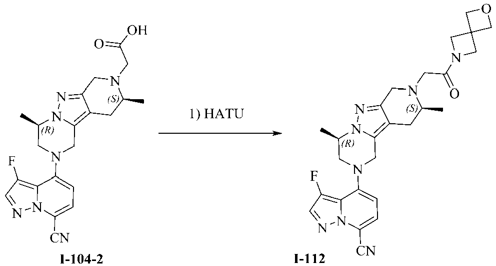

**Example I-112** was synthesized by following the protocols of **Example I-104.** MS: 505.1 (M+H)⁺. ¹H NMR (400 MHz, DMSO-*d₆*) δ 8.31 (d, *J* = 3.6 Hz, 1H), 7.74 (d, *J* = 8.0 Hz, 1H), 6.70 (d, *J* = 8.0 Hz, 1H), 4.67 (s, 4H), 4.53 - 4.39 (m, 2H), 4.36 (s, 3H), 4.05 (s, 2H), 4.04 - 3.97 (m, 1H), 3.62 (d, *J* = 2.4 Hz, 2H), 3.38 (s, 1H), 3.24 - 3.08 (m, 2H), 3.04 (q, *J* = 6.0 Hz, 1H), 2.63 (dd, *J* = 15.2, 4.8 Hz, 1H), 2.20 (dd, *J* = 15.2, 6.4 Hz, 1H), 1.49 (d, *J* = 6.4 Hz, 3H), 1.06 (d, *J* = 6.4 Hz, 3H).

### Example 1-113

### 4- ((4R, 9S) -4, 9- dimethyl-8- (2-oxo-2- (piperazine-1-xyl-ethyl) -3, 4, 7, 8, 9, 10-hexahydropyridine [3 ', 4 ': 3, 4] pyrazole [1, 5-a] pyrazine-2 (1 H) -yl) -3-fluoropyrazole [1, 5-a] pyridine-7-nitrile

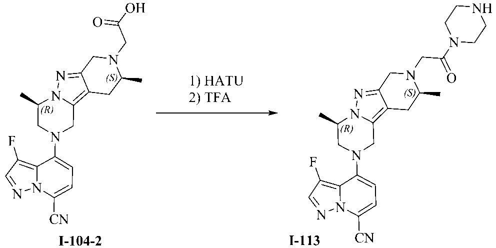

**Example 1-113** was synthesized by following the protocols of **Example 1-104.** MS: 492.6 (M+H)⁺. ¹H NMR (400 MHz, DMSO-*d₆*) δ 8.32 (d, *J =* 3.6 Hz, 1H), 7.74 (d, *J =* 8.0 Hz, 1H), 6.71 (d, *J* = 8.0 Hz, 1H), 4.46 (q, *J* = 15.2 Hz, 2H), 4.36 (d, *J* = 10.0 Hz, 1H), 4.01 (d, *J* = 11.2 Hz, 1H), 3.66 (s, 1H), 3.60 (s, 1H), 3.53 (s, 2H), 3.44 (t, *J* = 4.8 Hz, 2H), 3.42 - 3.32 (m, 4H), 3.12 (q, *J =* 5.6 Hz, 1H), 2.73 (s, 4H), 2.65 (dd, *J =* 15.2, 5.2 Hz, 1H), 2.23 (dd, *J =* 15.2, 5.2 Hz, 1H), 1.49 (d, *J =* 6.4 Hz, 3H), 1.05 (d, *J =* 6.4 Hz, 3H).

### Example 1-114

### 4-((4R,9S)-8-(L-prolyl)-4,9-dimethyl-3,4,7,8,9,10-hexahydropyrido[3',4':3,4]pyrazolo [1,5-a]pyrazin-2(1H)-yl)-3-fluoropyrazolo[1,5-a]pyridine-7-carbonitrile

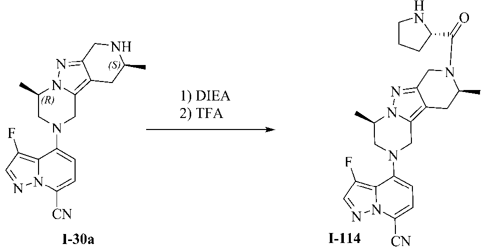

**Example I-114** was synthesized by following the protocols of **I-99 Example.** MS: 464.4 (M+H)⁺. ¹H NMR (400 MHz, DMSO-*d₆*) δ 8.36 (d, *J* = 3.5 Hz, 1H), 7.78 (dd, *J =* 8.1, 1.5 Hz, 1H), 6.75 (dd, *J =* 8.1, 3.2 Hz, 1H), 5.30 - 5.15 (m, 1H), 4.86 - 4.59 (m, 1H), 4.53 (s, 2H), 4.50-4.30 (m, 2H), 4.20 - 4.00 (m, 3H), 3.49 - 3.40 (m, 2H), 3.17-3.09 (m, 1.5H), 2.90-2.81 (m, 1.5H), 2.50-2.42 (m, 1H), 2.28-2.09 (m, 0.5H), 1.89 - 1.61 (m, 2.5H), 1.55 (dd, *J* = 6.4, 3.7 Hz, 3H), 1.20 (dd, *J* = 32.7, 6.8 Hz, 3H).

### Example I-115

### 4- ((4R, 9S) -4, 9-dimethyl-8- (2, 6-dinitrogen hydrazine [3.3] helium-2-carbonyl) -3, 4, 7, 8, 9, 10-hydropyridine [3 ', 4 ': 3, 4] pyrazole [1, 5-a] pyrazine-2 (1 H) -yl) -3-fluoropyrazole [1, 5-a] pyridine-7-nitrile

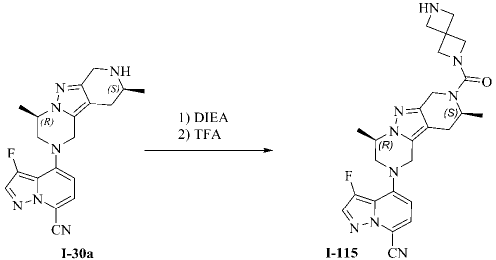

**Example I-115** was synthesized by following the protocols of **I-99 Example.** MS: 490.4 (M+H)⁺. ¹H NMR (400 MHz, DMSO-*d₆*) δ 8.32 (d, *J =* 3.6 Hz, 1H), 7.74 (d, *J =* 8.0 Hz, 1H), 6.70 (d, *J =* 8.1 Hz, 1H), 4.53 (d, *J =* 16.1 Hz, 1H), 4.49 - 4.45 (m, 2H), 4.38 (td, *J =* 11.0, 9.3, 5.8 Hz, 2H), 4.15 (d, *J =* 8.9 Hz, 2H), 4.00 (s, 2H), 3.98 (d, *J =* 2.3 Hz, 4H), 3.40 (d, *J* = 4.5 Hz, 1H), 2.72 (dd, *J* = 15.5, 5.9 Hz, 1H), 2.35 (d, *J* = 15.1 Hz, 1H), 1.51 (d, *J* = 6.4 Hz, 3H), 1.10 (d, *J =* 6.8 Hz, 3H).

### Example 1-116

### 4-((4R,9S)-4,9-dimethyl-8-(3,3,3-trifluoropropyl)-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

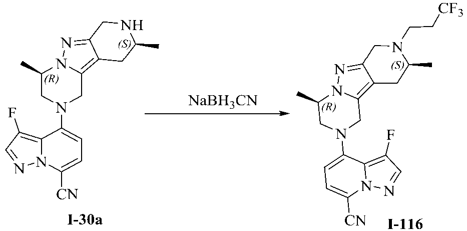

**Example I-116** was synthesized by following the protocols of **Example I-24.** MS: 463.1 (M+H)⁺. ¹H NMR (400 MHz, DMSO-*d₆*) δ 8.32 (d, *J =* 3.5 Hz, 1H), 7.74 (d, *J =* 8.0 Hz, 1H), 6.70 (d, *J* = 8.1 Hz, 1H), 4.56 - 4.32 (m, 3H), 4.02 (dd, *J* = 13.9, 4.2 Hz, 1H), 3.65 (s, 2H), 3.40 (s, 1H), 3.12-3.04 (m, 1H), 2.86 - 2.55 (m, 4H), 2.51 - 2.43 (m, 1H), 2.23 (dd, *J =* 15.2, 5.7 Hz, 1H), 1.50 (d, *J =* 6.4 Hz, 3H), 1.06 (d, *J =* 6.6 Hz, 3H).

### Example I-117

### 3-Fluoro-4-((4R,9S)-8-(2-hydroxy-2-methylpropyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

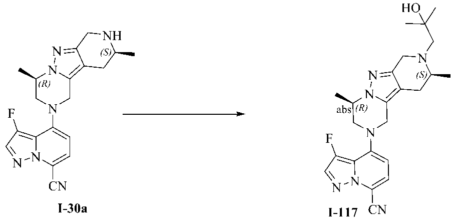

A reaction mixture of **Example I-30a** (150 mg, 0.41 mmol), isobutylene oxide (148 mg, 2.05 mmol) and TEA (125 mg, 1.23 mmol) in EtOH (5 mL) was stirred at 80 °C for 12 hours. The mixture was quenched with water and extracted with EtOAc, and the organic phase was washed with saturated saline, dried with Na₂SO₄ and concentrated. Purification by pre-HPLC gave **Example I-117** (49 Mg, 27.4%) as white solid. MS: 438.1 (M+H)⁺. ¹H NMR (400 MHz, DMSO-*d₆*) δ 8.31 (d, J = 3.6 Hz, 1H), 7.74 (d, J = 8.0 Hz, 1H), 6.71 (d, J = 8.1 Hz, 1H), 4.55 - 4.31 (m, 3H), 4.01 (dd, J = 13.5, 4.1 Hz, 1H), 3.78 (d, J = 1.7 Hz, 2H), 3.37 (d, J = 4.3 Hz, 1H), 3.11 (q, J = 5.9 Hz, 1H), 2.65-2.60 (m, 1H), 2.43-2.40 (m, 1H), 2.26-2.18 (m, 2H), 1.50 (d, J = 6.3 Hz, 3H), 1.10 (d, J = 6.3 Hz, 6H), 1.03 (d, J = 6.7 Hz, 3H).

### Example I-118

### 2-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-N,N-dimethylethane-1-sulfonamide

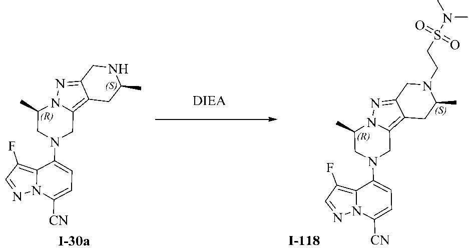

**Example 1-118** was synthesized by following the protocols of **Example 1-99.** MS: 501.1 (M+H)⁺. ¹H NMR (400 MHz, DMSO-*d₆*) δ 8.32 (d, J = 3.5 Hz, 1H), 7.74 (d, J = 8.0 Hz, 1H), 6.71 (d, J = 8.1 Hz, 1H), 4.61 - 4.54 (m, 1H), 4.52 - 4.37 (m, 4H), 4.22 (d, J = 16.1 Hz, 1H), 4.03 (dd, J = 13.7, 4.1 Hz, 1H), 3.45 - 3.39 (m, 1H), 3.38-3.21 (m, 1H), 3.24 - 3.17 (m, 1H), 2.86-2.79 (m, 1H), 2.62 - 2.55 (m, 2H), 2.40 (d, J = 15.4 Hz, 1H), 2.13 (s, 6H), 1.52 (d, J = 6.4 Hz, 3H), 1.20 (d, J = 6.9 Hz, 3H).

### Example I-119

### 4-((4R,9S)-4,9-dimethyl-8-(2-oxo-2-(2,6-diazaspiro[3.3]heptan-2-yl)ethyl)-3,4,7,8,9,10 -hexahydropyrido[3',4':3,4]pyrazolo[1,5-a]pyrazin-2(1H)-yl)-3-fluoropyrazolo[1,5-a] pyridine-7-carbonitrile

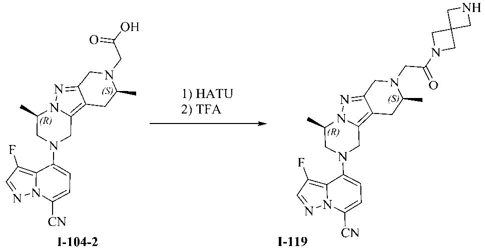

**Example I-119** was synthesized by following the protocols of **Example I-104.** MS: 504.5 (M+H)⁺. ¹H NMR (400 MHz, DMSO-*d₆*) δ 8.37 (s, 1H), 8.29 (d, J = 3.6 Hz, 1H), 7.71 (d, J = 8.0 Hz, 1H), 6.68 (d, J = 8.0 Hz, 1H), 4.50 - 4.37 (m, 2H), 4.35 (d, J = 10.3 Hz, 1H), 4.29 (s, 2H), 3.98 (s, 3H), 3.88 (s, 4H), 3.59 (s, 2H), 3.36 - 3.31 (m, 1H), 3.21 - 3.07 (m, 2H), 3.01 (q, J = 6.4 Hz, 1H), 2.61 (dd, J = 15.2, 4.8 Hz, 1H), 2.18 (dd, J = 15.2, 6.4 Hz, 1H), 1.46 (d, J = 6.4 Hz, 3H), 1.04 (d, J = 6.4 Hz, 3H).

### Example I-120

### 4-((4R,9S)-8-(D-prolyl)-4,9-dimethyl-3,4,7,8,9,10-hexahydropyrido [3',4':3,4] pyrazolo [1,5-a]pyrazin-2(1H)-yl)-3-fluoropyrazolo[1,5-a]pyridine-7-carbonitrile

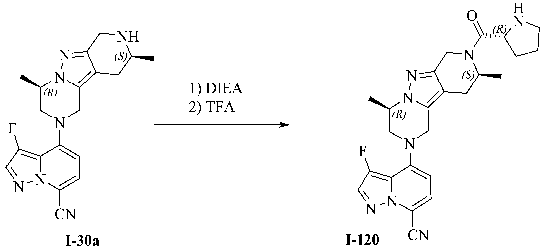

**Example I-120** was synthesized by following the protocols of **I-99 Example.** MS: 464.4 (M+H)⁺. ¹H NMR (400 MHz, DMSO-*d₆*) δ 8.29 (d, J = 3.6 Hz, 1H), 7.71 (d, J = 8.0 Hz, 1H), 6.68 (dd, J = 8.0, 1.5 Hz, 1H), 5.20 - 5.10 (m, 1H), 4.38 - 4.34 (m, 1H), 4.27 (d, J = 7.6 Hz, 1H), 3.99 (dd, J = 15.6, 8.8 Hz, 2H), 3.40 (dd, J = 13.6, 9.2 Hz, 1H), 3.08 (dt, J = 11.6, 6.4 Hz, 1H), 2.92 - 2.63 (m, 2H), 2.43 (dd, J = 25.6, 15.2 Hz, 1H), 2.23 (s, 1H), 1.84 - 1.58 (m, 3H), 1.50 (dd, J = 6.4, 2.8 Hz, 3H), 1.13 (dd, J = 36.4, 6.8 Hz, 3H).

### Example I-121

### 3-Fluoro-4-((4R,9S)-8-(2-(3-hydroxyazetidin-1-yl)-2-oxoethyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

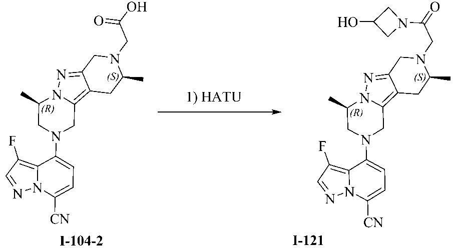

**Example I-121** was synthesized by following the protocols of **Example I-104.** MS: 479.1 (M+H)⁺. ¹H NMR (400 MHz, DMSO-*d₆*) δ 8.32 (d, J = 3.6 Hz, 1H), 7.74 (d, J = 8.0 Hz, 1H), 6.71 (d, J = 8.1 Hz, 1H), 5.70 (d, J = 6.1 Hz, 1H), 4.54 - 4.33 (m, 5H), 4.05 (dt, J = 16.4, 9.3 Hz, 2H), 3.93 (dd, J = 9.5, 4.3 Hz, 1H), 3.66 - 3.58 (m, 3H), 3.39-3.37 (m, 1H), 3.27 - 3.09 (m, 2H), 3.09 - 3.02 (m, 1H), 2.64 (dd, J = 15.1, 4.8 Hz, 1H), 2.20 (dd, J = 15.2, 6.3 Hz, 1H), 1.49 (d, J = 6.3 Hz, 3H), 1.06 (d, J = 6.5 Hz, 3H).

### Example I-122

### 4-((4R,9S)-4,9-dimethyl-8-((2R,6R)-6-methylmorpholine-2-carbonyl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

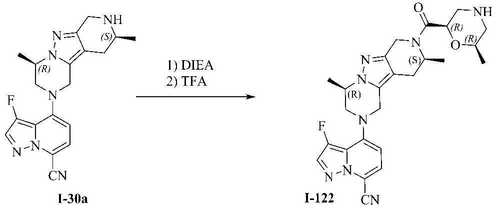

**Example I-122** was synthesized by following the protocols of **Example I-99.** MS: 494.4 (M+H)⁺. ¹H NMR (400 MHz, DMSO-*d₆*) δ 8.32 (d, J = 3.5 Hz, 1H), 8.22 (s, 1H), 7.74 (d, J = 7.9 Hz, 1H), 6.71 (dd, J = 8.1, 1.9 Hz, 1H), 5.22 - 4.96 (m, 1H), 4.74 - 4.63 (m, 1H), 4.49 (s, 2H), 4.45-4.38 (m, 1H), 4.36 - 4.23 (m, 1H), 4.05-3.98 (m, 1H), 3.89 (d, J = 16.8 Hz, 1H), 3.70-3.65 (m, 1H), 3.45-3.49(m, 1H), 2.80 (m, 5H), 2.49 - 2.27 (m, 1H), 1.53-1.47 (m, 3H), 1.19 (d, J = 6.6 Hz, 2H), 1.11-1.05 (m, 3H), 1.01 (d, J = 6.2 Hz, 1H).

### Example I-123

### 4-((4R,9S)-8-((3,3-difluorocyclobutyl)methyl)-4,9-dimethyl-3,4,7,8,9,10-hexahydro pyrido[3',4':3,4]pyrazolo[1,5-a]pyrazin-2(1H)-yl)-3-fluoropyrazolo[1,5-a]pyridine-7-carbonitrile

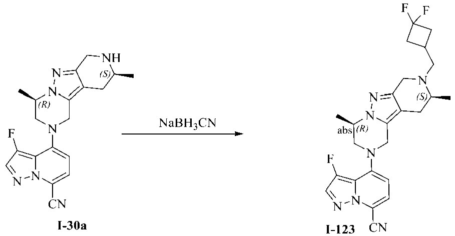

**Example I-123** was synthesized by following the protocols of **Example I-24.** MS: 471.1 (M+H)⁺. ¹H NMR (400 MHz, DMSO-*d₆*) δ 8.32 (d, *J* = 3.6 Hz, 1H), 7.74 (d, *J* = 8.0 Hz, 1H), 6.71 (d, *J* = 8.1 Hz, 1H), 4.53 - 4.32 (m, 3H), 4.02 (dd, *J* = 13.7, 4.1 Hz, 1H), 3.63-3.54 (m, 2H), 3.41-3.39 (m, 1H),3.05-2.99 (m, 1H), 2.71 - 2.64 (m, 2H), 2.63 - 2.57 (m, 3H), 2.40-2.30 (m, 1H), 2.27-2.19 (m, 3H), 1.49 (d, *J* = 6.4 Hz, 3H), 1.05 (d, *J* = 6.6 Hz, 3H).

### Example I-124

### 4-((4R,9S)-8-(cyanomethyl)-4,9-dimethyl-3,4,7,8,9,10-hexahydropyrido[3',4':3,4] pyrazolo[1,5-a]pyrazin-2(1H)-yl)-3-fluoropyrazolo[1,5-a]pyridine-7-carbonitrile

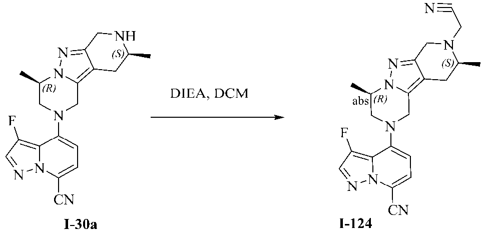

**Example I-124** was synthesized by following the protocols of **I-99 Example.** MS: 406.1 (M+H)⁺. ¹H NMR (400 MHz, DMSO-*d₆*) δ 8.32 (d, *J* = 3.5 Hz, 1H), 7.74 (d, *J* = 8.0 Hz, 1H), 6.71 (d, *J* = 8.1 Hz, 1H), 4.55 - 4.36 (m, 3H), 4.07 - 4.00 (m, 2H), 3.86 (d, *J =* 4.3 Hz, 1H), 3.82 (s, 1H), 3.55 (d, *J* = 14.4 Hz, 1H), 3.42-3.34 (m, 1H), 2.77 - 2.67 (m, 2H), 2.24 (dd, *J* = 15.0, 8.5 Hz, 1H), 1.49 (d, *J* = 6.3 Hz, 3H), 1.19 (d, *J* = 6.2 Hz, 3H).

### Example I-125

### 3-Fluoro-4-((4R,9S)-8-(2-methoxyethyl)-4,9-dimethyl-3,4,7,8,9,10-hexahydropyridine [3 ', 4 ': 3, 4] pyrazole [1,5-a] pyrazine-2(1H)-yl) pyrazole[1,5-a] pyridine-7-caronitrile

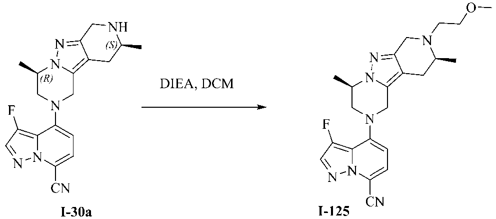

**Example I-125** was synthesized by following the protocols of **I-99 Example.** MS: 425.1 (M+H)⁺. ¹H NMR (400 MHz, DMSO-*d₆*) δ 8.32 (d, *J* = 3.6 Hz, 1H), 7.74 (d, *J* = 8.0 Hz, 1H), 6.70 (d, *J* = 8.1 Hz, 1H), 4.53 - 4.31 (m, 3H), 4.02 (dd, *J* = 13.6, 4.2 Hz, 1H), 3.64 (s, 2H), 3.47 (t, *J* = 6.0 Hz, 2H, 3.35 - 3.22 m), 1H, (3.27 s, 3H), 3.10 - 3.00 (m, 1H), 2.73 - 2.61 (m, 3H), 2.21 (dd, *J* = 15.2, 5.5 Hz, 1H), 1.49 (d, *J* = 6.4 Hz, 3H), 1.04 (d, *J* = 6.6 Hz, 3H.)

### Example I-126

### 4-((4R,9S)-4,9-dimethyl-8-(2,2,2-trifluoroethyl)-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

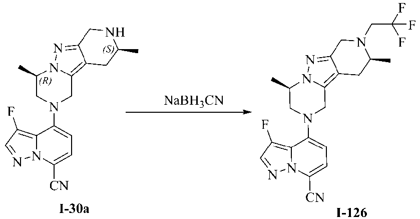

**Example I-126** was synthesized by following the protocols of **Example I-24.** MS: 449.1 (M+H)⁺. ¹H NMR (400 MHz, DMSO-*d₆*) δ 8.32 (d, *J* = 3.5 Hz, 1H), 7.74 (d, *J* = 8.0 Hz, 1H), 6.72 (d, *J =* 8.1 Hz, 1H), 4.55 - 4.32 (m, 3H), 4.03 (d, *J =* 10.9 Hz, 1H), 3.87-3.75 (m, 2H), 3.45-3.88 (m, 2H), 3.23 - 3.02 (m, 2H), 2.70-2.62 (m, 1H), 2.26 (dd, *J =* 15.5, 6.4 Hz, 1H), 1.50 (d, *J* = 6.4 Hz, 3H), 1.11 (d, *J* = 6.7 Hz, 3H).

### Example I-127

### 4-((4R,9S)-8-(2-(2,6-dimethylmorpholino)acetyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

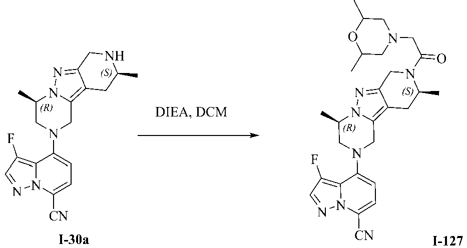

**Example I-127** was synthesized by following the protocols of **I-99 Example** MS: 521.1 (M+H)⁺. ¹H NMR (400 MHz, MeOH-*d₄*) δ 8.07 (d, *J* = 3.5 Hz, 1H), 7.49 (d, *J* = 7.9 Hz, 1H), 6.69 (d, *J* = 8.0 Hz, 1H), 5.38 - 5.08 (m, 1H), 4.53-4.44 (m, 3H), 4.12 - 3.96 (m, 2H), 3.82 - 3.70 (m, 1H), 3.49-3.40 (m, 2H), 3.36 (d, *J* = 1.6 Hz, 2H), 3.34 (d, *J* = 1.7 Hz, 2H), 3.03 - 2.73 (m, 2H), 2.61-2.48 (m, 1.5H), 2.32 - 2.17 (m, 0.5H), 1.94 - 1.87 (m, 1H), 1.62 (dd, *J* = 6.6, 2.7 Hz, 3H), 1.30 - 1.12 (m, 9H).

### Example I-128

### 4-((4R,9S)-4,9-dimethyl-8-(2-(4-methylpiperazin-1-yl)-2-oxoethyl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

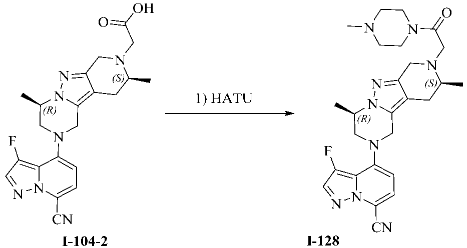

**Example I-128** was synthesized by following the protocols of **Example I-104.** MS: 479.1 (M+H)⁺. ¹H NMR (400 MHz, DMSO-*d₆*) δ 8.32 (d, J = 3.6 Hz, 1H), 7.74 (d, J = 8.0 Hz, 1H), 6.71 (d, J = 8.1 Hz, 1H), 4.55 - 4.33 (m, 3H), 4.06 - 3.97 (m, 1H), 3.67 (d, J = 15.1 Hz, 1H), 3.60 - 3.53 (m, 3H), 3.41 (s, 5H), 3.11 (q, J = 5.8 Hz, 1H), 2.65 (dd, J = 15.1, 5.0 Hz, 1H), 2.32 - 2.20 (m, 5H), 2.18 (s, 3H), 1.49 (d, J = 6.4 Hz, 3H), 1.05 (d, J = 6.5 Hz, 3H).

### Example I-129

### 4-((4R,9S)-8-(2-cyanoethyl)-4,9-dimethyl-3,4,7,8,9,10-hexahydropyridine[3',4':3,4] pyrazole[1,5-a] pyrazine-2 (1 H)-yl)-3-fluoropyrazole [1,5-a] pyridine-7-nitrile

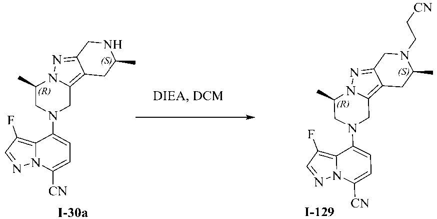

**Example I-129** was synthesized by following the protocols of **Example I-99.** MS: 419.1 (M+H)⁺.

### Example I-130

### 3-Fluoro-4-((4R,9S)-8-(2-(4-(2-hydroxyethyl)pyridoxine-1-yl)-2-oxyethyl)-4,9-dimethyl-3,4,7,8,9,10-hydropyridote [3 ', 4 ': 3, 4] pyrazole [1, 5-a] pyrazine-2 (1 H) - yl) pyrazole [1, 5-a] pyridine-7-nitrile

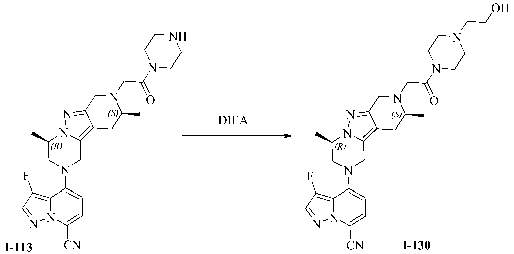

**Example I-130** was synthesized by following the protocols of **Example I-99.** MS: 492.6 (M+H)⁺. ¹H NMR (400 MHz, DMSO-*d₆*) δ 8.31 (d, *J* = 3.5 Hz, 1H), 7.74 (d, *J* = 8.0 Hz, 1H), 6.70 (d, *J* = 8.1 Hz, 1H), 4.55 - 4.33 (m, 4H), 4.01 (d, *J* = 11.3 Hz, 1H), 3.70 - 3.35 (m, 10H), 3.11 (q, *J =* 5.7 Hz, 1H), 2.65 (dd, *J* = 15.3, 5.0 Hz, 1H), 2.42 - 2.35 (m, 6H), 2.22 (dd, *J* = 15.2, 5.4 Hz, 1H), 1.49 (d, *J* = 6.4 Hz, 3H), 1.05 (d, *J* = 6.6 Hz, 3H).

### Example I-131

### 3-Fluoro-4-((4R, 9S)-8-(2-(4- (3-hydroxypropyl) pyridoxine-1-yl)-2-oxygenyl) -4, 9-dimethyl-3, 4, 7, 8, 9, 10-hydropyridoxine [3 ', 4 ': 3, 4] pyrazole [1, 5-a] pyrazine-2 (1 H) -yl) pyrazole [1, 5-a] pyridine-7-nitrile

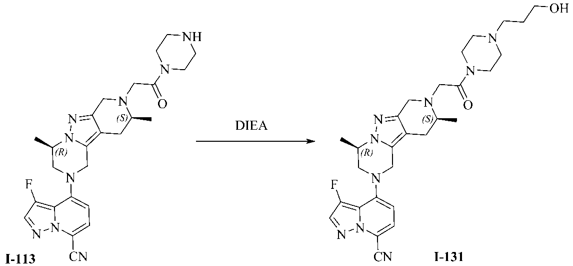

**Example I-131** was synthesized by following the protocols of **Example I-99.** MS: 550.1 (M+H)⁺. ¹H NMR (400 MHz, DMSO-*d₆*) δ 8.30 (d, *J =* 3.6 Hz, 1H), 7.73 (d, *J =* 8.0 Hz, 1H), 6.70 (d, *J* = 8.0 Hz, 1H), 4.53 - 4.39 (m, 2H), 4.36 (s, 1H), 4.00 (d, *J* = 13.5 Hz, 1H), 3.70 - 3.58 (m, 2H), 3.54 (d, *J* = 6.2 Hz, 2H), 3.43 (t, *J* = 6.3 Hz, 4H), 3.39 (s, 1H), 3.34 (s, 3H), 3.10 (q, *J* = 5.9 Hz, 1H), 2.64 (dd, *J* = 15.1, 5.0 Hz, 1H), 2.37 - 2.28 (m, 6H), 2.22 (dd, *J =* 15.2, 5.3 Hz, 1H), 1.58 (p, *J =* 6.6 Hz, 2H), 1.48 (d, *J* = 6.3 Hz, 3H), 1.04 (d, *J* = 6.5 Hz, 3H).

### Example I-132

### 5-((4R,9S)-4,9-dimethyl-3,4,7,8,9,10-hexahydropyrido[3',4':3,4]pyrazolo[1,5-a]pyrazin-2(1H)-yl)quinoline-8-carbonitrile

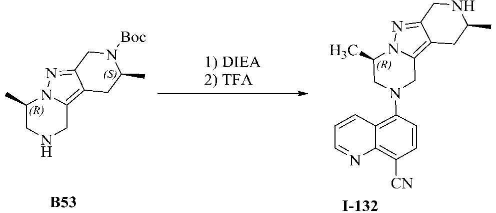

**Example I-132** was synthesized by following the protocols of **Example I-2.** MS: 359.1 (M+H)⁺. ¹H NMR (400 MHz, DMSO-*d₆*) δ 9.09 (dd, *J* = 4.2, 1.6 Hz, 1H), 8.62 (dd, *J =* 8.8, 1.6 Hz, 1H), 8.32 (d, *J* = 7.6 Hz, 2H), 7.72 (dd, *J* = 8.4, 4.0 Hz, 1H), 7.38 (d, *J* = 8.0 Hz, 1H), 4.60 (d, *J* = 5.6 Hz, 1H), 4.42 (s, 2H), 4.01 (t, *J* = 16.0 Hz, 2H), 3.91 (dd, *J* = 13.2, 4.0 Hz, 1H), 3.29 (dd, *J* = 13.2, 9.2 Hz, 1H), 3.13 - 3.03 (m, 1H), 2.67 (dd, *J* = 15.2, 4.0 Hz, 1H), 2.22 (dd, *J* = 15.2, 10.4 Hz, 1H), 1.51 (d, *J* = 6.4 Hz, 3H), 1.25 (d, *J* = 6.4 Hz, 3H).

### Example I-133

### 4-((4R,9S)-4,9-dimethyl-8-(oxazol-2-ylmethyl)-3,4,7,8,9,10-hexahydropyrido[3',4':3,4] pyrazolo[1,5-a]pyrazin-2(1H)-yl)-3-fluoropyrazolo[1,5-a]pyridine-7-carbonitrile

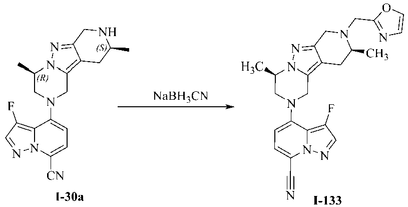

**Example 1-133** was synthesized by following the protocols of **Example 1-24.** MS: 447.1 (M+H)⁺. ¹H NMR (400 MHz, DMSO-*d₆*) δ 8.31 (d, *J* = 3.6 Hz, 1H), 8.10 (s, 1H), 7.73 (d, *J =* 8.0 Hz, 1H), 7.20 (s, 1H), 6.70 (d, *J =* 8.1 Hz, 1H), 4.56 - 4.29 (m, 3H), 4.07 - 3.89 (m, 2H), 3.88-3.78 (m, 1H), 3.74-3.54 (m, 2H), 3.06 (s, 1H), 2.69 (dd, *J =* 16.9, 3.8 Hz, 1H), 2.26 (dd, *J =* 15.9, 6.3 Hz, 1H), 1.48 (d, *J =* 6.4 Hz, 3H), 1.15 (d, *J =* 6.5 Hz, 3H).

### Example I-134

### 4-((4R,9S)-8-((1H-pyrazol-5-yl)methyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

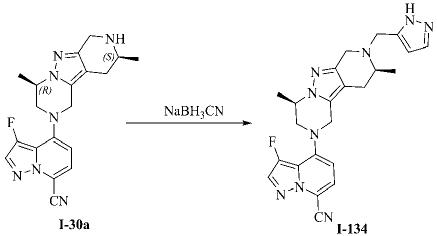

**Example I-134** was synthesized by following the protocols of **Example I-24.** MS: 447.1 (M+H)⁺. ¹H NMR (400 MHz, DMSO-*d₆*) δ 8.29 (d, *J* = 3.6 Hz, 1H), 8.14 (s, 0H), 7.71 (d, *J* = 8.0 Hz, 1H), 7.54 (d, *J* = 2.0 Hz, 1H), 6.68 (d, *J* = 8.0 Hz, 1H), 6.15 (d, *J =* 2.0 Hz, 1H), 4.44 (q, *J* = 15.2 Hz, 2H), 4.32 (ddd, *J* = 10.4, 6.4, 4.4 Hz, 1H), 3.98 (dd, *J =* 13.6, 4.4 Hz, 1H), 3.69 (s, 2H), 3.54 - 3.46 (m, 2H), 3.34 (m, 1H), 3.03 (q, *J =* 5.6 Hz, 1H), 2.66 (dd, *J* = 15.2, 5.2 Hz, 1H), 2.22 (dd, *J* = 15.2, 5.2 Hz, 1H), 1.45 (d, *J* = 6.4 Hz, 3H), 1.10 (d, *J* = 6.4 Hz, 3H).

### Example I-135

### 4-((4R,9S)-8-((1H-pyrazol-5-yl)methyl)-4,9-dimethyl-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

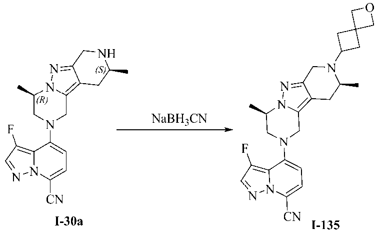

**Example I-135** was synthesized by following the protocols of **Example I-24.** MS: 462.1 (M+H)⁺. ¹H NMR (400 MHz, DMSO-*d₆*) δ 8.29 (d, *J* = 3.6 Hz, 1H), 8.14 (s, 1H), 7.71 (d, *J* = 8.0 Hz, 1H), 6.67 (d, *J* = 8.0 Hz, 1H), 4.57 (s, 2H), 4.43 (s, 2H), 4.42 (t, *J =* 6.4 Hz, 2H), 4.32 (td, *J* = 8.0, 6.4, 4.4 Hz, 1H), 3.98 (dd, *J* = 14.0, 4.4 Hz, 1H), 3.37 (s, 2H), 3.33 (s, 1H), 3.09 (dt, *J* = 8.0, 4.0 Hz, 1H), 2.95 (q, *J* = 7.6 Hz, 1H), 2.62 (dd, *J =* 15.2, 5.6 Hz, 1H), 2.35 (dt, *J* = 11.2, 5.6 Hz, 2H), 2.18 (dd, *J =* 15.2, 2.8 Hz, 1H), 1.93 (ddd, *J* = 12.0, 8.4, 2.0 Hz, 2H), 1.46 (d, *J* = 6.4 Hz, 3H), 0.91 (d, *J* = 6.4 Hz, 3H).

### Example I-136

### 4-((4R,9S)-8-(2-(4-cyclopropylpiperazin-1-yl)-2-oxoethyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

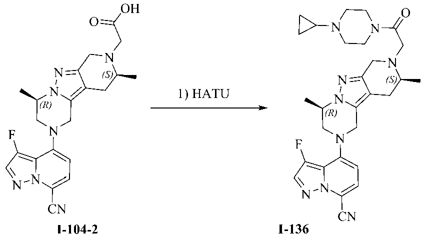

**Example I-136** was synthesized by following the protocols of **Example I-104.** MS: 532.7 (M+H)⁺. ¹H NMR (400 MHz, DMSO-*d₆*) δ 8.31 (d, *J =* 3.5 Hz, 1H), 7.73 (d, *J =* 8.0 Hz, 1H), 6.70 (d, *J* = 8.1 Hz, 1H), 4.57 - 4.32 (m, 3H), 4.01 (dd, *J* = 13.9, 4.1 Hz, 1H), 3.80-3.55 (m, 2H), 3.51-3.48 (m, 2H), 3.44-3.38 (m, 4H), 3.37 -3.34 (m, 3H), 3.16 (s, 1H), 2.73 - 2.63 (m, 1H), 2.50 - 2.47 (m, 2H), 2.25 (dd, *J =* 15.3, 5.3 Hz, 1H), 1.64 - 1.59 (m, 1H), 1.49 (d, *J =* 6.3 Hz, 3H), 1.07 (d, *J =* 6.5 Hz, 3H), 0.45 -0.41 (m, 2H), 0.35 -0.32 (m, 2H).

### Example I-137

### 4-((4R,9S)-8-((1H-pyrazol-4-yl)methyl)-4,9-dimethyl-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

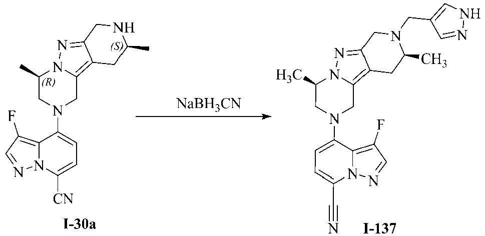

**Example I-137** was synthesized by following the protocols of **Example I-24.** MS: 447.1 (M+H)⁺.

### Example I-138

### 4-((4R,9S)-8-(2-(azetidin-1-yl)-2-oxoethyl)-4,9-dimethyl-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

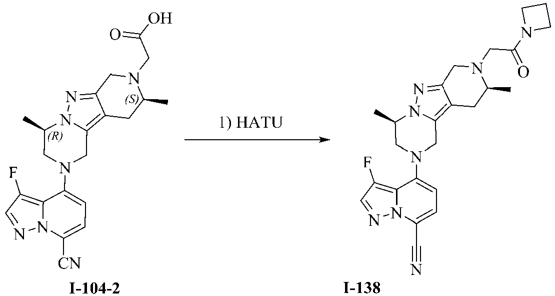

**Example I-138** was synthesized by following the protocols of **Example I-104.** MS: 464.1 (M+H)⁺. ¹H NMR (400 MHz, DMSO-*d₆*) δ 8.31 (d, J = 3.5 Hz, 1H), 7.73 (d, J = 8.0 Hz, 1H), 6.70 (d, J = 8.1 Hz, 1H), 4.55 - 4.32 (m, 3H), 4.19 (t, J = 7.7 Hz, 2H), 4.01 (dd, J = 13.8, 4.1 Hz, 1H), 3.87 (t, J = 7.7 Hz, 2H), 3.67 - 3.59 (m, 2H), 3.39 (s, 1H), 3.23 - 3.02 (m, 3H), 2.63 (dd, J = 15.2, 4.8 Hz, 1H), 2.23 - 2.14 (m, 3H), 1.49 (d, J = 6.4 Hz, 3H), 1.06 (d, J = 6.5 Hz, 3H).

### Example I-139

### 4-((4R,9S)-4,9-dimethyl-8-(2-morpholino-2-oxoethyl)-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

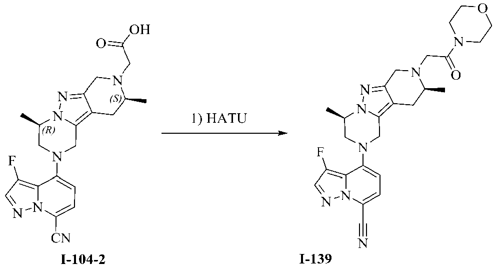

**Example I-139** was synthesized by following the protocols of **Example I-104.** MS: 493.5 (M+H)⁺. ¹H NMR (400 MHz, DMSO-*d₆*) δ 8.31 (d, *J =* 3.5 Hz, 1H), 7.73 (d, *J =* 8.0 Hz, 1H), 6.70 (d, *J* = 8.1 Hz, 1H), 4.53 - 4.32 (m, 3H), 4.01 (d, *J* = 11.0 Hz, 1H), 3.69-3.54 (m, 8H), 3.50 - 3.38 (m, 5H), 3.11 (q, *J* = 5.8 Hz, 1H), 2.65 (dd, *J* = 15.2, 5.0 Hz, 1H), 2.22 (dd, *J =* 15.2, 5.3 Hz, 1H), 1.48 (d, *J* = 6.4 Hz, 3H), 1.04 (d, *J* = 6.6 Hz, 3H).

### Example I-140

### 4-((4R,9S)-8-(2-(3,3-difluoroazetidin-1-yl)-2-oxoethyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

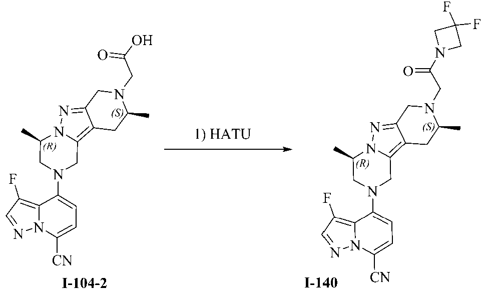

**Example I-140** was synthesized by following the protocols of **Example I-104.** MS: 499.5 (M+H)⁺. ¹H NMR (400 MHz, DMSO-*d₆*) δ 8.31 (d, *J* = 3.5 Hz, 1H), 7.73 (d, *J* = 8.0 Hz, 1H), 6.70 (d, *J* = 8.1 Hz, 1H), 4.65 (t, *J* = 12.5 Hz, 2H), 4.53 - 4.40 (m, 2H), 4.40 - 4.28 (m, 3H), 4.01 (d, *J =* 10.8 Hz, 1H), 3.69 - 3.57 (m, 2H), 3.38 (s, 0.5H), 3.33 (s, 0.5H), 3.25 (d, *J* = 15.1 Hz, 1H), 3.06 (s, 1H), 2.65 (d, *J* = 13.5 Hz, 0.5H), 2.24 - 2.19 (m, 0.5H), 2.09 (s, 2H), 1.48 (d, *J* = 6.4 Hz, 3H), 1.06 (d, *J* = 6.5 Hz, 3H).

### Example I-141

### 2-((4R,9S)-2-(8-cyanoquinolin-5-yl)-4,9-dimethyl-1,2,3,4,9,10-hexahydropyrido [3',4':3,4]pyrazolo[1,5-a]pyrazin-8(7H)-yl)acetamide

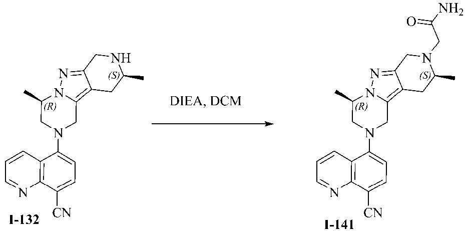

**Example I-141** was synthesized by following the protocols of **Example I-99,.** MS: 416.5 (M+H)⁺. ¹H NMR (400 MHz, DMSO-*d₆*) δ 9.09 (dd, *J* = 4.0, 1.6 Hz, 1H), 8.62 (dd, *J* = 8.4, 1.6 Hz, 1H), 8.32 (d, *J* = 8.0 Hz, 1H), 7.72 (dd, *J =* 8.4, 4.0 Hz, 1H), 7.38 (d, *J =* 8.0 Hz, 1H), 7.25 (d, *J =* 3.2 Hz, 1H), 7.18 (d, *J =* 3.2 Hz, 1H), 4.59 (d, *J =* 8.8 Hz, 1H), 4.48 - 4.35 (m, 2H), 3.89 (dd, *J =* 13.2, 4.4 Hz, 1H), 3.74 - 3.61 (m, 2H), 3.32 - 3.26 (m, 1H), 3.16 - 3.03 (m, 2H), 2.96 (d, *J =* 16.0 Hz, 1H), 2.66 (dd, *J =* 15.2, 4.8 Hz, 1H), 2.23 (dd, *J* = 15.2, 6.4 Hz, 1H), 1.51 (d, *J=* 6.4 Hz, 3H), 1.08 (d, *J =* 6.4 Hz, 3H).

### Example I-142

### 4-((4R,9S)-4,9-dimethyl-8-(tetrahydrofuran-3-yl)-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

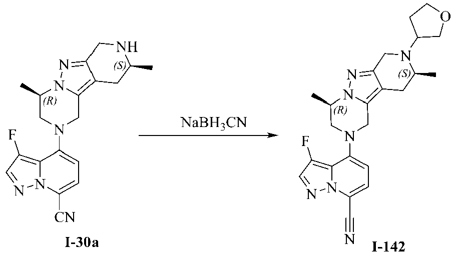

**I-142 Example** was synthesized by following the protocols of **Example I-24.** MS: 436.5 (M+H)⁺. ¹H NMR (400 MHz, DMSO-*d₆*) δ 8.33 - 8.29 (m, 2H), 7.73 (d, *J =* 8.0 Hz, 1H), 6.69 (d, *J =* 8.1 Hz, 1H), 4.50 - 4.40 (m, 2H), 4.37 - 4.32 (m, 1H), 4.00 (dd, *J* = 13.6, 4.1 Hz, 1H), 3.92- 3.87 (m, 1H), 3.84 - 3.81 (m, 1H), 3.73 - 3.65 (m, 1H), 3.64 - 3.60 (m, 1H), 3.54 - 3.50 (m, 1H), 3.47 - 3.42 (m, 1H), 3.40 - 3.34 (m, 1H), 3.32 - 3.27 (m, 1H), 3.26 - 3.12 (m, 1H), 2.80 - 2.70 (m, 1H), 2.24 - 2.19 (m, 1H), 2.14 - 2.00 (m, 1H), 1.78 - 1.72 (m, 1H), 1.51 - 1.46 (m, 3H), 1.00 - 0.97 (m, 3H).

### Example I-143

### 4-((4R,9S)-4,9-dimethyl-8-(oxetan-3-yl)-3,4,7,8,9,10-hexahydropyrido[3',4':3,4] pyrazolo[1,5-a]pyrazin-2(1H)-yl)-3-fluoropyrazolo[1,5-a]pyridine-7-carbonitrile

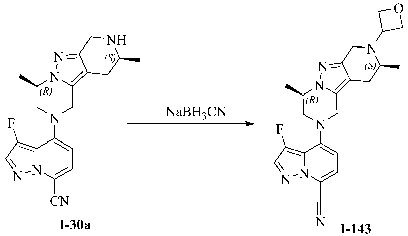

**Example I-143** was synthesized by following the protocols of **Example I-24.** MS: 422.1 (M+H)⁺. ¹H NMR (400 MHz, DMSO-*d₆*) δ 8.29 (d, *J* = 3.2 Hz, 1H), 7.71 (d, *J* = 8.0 Hz, 1H), 6.67 (d, *J* = 8.0 Hz, 1H), 4.55 - 4.48 (m, 4H), 4.47 - 4.37 (m, 2H), 4.36 - 4.28 (m, 1H), 3.99 (dd, *J* = 14.0, 4.0 Hz, 1H), 3.88 (p, *J* = 6.8 Hz, 1H), 3.48 (d, *J* = 14.8 Hz, 1H), 3.37 (s, 2H), 2.88 (h, *J* = 6.0 Hz, 1H), 2.62 (dd, *J* = 15.2, 5.2 Hz, 1H), 2.20 (dd, *J* = 15.2, 4.8 Hz, 1H), 1.46 (d, *J* = 6.4 Hz, 3H), 0.92 (d, *J* = 6.4 Hz, 3H).

### Example I-144

### 4-((4R,9S)-8-(2-((3S,5S)-3,5-dimethylpiperazin-1-yl)-2-oxoethyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

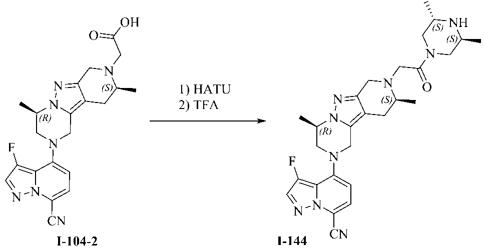

**Example I-144** was synthesized by following the protocols of **Example I-104.** MS: 520.5 (M+H)⁺. ¹H NMR (400 MHz, DMSO-*d₆*) δ 8.31 (d, *J* = 3.5 Hz, 1H), 7.74 (d, *J* = 8.0 Hz, 1H), 6.71 (d, *J* = 8.1 Hz, 1H), 4.55 - 4.31 (m, 3H), 4.02 (dd, *J* = 13.7, 4.1 Hz, 1H), 3.92 - 3.78 (m, 1H), 3.72-3.68 (m, 1H), 3.60 - 3.38 (m, 10H), 3.19 - 3.15 (m, 1H), 2.65 (dd, *J* = 15.3, 5.1 Hz, 1H), 2.24 (dd, *J* = 15.2, 5.4 Hz, 1H), 1.49 (d, *J* = 6.3 Hz, 3H), 1.20 (t, *J* = 5.8 Hz, 6H), 1.07 (d, *J* = 6.5 Hz, 3H).

### Example I-145

### 4-((4R,9S)-8-(2-((3S,5S)-3,5-dimethylpiperazin-1-yl)-2-oxoethyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

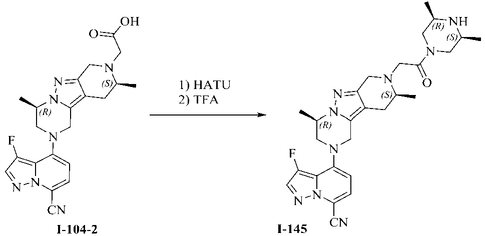

**Example I-145** was synthesized by following the protocols of **Example I-104.** MS: 520.5 (M+H)⁺. ¹H NMR (400 MHz, DMSO-*d₆*) δ 8.31 (d, *J* = 3.5 Hz, 1H), 7.74 (d, *J =* 8.0 Hz, 1H), 6.71 (d, *J* = 8.1 Hz, 1H), 4.55 - 4.32 (m, 4H), 4.18 (d, *J* = 13.4 Hz, 1H), 4.06 - 3.97 (m, 1H), 3.22 - 3.18 (m, 2H), 2.97 (t, *J* = 12.3 Hz, 1H), 2.75-2.62 (m, 2H), 2.59-2.56 (m, 1H), 2.27 (s, 1H, 1.49) d, (*J =* 6.4 Hz, 3H), 1.22 (dt, *J =* 11.7, 4.5 Hz, 6H), 1.08 (s, 3H).

### Example I-146

### 4-((4R,9S)-8-(2-((3R,5R)-3,5-dimethylpiperazin-1-yl)-2-oxoethyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

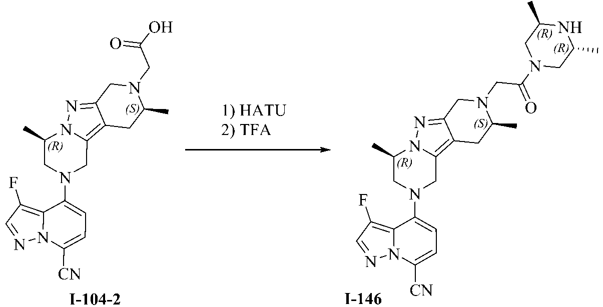

**Example I-146** was synthesized by following the protocols of **Example I-104.** MS: 520.5 (M+H)⁺. ¹H NMR (400 MHz, DMSO-*d₆*) δ 9.11 (s, 2H), 8.33 (d, *J* = 3.5 Hz, 1H), 7.76 (d, *J* = 8.0 Hz, 1H), 6.74 (d, *J* = 8.1 Hz, 1H), 4.63 - 4.24 (m, 6H), 4.07 (d, *J* = 13.2 Hz, 1H), 3.92 - 3.69 (m, 4H), 3.60 (s, 1H), 3.22 (s, 1H), 2.98 (s, 1H), 2.69 (s, 1H), 1.52 (d, *J* = 6.3 Hz, 3H), 1.45 - 1.29 (m, 3H), 1.23 (d, *J* = 7.7 Hz, 6H).

### Example I-149

### 3-Fluoro-4-((4R,9S)-8-(2-((R)-3-fluoropyrrolidin-1-yl)-2-oxoethyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

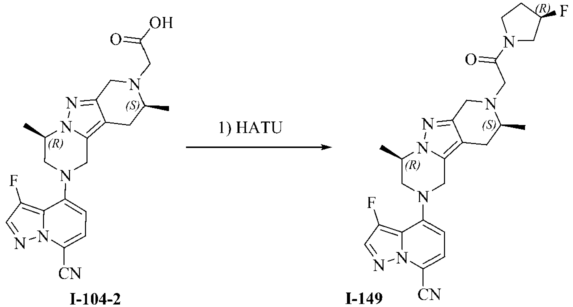

**Example I-149** was synthesized by following the protocols of **Example I-104.** MS: 496.3 (M+H)⁺. ¹H NMR (400 MHz, DMSO-*d₆*) δ 8.31 (d, *J =* 3.6 Hz, 1H), 7.73 (d, *J =* 8.0 Hz, 1H), 6.70 (d, *J* = 8.1 Hz, 1H), 5.46 - 5.22 (m, 1H), 4.55 - 4.31 (m, 3H), 4.04 - 3.36 (m, 9H), 3.32 - 3.29 (m, 1H), 3.15 (s, 1H), 2.71 - 2.61 (m, 1H), 2.30 - 1.94 (m, 3H), 1.52 - 1.44 (m, 3H), 1.08 (t, *J =* 5.7 Hz, 3H).

### Example I-150

### 4-((4R,9S)-8-(2-cyano-2-methylpropyl)-4,9-dimethyl-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

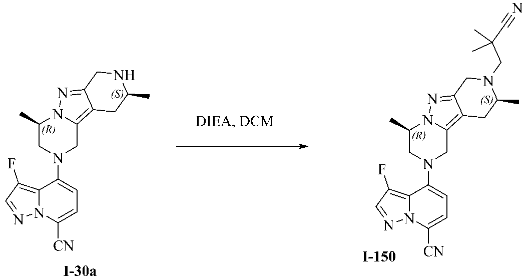

**Example I-150** was synthesized by following the protocols of **Example I-99.** MS: 447.4 (M+H)⁺. ¹H NMR (400 MHz, DMSO-*d₆*) δ 8.31 (d, J = 3.5 Hz, 1H), 7.73 (d, J = 8.0 Hz, 1H), 6.71 (d, J = 8.1 Hz, 1H), 4.54 - 4.31 (m, 3H), 4.01 (dd, J = 13.5, 4.2 Hz, 1H), 3.82 (s, 2H), 3.44 - 3.35 (m, 1H), 3.18-3.14 (m, 1H), 2.72 - 2.62 (m, 2H), 2.52-2.47 (m, 1H), 2.23 (dd, J = 15.3, 4.9 Hz, 1H), 1.50 (d, J = 6.4 Hz, 3H), 1.29 (d, J = 3.2 Hz, 6H), 1.06 (d, J = 6.6 Hz, 3H).

### Example I-151

### 4-((4R,9S)-4,9-dimethyl-8-(2-oxo-2-(pyrrolidin-1-yl)ethyl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

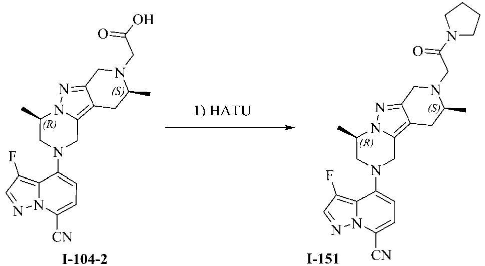

**Example 1-151** was synthesized by following the protocols of **Example** I**-104.** MS: 477.3 (M+H)⁺. ¹H NMR (400 MHz, DMSO-*d₆*) δ 8.31 (d, *J* = 3.5 Hz, 1H), 7.73 (d, *J* = 8.0 Hz, 1H), 6.70 (d, *J* = 8.1 Hz, 1H), 4.54 - 4.30 (m, 3H), 4.01 (dd, *J* = 13.8, 4.2 Hz, 1H), 3.67 (q, *J* = 15.1 Hz, 2H), 3.50 (t, *J* = 6.7 Hz, 2H), 3.38 (d, *J* = 8.7 Hz, 3H), 3.27 (d, *J =* 14.9 Hz, 2H), 3.15 - 3.11 (m, 1H), 2.63 (dd, *J =* 15.1, 4.8 Hz, 1H), 2.20 (dd, *J =* 15.1, 6.2 Hz, 1H), 1.91 - 1.81 (m, 2H), 1.80 - 1.75 (m, 2H), 1.48 (d, *J* = 6.3 Hz, 3H), 1.07 (d, *J* = 6.5 Hz, 3H).

### Example I-152

### 4-((4R,9S)-8-(2-(3,3-difluoropyrrolidin-1-yl)-2-oxoethyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

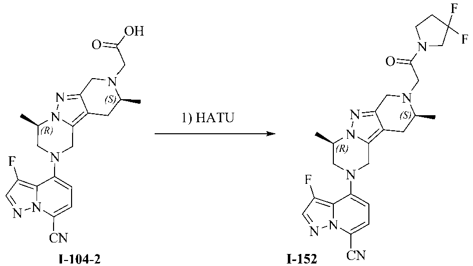

**Example 1-152** was synthesized by following the protocols of **Example 1-104.** MS: 513.3 (M+H)⁺. ¹H NMR (400 MHz, DMSO-*d₆*) δ 8.31 (d, *J* = 3.5 Hz, 1H), 7.74 (d, *J* = 8.0 Hz, 1H), 6.71 (d, *J* = 8.1 Hz, 1H), 4.56 - 4.33 (m, 3H), 4.03 (t, *J* = 12.9 Hz, 2H), 3.90 - 3.50 (m, 5H), 3.41 - 3.34 (m, 3H), 3.17 (s, 1H), 2.84 - 2.55 (m, 1H), 2.51 - 2.15 (m, 3H), 1.49 (d, *J* = 6.3 Hz, 3H), 1.10 (s, 3H).

### Example I-153

### 3-Fluoro-4-((4R,9S)-8-(2-((S)-3-fluoropyrrolidin-1-yl)-2-oxoethyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

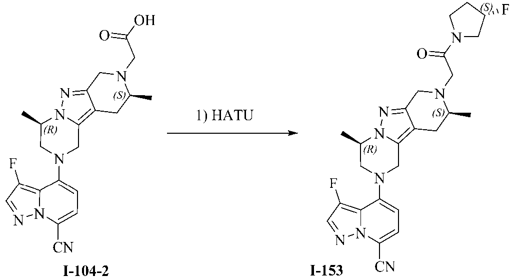

**Example I-153** was synthesized by following the protocols of **Example I-104.** MS: 496.3 (M+H)⁺. ¹H NMR (400 MHz, DMSO-*d₆*) δ 8.31 (d, *J =* 3.5 Hz, 1H), 7.73 (d, *J =* 8.0 Hz, 1H), 6.70 (d, *J =* 8.1 Hz, 1H), 5.34 (d, *J =* 53.2, 26.3 Hz, 1H), 4.55 - 4.31 (m, 3H, 4.05 - 3.97 m, 1H), 3.95 - 3.72 (m, 1H), 3.72 - 3.48 (m, 4H), 3.45 - 3.35 (m, 3H), 3.34 - 3.28 (m, 2H), 3.15 - 3.09 (m, 1H), 2.64 (dd, *J =* 15.0, 4.8 Hz, 1H), 2.22 - 1.98 (m, 2H, 1.49 d, *J =* 6.3 Hz, 3H), 1.08 (dd, *J =* 6.6, 1.8 Hz, 3H).

### Example I-154

### N-(tert-butyl)-2-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)acetamide

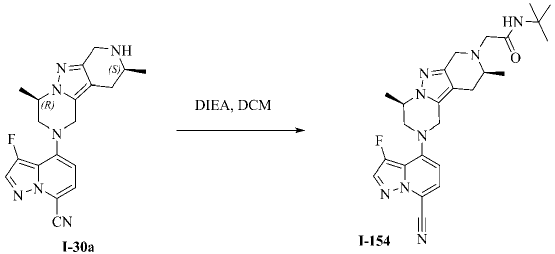

**Example I-154** was synthesized by following the protocols of **Example I-99.** MS: 480.4 (M+H)⁺. ¹H NMR (400 MHz, DMSO-*d₆*) δ 8.30 (d, *J =* 3.5 Hz, 1H), 7.73 (d, *J =* 8.0 Hz, 1H), 7.23 (s, 1H), 6.70 (d, *J =* 8.1 Hz, 1H), 4.56 - 4.31 (m, 3H), 4.01 (dd, *J =* 13.8, 4.1 Hz, 1H), 3.75 - 3.59 (m, 2H), 3.38 (d, *J =* 9.4 Hz, 1H), 3.13 - 3.01 (m, 2H), 2.89 (d, *J =* 16.0 Hz, 1H), 2.64 (dd, *J =* 15.3, 4.9 Hz, 1H), 2.24 (dd, *J =* 15.3, 6.3 Hz, 1H), 1.49 (d, *J =* 6.4 Hz, 3H), 1.30 (s, 9H), 1.07 (d, *J =* 6.6 Hz, 3H) .

### Example I-155

### 4-((4R,9S)-4,9-dimethyl-8-((R)-2-oxopyrrolidin-3-yl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

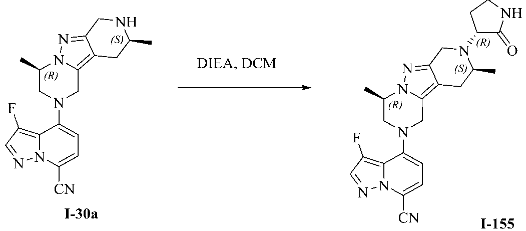

**Example I-155** was synthesized by following the protocols of **Example I-99.** MS: 449.4 (M+H)⁺. ¹H NMR (400 MHz, DMSO-*d₆*) δ 8.30 (d, *J* = 3.5 Hz, 1H), 7.72 (d, *J* = 8.0 Hz, 1H), 7.68 (s, 1H), 6.69 (d, *J* = 8.1 Hz, 1H), 4.54 - 4.28 (m, 3H), 4.04 - 3.94 (m, 1H), 3.76-3.72 (m, 1H), 3.69 - 3.59 (m, 2H), 3.47-3.43 (m, 1H), 3.40 - 3.34 (m, 1H), 3.25 - 3.09 (m, 2H), 2.64 (dd, *J* = 14.9, 4.7 Hz, 1H), 2.17 (dd, *J* = 15.2, 6.0 Hz, 2H), 2.07 - 1.96 (m, 1H), 1.48 (d, *J* = 6.3 Hz, 3H), 1.14 (d, *J* = 6.4 Hz, 3H).

### Example I-156

### 2-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)propanamide

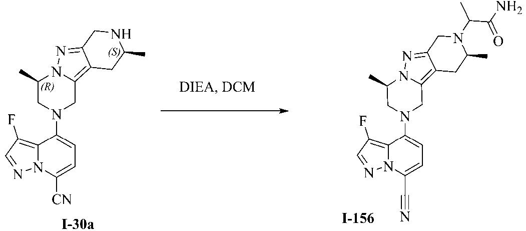

**Example 1-156** was synthesized by following the protocols of **Example I-99.** MS: 437.4 (M+H)⁺.¹H NMR (400 MHz, DMSO-*d₆*) δ 8.31 (d, *J* = 3.5 Hz, 1H), 7.74 (d, *J* = 7.9 Hz, 1H), 7.09 (d, *J* = 63.8 Hz, 1H), 6.71 (d, *J* = 8.2 Hz, 1H), 4.52 - 4.38 (m, 3H), 4.11 - 3.43 (m, 3H), 3.41 - 3.37 (m, 3H), 3.17 (s, 1H), 2.72 (d, *J =* 21.4 Hz, 1H), 2.31 (d, *J =* 28.7 Hz, 0.5H), 1.49 (dd, *J =* 6.6, 2.0 Hz, 3H), 1.46 - 0.84 (m, 5.5H).

### Example I-157

### 4-((4R,9S)-4,9-dimethyl-8-((S)-2-oxopyrrolidin-3-yl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

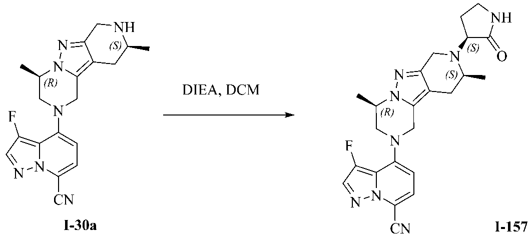

**Example I-157** was synthesized by following the protocols of **Example I-99.** MS: 449.4 (M+H)⁺. 1H NMR (400 MHz, DMSO-*d₆*) δ 8.30 (d, *J =* 3.6 Hz, 1H), 7.78 - 7.67 (m, 2H), 6.69 (d, *J* = 8.1 Hz, 1H), 4.51 - 4.30 (m, 3H), 4.03 - 3.96 (m, 1H), 3.74 - 3.55 (m, 3H), 3.37 (d, *J* = 9.3 Hz, 1H), 3.22 - 3.12 (m, 2H), 3.00 - 2.95 (m, 1H), 2.67 (dd, *J =* 15.0, 4.5 Hz, 1H), 2.22 (dd, *J =* 15.0, 6.8 Hz, 1H), 2.09 - 2.03 (m, 2H), 1.47 (d, *J =* 6.3 Hz, 3H), 1.15 (d, *J =* 6.3 Hz, 3H)

### Example I-158

### 4-((4R,9S)-8-((R)-2,3-dihydroxypropyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

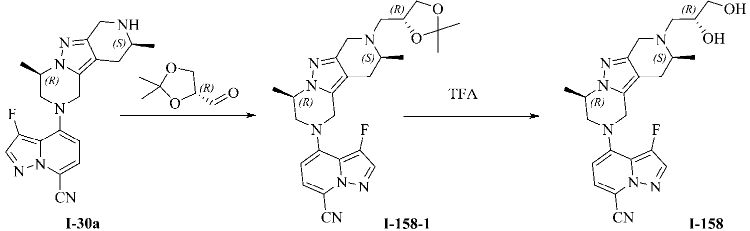

**Example I-158-1** was synthesized by following the protocols of **Example I-24.** MS: 480.4 (M+H)⁺. ¹H NMR (400 MHz, DMSO-*d₆*) δ 8.31 (d, *J* = 3.5 Hz, 1H), 7.73 (d, *J* = 8.0 Hz, 1H), 6.70 (d, *J* = 8.1 Hz, 1H), 4.53 - 4.31 (m, 3H), 4.23 (t, *J* = 6.2 Hz, 1H), 4.06 - 3.97 (m, 2H), 3.75 - 3.58 (m, 3H), 3.39 (s, 1H), 3.07 (d, *J* = 6.3 Hz, 1H), 2.67 - 2.56 (m, 3H), 2.21 (dd, *J* = 15.2, 5.4 Hz, 1H), 1.49 (d, *J* = 6.3 Hz, 3H), 1.34 (s, 3H), 1.29 (s, 3H), 1.04 (d, *J* = 6.5 Hz, 3H).

**Example I-158** was synthesized by following the protocols of **Example I-2.** MS: 440.2 (M+H)⁺. ¹H NMR (400 MHz, Methanol-*d₄*) δ 8.45 (s, 1H), 8.07 (d, *J* = 3.6 Hz, 1H), 7.49 (d, *J* = 7.9 Hz, 1H), 6.69 (d, *J* = 8.0 Hz, 1H), 4.59 - 4.43 (m, 3H), 4.32 (s, 2H), 4.14 - 3.98 (m, 2H), 3.79 (d, *J* = 5.9 Hz, 1H), 3.71 - 3.50 (m, 3H), 3.43 - 3.35 (m, 2H), 3.13 - 2.93 (m, 3H), 2.61 (dd, *J* = 16.3, 7.0 Hz, 1H), 1.61 (d, *J =* 6.4 Hz, 3H), 1.43 (d, *J =* 6.7 Hz, 3H).

### Example I-159

### 4-((4R,9S)-8-(1,3-dihydroxypropan-2-yl)-4,9-dimethyl-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

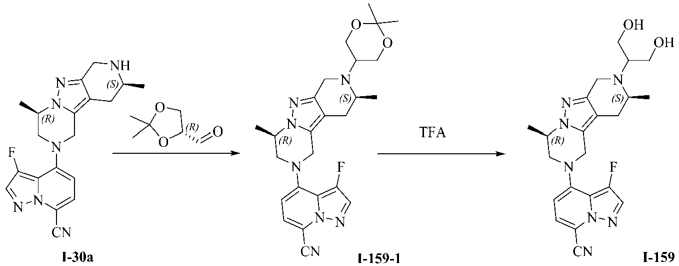

**Example I-159** was synthesized by following the protocols of **Example I-158.** MS: 440.3 (M+H)⁺. ¹H NMR (400 MHz, DMSO-*d₆*) δ 8.33 (d, *J =* 3.5 Hz, 1H), 7.76 (d, *J =* 8.0 Hz, 1H), 6.73 (d, *J =* 8.0 Hz, 1H), 5.49-5.40 (m, 2H), 4.53-4.33(m, 3H), 4.05 (d, *J =* 12.7 Hz, 2H), 3.87 (s, 4H), 3.47-3.42 (m, 3H), 3.33-3.28 (m, 1H), 3.04 (d, *J =* 16.9 Hz, 1H), 2.76 - 2.55 (m, 1H), 1.51 (d, *J =* 6.3 Hz, 3H), 1.44 (s, 1.5H), 1.28 (q, *J =* 5.2, 4.0 Hz, 1.5H).

### Example I-160

### 4-((4R,9S)-8-((S)-2,3-dihydroxypropyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

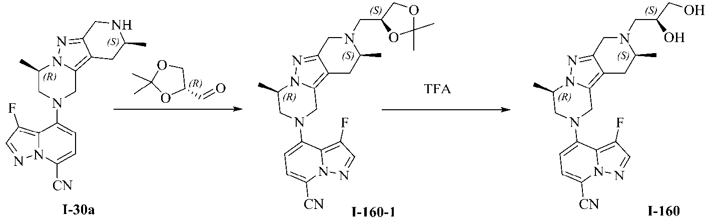

**Example I-160** was synthesized by following the protocols of **Example I-158.** MS: 440.3 (M+H)⁺. ¹H NMR (400 MHz, DMSO-*d₆*) δ 8.32 (d, J = 3.5 Hz, 1H), 7.75 (d, J = 8.0 Hz, 1H), 6.72 (d, J = 8.0 Hz, 1H), 4.60-4.57 (m, 1H), 4.49-4.39 (m, 4H), 4.05 (dd, J = 13.7, 4.0 Hz, 1H), 3.95 (s, 1H), 3.80 (s, 1H), 3.48-3.33 (m, 5H), 3.28-3.14(m,1H), 2.95 (d, J = 15.9 Hz, 2H), 2.59 (d, J = 15.0 Hz, 1H), 1.52 (d, J = 6.4 Hz, 3H), 1.34 (d, J = 6.3 Hz, 3H).

### Example I-161

### (R)-2-(2-(7-cyano-3-fluoropyrazolo[1,5-a]pyridin-4-yl)-4,9,9-trimethyl-1,2,3,4,9,10-hexahydropyrido[3',4':3,4]pyrazolo[1,5-a]pyrazin-8(7H)-yl)-N,N-dimethylacetamide

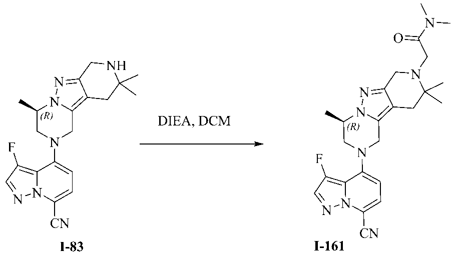

**Example I-161** was synthesized by following the protocols of **Example I-99.** MS: 466.3 (M+H)⁺.

### Example I-162

### 2-((4R)-2-(7-cyano-3-fluoropyrazolo[1,5-a]pyridin-4-yl)-9-isopropyl-4-methyl-1,2,3,4,9,10-hexahydropyrido[3',4':3,4]pyrazolo[1,5-a]pyrazin-8(7H)-yl)acetamide

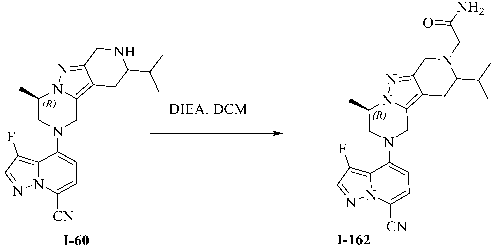

**Example I-162** was synthesized by following the protocols of **Example I-99.** MS: 451.3 (M+H)⁺. ¹H NMR (400 MHz, DMSO-*d₆*) δ 8.31 (d, *J* = 3.5 Hz, 1H), 7.75 (dd, *J =* 8.0, 2.4 Hz, 1H), 7.25 (s, 1H), 6.72 (dd, *J* = 8.1, 2.3 Hz, 1H), 4.64 - 4.31 (m, 3H), 4.09 - 3.97 (m, 1H), 3.89 - 3.60 (m, 2H), 3.43 - 3.35 (m, 2H), 3.18 - 3.13 (m, 1H), 2.69 (s, 1H), 2.31 (d, *J* = 32.6 Hz, 1H), 1.71 (s, 1H), 1.50 (d, *J* = 6.1 Hz, 3H), 1.30 - 25 (m, 2H), 1.07 (d, *J =* 6.4 Hz, 3H), 0.95 (d, *J =* 6.4 Hz, 3H).

### Example I-163

### 2-((4R,9R)-2-(7-cyano-3-fluoropyrazolo[1,5-a]pyridin-4-yl)-9-(hydroxymethyl)-4-methyl-1,2,3,4,9,10-hexahydropyrido[3',4':3,4]pyrazolo[1,5-a]pyrazin-8(7H)-yl)-N,N-dimethylacetamide

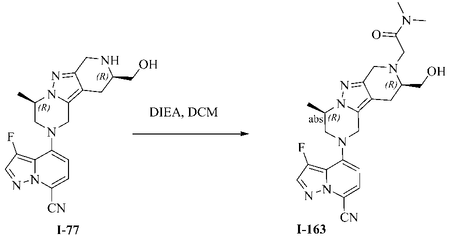

**Example I-163** was synthesized by following the protocols of **Example I-99.** MS: 467.3 (M+H)⁺. ¹H NMR (400 MHz, DMSO-*d₆*) δ 8.32 (d, J = 3.5 Hz, 1H), 7.74 (d, J = 8.0 Hz, 1H), 6.72 (d, J = 8.1 Hz, 1H), 4.67 - 4.33 (m, 3H), 4.03 (dd, J = 13.8, 4.2 Hz, 1H), 3.70 (s, 2H), 3.62-3.58 (m, 1H), 3.54 - 3.38 (m, 6H), 3.06 (s, 3H), 3.02 (t, J = 5.9 Hz, 1H), 2.85 (s, 3H), 2.58 (d, J = 5.2 Hz, 0.5H), 2.43 (dd, J = 15.4, 6.0 Hz, 0.5H), 1.50 (d, J = 6.3 Hz, 3H).

### Example I-164

### 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)cyclobutane-1-carboxamide

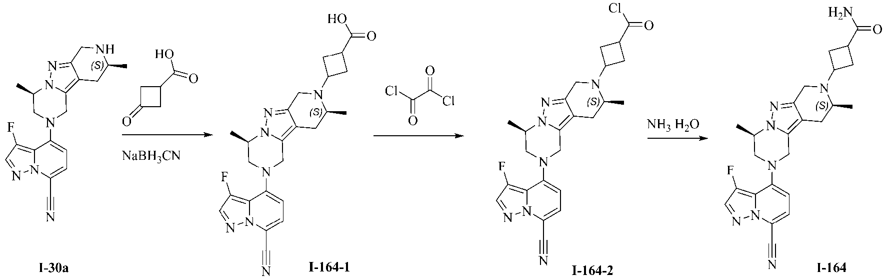

**Example 1-164-1** was synthesized by following the protocols of **Example I-24.** MS: 464.4 (M+H)⁺.

### Example I-164-2 Preparation

A reaction mixture of **Example I-164-1** (50 mg, 0.108 mmol), oxalyl chloride in DCM (3 mL) and DMF (1 drop) was stirred at 0°C for 1.5 hours. The mixture was concentrated to give **intermediate** I **-164-2** which was directly used in the next reaction.

### Example I-164 Preparation

A reaction mixture of **intermediate I-164-2** (52 mg, 0.108 mmol) and NH₃.H₂O (0.5 mL) in THF (5 mL) was stirred at 25 °C for 2 hours. The mixture was quenched with water and extracted with EtOAc, and the organic phase was washed with saturated saline, dried with Na₂SO₄ and concentrated. Purification by pre-HPLC gave **Example I-164**(10.4 mg) as white solid. MS: 464.3 (M+H)⁺. ¹H NMR (400 MHz, DMSO-*d₆*) δ 8.31 (d, *J* = 3.5 Hz, 1H), 7.72 (d, *J=* 8.0 Hz, 1H), 7.19 (s, 1H),6.74-6.68 (m, 2H), 4.52 - 4.28 (m, 3H), 4.00 (dd, *J* = 13.6, 4.2 Hz, 1H), 3.48-3.35(m, 3H), 3.13 - 3.02 (m, 2H), 2.71 - 2.54 (m, 2H), 2.23 - 2.12 (m, 3H), 2.06-1.89(m, 2H), 1.48 (d, *J=* 6.4 Hz, 3H), 0.95 (dd, *J* = 6.7, 4.2 Hz, 3H).

### Example I-165

### 2-((4R,7S,9R)-2-(7-cyano-3-fluoropyrazolo[1,5-a]pyridin-4-yl)-4,7,9-trimethyl-1,2,3,4,9,10-hexahydropyrido[3',4':3,4]pyrazolo[1,5-a]pyrazin-8(7H)-yl)acetamide

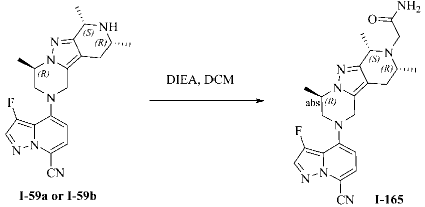

**Example I-165** was synthesized by following the protocols of **Example I-99.** MS: 437.2 (M+H)⁺. ¹H NMR (400 MHz, DMSO-*d₆*) δ 8.31 (d, *J =* 3.4 Hz, 1H), 7.74 (d, *J =* 8.1 Hz, 1H), 7.21 (d, *J* = 53.4 Hz, 1H), 6.72 (d, *J* = 8.2 Hz, 1H), 4.52 - 4.44 (m, 3H), 4.00 (d, *J* = 11.2 Hz, 2H), 3.40 - 3.35(m, 3H), 3.13 (s, 1H), 3.01 - 2.75 (m, 1H), 2.39 (dd, *J* = 39.7, 13.1 Hz, 1H), 1.48 (d, *J* = 6.4 Hz, 3H), 1.39 - 1.28 (m, 3H), 1.28 - 1.13 (m, 3H).

### Example I-166

### 2-((4R,7S,9R)-2-(7-cyano-3-fluoropyrazolo[1,5-a]pyridin-4-yl)-4,7,9-trimethyl-1,2,3,4,9,10-hexahydropyrido[3',4':3,4]pyrazolo[1,5-a]pyrazin-8(7H)-yl)-N,N-dimethylacetamide

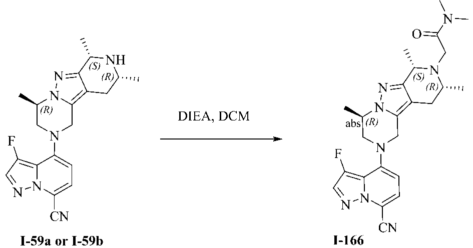

**Example I-166** was synthesized by following the protocols of **Example I-99.** MS: 466.2 (M+H)⁺. ¹H NMR (400 MHz, DMSO-*d₆*) δ 8.30 (d, *J =* 3.5 Hz, 1H), 7.73 (d, *J =* 8.0 Hz, 1H), 6.70 (d, *J =* 8.1 Hz, 1H), 4.48 - 4.37 (m, 3H), 4.16 (q, *J =* 6.7 Hz, 1H), 4.00 (dd, *J =* 13.7, 4.2 Hz, 1H), 3.56 (s, 2H), 3.03 (s, 3H), 2.83 (s, 3H), 2.48 (d, *J =* 4.1 Hz, 1H), 2.23 (dd, *J =* 14.9, 8.6 Hz, 1H), 1.47 (d, *J =* 6.3 Hz, 3H), 1.33 (d, *J =* 6.7 Hz, 3H), 1.10 (d, *J* = 6.5 Hz, 3H).

### Example I-167

### 2-((4R,7R,9S)-2-(7-cyano-3-fluoropyrazolo[1,5-a]pyridin-4-yl)-4,7,9-trimethyl-1,2,3,4,9,10-hexahydropyrido[3',4':3,4]pyrazolo[1,5-a]pyrazin-8(7H)-yl)-N,N-dimethylacetamide

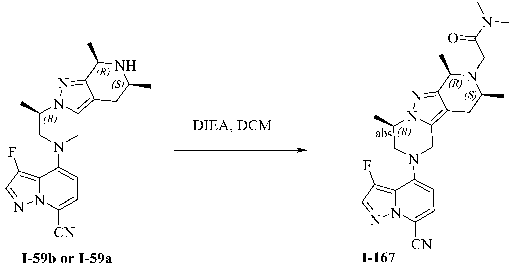

**Example I-167** was synthesized by following the protocols of **Example I-99.** MS: 465.4 (M+H)⁺. ¹H NMR (400 MHz, DMSO-*d₆*) δ 8.31 (d, *J =* 3.5 Hz, 1H), 7.73 (d, *J* = 8.0 Hz, 1H), 6.70 (d, *J* = 8.1 Hz, 1H), 4.50 (d, *J* = 15.5 Hz, 1H), 4.43 - 4.31 (m, 2H), 4.20 (d, *J* = 6.7 Hz, 1H), 4.01 (dd, *J* = 13.8, 4.1 Hz, 1H), 3.60 (d, *J* = 3.1 Hz, 2H), 3.39 (s, 2H), 3.02 (s, 3H), 2.83 (s, 3H), 2.54 (d, *J =* 3.5 Hz, 1H), 2.50 (s, 0H), 2.17 (dd, *J =* 14.8, 9.5 Hz, 1H), 1.49 (d, *J* = 6.3 Hz, 3H), 1.34 (d, *J* = 6.6 Hz, 3H), 1.11 (d, *J* = 6.4 Hz, 3H).

### Example I-168

### 4-((4R,9S)-8-(azetidin-3-yl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

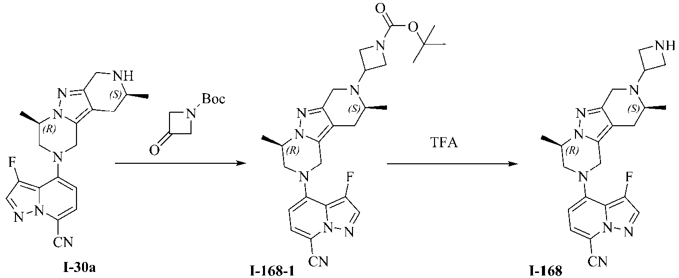

**Example I-168** was synthesized by following the protocols of **Example I-158.** MS: 421.3 (M+H)⁺. ¹H NMR (400 MHz, DMSO-*d₆*) δ 8.75 (d, *J =* 81.5 Hz, 1H), 8.32 (d, *J =* 3.5 Hz, 1H), 7.74 (d, *J* = 8.0 Hz, 1H), 6.70 (d, *J* = 8.1 Hz, 1H), 4.54 - 4.33 (m, 3H), 4.04 - 3.98 (m, 5H), 3.88 (s, 1H), 3.70 (s, 1H), 3.41 - 3.35 (m, 2H), 3.21 (s, 1H), 2.76 - 2.66 (m, 1H), 2.35 - 2.26 (m, 1H), 1.50 (d, *J =* 6.4 Hz, 3H), 1.02 (d, *J =* 6.6 Hz, 3H).

### Example I-169

### 4-((4R)-12-(cyanomethyl)-4-methyl-1,3,4,7,8,9,10,11-octahydro-2H-7,10-epiminocyclohepta[3,4]pyrazolo[1,5-a]pyrazin-2-yl)-3-fluoropyrazolo[1,5-a]pyridine-7-carbonitrile

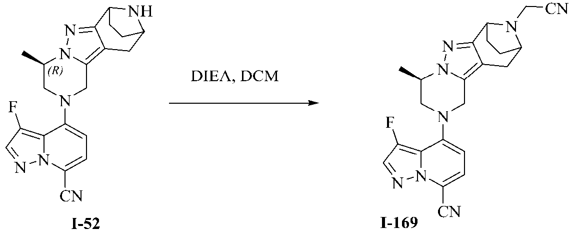

**Example I-169** was synthesized by following the protocols of **Example I-99.** MS: 418.4 (M+H)⁺. ¹H NMR (400 MHz, DMSO-*d₆*) δ 8.31 (d, *J* = 3.5 Hz, 1H), 7.72 (dd, *J =* 8.0, 4.3 Hz, 1H), 6.70 (t, *J* = 8.0 Hz, 1H), 4.54 - 4.31 (m, 3H), 4.08 - 3.97 (m, 2H), 3.64 - 3.47 (m, 3H), 3.37 (s, 1H), 3.32 (d, *J* = 4.7 Hz, 1H), 2.84 - 2.72 (m, 1H), 2.28 - 2.09 (m, 3H), 1.72 - 1.70 (m, 1H), 1.49 (dd, *J* = 6.3, 1.3 Hz, 3H).

### Example I-170

### 4-((4R,9S)-8-((S)-2-amino-3,3,3-trifluoropropyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

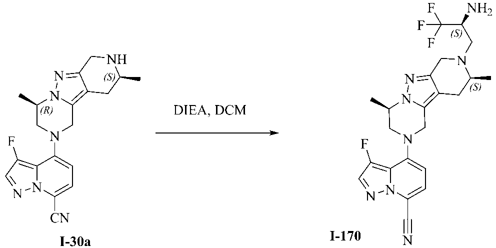

**Example I-170** was synthesized by following the protocols of **Example I-99.** MS: 477.4 (M+H)⁺. ¹H NMR (400 MHz, DMSO-*d₆*) δ 8.32 (d, *J* = 3.5 Hz, 1H), 7.74 (d, *J* = 8.0 Hz, 1H), 6.71 (d, *J=* 8.1 Hz, 1H), 4.57 - 4.29 (m, 3H), 4.02 (dd, *J* = 13.7, 4.1 Hz, 1H), 3.75 (d, *J* = 15.4 Hz, 1H), 3.63 - 3.48 (m, 3H), 3.41-3.38 (m, 2H), 3.17 - 3.13 (m, 1H), 2.70 - 2.61 (m, 2H), 2.56-2.50 (m, 1H), 2.23 (dd, *J* = 15.3, 5.6 Hz, 1H), 1.50 (d, *J* = 6.4 Hz, 3H), 1.06 (d, *J* = 6.6 Hz, 3H).

### Example I-171

### 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)azetidine-1-carboxamide

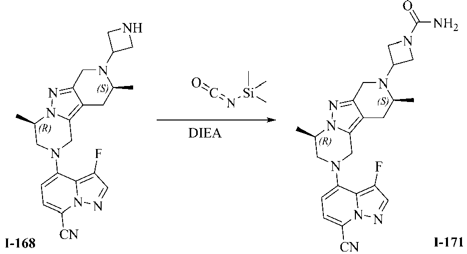

**Example I-171** was synthesized by following the protocols of **Example I-9.** MS: 464.4 (M+H)⁺. ¹H NMR (400 MHz, DMSO-*d₆*) δ 8.31 (d, *J* = 3.6 Hz, 1H), 7.73 (d, *J* = 8.0 Hz, 1H), 6.69 (d, *J=* 8.1 Hz, 1H), 5.83 (s, 2H), 4.54 - 4.31 (m, 3H), 4.01 (dd, *J* = 13.8, 4.2 Hz, 1H), 3.86-3.80 (m, 2H), 3.66-3.62 (m, 2H), 3.55-3.48 (m, 3H), 3.41 (s, 1H), 3.08-3.04 (m, 1H), 2.67 (dd, *J=* 15.2, 5.2 Hz, 1H), 2.23 (dd, *J* = 15.1, 3.3 Hz, 1H), 1.49 (d, *J* = 6.4 Hz, 3H), 0.97 (d, *J* = 6.6 Hz, 3H).

### Example I-172

### 2-((4R,7R,9S)-2-(7-cyano-3-fluoropyrazolo[1,5-a]pyridin-4-yl)-4,7,9-trimethyl-1,2,3,4,9,10-hexahydropyrido[3',4':3,4]pyrazolo[1,5-a]pyrazin-8(7H)-yl)acetamide

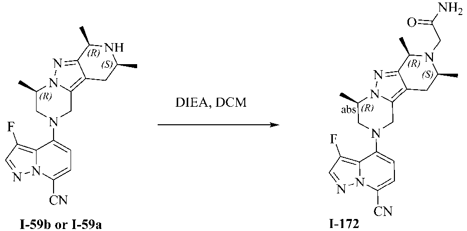

**Example I-172** was synthesized by following the protocols of **Example I-99.** MS: 437.4 (M+H)⁺.

### Example I-173

### 4-((4R,9S)-8-(1-acetylazetidin-3-yl)-4,9-dimethyl-3,4,7,8,9,10-hexahydropyrido [3',4':3,4]pyrazolo[1,5-a]pyrazin-2(1H)-yl)-3-fluoropyrazolo[1,5-a]pyridine-7-carbonitrile

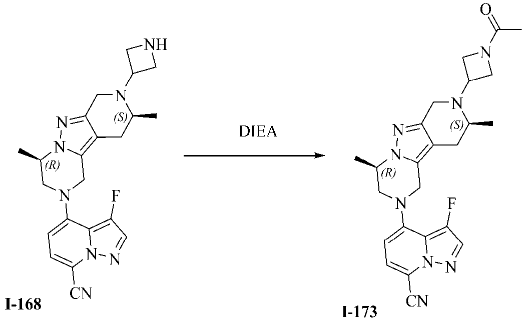

**Example 1-173** was synthesized by following the protocols of **Example 1-99.** MS: 463.4 (M+H)⁺. ¹H NMR (400 MHz, DMSO-*d₆*) δ 8.31 (d, J = 3.5 Hz, 1H), 7.73 (d, J = 8.0 Hz, 1H), 6.69 (d, J = 8.1 Hz, 1H), 4.53 - 4.32 (m, 3H), 4.21 - 4.12 (m, 1H), 4.06 - 3.96 (m, 2H), 3.88 (t, J = 8.4 Hz, 1H), 3.68 (dd, J = 9.5, 5.9 Hz, 1H), 3.64 - 3.50 (m, 3H), 3.42 - 3.37 (m, 1H), 3.11 (d, J = 5.6 Hz, 1H), 2.73 - 2.64 (m, 1H), 2.28 - 2.20 (m, 1H), 1.77 (s, 3H), 1.49 (d, J = 6.4 Hz, 3H), 0.98 (dd, J = 6.7, 2.8 Hz, 3H).

### Example I-174

### 3-Fluoro-4-((4R,9S)-8-(2-hydroxycyclobutyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

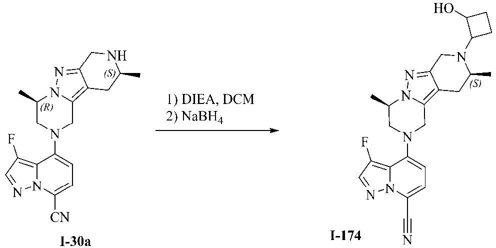

**Example I-174** was synthesized by following the protocols of **Example 1-99.** MS: 436.4 (M+H)⁺. ¹H NMR (400 MHz, DMSO-*d₆*) δ 8.35 (s, 1H), 8.32 (d, J = 3.6 Hz, 1H), 7.73 (d, J = 8.0 Hz, 1H), 6.71 (d, J = 8.1 Hz, 1H), 4.48 - 4.34 (m, 3H), 4.00 (s, 1H), 3.76 - 3.67 (m, 2H), 3.52 - 3.38 (m, 2H), 3.31 - 3.14 (m, 1H), 2.97 - 2.85 (m, 1H), 2.77 - 2.63 (m, 1H), 2.20 (dt, J = 14.9, 2.8 Hz, 1H), 1.98 (d, J = 9.1 Hz, 1H), 1.83 - 1.72 (m, 1H), 1.50 (dd, J = 6.4, 1.8 Hz, 3H), 1.43 - 1.33 (m, 1H), 1.18 (d, J = 9.2 Hz, 1H), 0.98 (dd, J = 12.8, 6.6 Hz, 3H).

### Example 1-175

### 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-N,N-dimethylazetidine-1-carboxamide

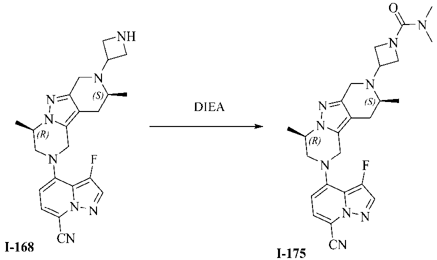

**Example I-175** was synthesized by following the protocols of **Example I-99.** MS: 493.4 (M+H)⁺. ¹H NMR (400 MHz, DMSO-*d₆*) δ 8.31 (d, *J* = 3.5 Hz, 1H), 7.73 (d, *J* = 8.0 Hz, 1H), 6.69 (d, *J* = 8.1 Hz, 1H), 4.53 - 4.29 (m, 3H), 4.05 - 3.97 (m, 1H), 3.94-3.89 (m, 2H), 3.75 (t, *J* = 6.8 Hz, 2H), 3.52-3.48 (m, 3H), 3.40-3.34 (m, 1H), 3.07 (s, 1H), 2.77 (s, 6H), 2.67 (dd, *J* = 15.2, 5.3 Hz, 1H), 2.23 (dd, *J* = 15.5, 3.3 Hz, 1H), 1.48 (d, *J* = 6.3 Hz, 3H), 0.96 (d, *J* = 6.5 Hz, 3H).

### Example I-176

### Methyl 3-(((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)methyl) bicyclo[1.1.1]pentane-1-carboxylate

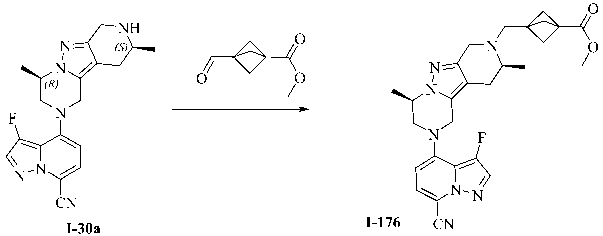

**Example I-176** was synthesized by following the protocols of **Example I-24.** MS: 504.4 (M+H)⁺.

### Example I-177

### N-(2-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)ethyl)acetamide

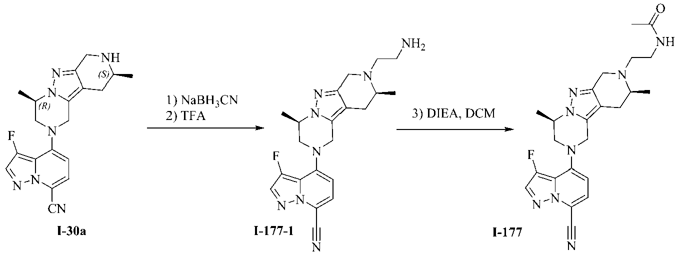

**Example I-177** was synthesized by following the protocols of **Example I-158** and **Example** s **I-99.** MS: 451.4 (M+H)⁺. ¹H NMR (400 MHz, MeOH-*d₄*) δ 8.07 (d, *J* = 3.5 Hz, 1H), 7.49 (d, *J* = 7.9 Hz, 1H), 6.69 (d, *J* = 7.9 Hz, 1H), 4.57 - 4.43 (m, 3H), 4.31 (s, 2H), 4.09 (dd, *J* = 13.5, 4.2 Hz, 1H), 3.85 - 3.72 (m, 1H), 3.57 (t, *J* = 6.1 Hz, 2H), 3.43 - 3.37 (m, 1H), 3.29 - 3.20 (m, 1H), 3.19 - 3.10 (m, 1H), 3.00 (dd, *J* = 16.2, 5.0 Hz, 1H), 2.64 (dd, *J* = 16.2, 5.3 Hz, 1H), 2.02 (s, 3H), 1.61 (d, *J* = 6.4 Hz, 3H), 1.39 (d, *J* = 6.6 Hz, 3H).

### Example I-178

### (R)-2-(2-(7-Cyano-3-fluoropyrazolo[1,5-a]pyridin-4-yl)-4,7,7-trimethyl-1,2,3,4,9,10-hexahydropyrido[3',4':3,4]pyrazolo[1,5-a]pyrazin-8(7H)-yl)-N,N-dimethylacetamide

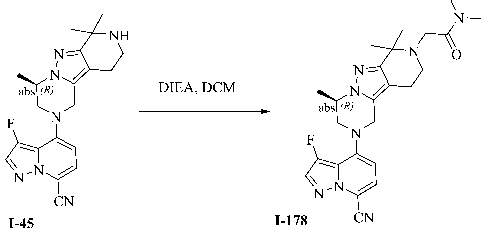

**Example I-178** was synthesized by following the protocols of **Example I-99.** MS: 465.4 (M+H)⁺. ¹H NMR (400 MHz, DMSO-*d₆*) δ 8.31 (d, *J* = 3.5 Hz, 1H), 7.73 (d, *J =* 8.0 Hz, 1H), 6.72 (d, *J* = 8.1 Hz, 1H), 4.51 - 4.31 (m, 3H), 4.00 (dd, *J* = 13.6, 4.1 Hz, 1H), 3.35 - 3.31 (m, 2H), 3.11 (s, 3H), 3.00 - 2.88 (m, 1H), 2.85 (s, 3H), 2.72 (t, *J* = 6.0 Hz, 2H), 2.45 - 2.40 (mz, 2H), 1.48 (d, *J =* 6.3 Hz, 3H), 1.35 (d, *J =* 12.4 Hz, 6H).

### Example I-179

### 2-((4R,7S)-2-(7-cyano-3-fluoropyrazolo[1,5-a]pyridin-4-yl)-4,7-dimethyl-1,2,3,4,9,10-hexahydropyrido[3',4':3,4]pyrazolo[1,5-a]pyrazin-8(7H)-yl)acetamide

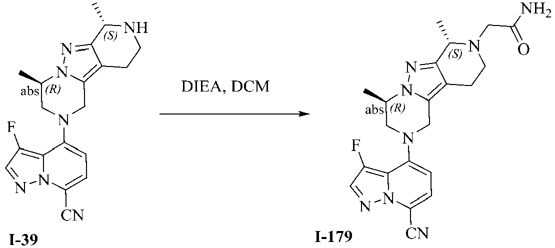

**Example I-179** was synthesized by following the protocols of **Example I-99.** MS: 423.4 (M+H)⁺. ¹H NMR (400 MHz, DMSO-*d₆*) δ 8.31 (d, *J =* 3.5 Hz, 1H), 7.73 (d, *J =* 8.0 Hz, 1H), 7.27 - 7.11 (m, 2H), 6.72 (t, *J* = 8.1 Hz, 1H), 4.52-4.33 (m, 3H), 4.03-3.98 (m, 1H), 3.79 - 3.42 (m, 2H), 3.26 - 2.87 (m, 4H), 2.69-2.63 (m, 1H), 2.49 (d, *J =* 5.7 Hz, 1H), 1.48 (dd, *J =* 6.4, 4.2 Hz, 3H), 1.33 (d, *J =* 6.5 Hz, 2H), 1.08 (d, *J =* 6.6 Hz, 1H).

### Example 1-180

### 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)propanamide

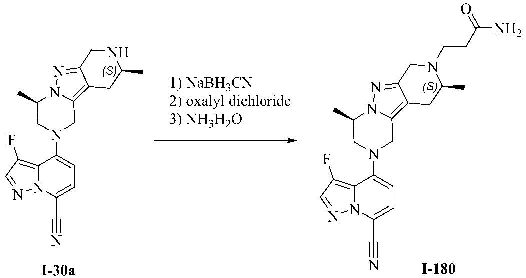

**Example I-180** was synthesized by following the protocols of **Example I-164.** MS: 437.4 (M+H)⁺. ¹H NMR (400 MHz, DMSO-*d₆*) δ 8.37 (d, *J* = 3.4 Hz, 1H), 7.79 (d, *J =* 7.9 Hz, 1H), 7.46 (s, 1H), 6.82 (s, 1H), 6.76 (d, *J =* 8.0 Hz, 1H), 4.58-4.40 (m, 3H), 4.11 - 4.04 (m, 1H), 3.67 (s, 2H), 3.46-3.40 (m, 1H), 3.13-3.08 (m, 1H), 2.81 (q, *J* = 6.5 Hz, 2H), 2.83-2.78 (m, 1H), 2.36 - 2.25 (m, 3H), 1.55 (d, *J =* 6.3 Hz, 3H), 1.11 (d, *J =* 6.4 Hz, 3H).

### Example I-181

### 4-((4R)-4,12-dimethyl-1,3,4,7,8,9,10,11-octahydro-2H-7,10-epiminocyclohepta[3,4]pyrazolo[1,5-a]pyrazin-2-yl)-3-fluoropyrazolo[1,5-a]pyridine-7-carbonitrile

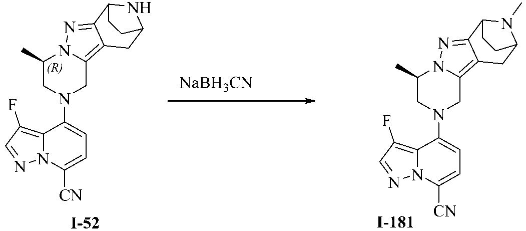

**Example 1-181** was synthesized by following the protocols of **Example 1-24.** MS: 392.4 (M+H)⁺. ¹H NMR (400 MHz, DMSO-*d₆*) δ 8.31 (d, *J* = 3.4 Hz, 1H), 7.74 (dd, *J =* 8.0, 5.4 Hz, 1H), 6.69 (dd, *J* = 8.1, 3.3 Hz, 1H), 4.64 - 4.29 (m, 3H), 4.05 - 3.90 (m, 2H),3.48-3.45(m, 1H), 3.40-3.27 (m, 1H), 2.93 (dd, *J =* 16.7, 4.5 Hz, 1H), 2.27 (d, *J* = 16.1 Hz, 1H), 2.23 (d, *J* = 8.9 Hz, 3H), 2.19 - 2.02 (m, 2H), 1.669-1.62 (m, 1H), 1.48 *(t, J* = 5.9 Hz, 3H), 1.45-1.41 (m, 1H).

### Example I-182

### (R)-2-(2-(7-cyano-3-fluoropyrazolo[1,5-a]pyridin-4-yl)-4,7,7-trimethyl-1,2,3,4,9,10-hexahydropyrido[3',4':3,4]pyrazolo[1,5-a]pyrazin-8(7H)-yl)acetamide

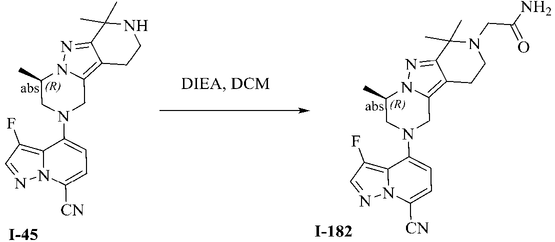

**Example I-182** was synthesized by following the protocols of **Example I-99.** MS: 437.6 (M+H)⁺. ¹H NMR (400 MHz, DMSO-*d₆*) δ 8.31 (d, *J* = 3.5 Hz, 1H), 7.73 (d, *J =* 8.0 Hz, 1H), 7.21 (dd, *J* = 20.0, 3.7 Hz, 2H), 6.73 (d, *J* = 8.1 Hz, 1H), 4.49 - 4.38 (m, 3H), 4.00 (d, *J* = 10.9 Hz, 1H), 3.37 (d, *J =* 9.0 Hz, 1H), 3.32 (s, 1H), 3.11 - 2.93 (m, 2H), 2.75 - 2.67 (m, 2H), 2.50 (s, 1H), 1.48 (d, *J =* 6.3 Hz, 3H), 1.33 (d, *J =* 17.2 Hz, 6H).

### Example I-183

### 2-((4R,7R)-2-(7-cyano-3-fluoropyrazolo[1,5-a]pyridin-4-yl)-4,7-dimethyl-1,2,3,4,9,10-hexahydropyrido[3',4':3,4]pyrazolo[1,5-a]pyrazin-8(7H)-yl)-N,N-dimethylacetamide

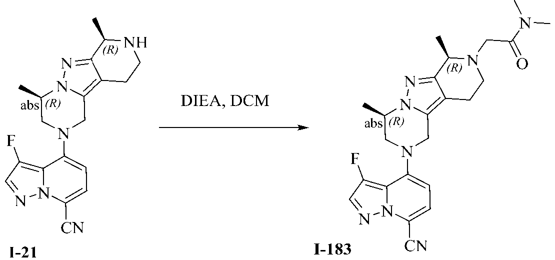

**Example I-183** was synthesized by following the protocols of **Example I-99.** MS: 451.4 (M+H)⁺. ¹H NMR (400 MHz, DMSO-*d₆*) δ 8.30 (d, *J =* 3.5 Hz, 1H), 7.72 (d, *J* = 8.0 Hz, 1H), 6.71 (d, *J* = 8.1 Hz, 1H), 4.54 - 4.32 (m, 3H), 4.01 (dd, *J* = 13.8, 4.1 Hz, 1H), 3.75 - 3.71 (m, 1H), 3.55 (d, *J* = 14.1 Hz, 1H), 3.38 - 3.34 (m, 1H), 3.26 - 3.22 (m, 1H), 3.06 (s, 3H), 2.98 - 2.92 (m, 1H), 2.84 (s, 3H), 2.70 - 2.64 (m, 1H), 2.54 (d, *J* = 4.5 Hz, 0H), 2.49 (d, *J* = 5.1 Hz, 1H), 2.45 - 2.35 (m, 1H), 1.49 (d, *J* = 6.4 Hz, 3H), 1.34 (d, *J* = 6.5 Hz, 3H).

### Example I-184

### 4-((4R,9S)-8-((3-aminooxetan-3-yl)methyl)-4,9-dimethyl-3,4,7,8,9,10-hexahydro pyrido[3',4':3,4]pyrazolo[1,5-a]pyrazin-2(1H)-yl)-3-fluoropyrazolo[1,5-a]pyridine-7-carbonitrile

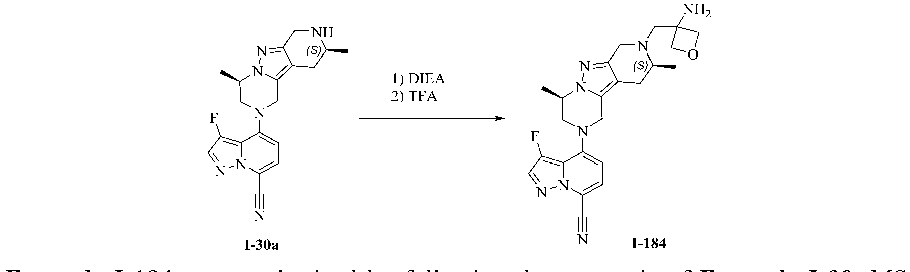

**Example I-184** was synthesized by following the protocols of **Example I-99.** MS: 450.9 (M+H)⁺. ¹H NMR (400 MHz, DMSO-*d₆*) δ 8.31 (d, *J* = 3.5 Hz, 1H), 7.73 (d, *J* = 8.0 Hz, 1H), 6.71 (d, *J* = 8.1 Hz, 1H), 4.53 - 4.31 (m, 7H), 4.01 (d, *J* = 11.8 Hz, 1H), 3.63 - 3.53 (m, 2H), 3.39 (d, *J* = 9.3 Hz, 3H), 3.06 (q, *J* = 5.8 Hz, 1H), 2.71 (d, *J=* 26.2 Hz, 3H), 2.24 (dd, *J* = 15.2, 4.7 Hz, 1H), 1.49 (d, *J* = 6.3 Hz, 3H), 1.05 (d, *J* = 6.6 Hz, 3H).

### Example I-185

### 4-((4R)-12-cyclopropyl-4-methyl-1,3,4,7,8,9,10,11-octahydro-2H-7,10-epiminocyclo hepta[3,4]pyrazolo[1,5-a]pyrazin-2-yl)-3-fluoropyrazolo[1,5-a]pyridine-7-carbonitrile

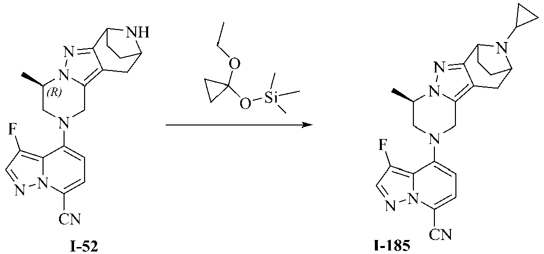

**Example I-185** was synthesized by following the protocols of **Example 1-96.** MS: 418.4 (M+H)⁺. ¹H NMR (400 MHz, DMSO-*d₆*) δ 8.33 (t, *J* = 3.2 Hz, 1H), 7.78 (t, *J* = 7.0 Hz, 1H), 6.68 (d, *J* = 8.1 Hz, 1H), 4.90 (d, *J* = 80.0 Hz, 1H), 4.65 - 4.27 (m, 3H), 4.12 - 3.93 (m, 1H), 3.43 - 3.38 (m, 2H), 3.34 - 3.10 (m, 4H), 2.93 (s, 1H), 2.56 - 2.51 (m, 1H), 2.34 - 1.80 (m, 2H), 1.52 - 1.47 (m, 3H), 1.29 - 0.82 (m, 4H).

### Example I-186

### 3-Fluoro-4-((4R,9S)-4-methyl-9-(morpholine-4-carbonyl)-3,4,7,8,9,10-hexahydro pyrido[3',4':3,4]pyrazolo[1,5-a]pyrazin-2(1H)-yl)pyrazolo[1,5-a]pyridine-7-carbonitrile

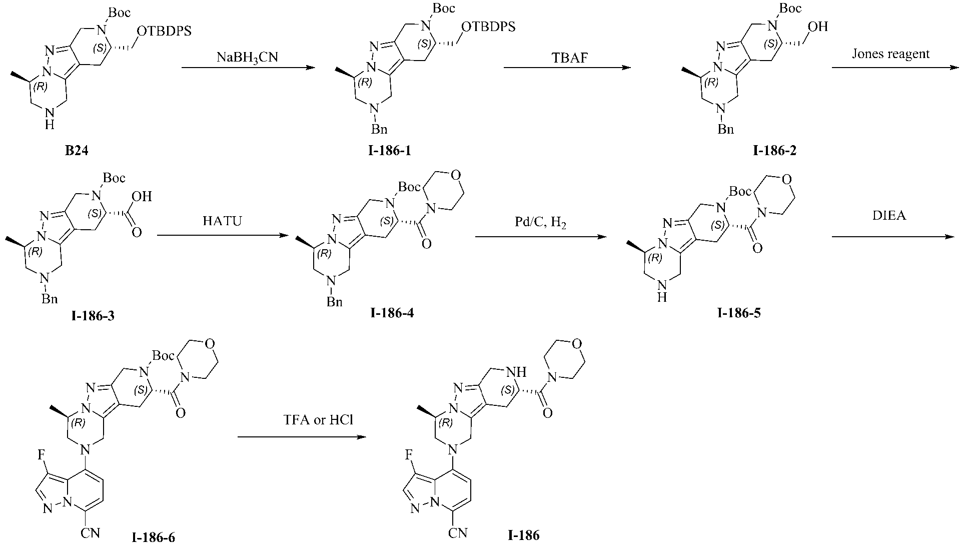

### Step 1: Preparation of I-186-1

**Intermediate I-186-1** was synthesized by following the protocols of **Example I-24.** MS: 651.4 (M+H)⁺.

### Step 2: Preparation of I-186-2

**Intermediate I-186-2**was synthesized by following the protocols of **Example I-33-2.** MS: 413.4 (M+H)⁺.

### Step 3: Preparation of 1-186-3

A reaction mixture of **Intermediate I-186-2** (580 mg, 1.41 mmol) and Jones reagents (1.5 mmol) in acetone (5 mL) was stirred at 0 °C for 3 hours. The mixture was quenched with water and extracted with EtOAc, and the organic phase was washed with saturated saline, dried with Na₂SO₄ and concentrated. Purification by flash chromatography gave **Intermediate I-186-3** (230 mg, 38.4%) as yellow solid. MS: 427.3 (M+H)⁺.

### Step 4: Preparation of I-186-4

**Intermediate I-186-4** was synthesized by following the protocols of **Example I-112.** MS: 496.1 (M+H)⁺.

### Step 5: Preparation of I-186-5

**Intermediate I-186-5** was synthesized by following the protocols of **Intermediate B-**33. MS: 406.1 (M+H)⁺.

### Step6: Preparation of I-186

**Example I-186** was synthesized by following the protocols of **Example I-2.** MS: 465.1 (M+H)⁺. ¹H NMR (400 MHz, DMSO-*d₆*) δ 8.31 (d, *J* = 3.5 Hz, 1H), 7.74 (d, *J* = 8.0 Hz, 1H), 6.72 (d, *J=* 8.1 Hz, 1H), 4.56 - 4.31 (m, 3H), 4.00 (dd, *J* = 13.6, 4.1 Hz, 1H), 3.88 - 3.78 (m, 3H), 3.68 - 3.54 (m, 8H), 3.35 (d, *J* = 4.7 Hz, 2H), 2.66 - 2.54 (m, 2H), 1.47 (d, *J* = 6.3 Hz, 3H).

### Example I-187

### 3-Fluoro-4-((4R,8R)-8-(3-hydroxypyrrolidin-1-yl)-4,8-dimethyl-3,4,7,8,9,10-hexahydropyrazino[1,2-b]indazol-2(1H)-yl)pyrazolo[1,5-a]pyridine-7-carbonitrile

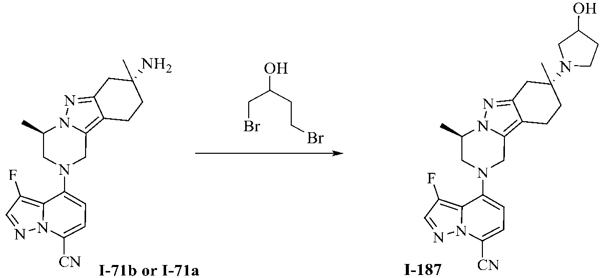

**Example I-187** was synthesized by following the protocols of Intermediate **B15-2.** MS: 450.4 (M+H)⁺.

### Example I-188

### 3-Fluoro-4-((4R,8S)-8-(3-hydroxypyrrolidin-1-yl)-4,8-dimethyl-3,4,7,8,9,10-hexahydropyrazino[1,2-b]indazol-2(1H)-yl)pyrazolo[1,5-a]pyridine-7-carbonitrile

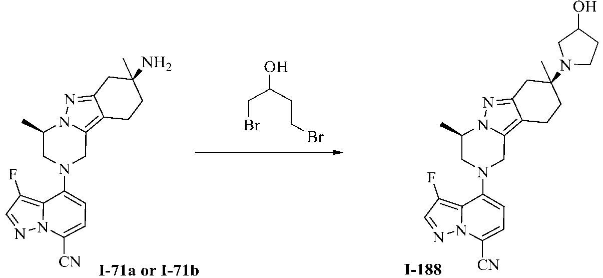

**Example I-188** was synthesized by following the protocols of Intermediate **B15-2.** MS: 450.4 (M+H)⁺.

### Example 1-189

### 3-Fluoro-4-((4R,9S)-9-(3-hydroxypyrrolidin-1-yl)-4,9-dimethyl-3,4,7,8,9,10-hexahydropyrazino[1,2-b]indazol-2(1H)-yl)pyrazolo[1,5-a]pyridine-7-carbonitrile

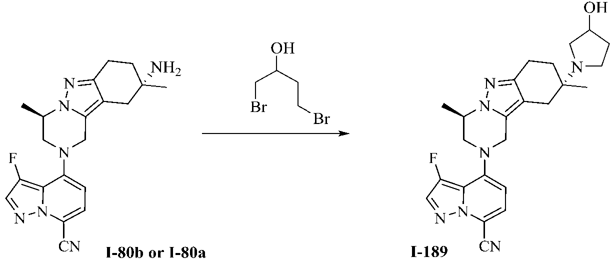

**Example I-189** was synthesized by following the protocols of Intermediate **B15-2.** MS: 450.4 (M+H)⁺.

### Example I-190

### 3-Fluoro-4-((4R,9R)-9-(3-hydroxypyrrolidin-1-yl)-4,9-dimethyl-3,4,7,8,9,10-hexahydropyrazino[1,2-b]indazol-2(1H)-yl)pyrazolo[1,5-a]pyridine-7-carbonitrile

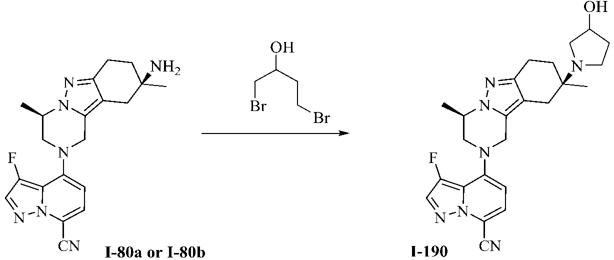

**Example I-190** was synthesized by following the protocols of Intermediate **B15-2.** MS: 450.4 (M+H)⁺.

### Example I-191

### 3-(((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)methyl)bicyclo[1.1.1] pentane-1-carboxylic acid

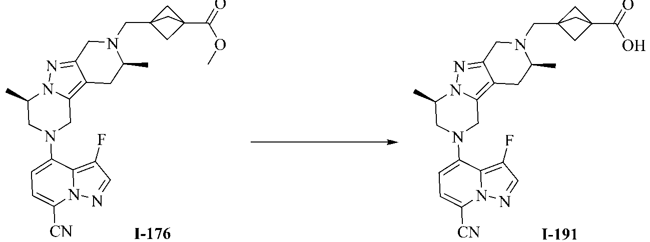

**Example I-191** was synthesized by following the protocols of Intermediate **B10-3.** MS: 490.5 (M+H)⁺.

### Example I-192

### Methyl 1-(((4R,9S)-2-(7-cyano-3-fluoropyrazolo [1,5-a]pyridin-4-yl)-4,9-dimethyl-1,2,3,4,9,10-hexahydropyrido[3',4':3,4]pyrazolo[1,5-a]pyrazin-8(7H)-yl)methyl) cyclopropane-1-carboxylate

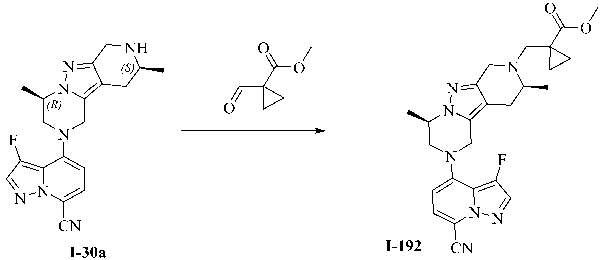

**Example I-192** was synthesized by following the protocols of **Example I-24.**MS: 478.5 (M+H)⁺.

### Example I-193

### 3-(((4R,9S)-2-(7-cyano-3-fluoropyrazolo [1,5-a]pyridin-4-yl)-4,9-dimethyl-1,2,3,4,9,10-hexahydropyrido[3',4':3,4]pyrazolo[1,5-a]pyrazin-8(7H)-yl)methyl)bicyclo[1.1.1] pentane-1-carboxamide

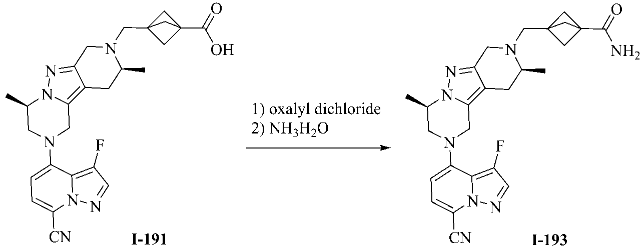

**Example I-193** was synthesized by following the protocols of **Example I-164**. MS: 489.5 (M+H)⁺.

### Example 1-194

### Methyl 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-alpyrazin-8(7H)-yl)-2,2-dimethyl propanoate

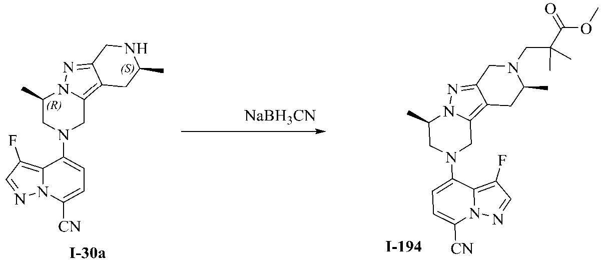

**Example I-194** was synthesized by following the protocols of **Example I-99**. MS: 479.9 (M+H)⁺. ¹H NMR (400 MHz, DMSO-*d*₆) δ 8.30 (d, *J =* 3.5 Hz, 1H), 7.72 (d, *J =* 8.0 Hz, 1H), 6.70 (d, *J =* 8.1 Hz, 1H), 4.45 (q, *J =* 15.5 Hz, 2H), 4.35 (s, 1H), 4.00 (d, *J =* 13.6 Hz, 1H), 3.60 (s, 3H), 3.59 (s, 2H), 3.04 (s, 1H), 2.68 (d, *J* = 14.4 Hz, 2H), 2.51 (s, 2H), 2.19 (d, *J* = 14.7 Hz, 1H), 1.49 (d, *J* = 6.4 Hz, 3H), 1.12 (s, 6H), 0.98 (d, *J* = 6.6 Hz, 3H).

### Example I-195 and Example I-196

### 3-((4R,95)-2-(7-carbamoyl-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-2,2-dimethylpropanoic acid (Example I-195)

### 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-2,2-dimethylpropanoic acid (Example I-196)

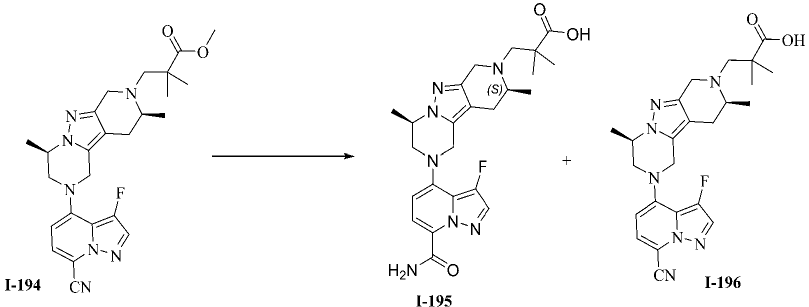

**Example 1-195 and Example 1-196** were synthesized by following the protocols of Intermediate **B10-3. Example I-195:** MS: 483.9(M+H)⁺. **Example I-196:** MS: 465.9 (M+H)⁺.¹H NMR (400 MHz, DMSO-*d*₆) δ 8.31 (d, *J* = 3.5 Hz, 1H), 7.73 (d, *J* = 8.0 Hz, 1H), 6.70 (d, *J =* 8.1 Hz, 1H), 4.46 (q, *J =* 15.3 Hz, 2H), 4.36 (s, 1H), 4.06 - 3.94 (m, 1H), 3.67 (d, *J=* 2.3 Hz, 2H), 3.41-3.38 (m, 1H), 3.18 - 3.00 (m, 2H), *J =* 15.2, 3.9 Hz, 1H), 1.49 (d, *J =* 6.4 Hz, 3H), 1.09 (d, *J =* 3.4 Hz, 6H), 1.00 (d, *J =* 6.6 Hz, 3H).

### Example I-197

### 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-N,N,2,2-tetramethyl propanamide

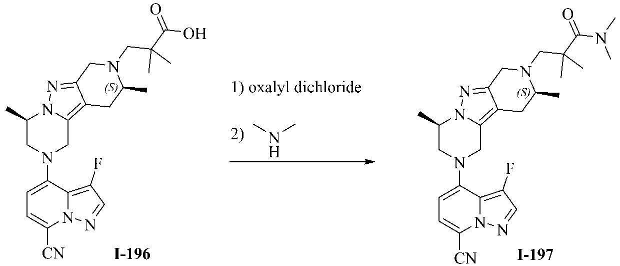

**Example I-197** was synthesized by following the protocols of **Example I-164**. MS: 492.9 (M+H)⁺. ¹H NMR (400 MHz, DMSO-*d*₆) δ 8.30 (d, *J =* 3.5 Hz, 1H), 7.73 (d, *J* = 8.0 Hz, 1H), 6.70 (d, *J* = 8.1 Hz, 1H), 4.45 (q, *J=* 15.2 Hz, 2H), 4.36 (dt, *J* = 10.3, 5.0 Hz, 1H), 4.04 - 3.96 (m, 1H), 3.58 (d, *J* = 3.7 Hz, 2H), 3.55-3.45 (m, 1H), 3.07 (q, *J* = 5.5 Hz, 1H), 2.96 (s, 6H), 2.64 (q, *J=* 14.1 Hz, 3H), 2.20 (dd, *J* = 15.2, 4.1 Hz, 1H), 1.49 (d, *J* = 6.4 Hz, 3H), 1.20 (d, *J* = 4.3 Hz, 6H), 0.99 (d, *J* = 6.7 Hz, 3H).

### Example 1-198

### 3-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-2,2-dimethyl propanamide

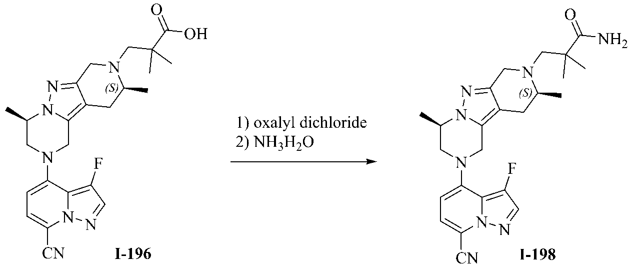

**Example I-198** was synthesized by following the protocols of **Example I-164**. MS: 465.4 (M+H)⁺. ¹H NMR (400 MHz, DMSO-*d*₆) δ 8.30 (d, *J =* 3.6 Hz, 1H), 7.73 (d, *J =* 8.0 Hz, 1H), 7.06 (s, 1H), 6.78 (s, 1H), 6.70 (d, *J =* 8.1 Hz, 1H), 4.45 (q, *J =* 15.3 Hz, 2H), 4.35 (q, *J =* 9.6, 7.0 Hz, 1H), 3.64 (s, 2H), 3.55 - 3.45 (m, 2H),3.08 (q, *J =* 5.4 Hz, 1H), 2.69 - 2.55 (m, 2H), 2.46 (d, *J=* 13.8 Hz, 1H), 2.19 (dd, *J =* 15.1, 3.9 Hz, 1H), 1.49 (d, *J =* 6.4 Hz, 3H), 1.06 (d, *J =* 7.1 Hz, 6H), 0.99 (d, *J =* 6.6 Hz, 3H).

### Example I-199

### 2-((4R,7R)-2-(7-cyano-3-fluoropyrazolo[1,5-a]pyridin-4-yl)-4,7-dimethyl-1,2,3,4,9,10-hexahydropyrido[3',4':3,4]pyrazolo[1,5-a]pyrazin-8(7H)-yl)acetamide

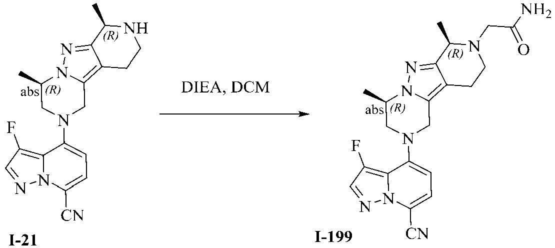

**Example I-199** was synthesized by following the protocols of **Example I-99**. MS: 423.4 (M+H)⁺. ¹H NMR (400 MHz, MeOH-*d₄*) δ 8.08 (d, *J* = 3.6 Hz, 1H), 7.50 (d, *J* = 7.9 Hz, 1H), 6.71 (d, *J* = 7.9 Hz, 1H), 4.68 (s, 1H), 4.58 - 4.46 (m, 3H), 4.16 - 3.92 (m, 3H), 3.74 (s, 1H), 3.63 - 3.48 (m, 1H), 3.40 (dd, *J* = 13.6, 8.6 Hz, 1H), 2.95 (d, *J* = 6.4 Hz, 2H), 1.75 (d, *J* = 6.8 Hz, 3H), 1.61 (d, *J* = 6.4 Hz, 3H).

### Example I-200

### 3-Fluoro-4-((4R,9S)-8-(2-methoxy-3,4-dioxocyclobut-1-en-1-yl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

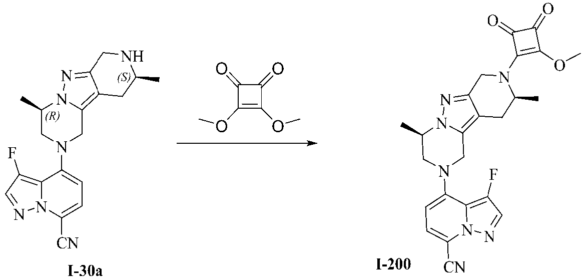

**Example I-200** was synthesized by following the protocols of **Example I-99**. MS: 476.4 (M+H)⁺.¹H NMR (400 MHz, DMSO-d6) δ 8.30 (d, *J =* 3.5 Hz, 1H), 7.72 (d, *J =* 8.0 Hz, 1H), 6.70 (d, *J* = 8.1 Hz, 1H), 4.60 (d, *J* = 18.5 Hz, 1H), 4.49 (s, 3H), 4.37 (d, *J=* 23.3 Hz, 5H), 4.06 - 3.97 (m, 1H), 3.42 (dd, *J* = 13.7, 9.1 Hz, 2H), 2.90 (s, 1H), 1.51 (d, *J* = 6.4 Hz, 3H), 1.27 (d, *J =* 6.9 Hz, 3H).

### Example I-201

### 1-(((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)methyl)cyclopropane-1-carboxylic acid

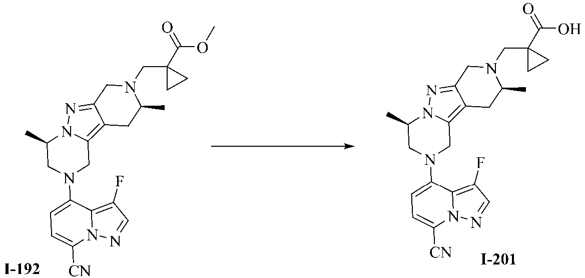

**Example I-201** was synthesized by following the protocols of **Example I-191**. MS: 464.5 (M+H)⁺.

### Example I-202

### 3-(((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)methyl)-N,N-dimethyl bicyclo[1.1.1]pentane-1-carboxamide

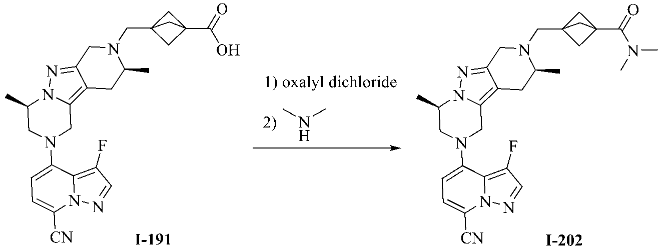

**Example I-202** was synthesized by following the protocols of **Example I-164**. MS: 517.6 (M+H)⁺.

### Example 1-203

### 1-(((4R,95)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)methyl)-N,N-dimethyl cyclopropane-1-carboxamide

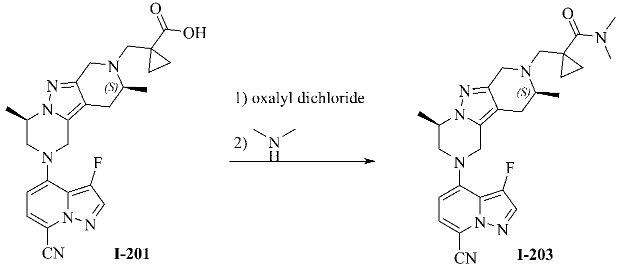

**Example I-203** was synthesized by following the protocols of Example **I-164**. MS: 491.6 (M+H)⁺.

### Example I-204

### 1-(((4R,9S)-2-(7-cyano-3-fluoropyrazolo [1,5-a]pyridin-4-yl)-4,9-dimethyl-1,2,3,4,9,10-hexahydropyrido[3',4':3,4]pyrazolo[1,5-a]pyrazin-8(7H)-yl)methyl)cyclopropane-1-carboxamide

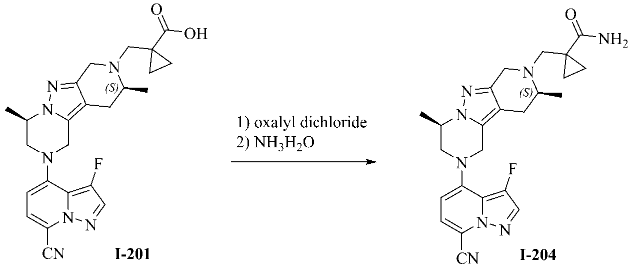

**Example I-204** was synthesized by following the protocols of **Example I-164.** MS: 463.5 (M+H)⁺.

### Example I-205

### 4-((4R,9S)-8-((1-(azetidine-1-carbonyl)cyclopropyl)methyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

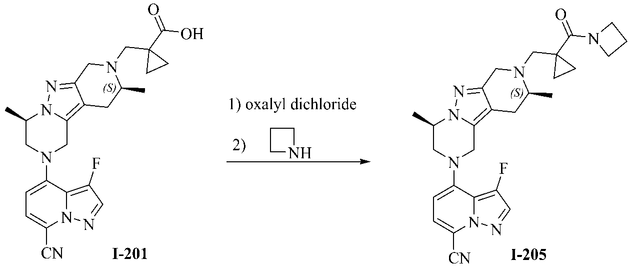

**Example I-205** was synthesized by following the protocols of **Example I-164**. MS: 503.6 (M+H)⁺.

### Example I-206

### 4-((4R,9S)-4,9-dimethyl-8-((1-(pyrrolidine-1-carbonyl)cyclopropyl)methyl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

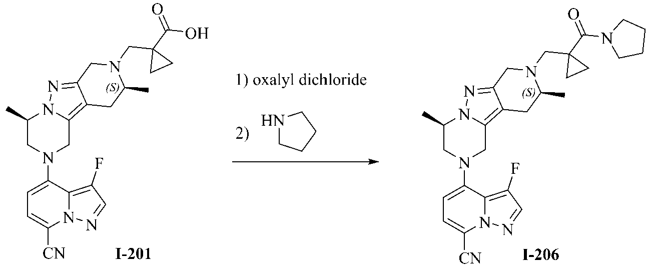

**Example I-206** was synthesized by following the protocols of **I-164**. MS: 517.6 (M+H)⁺.

### Example I-207

### 4-((4R,9S)-4,9-dimethyl-8-((1-(morpholine-4-carbonyl)cyclopropyl)methyl)-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

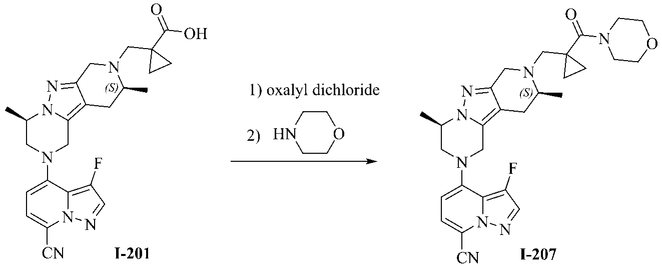

**Example I-207** was synthesized by following the protocols of **I-164**. MS: 533.6 (M+H)⁺.

### Example I-208

### 4-((4R,9S)-8-(3-(azetidin-1-yl)-2,2-dimethyl-3-oxopropyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

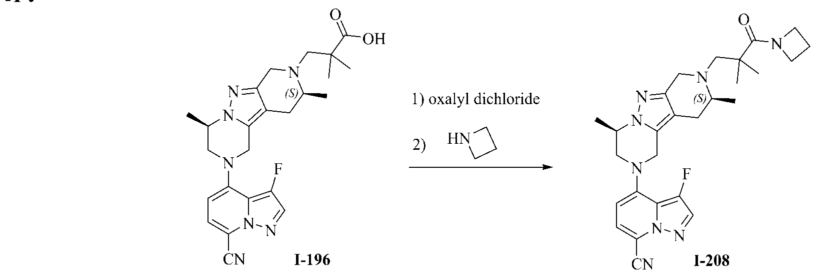

**Example I-208** was synthesized by following the protocols of **I-164**. MS: 504.9 (M+H)⁺. ¹H NMR (400 MHz, DMSO-*d*₆) δ 8.30 (d, *J* = 3.5 Hz, 1H), 7.73 (d, *J* = 8.1 Hz, 1H), 6.70 (d, *J =* 8.1 Hz, 1H), 4.45 (q, *J =* 15.5 Hz, 3H), 4.36 (s, 2H), 4.05 - 3.97 (m, 1H), 3.83 (s, 2H), 3.63 (s, 2H), 3.06 (q, *J* = 5.7 Hz, 1H), 2.70 - 2.56 (m, 2H), 2.43 (d, *J* = 13.8 Hz, 1H), 2.25 - 2.06 (m, 3H), 1.49 (d, *J* = 6.4 Hz, 3H), 1.26 (s, 2H), 1.09 (d, *J* = 5.1 Hz, 6H), 1.00 (d, *J* = 6.6 Hz, 3H).

### Example I-209

### 4-((4R,9S)-8-(2,2-dimethyl-3-oxo-3-(pyrrolidin-1-yl)propyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

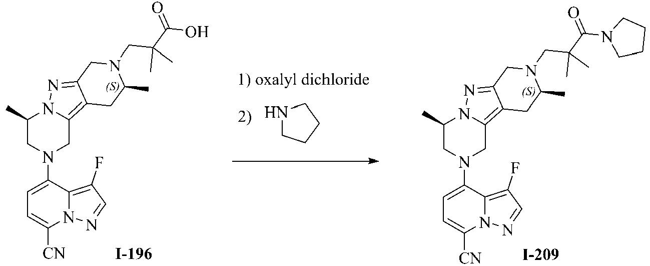

**Example I-209** was synthesized by following the protocols of **I-164**. MS: 518.9 (M+H)⁺. ¹H NMR (400 MHz, DMSO-*d*₆) δ 8.30 (d, *J* = 3.6 Hz, 1H), 7.72 (d, *J* = 8.0 Hz, 1H), 6.69 (d, *J =* 8.2 Hz, 1H), 4.52 - 4.39 (m, 2H), 4.35 (s, 1H), 4.00 (d, *J =* 11.5 Hz, 1H), 3.70 - 3.49 (m, 4H), 3.42 - 3.38 (m, 1H), 3.30 - 3.22 (m, 1H)3.04 (q, *J=* 5.9 Hz, 1H), 2.64 - 2.55 (m, 3H), 2.19 (dd, *J* = 15.2, 4.9 Hz, 1H), 1.75 (s, 4H), 1.48 (d, *J* = 6.3 Hz, 3H), 1.18 (d, *J =* 9.7 Hz, 6H), 1.00 (d, *J* = 6.7 Hz, 3H).

### Example I-210

### 3-Fluoro-4-((4R,9S)-8-((3R,4S)-4-hydroxytetrahydrofuran-3-yl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

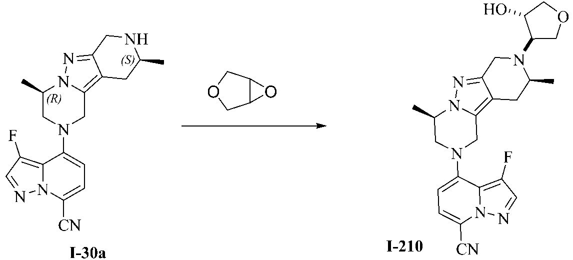

**Example 1-210** was synthesized by following the protocols of **Example I-99**. MS: 451.9 (M+H)⁺. ¹H NMR (400 MHz, DMSO-*d*₆) δ 8.31 (d, *J =* 3.5 Hz, 1H), 7.73 (d, *J =* 8.0 Hz, 1H), 6.70 (d, *J =* 8.1 Hz, 1H), 5.10 (s, 1H), 4.45 (d, *J =* 5.9 Hz, 2H), 4.36 (s, 1H), 4.17 (s, 1H), 3.99 (td, *J =* 10.6, 9.0, 5.4 Hz, 2H), 3.86 - 3.75 (m, 2H), 3.57 - 3.50 (m, 4H), 3.19 - 3.03 (m, 2H), 2.78-2.70 (m, 1H), 2.22 (d, *J=* 15.2 Hz, 1H), 1.49 (dd, *J=* 6.4, 2.6 Hz, 3H), 1.00 (dd, *J =* 14.0, 6.5 Hz, 3H).

### Example I-211

### 4-((4R,9S)-8-(2-amino-3,4-dioxocyclobut-1-en-1-yl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

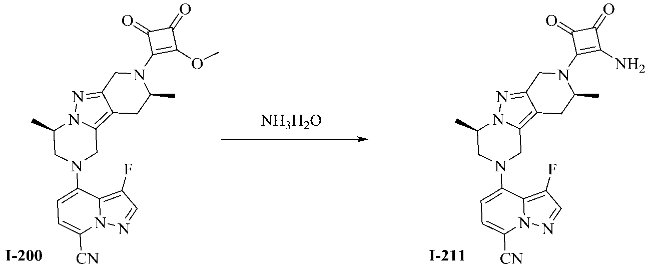

**Example I-211** was synthesized by following the protocols of **Example I-99**. MS: 461.4 (M+H)⁺. ¹H NMR (400 MHz, DMSO-*d*₆) δ 8.30 (d, *J* = 3.5 Hz, 1H), 7.81 (s, 2H), 7.72 (d, *J =* 8.0 Hz, 1H), 6.70 (d, *J =* 8.1 Hz, 1H), 4.55 - 4.45 (m, 3H), 4.40 (s, 1H), 4.02 (d, *J =* 11.5 Hz, 1H), 3.49 - 3.39 (m, 2H), 2.94 - 2.82 (m, 1H), 2.59 - 2.54 (m, 1H), 2.47 (d, *J* = 15.3 Hz, 1H), 1.52 (d, *J =* 6.4 Hz, 3H), 1.23 (d, *J =* 6.8 Hz, 3H).

### Example I-212

### 4-((4R,9S)-8-(2-(dimethylamino)-3,4-dioxocyclobut-1-en-1-yl)-4,9-dimethyl-3,4,7,8,9, 10-hexahydropyrido[3',4':3,4]pyrazolo[1,5-a]pyrazin-2(1H)-yl)-3-fluoropyrazolo[1,5-a]pyridine-7-carbonitrile

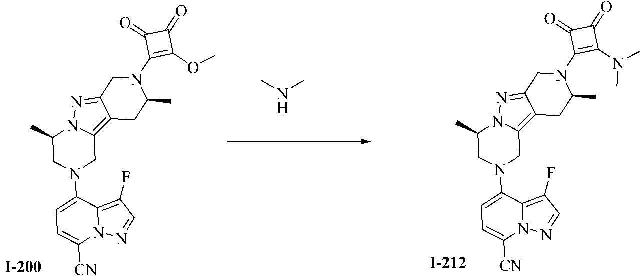

**Example I-212** was synthesized by following the protocols of **Example I-99**. MS: 489.4 (M+H)⁺. ¹H NMR (400 MHz, DMSO-*d*₆) δ 8.31 (d, *J* = 3.5 Hz, 1H), 7.73 (d, *J =* 8.0 Hz, 1H), 6.70 (d, *J =* 8.1 Hz, 1H), 4.81 - 4.70 (m, 2H), 4.57 (d, *J =* 16.1 Hz, 1H), 4.50 (d, *J =* 8.2 Hz, 2H), 4.39 (q, *J* = 9.9, 7.3 Hz, 1H), 4.05 - 3.97 (m, 1H), 3.43 (d, *J=* 8.8 Hz, 1H), 3.22 (s, 6H), 2.91 (dd, *J =* 15.5, 5.9 Hz, 1H), 1.51 (d, *J =* 6.4 Hz, 3H), 1.27 (d, *J =* 6.8 Hz, 3H).

### Example I-213

### 3-Fluoro-4-((4R,9S)-8-((1R,2R)-2-hydroxycyclopentyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

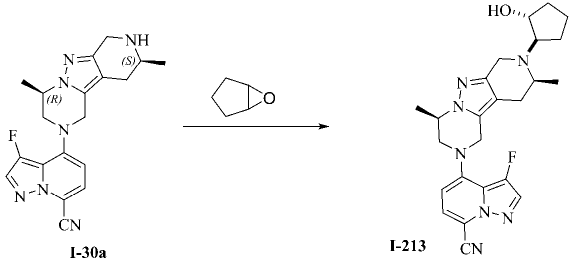

**Example I-213** was synthesized by following the protocols of **Example 1-99.** MS: 449.9 (M+H)⁺. ¹H NMR (400 MHz, DMSO-*d*₆) δ 8.30 (d, *J* = 3.5 Hz, 1H), 7.72 (d, *J* = 8.0 Hz, 1H), 6.69 (d, *J=* 8.1 Hz, 1H), 4.59 (dd, *J* = 9.2, 5.3 Hz, 1H), 4.44 (s, 2H), 4.34 (s, 1H), 4.00 (d, *J* = 11.9 Hz, 2H), 3.75 (d, *J* = 15.0 Hz, 1H), 3.58 - 3.37 (m, 3H), 2.81 - 2.68 (m, 2H), 2.23 - 2.15 (m, 1H), 1.91 - 1.77 (m, 2H), 1.66 - 1.34 (m, 8H), 0.97 (t, *J* = 6.0 Hz, 3H).

### Example I-214

### 4-((4R)-8-amino-4-methyl-1,3,4,7,8,9,10,11-octahydro-2H-cyclohepta[3,4]pyrazolo[1,5-a]pyrazin-2-yl)-3-fluoropyrazolo[1,5-a]pyridine-7-carbonitrile

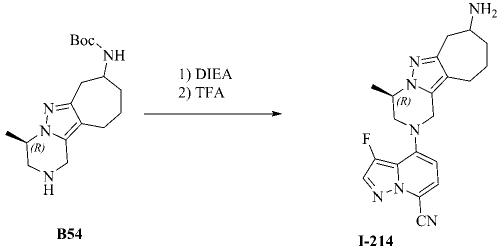

**Example I-214** was synthesized by following the protocols of **Example I-2**. MS: 380.1 (M+H)⁺. ¹H NMR (400 MHz, DMSO-*d₆*) δ 8.31 (d, J = 3.6 Hz, 1H), 8.02 - 7.88 (m, 3H), 7.75 (d, J = 8.0 Hz, 1H), 6.73 (d, J = 8.0 Hz, 1H), 4.51 (t, J = 15.2 Hz, 1H), 4.37 (dd, J = 24.8, 15.2 Hz, 2H), 4.00 (dt, J = 12.8, 5.6 Hz, 1H), 3.35 - 3.26 (m, 1H), 3.22 - 3.06 (m, 2H), 2.81 (dt, J = 14.2, 10.8 Hz, 1H), 2.68 - 2.57 (m, 1H), 2.32 (dt, J = 32.5, 13.6 Hz, 1H), 2.18 (s, 1H), 1.91 (q, J = 15.2 Hz, 1H), 1.85 - 1.69 (m, 1H), 1.48 (d, J = 6.4 Hz, 3H), 1.46 - 1.30 (m, 1H).

### Example I-215

### 4-((4R,9S)-8-ethyl-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

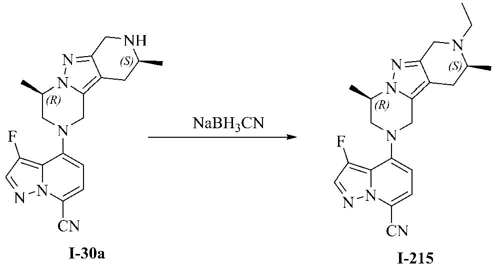

**Example I-215** was synthesized by following the protocols of **Example I-24.**MS: 394.1 (M+H)⁺.

### Example I-216

### 2-(((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)methyl)cyclopropane-1-carboxamide

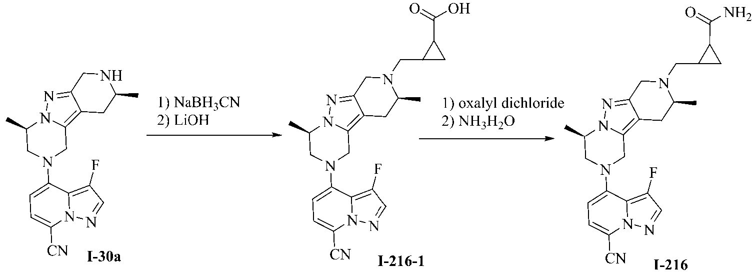

**Example I-216** was synthesized by following the protocols of **Example I-198**. MS: 394.1 (M+H)⁺. ¹H NMR (400 MHz, DMSO-*d*₆) δ 8.32 (d, *J =* 3.5 Hz, 1H), 7.74 (d, *J =* 8.0 Hz, 1H), 7.62 (s, 1H), 6.90 (s, 1H), 6.71 (d, *J* = 8.1 Hz, 1H), 4.58 - 4.37 (m, 3H), 4.30 - 3.98 (m, 2H), 3.42 - 3.35 (m, 4H), 2.93 (s, 3H), 1.51 (d, *J =* 6.3 Hz, 5H), 1.25 (s, 3H), 1.01 (s, 1H), 0.79 (s, 1H).

### Example I-221

### 2-((4R,9S)-2-(7-cyano-3-fluoropyrazolo[1,5-a]pyridin-4-yl)-4,9-dimethyl-1,2,3,4,9,10-hexahydropyrido[3',4':3,4]pyrazolo[1,5-a]pyrazin-8(7H)-yl)-N,N-dimethylacetamide-d2

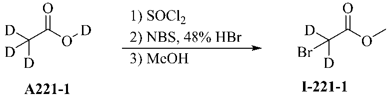

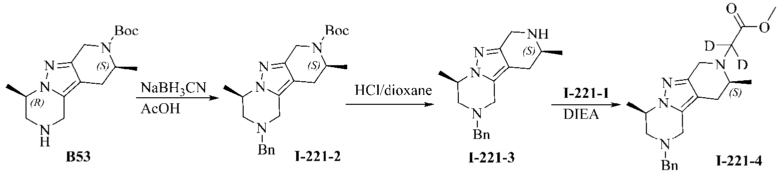

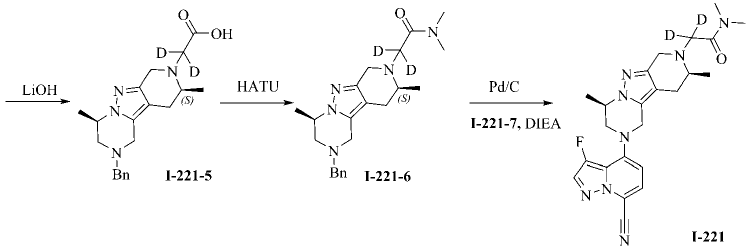

### Step1: Preparation of Intermediate I-221-1

A reaction mixture of SOCl₂ (11.14 g, 94 mmol), AcOH-D₄ (5 g, 78 mmol) in CHCl₃ (8 mL) was stirred at 85 °C for 0.5 hour. Then the reaction was cooled to 25°C. CHCl₃ (75 mL), NBS (16.7 g, 94 mmol) and 6 drops of HBr solution (48%) were then added to the mixture. The resulting mixture was stirred at 25°C for 1 hour. The mixture was quenched with icy water then extracted with EtOAc, and the organic phase was washed with saturated saline, dried with Na₂SO₄ and concentrated to give **Intermediate I-221-1** (4 g, 33.1%) as colorless oil.

**Steps 2-8: Intermediate I-221-3** was synthesized by following the protocols of **I-177-1.** MS: 297.1 (M+H)⁺. **Intermediate I-221-4** was synthesized by following the protocols of **Example I-99**. MS: 371.1 (M+H)⁺. **Intermediate I-221-5** was synthesized by following the protocols of **Intermediate B10-3.** MS: 357.2 (M+H)⁺. **Intermediate I-221-6** was synthesized by following the protocols of **Intermediate B10-4.** MS: 384.2 (M+H)⁺. **Intermediate 1-221-7** was synthesized by following the protocols of **Intermediate B4-1.** MS: 294.1 (M+H)⁺.

**Example I-221** was synthesized by following the protocols of **Intermediate I-1**. MS: 453.1 (M+H)⁺. ¹H NMR (400 MHz, DMSO-*d*₆) δ 8.29 (d, *J* = 3.6 Hz, 1H), 7.71 (d, *J* = 8.0 Hz, 1H), 6.68 (d, *J* = 8.0 Hz, 1H), 4.50 - 4.37 (m, 2H), 4.33 (q, *J* = 7.2, 4.8 Hz, 1H), 4.03 - 3.96 (m, 1H), 3.61 (d, *J=* 16.8 Hz, 2H), 3.37 (s, 1H), 3.12 - 3.07 (m, 1H),3.02 (s, 3H), 2.81 (s, 3 H), 2.62 (dd, *J=* 15.2, 4.8 Hz, 1H), 2.19 (dd, *J =* 15.2, 6.0 Hz, 1H), 1.46 (d, *J* = 6.4 Hz, 3H), 1.04 (d, *J* = 6.4 Hz, 3H).

### Example I-222

### 2-((4R)-2-(7-cyano-3-fluoropyrazolo[1,5-a]pyridin-4-yl)-4,7,10-trimethyl-1,2,3,4,9,10-hexahydropyrido[3',4':3,4]pyrazolo[1,5-a]pyrazin-8(7H)-yl)acetamide

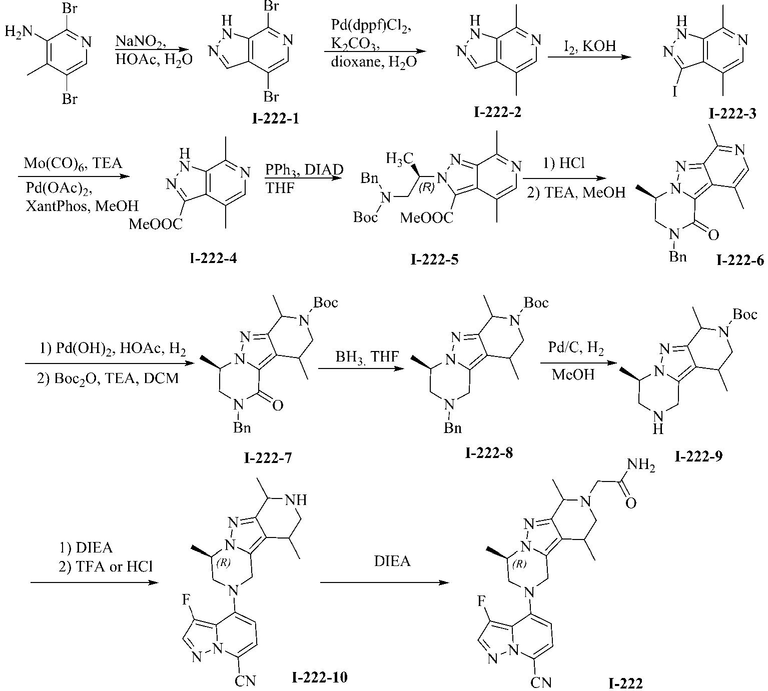

**Intermediate I-222-9** was synthesized by following the protocols of **Intermediate B47.** MS: 321.1 (M+H)⁺. **Intermediate 1-222-10** was synthesized by following the protocols of **Example I-30.** MS: 380.1 (M+H)⁺.

**Example I-222** was synthesized by following the protocols of **Example I-99**. MS: 437.1 (M+H)⁺. ¹H NMR (400 MHz, DMSO-*d₆*) δ 8.29 (d, *J =* 3.6 Hz, 1H), 7.71 (d, *J =* 8.0 Hz, 1H), 7.23 - 7.06 (m, 2H), 6.75 (d, *J* = 8.0 Hz, 1H), 4.59 - 4.27 (m, 3H), 4.04 - 3.89 (m, 1H), 3.61 (q, *J* = 6.4 Hz, 1H), 3.24 (d, *J* = 28.4 Hz, 1H), 3.12 (d, *J* = 16.0 Hz, 1H), 2.98 (d, *J* = 16.0 Hz, 1H), 2.85 (dt, *J =* 12.4, 6.0 Hz, 1H), 2.69 (dd, *J =* 12.4, 4.8 Hz, 1H), 2.56 (dd, *J =* 12.4, 6.4 Hz, 1H), 1.52 - 1.42 (m, 3H), 1.31 (d, *J =* 6.4 Hz, 3H), 1.20 - 1.10 (m, 3H).

### Example I-223

### 3-Fluoro-4-((4R,9S)-8-(3-(hydroxymethyl)bicyclo[1.1.1]pentan-1-yl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

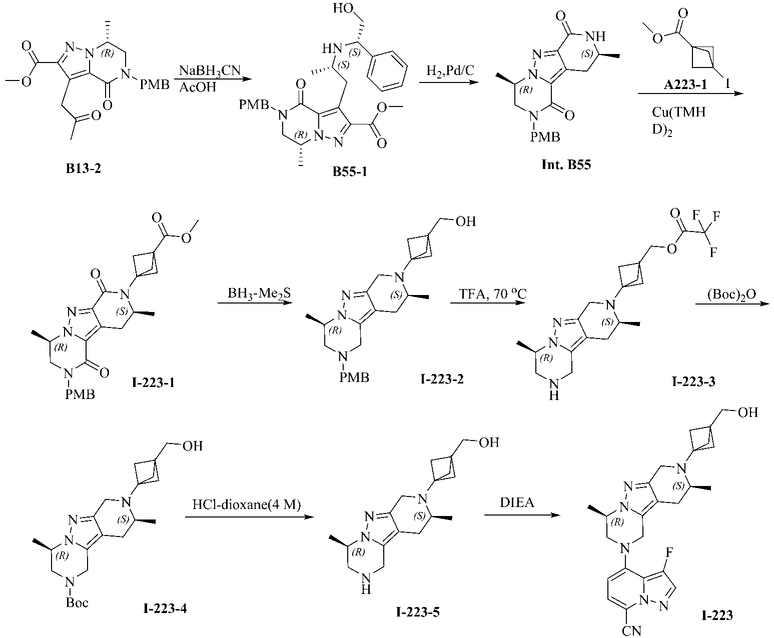

### Step 1: Preparation of Intermediate B55

**Intermediate B55** was synthesized by following the protocols of **Intermediate B13-4.** MS: 354.8 (M+H)⁺.

### Step 2: Preparation of Intermediate I-223-1

A reaction mixture of Cu(TMHD)₂ (951 mg, 2.21 mmol), K₃PO₄ (1.4 g, 6.64 mmol), **Intermediate B-55** (800 mg, 2.21 mmol) and **Intermediate A223-1** (669 mg, 2.65 mmol) in DMF (10 mL) was stirred at 110 °C (in a microwave reactor) for 2 hours. The mixture was quenched with water then extracted with EtOAc, and the organic phase was washed with saturated saline, dried with Na₂SO₄ and concentrated. Purification by flash chromatography gave **Intermediate I-223-1** (420 mg, 31%) as yellow solid. MS: 478.9 (M+H)⁺.

### Step 3: Preparation of Intermediate I-223-2

**Intermediate I-223-2**was synthesized by following the protocols of **Intermediate B2.** MS: 422.9 (M+H)⁺.

### Step 4: Preparation of Intermediate I-223-3

A reaction mixture of **Intermediate I-223-2**(350 mg, 0.76 mmol) in TFA (10 mL) was stirred at 70 °C for 48 hours. The mixture was concentrated under vacuum to give **Intermediate I-223-3** (390 mg, 100%) as yellow solid. MS: 398.9&302.8 (M+H)⁺.

### Step 5: Preparation of Intermediate I-223-4

A reaction mixture of **Intermediate I-223-3** (350 mg, 0.88 mmol), TEA (4 mL) and Boc₂O (383 mg, 1.8 mmol) in DCM (15 mL) was stirred at 25 °C for 2 hours. The mixture was quenched with water then extracted with EtOAc, and the organic phase was washed with saturated saline, dried with Na₂SO₄ and concentrated. Purification by flash chromatography gave **Intermediate I-223-4** as yellow solid. MS: 402.9 (M+H)⁺.

### Step 6: Preparation of Intermediate 1-223-5

A reaction mixture of **Intermediate I-223-4** (220 mg, 0.52 mmol) and HCl (4 M, 1 mL) in MeOH (2 mL) was stirred at 25 °C for 2 hours. The mixture was concentrated under vacuum to give **Intermediate I-223-5** (180 mg) as yellow solid. MS: 302.9 (M+H)⁺.

### Step 7: Intermediate I-223 Preparation

**Example I-223** was synthesized by following the protocols of **Example I-1**. MS: 461.9 (M+H)⁺. ¹H NMR (400 MHz, DMSO-*d*6) δ 8.32 (d, *J =* 3.5 Hz, 1H), 7.73 (d, *J =* 8.0 Hz, 1H), 6.69 (d, *J =* 8.1 Hz, 1H), 4.50 (d, *J =* 9.2 Hz, 1H), 4.45 (d, *J=* 4.1 Hz, 1H), 4.34 (s, 0H), 4.11 - 3.92 (m, 1H),3.70 - 3.43 (m, 3H), 3.35 - 3.21 (m, 1 H), 2.71 (dd, *J* = 14.9, 6.0 Hz, 1H), 2.22 (d, *J =* 14.8 Hz, 1H), 1.77 - 1.58 (m, 6H), 1.49 (d, *J =* 6.3 Hz, 3H), 0.99 (d, *J =* 6.6 Hz, 3H).

### Example I-224

### 4-((4R,9S)-8-((R)-2,3-dihydroxy-2-methylpropyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

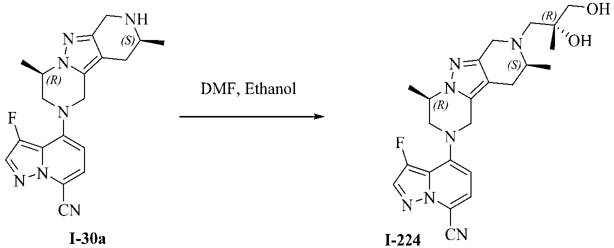

A reaction mixture of **Example I-30a** (250 mg, 0.684 mmol), (R)-(2-methyloxiran-2-yl)methanol (121 mg, 1.368 mmol) in DMF (2 mL) and EtOH (2 mL) was stirred at 100 °C for 15 hours. The mixture was quenched with water then extracted with EtOAc, and the organic phase was washed with saturated saline, dried with Na₂SO₄ and concentrated. Purification by pre-HPLC gave **Example I-224** (71 mg, 22.9%) as white solid.
MS: 453.9 (M+H)⁺. ¹H NMR (400 MHz, DMSO-*d*₆) δ 8.26 (d, *J* = 3.6 Hz, 1H), 8.16 (s, 1H), 7.68 (d, *J* = 8.0 Hz, 1H), 6.65 (d, *J* = 8.1 Hz, 1H), 4.48 - 4.27 (m, 4H), 3.96 (d, *J =* 12.2 Hz, 1H), 3.73 (d, *J =* 3.1 Hz, 3H), 3.34 - 3.20 (m, 4H), 3.08 (q, *J=* 5.8 Hz, 1H), 2.58 (dd, *J* = 15.2, 5.1 Hz, 1H), 2.43 (s, 1H), 2.30 (d, *J=* 13.8 Hz, 1H), 2.15 (dd, *J* = 15.2, 5.2 Hz, 1H), 1.44 (d, *J* = 6.4 Hz, 3H), 0.98 (d, *J* = 7.1 Hz, 6H).

### Example 1-225

### 3-Fluoro-4-((4R,9S)-8-(3-hydroxy-2-(hydroxymethyl)-2-methylpropyl)-4,9-dimethyl-3,4,7,8,9,10-hexahydropyrido[3',4':3,4]pyrazolo[1,5-a]pyrazin-2(1H)-yl)pyrazolo[1,5-a]pyridine-7-carbonitrile

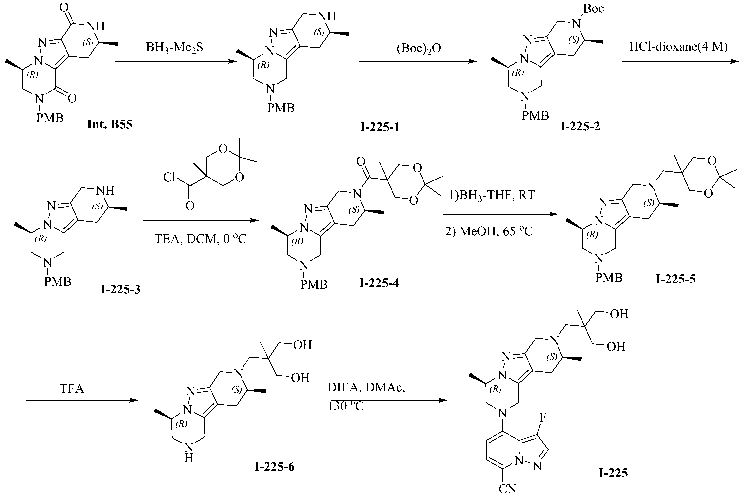

### Steps 1-3: Preparation of Intermediates I-225-3, I-225-4, I-225-5

**Intermediate I-225-3** was synthesized by following the protocols of **Intermediate I-223-5.** MS: 326.9 (M+H)⁺. **Intermediate I-225-4** was synthesized by following the protocols of **Intermediate 1-6.** MS: 482.9 (M+H)⁺. **Intermediate 1-225-5** was synthesized by following the protocols of **Intermediate B2**. MS: 468.9 (M+H)⁺.

### Step 4: Preparation of Intermediate I-225-6

A reaction mixture of **Intermediate I-225-5** (750 mg, 1.6 mmol) in TFA (15 mL) was stirred at 70°C for 48 hours. The mixture was quenched with water and extracted with EtOAc, and the organic phase was washed with saturated saline, dried with Na₂SO₄ and concentrated to give **Intermediate I-225-6** (500 mg, 100%) which was directly used in the next reaction. MS: 308.9 (M+H)⁺.

**Example I-225** was synthesized by following the protocols of **Intermediate I-1.** MS: 465.9 (M+H)⁺. ¹H NMR (400 MHz, DMSO-*d*₆) δ 8.26 (d, *J =* 3.5 Hz, 1H), 7.69 (d, *J =* 8.0 Hz, 1H), 6.66 (d, *J =* 8.1 Hz, 1H), 4.47 (s, 2H), 4.41 (d, *J* = 15.1 Hz, 2H), 4.31 (s, 1H), 3.96 (d, *J=* 11.8 Hz, 1H), 3.63 (s, 2H), 3.23 (s, 4H), 3.05 (s, 1H), *J =* 15.2, 5.2 Hz, 1H), 2.43 (d, *J* = 14.0 Hz, 1H), 2.23 (d, *J=* 14.0 Hz, 1H), 2.16 (dd, *J* = 15.2, 4.3 Hz, 1H), 1.45 (d, *J* = 6.4 Hz, 3H), 0.97 (d, *J =* 6.7 Hz, 3H), 0.71 (s, 3H).

### Example I-226

### 4-((4R,9S)-8-((S)-2,3-dihydroxy-2-methylpropyl)-4,9-dimethyl-3,4,7,8,9,10-hexahydropyrido[3',4':3,4]pyrazolo[1,5-a]pyrazin-2(1H)-yl)-3-fluoropyrazolo[1,5-a]pyridine-7-carbonitrile

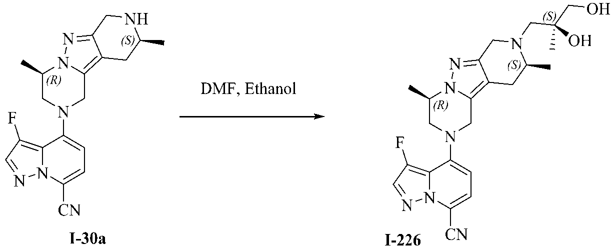

**Example I-226** was synthesized by following the protocols of **Example I-224.** MS: 453.9 (M+H)⁺. ¹H NMR (400 MHz, DMSO-*d*₆) δ 8.31 (d, *J* = 3.6 Hz, 1H), 7.73 (d, *J* = 8.0 Hz, 1H), 6.70 (d, *J* = 8.1 Hz, 1H), 4.45 (q, *J* = 15.3 Hz, 2H), 4.33 (d, *J* = 10.2 Hz, 1H), 4.04 - 3.97 (m, 1H), 3.77 (d, *J* = 3.0 Hz, 2H), 3.39 - 3.33 (m, 5H), 3.27 (d, *J* = 2.9 Hz, 2H), 3.12 (q, *J=* 5.9 Hz, 1H), 2.62 (dd, *J* = 15.2, 5.1 Hz, 1H), 2.48 (d, *J* = 13.7 Hz, 1H), 2.34 (d, *J=* 13.8 Hz, 1H), 2.20 (dd, *J* = 15.2, 5.3 Hz, 1H), 1.49 (d, *J* = 6.4 Hz, 3H), 1.02 (d, *J* = 7.0 Hz, 6H).

### Example I-227

### 4-((4R,7R,9S)-8-((R)-2,3-dihydroxypropyl)-4,7,9-trimethyl-3,4,7,8,9,10-hexahydro pyrido[3',4':3,4]pyrazolo[1,5-a]pyrazin-2(1H)-yl)-3-fluoropyrazolo[1,5-a]pyridine-7-carbonitrile

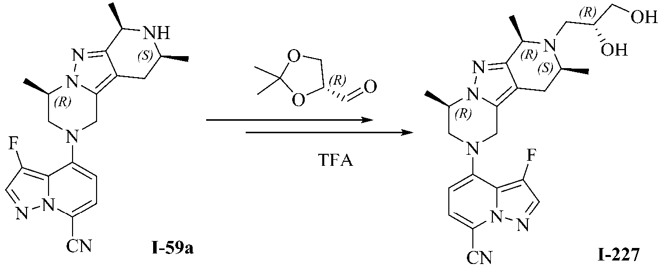

**Example I-227** was synthesized by following the protocols of **Example I-158.** MS: 454.3 (M+H)⁺.

### Evaluation of biological efficacy at cellular functional levels

HEK-Blue cells with stable expression of human TLR7 or TLR8 were used in the evaluation of inhibitory activity of compounds in the present invention against TLR7 or TLR8, by using its ability to induce the SEAP reporting gene under the control of the fusion of IFN-β minimum promoters into five NF-κB and ap-1 binding sites, as follows:
hTLR7 HEK-Blue cells (Invivogen, 80, 000 cells per well) or hTLR8 HEK-Blue cells (60, 000 cells per well) were added to a 96-well cell culture plate. A solution of the testing compounds were added to each well, with the final concentration of tested compounds in the medium ranged from 0.001 to 36 µM and the culture was incubated for 15 minutes. M5049 was used as positive controls in the testing.

Then a TLR7 ligand (Gardiquimod, purchased from MedChemExpress, with final concentration of ligand in cell culture medium kept at 10 µM) or a TLR8 ligand (purchased from MedChemExpress with final concentration in cell culture media at 10 µM) and the culture was incubated for 20 hours. According to the manufacturer's instructions, a HEK-Blue reagent Quanti-blue (Invivogen) was used to detect the SEAP levels in cell cultures. The half effective concentration or IC₅₀ was calculated by using GraphPad Prism. The IC₅₀ values were defined as A and B (Against hTLR7 EC₅₀(µM): A < 0.05 µM, B > 0.05 µM; against hTLR8 EC ₅₀(µM): A < = 0.1 µM, B > 0.1 µM; -, as untested).

**Table 1: Activities of examples in this invention to hTLR7/8 in HEK-Blue Cells**

| Example code | HEK-Blue hTLR7 IC₅₀ (µM) | HEK-Blue hTLR8 IC₅₀ (µM) |
|---|---|---|
| **M5049** | 0.064 | 0.15 |
| **I-2** | A | A |
| **I-5** | A | A |
| **I-9** | B | - |
| **I-11** | A | A |
| **I-11a** | A | A |
| **I-11b** | A | A |
| **I-12** | A | A |
| **I-17** | - | B |
| **I-19** | - | B |
| **I-20** | - | A |
| **I-20a** | A | A |
| **I-20b** | A | A |
| **I-21** | A | A |
| **I-23** | B | - |
| **I-25** | A | - |
| **I-27** | B | A |
| **I-28** | B | A |
| **I-29** | - | A |
| **I-30** | A | A |
| **I-30a** | A | A |
| **I-30b** | A | A |
| **I-31** | - | A |
| **I-32** | - | A |
| **I-33** | B | A |
| **I-34** | A | A |
| **I-35** | - | A |
| **I-36** | - | A |
| **I-37** | - | A |
| **I-38** | - | B |
| **I-39** | A | A |
| **I-40** | B | A |
| **I-43** | A | A |
| **I-44** | - | A |
| **I-45** | A | A |
| **I-46** | - | A |
| **I-47** | - | B |
| **I-49** | B | - |
| **I-52** | A | A |
| **I-53** | A | - |
| **I-54** | A | - |
| **I-56** | B | - |
| **I-59** | A | B |
| **I-59a** | A | B |
| **I-59b** | A | A |
| **I-60** | A | B |
| **I-61** | A | B |
| **I-62** | B | - |
| **I-67** | B | - |
| **I-68** | B | A |
| **I-69** | - | A |
| **I-70** | - | A |
| **I-71** | - | A |
| **I-71a** | A | A |
| **I-71b** | B | A |
| **I-74** | B | B |
| **I-76** | B | B |
| **I-77** | B | A |
| **I-78** | - | A |
| **I-79** | A | A |
| **I-80** | B | - |
| **I-80a** | B | B |
| **I-80b** | B | B |
| **I-82** | A | B |
| **I-83** | A | A |
| **I-85** | B | A |
| **I-86** | A | - |
| **I-87** | A | A |
| **I-88a** | A | A |
| **I-88b** | A | B |
| **I-91** | A | A |
| **I-95** | B | B |
| **I-99** | A | B |
| **I-101** | B | - |
| **I-102** | A | - |
| **I-106** | A | A |
| **I-109** | - | A |
| **I-111** | A | A |
| **I-112** | B | - |
| **I-113** | A | - |
| **I-114** | - | A |
| **I-117** | A | A |
| **I-118** | A | A |
| **I-120** | B | A |
| **I-122** | - | A |
| **I-124** | A | A |
| **I-125** | A | A |
| **I-128** | A | A |
| **I-129** | A | A |
| **I-130** | A | - |
| **I-132** | A | A |
| **I-133** | B | - |
| **I-134** | A | A |
| **I-135** | A | A |
| **I-136** | A | A |
| **I-137** | A | A |
| **I-138** | A | B |
| **I-139** | A | B |
| **I-140** | A | B |
| **I-141** | B | B |
| **I-142** | A | B |
| **I-144** | A | B |
| **I-145** | A | A |
| **I-146** | A | B |
| **I-149** | A | B |
| **I-151** | A | B |
| **I-152** | A | B |
| **I-153** | A | B |
| **I-154** | B | B |
| **I-155** | A | B |
| **I-156** | B | B |
| **I-157** | B | B |
| **I-158** | A | A |
| **I-159** | B | B |
| **I-160** | B | B |
| **I-164** | A | B |
| **I-165** | A | B |
| **I-174** | A | B |
| **I-184** | B | B |
| **I-187** | A | A |
| **I-188** | B | A |
| **I-192** | B | A |
| **I-193** | B | A |
| **I-198** | B | B |
| **I-202** | B | A |
| **I-204** | A | B |
| **I-214** | - | A |
| **I-224** | A | A |
| **I-225** | B | B |
| **I-226** | A | B |

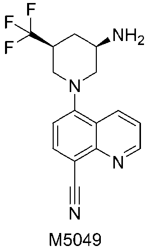

The results showed that the compounds in this invention have excellent TLR7 and/or TLR8 inhibitory activities at cellular levels.

### Evaluation of pharmacokinetics (PK) in mice

The pharmacokinetic study was carried out by Shanghai Medicilon Inc. Each testing compound was dissolved in a solvent of 10% DMSO + 40% PEG400 + 50% Saline (0.9% saline) and administered intravenously (i.v., 2 mg/kg) or through oral gavage (po, 5 mg/kg) to ICR mice. The mice were fasted overnight (10-14 hours) before compound was administered and were fed with food 4 hours after drug administration. Blood samples were collected at multiple time points (0.083, 0.25, 0.5, 1, 4.6 hours) in heparin-coated tubes. The plasma concentration of testing samples was analyzed by LC-MS/MS, and the PK parameters were calculated by Phoenix WinNonlin.

In conclusion: Experimental data showed that the compounds of the present invention with pharmacokinetic properties in mice, specifically, with plasma drug exposure sufficient to achieve in vivo efficacy.

All of the literature referred to in the present invention is cited as reference in the application. Furthermore, it should be understood that after reading the above-mentioned teachings of the present invention, a skilled person in the field may make various alterations or modifications to the present invention, and these equivalent forms fall equally within the scope of the claims annexed to this application.

## Claims

1. A compound shown in formula I, a pharmaceutically acceptable salt, a racemate, an R-isomer, an S-isomer, or mixtures thereof: 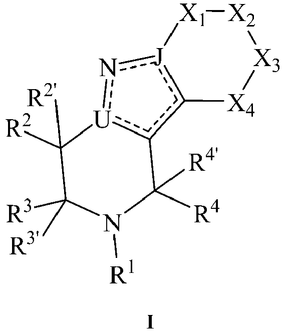 wherein,
the dashed line is a chemical bond or none; and 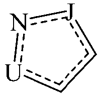 is an aromatic ring;
U is selected from the group consisting of: C and N;
J is selected from the group consisting of: C and N;
X₁, X₂, X₃, and X₄ are each independently selected from the group consisting of: bond, - O-, -S-, -S(=O)-, -S(=O)₂-, -C(=O)-, -C(R⁵R⁵')-, -C(R¹⁵)(L₁-NR¹⁶R¹⁶')-, -N(R⁸)-, and CH₂CH₂;
R¹ is selected from the group consisting of: substituted or unsubstituted fused 5, 6 membered heteroaryl, and substituted or unsubstituted fused 6, 6 membered heteroaryl;
R², R³, R⁴, R²', R³', and R⁴' are each independently selected from the group consisting of: H, deuterium, substituted or unsubstituted C₁₋₆ alkoxy, carboxyl, NH₂, substituted or unsubstituted C₁₋₆ alkyl, halogen, cyano, substituted or unsubstituted C₂₋₆ ester group, substituted or unsubstituted C₁₋₆ alkylene-COOH, substituted or unsubstituted C₂₋₆ acylamino, substituted or unsubstituted (CH₂)C(O)N(R¹⁷)₂, substituted or unsubstituted C₃₋₇ cycloalkyl, hydroxyl, substituted or unsubstituted C₁₋₉ heteroalkyl, substituted or unsubstituted 3-to 9- membered heterocycloalkyl; or R², R³, R⁴, R²', R³', and R⁴' located on the same carbon atom together form=O; or R², R²' and the carbon atom to which they attached, R³, R³' and the carbon atom to which they attached, or R⁴, R⁴' and the carbon atom to which they attached together form a substituted or unsubstituted 3- to 6- membered cycloalky, or a substituted or unsubstituted 4- to 6- membered heterocyclyl;
R⁵ and R⁵' are each independently selected from the group consisting of: H, deuterium, substituted or unsubstituted C₁₋₆ alkoxy, carboxyl, NH₂, halogenated C₁₋₆ alkyl, substituted or unsubstituted C₁₋₆ alkyl, halogen, cyano, substituted or unsubstituted C₂₋₆ ester group, substituted or unsubstituted C₁₋₆ alkylene-COOH, substituted or unsubstituted C₂₋₆ acylamino, substituted or unsubstituted (CH₂)C(O)N(R¹⁷)₂, substituted or unsubstituted C₃₋₇ cycloalkyl, hydroxyl, substituted or unsubstituted C₆-C₁₀ aryl-(CH₂)-, substituted or unsubstituted 5-to 12- membered heteroaryl-(CH₂)-, substituted or unsubstituted C₆-C₁₀ aryl, substituted or unsubstituted 5-to 12- membered heteroaryl, substituted or unsubstituted C₁. ₉ heteroalkyl, substituted or unsubstituted 3-to 9- membered heterocycloalkyl, substituted or unsubstituted 3-to 9- membered heterocycloalkyl-(CH₂)-, substituted or unsubstituted 3-to 9- membered heterocycloalkyl-C(=O); or each R⁵, and R⁵' located on the same carbon atom together form =O;
or R⁵, R⁵' and the carbon atom to which they attached together form a substituted or unsubstituted 3- to 6- membered cycloalky, or a substituted or unsubstituted 4- to 6-membered heterocyclyl;
or two of R⁵, R⁵' and R⁸ located on different atom together with the carbon or nitrogen atom to which they attached (optionally including the ring atom between the carbon atom or nitrogen atom to which they attached) together form a substituted or unsubstituted 3- to 6- membered cycloalky, or a substituted or unsubstituted 4- to 9- membered heterocyclyl;
L₁ is a chemical bond or C₁~C₆ alkylene, preferably a chemical bond;
R¹⁵ is independently selected from the group consisting of: H, deuterium, C₁₋₆ alkoxy, halogenated C₁₋₆ alkyl, C₁₋₆ alkyl, halogen, cyano, C₃₋₇ cycloalkyl, hydroxyl, hydroxyl substituted C₁₋₆ alkyl; or R¹⁵ and R¹⁶, R¹⁵ and R¹⁶' together form a substituted or unsubstituted 4- to 6- membered heterocycloalkyl; or R¹⁵, L₁ and the carbon atom to which they attached together form a substituted or unsubstituted 3- to 6- membered cycloalky, or a substituted or unsubstituted 4- to 6- membered heterocyclyl;
R¹⁶ and R¹⁶' are each independently selected from the group consisting of: H, deuterium, substituted or unsubstituted 3- to 9- membered carbocyclyl (saturated, unsaturated monocyclic ring, spiro ring, fused ring, or bridged ring), substituted or unsubstituted 4-to 9- membered heterocyclyl (saturated, unsaturated monocyclic ring, spiro ring, fused ring, or bridged ring), substituted or unsubstituted C₁₋₆ alkyl, benzyl, - (CH₂)ₙN(R⁸⁻¹R⁸⁻²), -(CH₂)_{nC}(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, -C(O)(CH₂)ₙR⁸⁻³, C₁₋₆ alkyl NHS(O)₂-, C₁₋₆ alkyl NHC(O)-, -C(O)(CH₂)ₙN(R⁸⁻¹R⁸⁻²), -SO₂(CH₂)ₙN(R⁸⁻¹R⁸⁻²), (CH₂)ₙSO₂N(⁸⁻¹R⁸⁻²), C₁-C₆ alkoxy, C₁-C₆ alkyl-amido, C₆-C₁₀ arylbenzyl, and 5 or 6 membered heteroarylbenzyl;
or R¹⁶ and R¹⁶' together form a substituted or unsubstituted 4-to 8- membered heterocycloalkyl;
R¹⁷ is selected from the group consisting of: H, substituted or unsubstituted C₁₋₆ alkyl, substituted or unsubstituted C₁₋₉ heteroalkyl, substituted or unsubstituted C₆-C₁₀ aryl, substituted or unsubstituted 5- or 6-membered heteroaryl, substituted or unsubstituted 3- to 9- membered heterocycloalkyl, and substituted or unsubstituted C₃₋₇ cycloalkyl;
R⁸ is selected from the group consisting of: H, deuterium, substituted or unsubstituted 3- to 9- membered carbocyclyl, substituted or unsubstituted 4- to 9- membered heterocyclyl, substituted or unsubstituted 3- to 9- membered carbocyclyl-(CH₂)-, substituted or unsubstituted 4- to 9- membered heterocyclyl-(CH₂)-, substituted or unsubstituted C₁₋₆ alkyl, substituted or unsubstituted benzyl, and substituted or unsubstituted group selected from the group consisting of: -(CH₂)ₙN(R⁸⁻¹R⁸⁻²), -(CH₂)_{nC}(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₘSO₂R⁸⁻¹, -(CH₂)ₙC(O)R⁸⁻³, - C(O)(CH₂)ₙR⁸⁻³, C₁₋₆ alkyl NHS(O)₂-, C₁₋₆ alkyl NHC(O)-, -C(O)(CH₂)ₙN(R⁸⁻¹R⁸⁻²), - SO₂(CH₂)ₙN(R⁸⁻¹R⁸⁻²), (CH₂)ₙSO₂N(R⁸⁻¹R⁸⁻²), C₁-C₆ alkoxy, C₁-C₂ alkyl-amido, substituted or unsubstituted C₆-C₁₀ aryl-(CH₂)-, substituted or unsubstituted 5- to 12- membered heteroaryl-(CH₂)-; wherein, when a group is substituted, the "substituted" can be located at the CH, CH₂, or CH₃ position of the group;
wherein, the "substituted" refers to one or more hydrogen atoms on the group are substituted with substituents selected from the group consisting of: halogen, hydroxyl, carboxyl, benzyl, oxo(=O), -(CH₂)ₙN(R⁸⁻¹R⁸⁻²), -(CH₂)ₙC(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, - C(O)(CH₂)ₙR⁸⁻³, amino, C₁₋₆ alkyl NHS(O)₂-, C₁₋₆ alkyl NHC(O)-, -C(O)(CH₂)ₙN(R⁸⁻¹R⁸⁻²), -SO₂(CH₂)ₙN(R⁸⁻¹R⁸⁻²), (CH₂)ₙSO₂N(R⁸⁻¹R⁸⁻²), nitro, cyano, unsubstituted or halogenated C₁-C₆ alkyl, C₁-C₆ alkyl-OH, C₂-C₁₀ alkenyl, C₁-C₆ alkoxy, C₁-C₆ alkyl-amido, C₆-C₁₀ aryl, 5 or 6 membered heteroaryl, -O-(C₆-C₁₀ aryl), -O- (5 or 6 membered heteroaryl), and unsubstituted or halogenated C₃₋₇ heterocycloalkyl;
R⁸⁻¹ and R⁸⁻² are selected from the group consisting of: hydrogen, C₁₋₆ alkyl, C₁₋₆ haloalkyl, C₃₋₆ cycloalkyl, 4- to 6- membered heterocycloalkyl, or R⁸⁻¹, R⁸⁻² and the nitrogen atom to which they attached together form 4- to 6- membered heterocycloalkyl or 7- to 10-membered heterocycloalkyl; the cycloalkyl and heterocycloalkyl can be substituted with one or more substituents; wherein R⁸⁻³ is selected from the group consisting of: hydrogen, C₁₋₆ alkyl, C₁₋₆ alkoxy, C₁₋₆ haloalkyl, C₃₋₆ cycloalkyl, and 4- to 6- membered heterocycloalkyl; and the cycloalkyl and heterocycloalkyl can be substituted with one or more substituents selected from the group consisting of: halogen, OH, C₁₋₆ alkyl, C₁₋₆ alkyl substituted with C₁₋₆ alkoxy, C₁₋₆ alkyl substituted with hydroxyl, C₃₋₆ cycloalkyl, and 4- to 6- membered heterocycloalkyl; n is 0, 1, 2, 3, 4, 5, or 6;
unless otherwise specified, when appeared alone or as part of other groups, the heterocycloalkyl or heteroaryl has 1, 2, or 3 heteroatoms selected from N, O, and S; the heteroalkyl is an alkyl group in which 1-3 CH₂ or CH on the carbon chain are substituted by heteroatoms selected from the group consisting of: NH, N, O, and S; the cycloalkyl and heterocycloalkyl include saturated monocyclic ring, spiro ring, fused ring, and bridged ring; and the carbocyclyl and heterocyclyl include saturated or partially unsaturated non-aromatic rings, including monocyclic ring, spiro ring, fused ring, and bridged ring.

2. The compound of formula I according to claim 1, the pharmaceutically acceptable salt, the racemate, the R-isomer, the S-isomer, or mixtures thereof, wherein, the compound of formula I has a structure as shown in formula I-1', formula 1-2', formula I-3', or formula I-4': 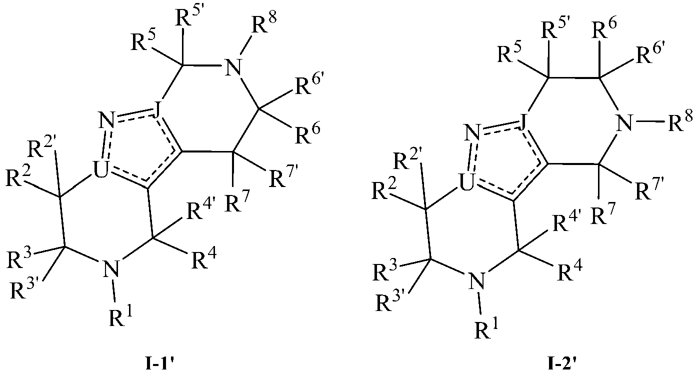 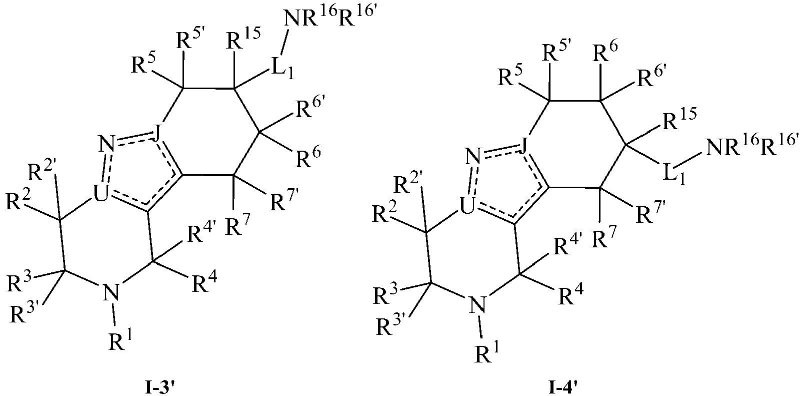
R⁵, R⁶, R⁷, R⁵', R⁶', and R⁷' are each independently selected from the group consisting of: H, deuterium, substituted or unsubstituted C₁₋₆ alkoxy, halogenated C₁₋₆ alkyl, substituted or unsubstituted C₁₋₆ alkyl, halogen, cyano, substituted or unsubstituted C₁₋₆ ester group, substituted or unsubstituted C₁₋₆ carboxylic acid, substituted or unsubstituted C₁₋₆ acylamino, substituted or unsubstituted C₃₋₇ cycloalkyl, hydroxyl, hydroxyl substituted C₁₋₆ alkyl, substituted or unsubstituted C₆-C₁₀ arylbenzyl, substituted or unsubstituted 5 or 6 membered heteroarylbenzyl, substituted or unsubstituted C₆-C₁₀ aryl, substituted or unsubstituted 5 or 6 membered heteroaryl, C₁₋₉ heteroalkyl with 1 to 3 heteroatoms, wherein the heteroatoms are selected from one or more of NH, N, O, and S; or two of R⁵, R⁶, R⁷ , R⁵', R⁶', and R⁷' located on the same carbon atom together form=O;
or R⁵, R⁵' and the carbon atom to which they attached, R⁶, R⁶' and the carbon atom to which they attached, or R⁷, R⁷' and the carbon atom to which they attachedtogether form a substituted or unsubstituted 3- to 6- membered cycloalky, or a substituted or unsubstituted 4- to 6- membered heterocyclyl;
or R⁵ and R⁶, R⁵ and R⁷, or R⁶ and R⁷ and the carbon atom to which they attached (optionally including the ring atom between the carbon atoms or nitrogen atom to which they attached) together form a substituted or unsubstituted 3- to 6- membered cycloalky, or a substituted or unsubstituted 4- to 9- membered heterocyclyl;
the definitions of the remaining groups are as described in claim 1.

3. The compound of formula I according to claims 1-2, the pharmaceutically acceptable salt, the racemate, the R-isomer, the S-isomer, or mixtures thereof, wherein, R¹ is selected from the group consisting of: 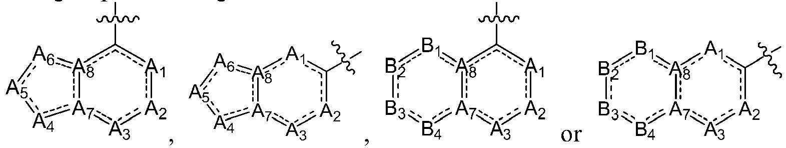
wherein, A₁, A₂, A₃, A₄, A₅, A₆, B₁, B₂, B₃, and B₄ are each independently selected from the group consisting of: CR, N, and NR¹⁴;
A₇ and A₈ are independently selected from the group consisting of: C, and N;
R¹⁴ is selected from the group consisting of: H, C₁₋₆ alkyl, C₁₋₆ alkyl substituted with halogen, and C₃₋₇ cycloalkyl; and
each R is independently selected from the group consisting of: H, halogen, cyano, C₁₋₆ alkyl, C₁₋₆ alkoxy, C₃₋₇ cycloalkyl, C₁₋₆ alkyl substituted with Ra, and C₁₋₆ alkoxy substituted with Rb; wherein, Ra and Rb are each independently selected from the group consisting of: OH, halogen, cyano, C₁₋₃ alkyl, and C₁₋₃ alkoxy.

4. The compound of formula I according to claims 1-2, the pharmaceutically acceptable salt, the racemate, the R-isomer, the S-isomer, or mixtures thereof, wherein, R¹ is selected from the group consisting of: 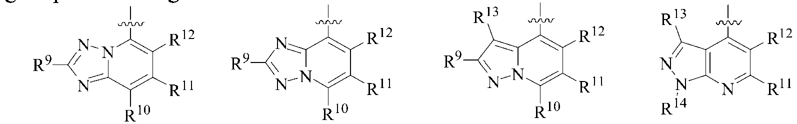 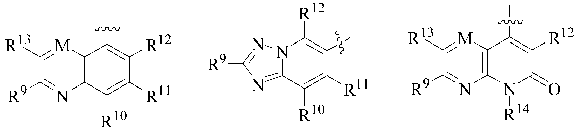
R⁹, R¹⁰, R¹¹, R¹², and R¹³ are each independently selected from the group consisting of: H, halogen, cyano, C₁₋₆ alkyl, C₁₋₆ alkoxy, C₃₋₇ cycloalkyl, C₁₋₆ alkyl substituted Ra, and C₁₋₆ alkoxy substituted with Rb; wherein, Ra and Rb are each independently selected from the group consisting of: OH, halogen, cyano, C₁₋₃ alkyl and C₁₋₃ alkoxy;
R¹⁴ is selected from the group consisting of: H, C₁₋₆ alkyl, C₁₋₆ alkyl substituted with halogen, and C₃₋₇ cycloalkyl; and
M is selected from the group consisting of: CH and N.

5. The compound of formula I according to claims 1-2, the pharmaceutically acceptable salt, the racemate, the R-isomer, the S-isomer, or mixtures thereof, wherein, the compound of formula I has a structure shown in formula II-1, formula II-2, formula II-2', formula II-3 or formula 11-4: 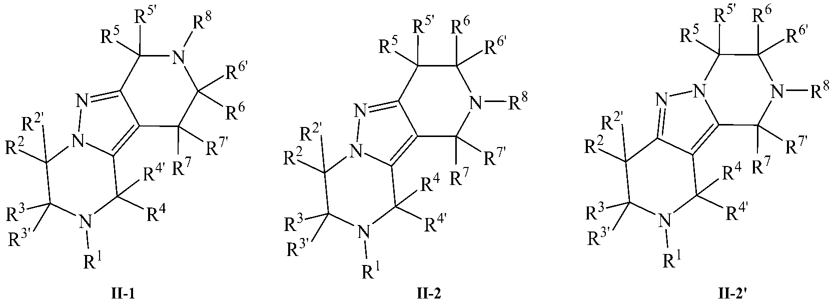 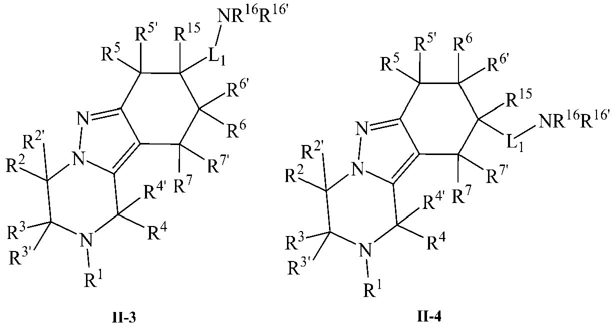 wherein, the definitions of each group are as described in claim 1.

6. The compound according to any one of claims 1-5, the pharmaceutically acceptable salt, the racemate, the R-isomer, the S-isomer, or mixtures thereof, wherein, the compound of formula I has a structure as shown in formula III-1, formula III-2, formula III-2', formula III-3 or formula III-4; 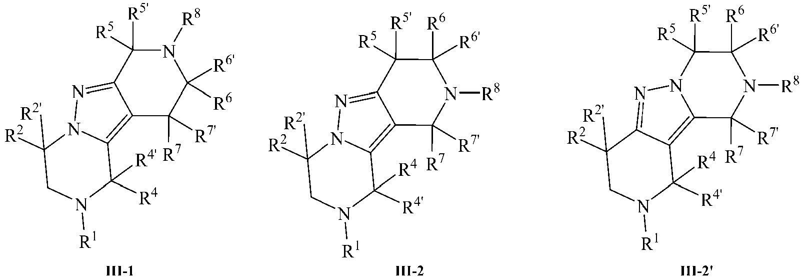 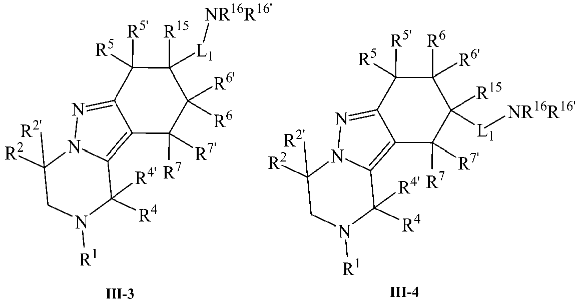 the definitions of each group are as described in claim 1.

7. The compound according to claim 6, the pharmaceutically acceptable salt, the racemate, the R-isomer, the S-isomer, or mixtures thereof, wherein, in the compounds of formula III-1, III-2, III-2', III-3, or III-4, R², R^{2'}, R⁴, R^{4'}, and R¹⁵ are each independently selected from the group consisting of: H, halogen, C₁₋₆ alkoxy, halogenated C₁₋₆ alkyl, C₁₋₆ alkyl, cyano, and C₃₋₇ cycloalkyl;
R⁵, R⁶, R⁷, R^{5'}, R^{6'}, and R⁷' are each independently selected from the group consisting of: H, deuterium, substituted or unsubstituted C₁₋₆ alkoxy, halogenated C₁₋₆ alkyl, substituted or unsubstituted C₁₋₆ alkyl, cyano, substituted or unsubstituted C₂₋₆ ester group, substituted or unsubstituted C₁₋₆ alkyl-COO, substituted or unsubstituted C₂₋₆ acylamino, substituted or unsubstituted (CH₂)C(O)N(R¹⁷)₂, substituted or unsubstituted C₃₋₇ cycloalkyl, hydroxyl, C₁₋₆ alkyl substituted with hydroxyl, substituted or unsubstituted benzyl, substituted or unsubstituted 5-to 9- membered heteroaryl-(CH₂)-, substituted or unsubstituted phenyl, substituted or unsubstituted 5-to 9- membered heteroaryl, substituted or unsubstituted C₁₋₉ heteroalkyl, substituted or unsubstituted 3-to 9- membered heterocycloalkyl, and substituted or unsubstituted 3-to 9- membered heterocycloalkyl-(CH₂)-, wherein the heteroatoms are selected from one or more of NH, N, O, and S; R¹⁷ is selected from the group consisting of: H, and substituted or unsubstituted C₁₋₆ alkyl;
or R⁵, R^{5'} and the carbon atom to which they attached, R⁶ and R⁶' and the carbon atom to which they attached, or R⁷ and R^{7'} and the carbon atom to which they attached together form a substituted or unsubstituted 3-to 6- membered cycloalkyl, or a substituted or unsubstituted 4-to 6- membered heterocyclyl;
or R⁵ and R⁶, R⁵ and R⁷, or R⁶ and R⁷ form a substituted or unsubstituted 3-to 6-membered cycloalkyl, or a substituted or unsubstituted 4-to 7- membered heterocyclyl;
or R⁶ and R⁶' together form=O; or R⁵ and R⁵' together form=O.

8. The compound according to any one of claims 1-7, the pharmaceutically acceptable salt, the racemate, the R-isomer, the S-isomer, or mixtures thereof, wherein,
R¹⁶ and R¹⁶' are each independently selected from the group consisting of: H, deuterium, substituted or unsubstituted C₁₋₆ alkyl, and benzyl;
or R¹⁶ and R^{16'} together form a substituted or unsubstituted 4-to 6- membered heterocycloalkyl;
R⁸ is selected from the group consisting of: H, deuterium, substituted or unsubstituted 3-to 9- membered carbocyclyl, substituted or unsubstituted 4-to 9- membered heterocyclyl, substituted or unsubstituted C₁₋₆ alkyl, and substituted or unsubstituted group selected from the group consisting of: -(CH₂)ₙN(R⁸⁻¹R⁸⁻²), -(CH₂)_{nC}(O)N(R⁸⁻¹R⁸⁻²), -(CD₂)ₙC(O)N(R⁸⁻¹R⁸⁻²), C(CH₃)₂N(R⁸⁻¹R⁸⁻²), -(CH₂)ₙC(O)OH, (CH₂)ₙSO₂R⁸⁻¹, -(CH₂)ₙC(O)R⁸⁻³, - C(O)(CH₂)ₙR⁸⁻³, C₁₋₆ alkyl NHS(O)₂-, C₁₋₆ alkyl NHC(O)-, -C(O)(CH₂)ₙN(R⁸⁻¹R⁸⁻²), - SO₂(CH₂)ₙN(R⁸⁻¹R⁸⁻²), -(CH₂)ₙSO₂N(R⁸⁻¹R³⁻²), C₁-C₆ alkoxy, C₁-C₂ alkyl-amido, C₆-C₁₀ aryl-(CH₂)-, and 5-to 9- membered heteroaryl-(CH₂)-;
wherein, R⁸⁻¹ and R⁸⁻² are selected from the group consisting of: hydrogen, C₁₋₆ alkyl, or R⁸⁻¹, R⁸⁻² and the N to which they attached together form a 4-to 6 -membered heterocycloalkyl, the cycloalkyl and heterocycloalkyl can be substituted with one or more halogens; R⁸⁻³ is selected from the group consisting of: hydrogen, C₁₋₆ alkyl, C₁₋₆ alkoxy, C₁₋₆ haloalkyl, C₃₋₆ cycloalkyl, and 4-to 6- membered heterocycloalkyl, the cycloalkyl and heterocycloalkyl can be substituted with one or more halogens or C₁₋₆ alkyl; n is 0, 1, 2, or 3.

9. The compound of formula I according to claim 1, the pharmaceutically acceptable salt, the racemate, the R-isomer, the S-isomer, or mixtures thereof, wherein, the compound is selected from the group consisting of:

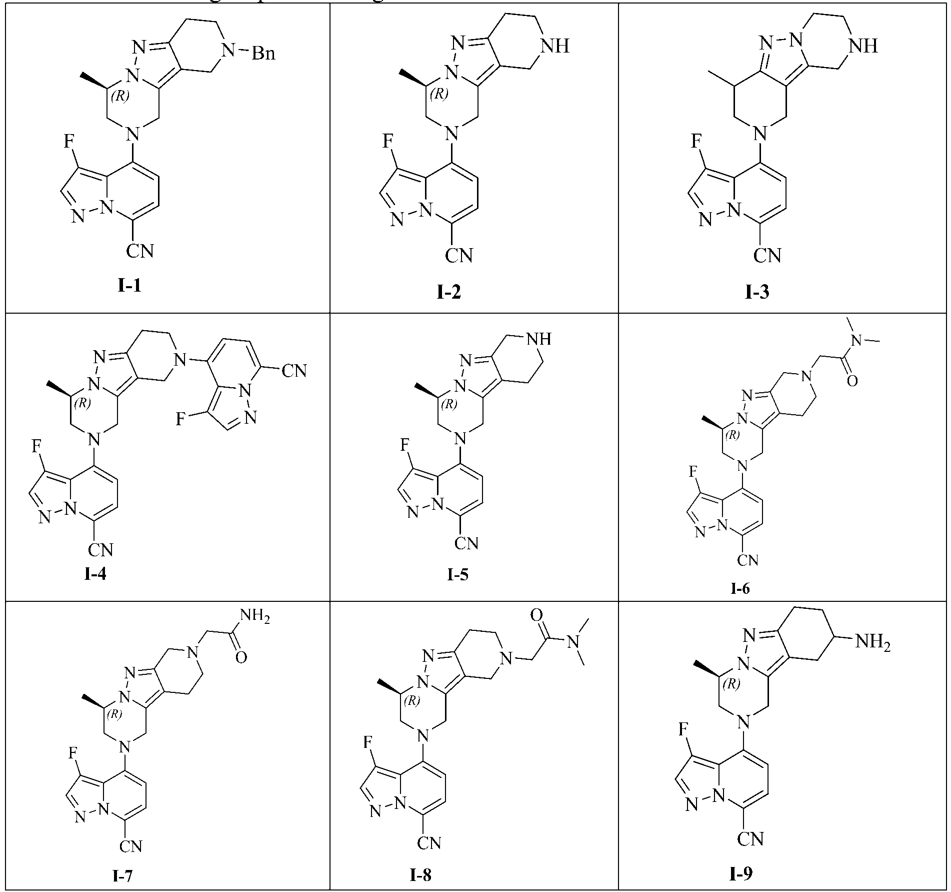

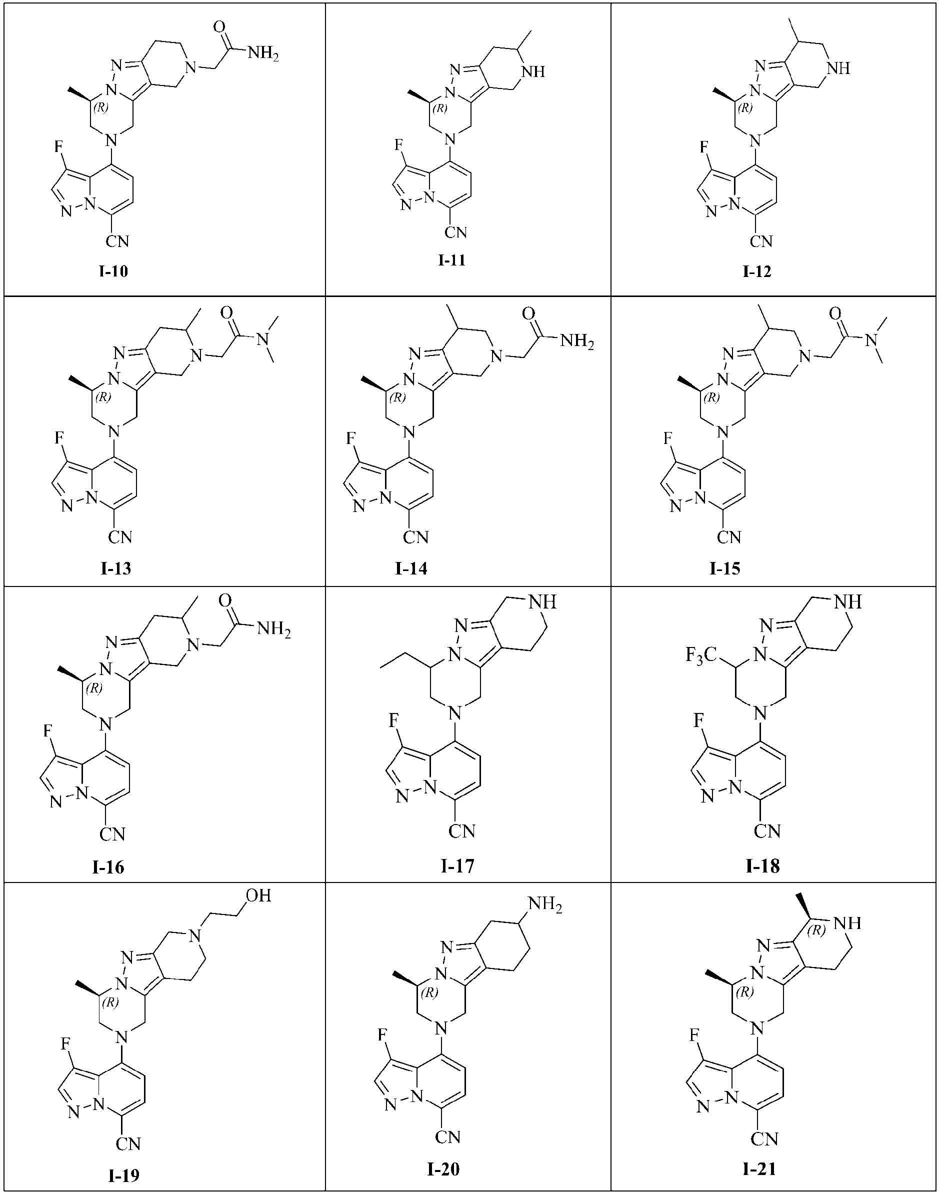

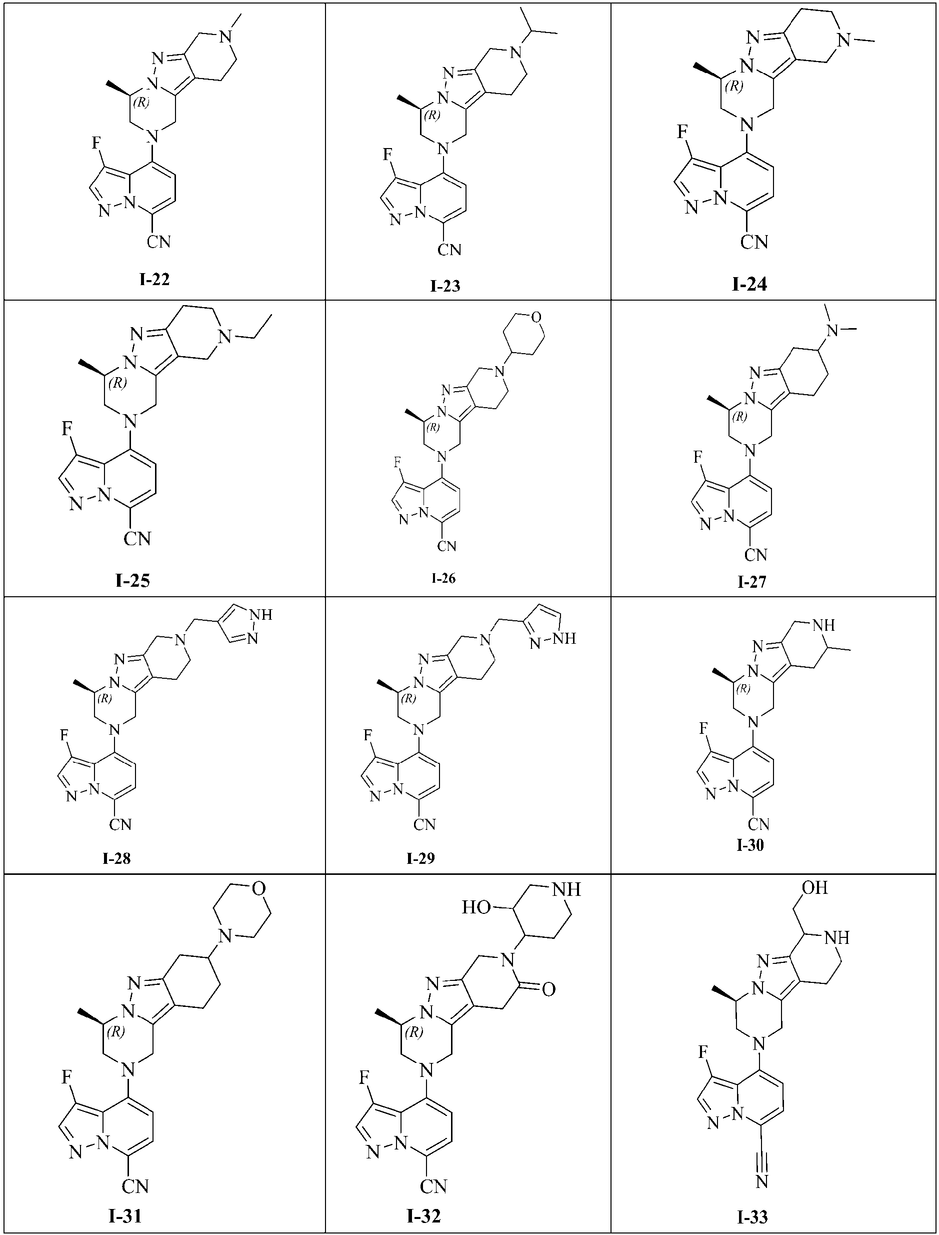

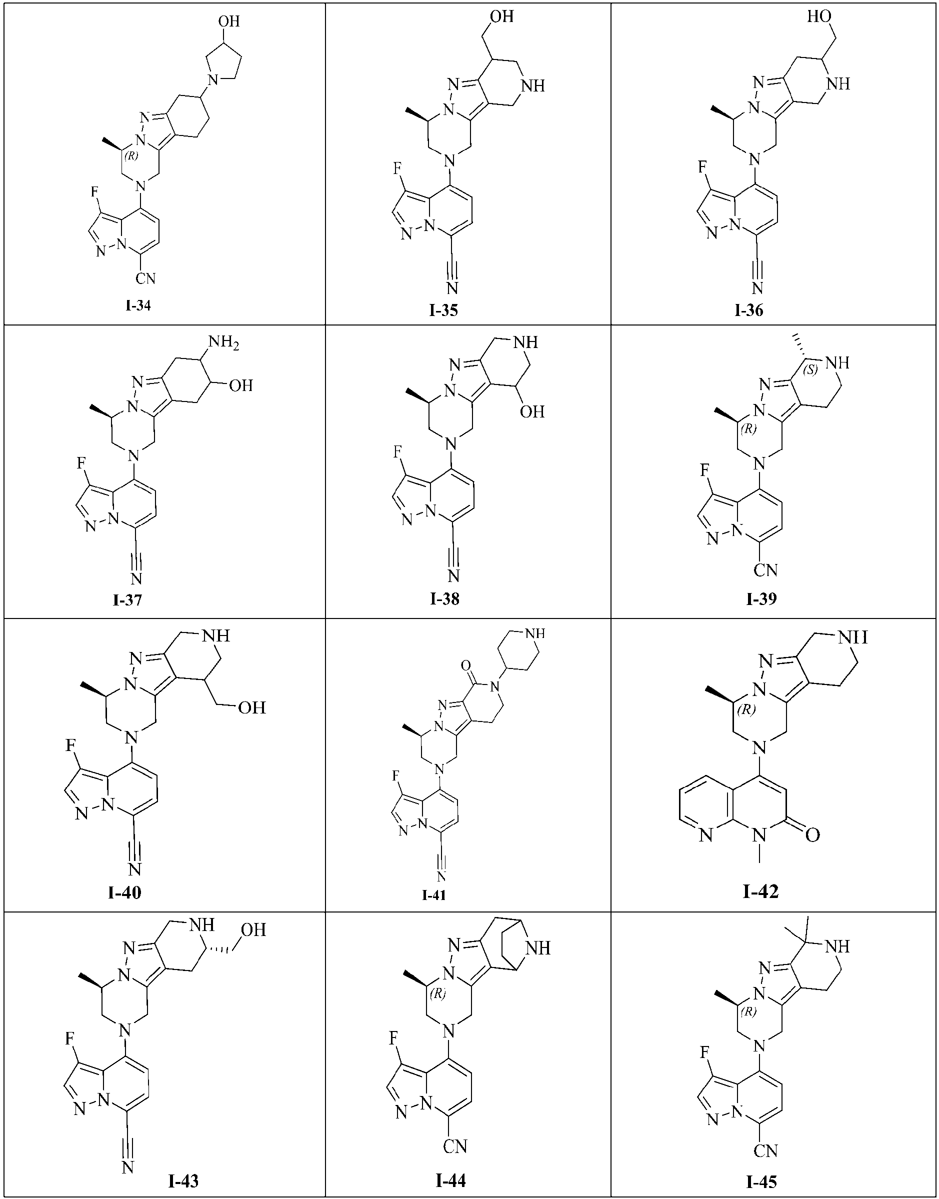

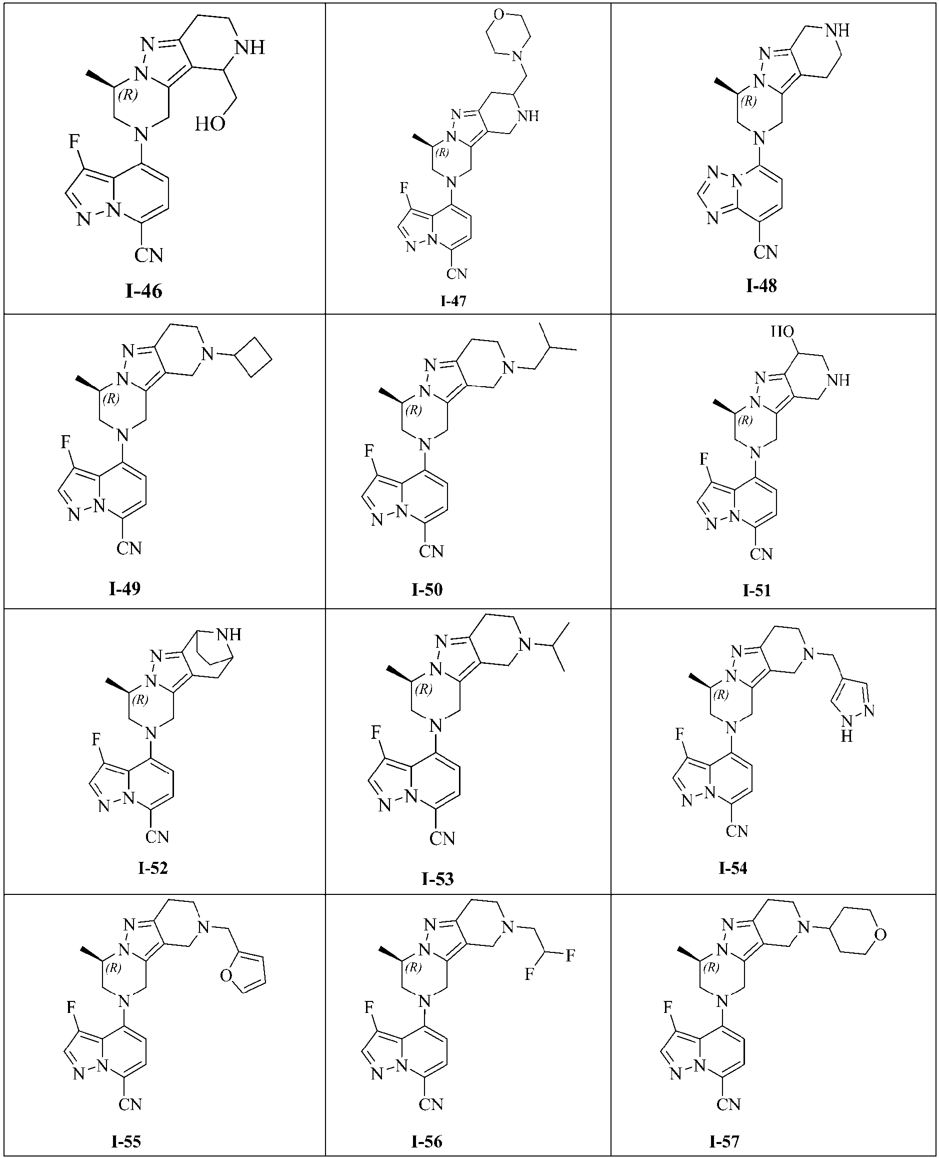

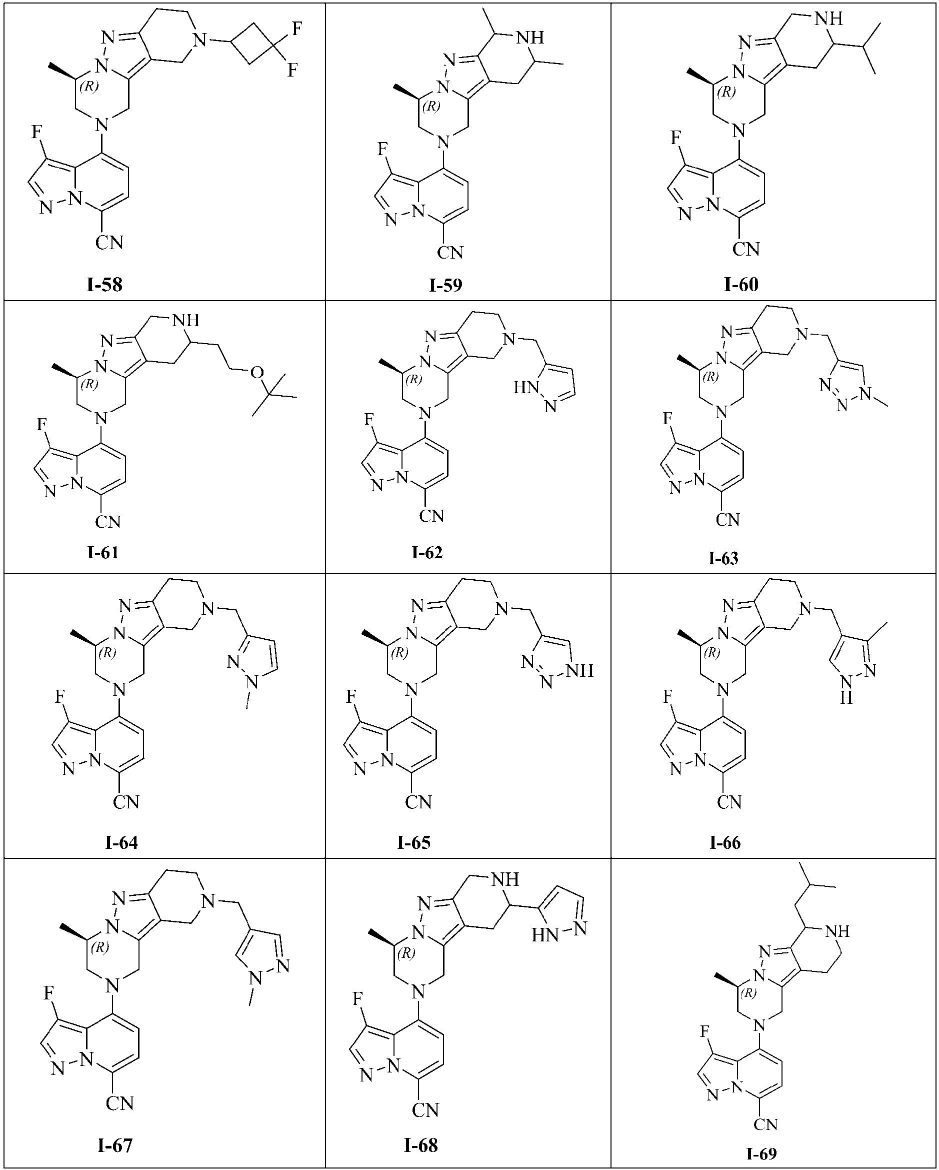

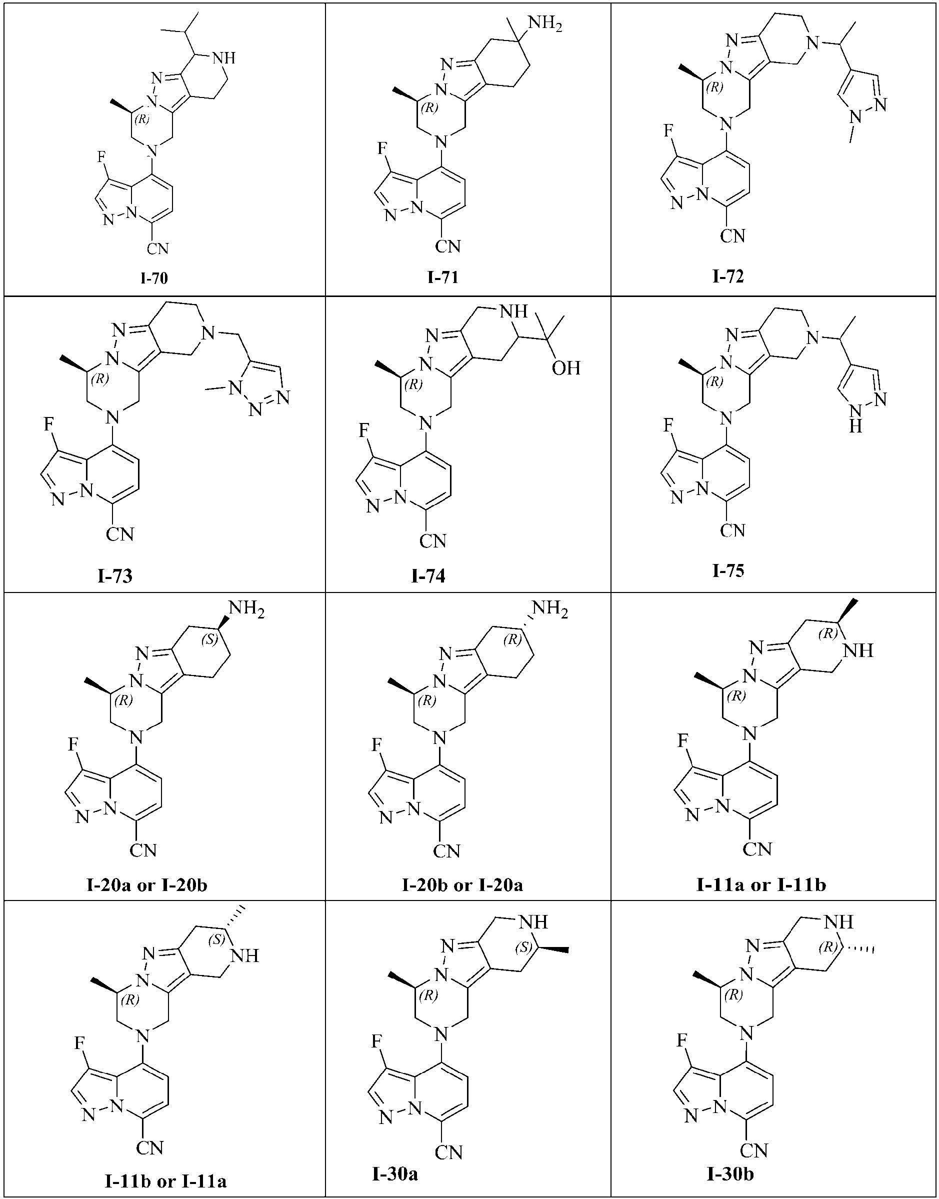

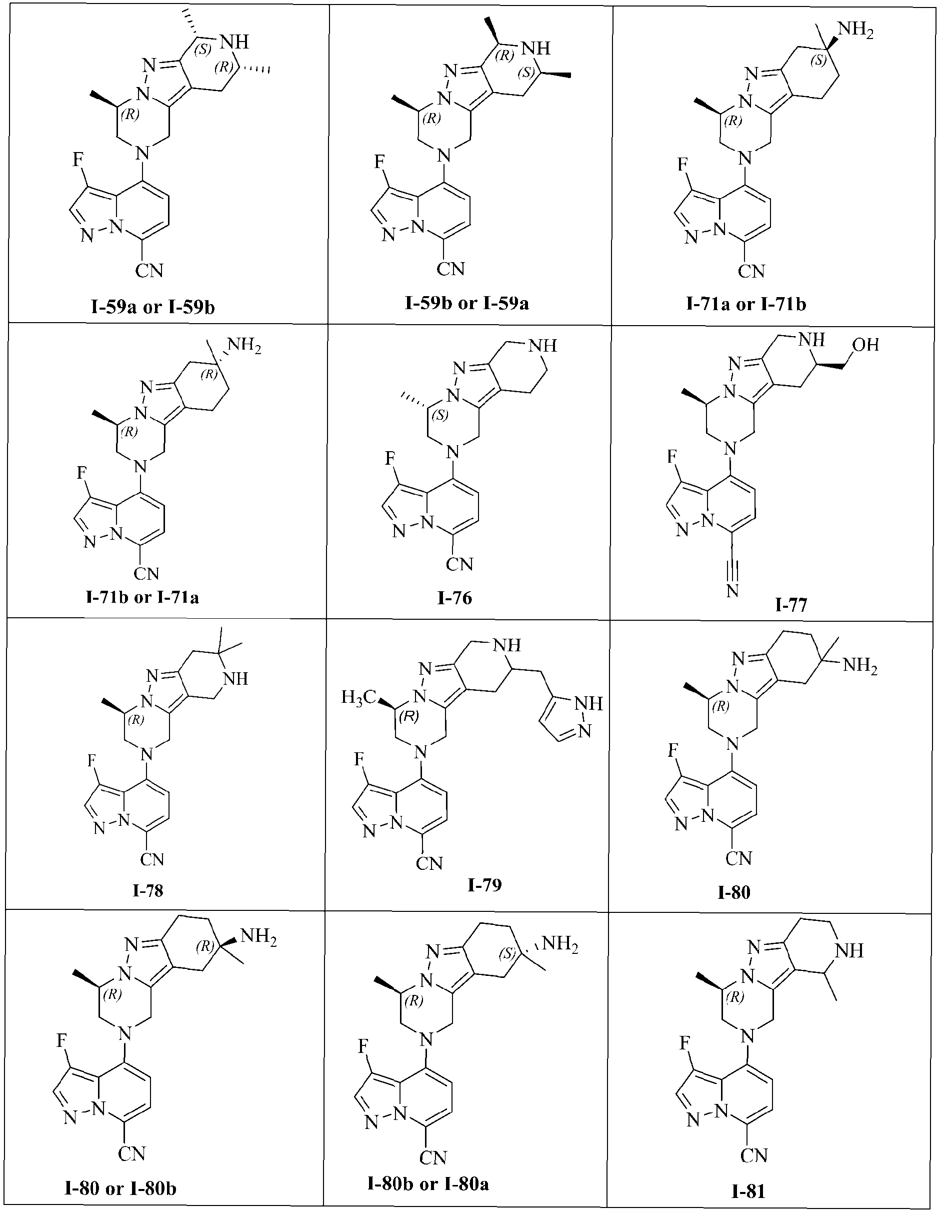

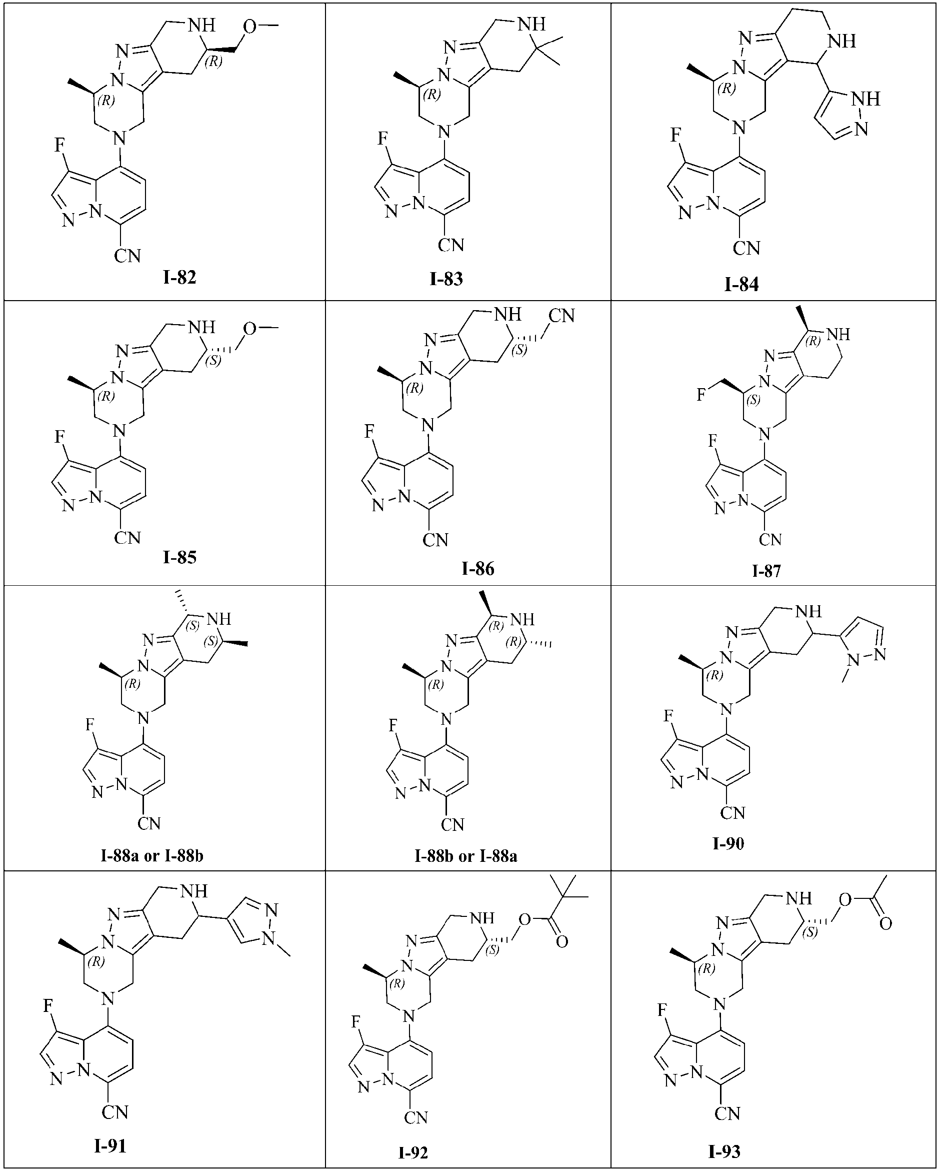

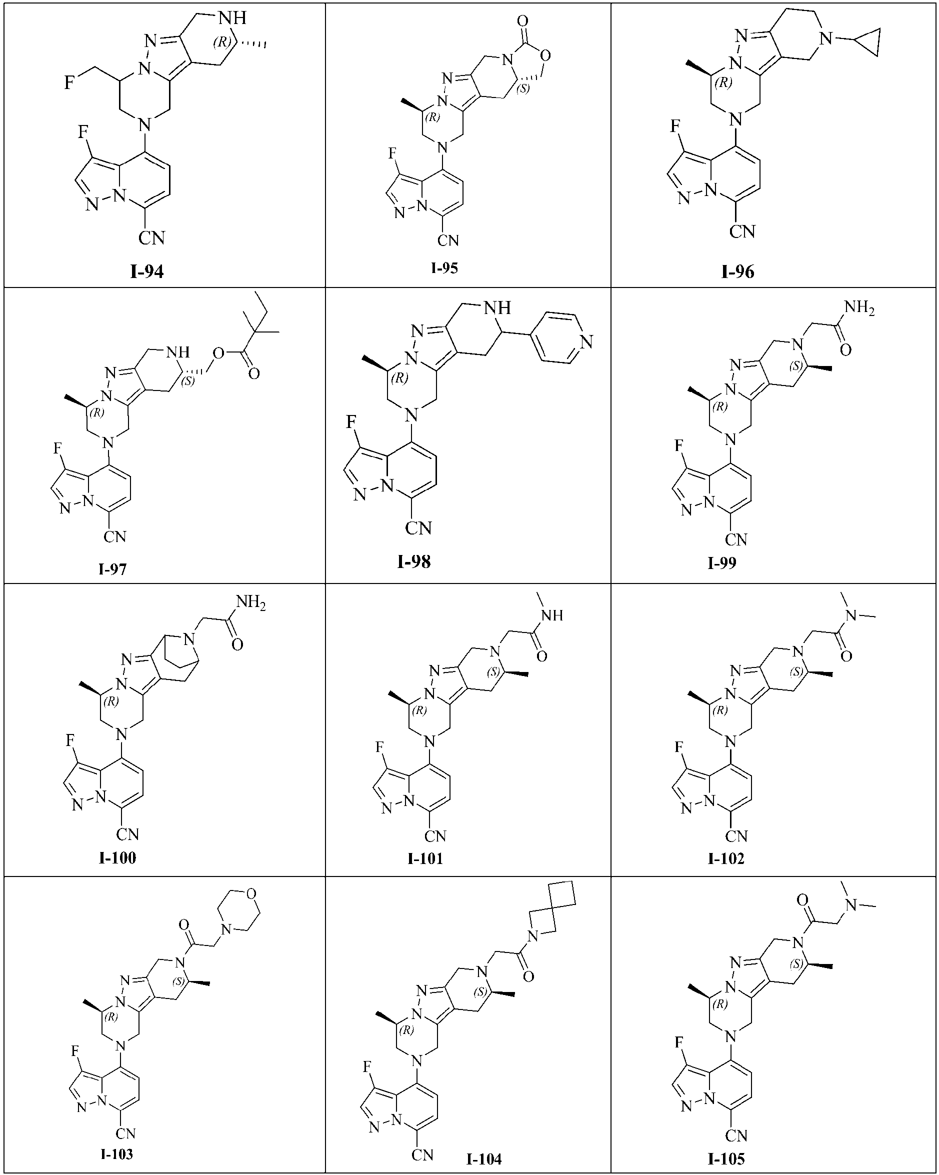

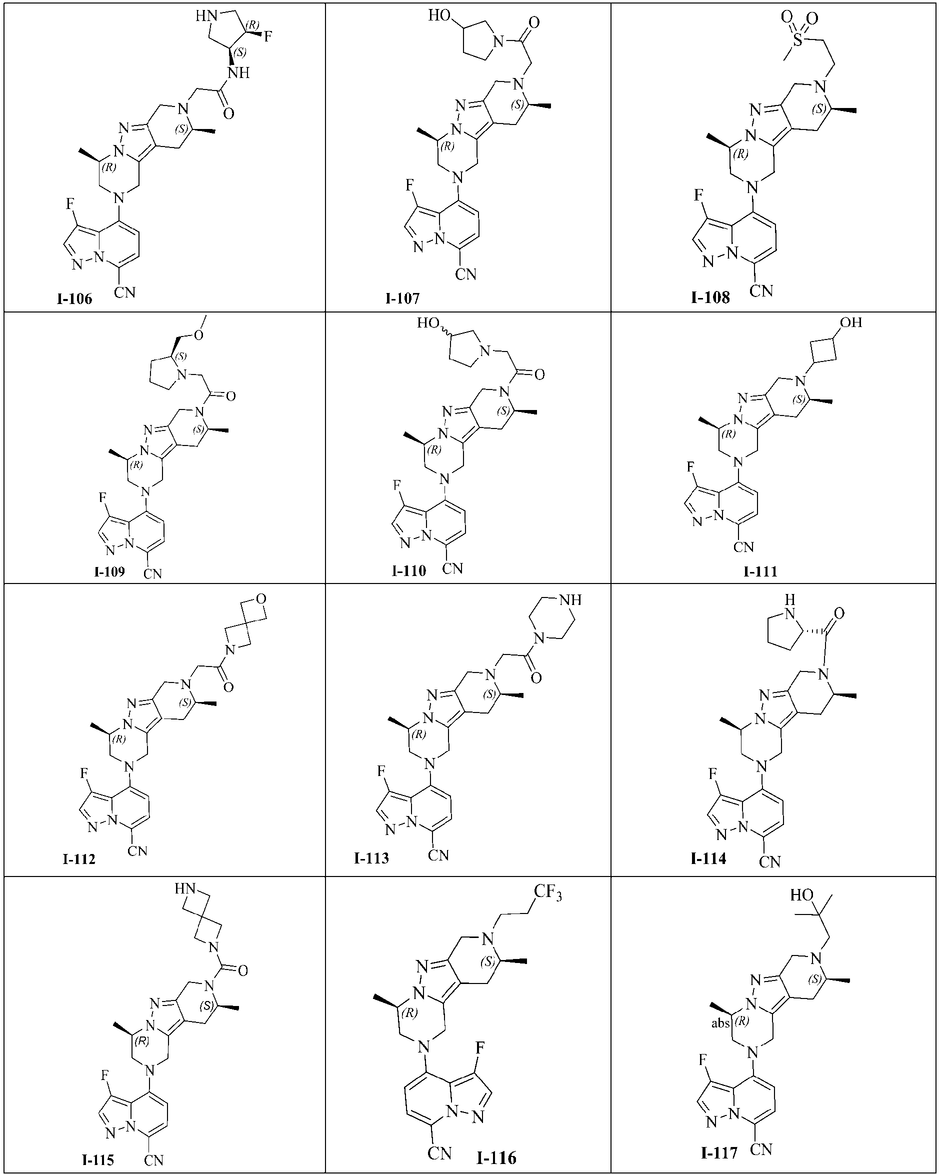

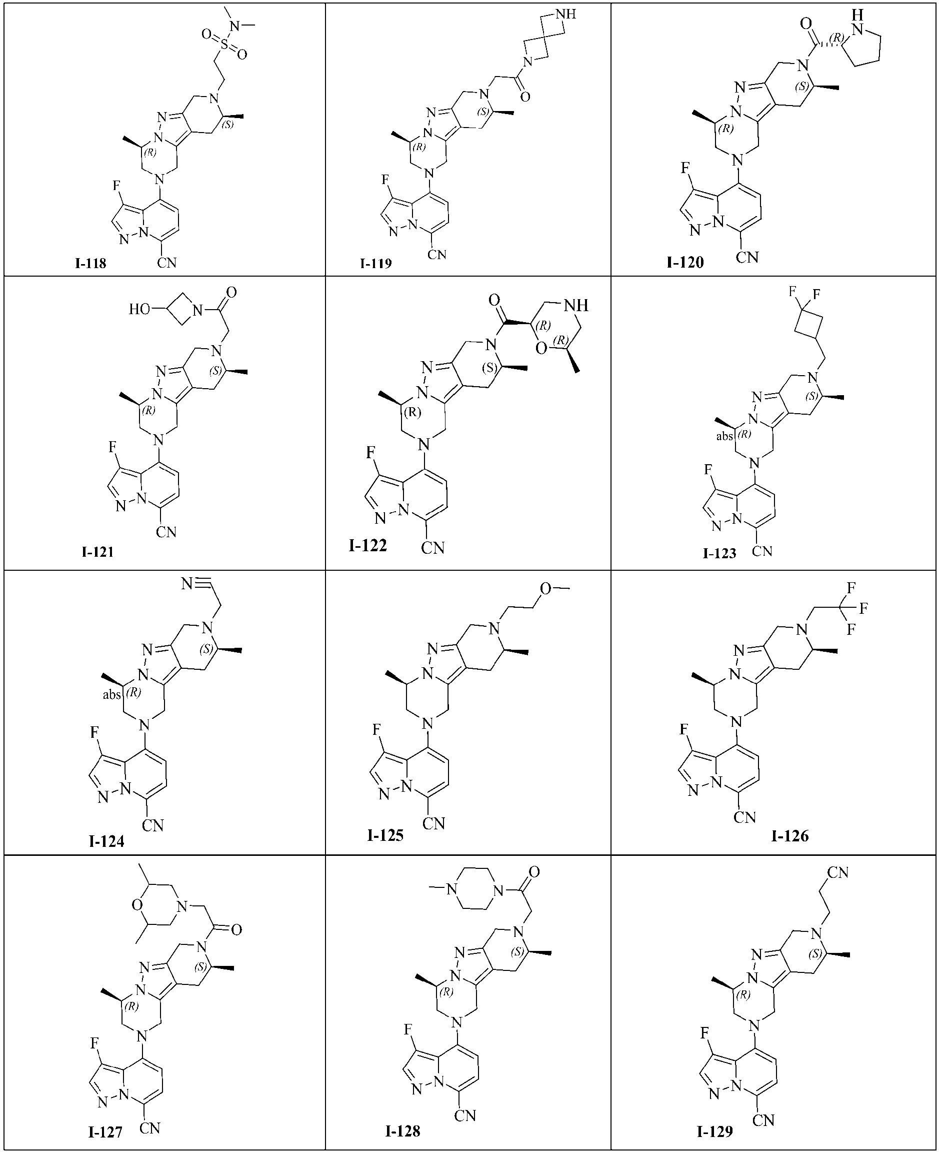

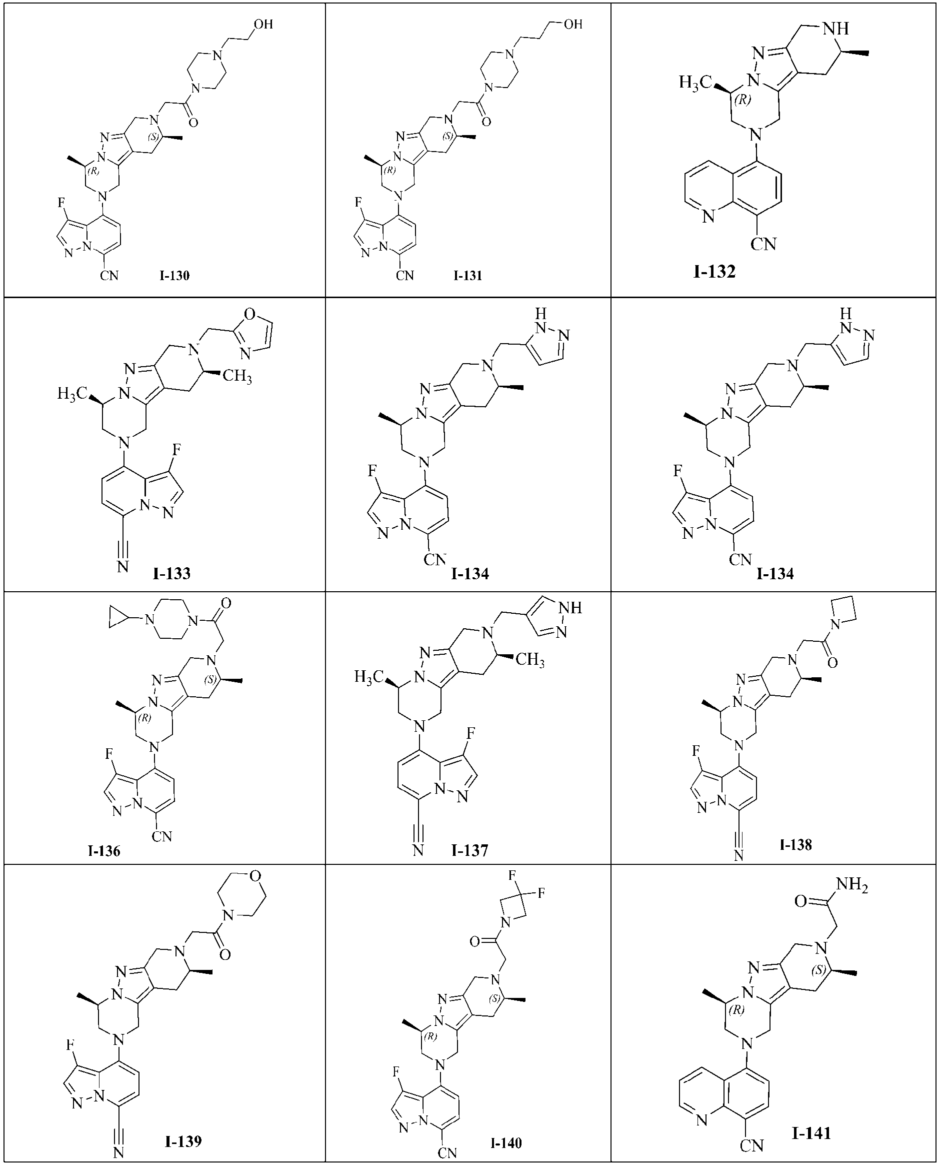

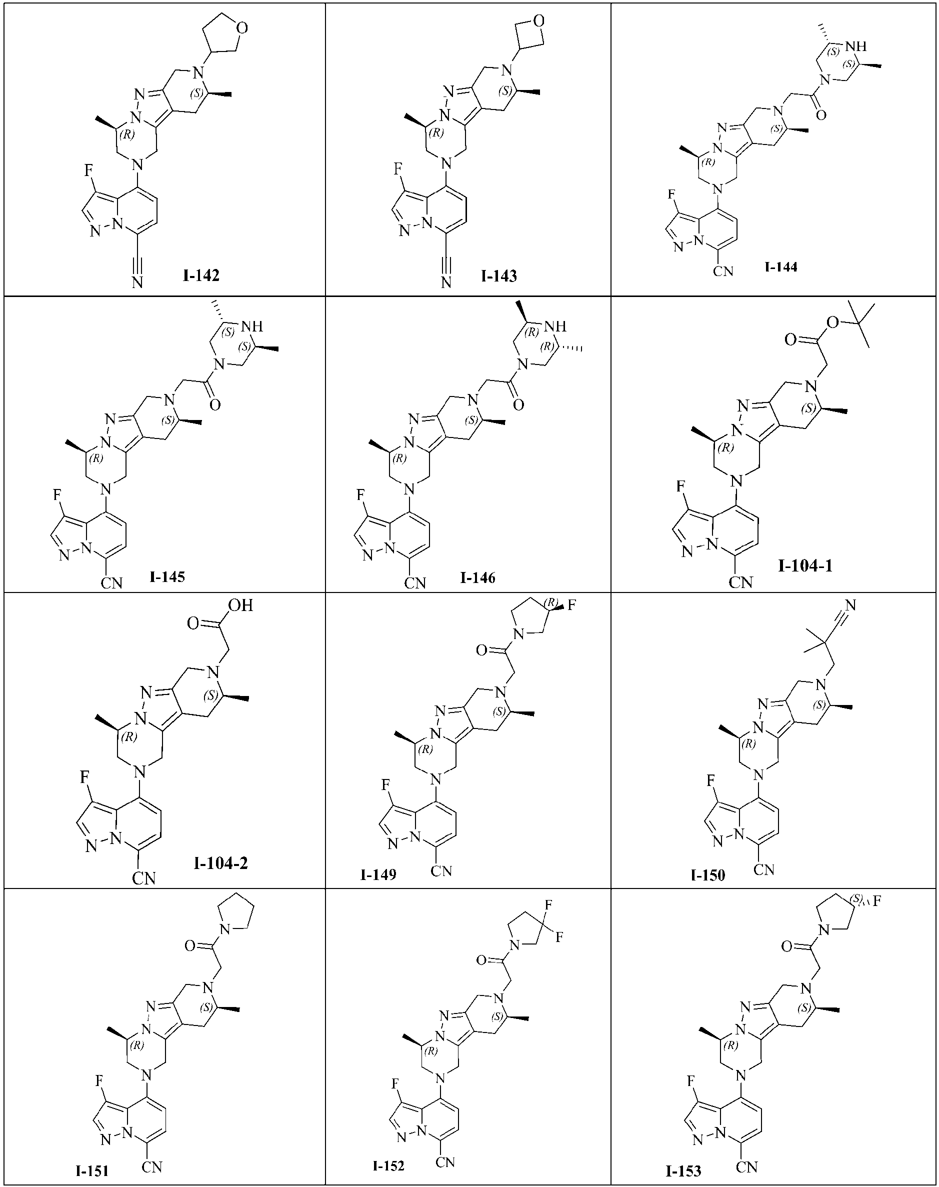

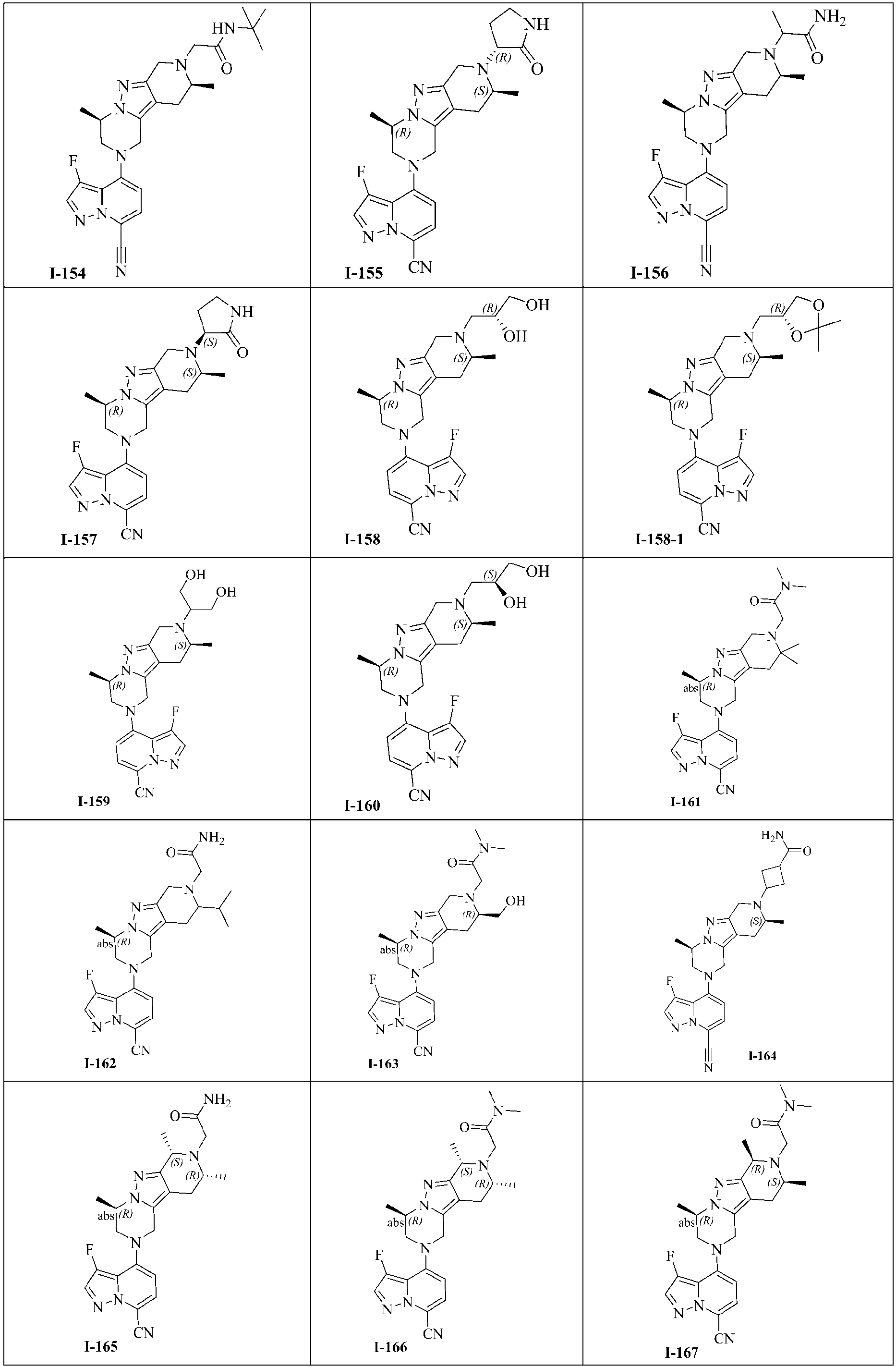

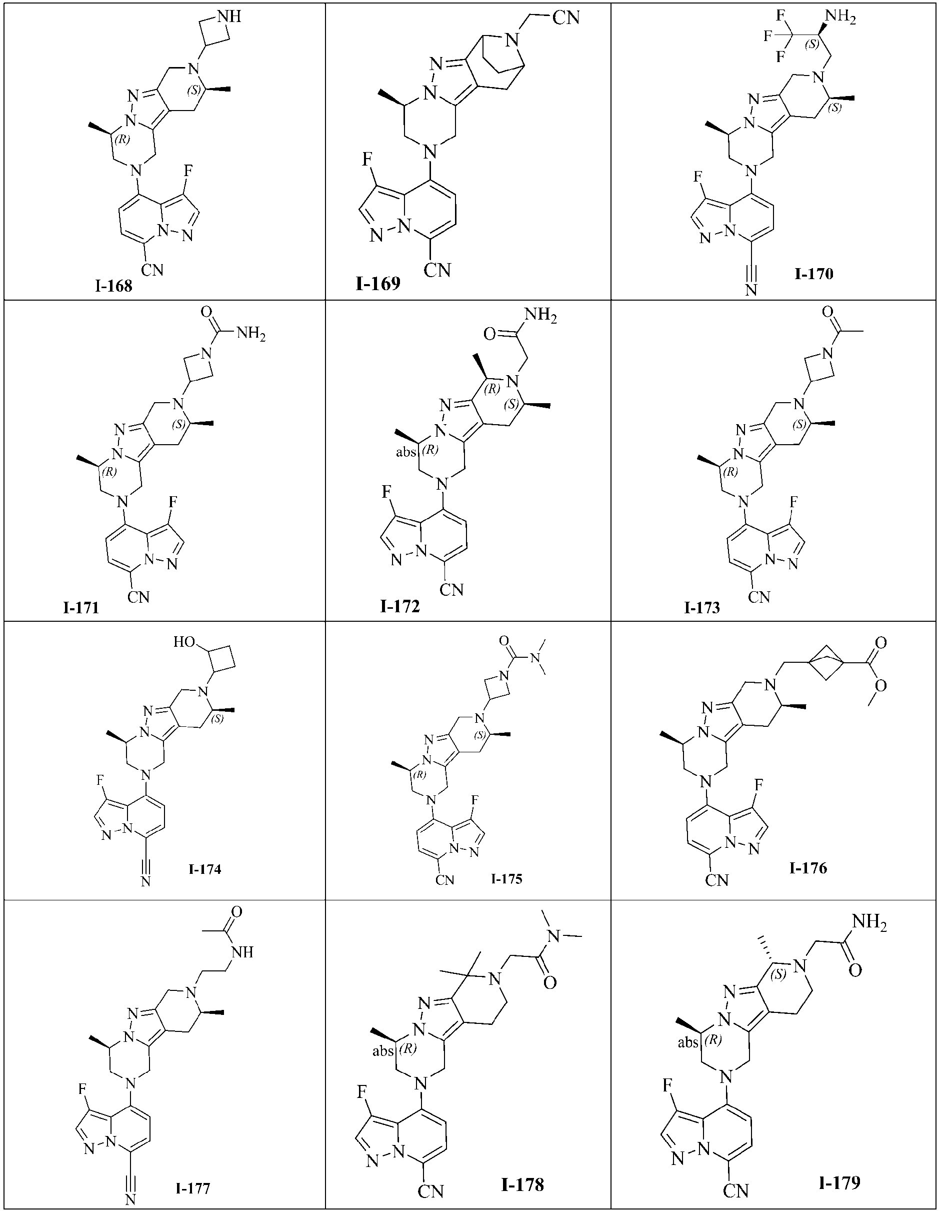

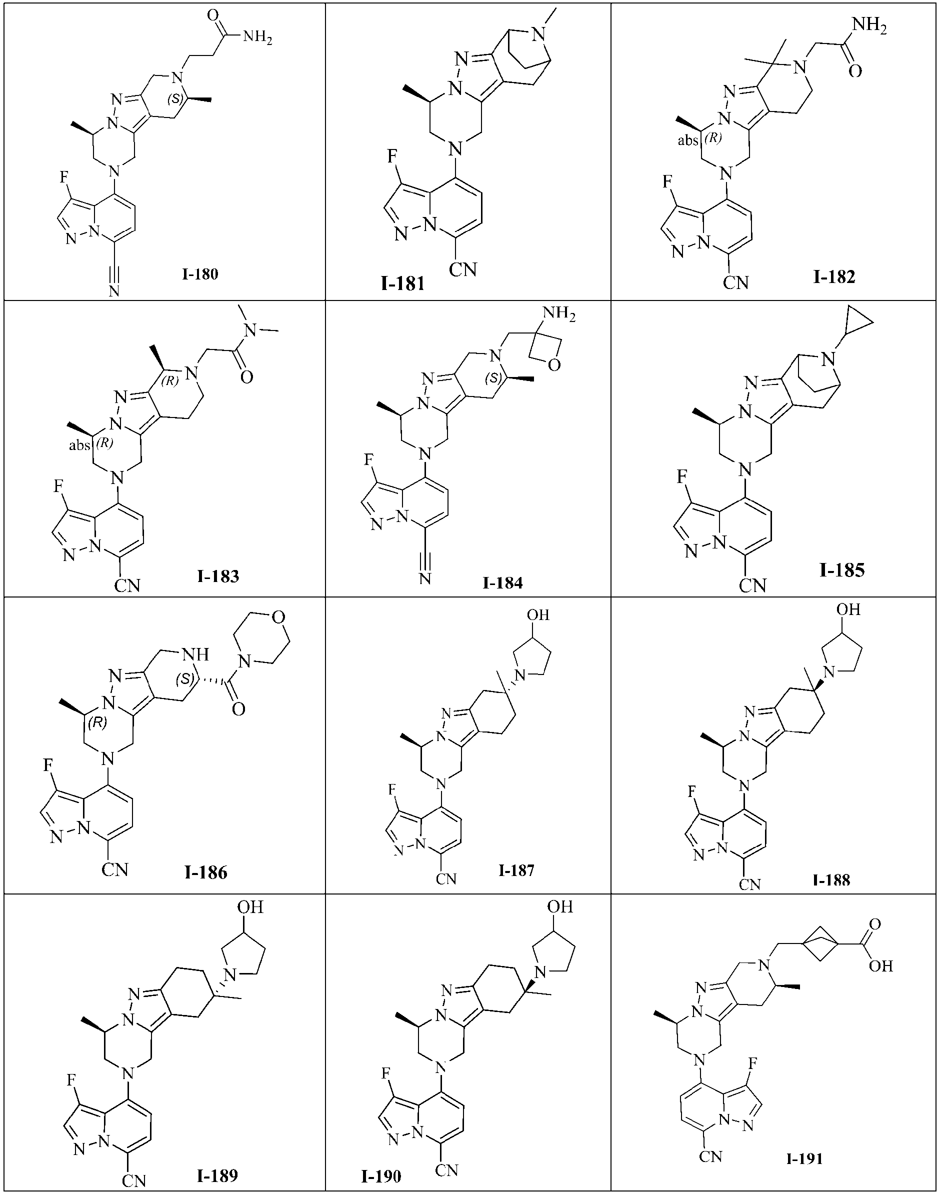

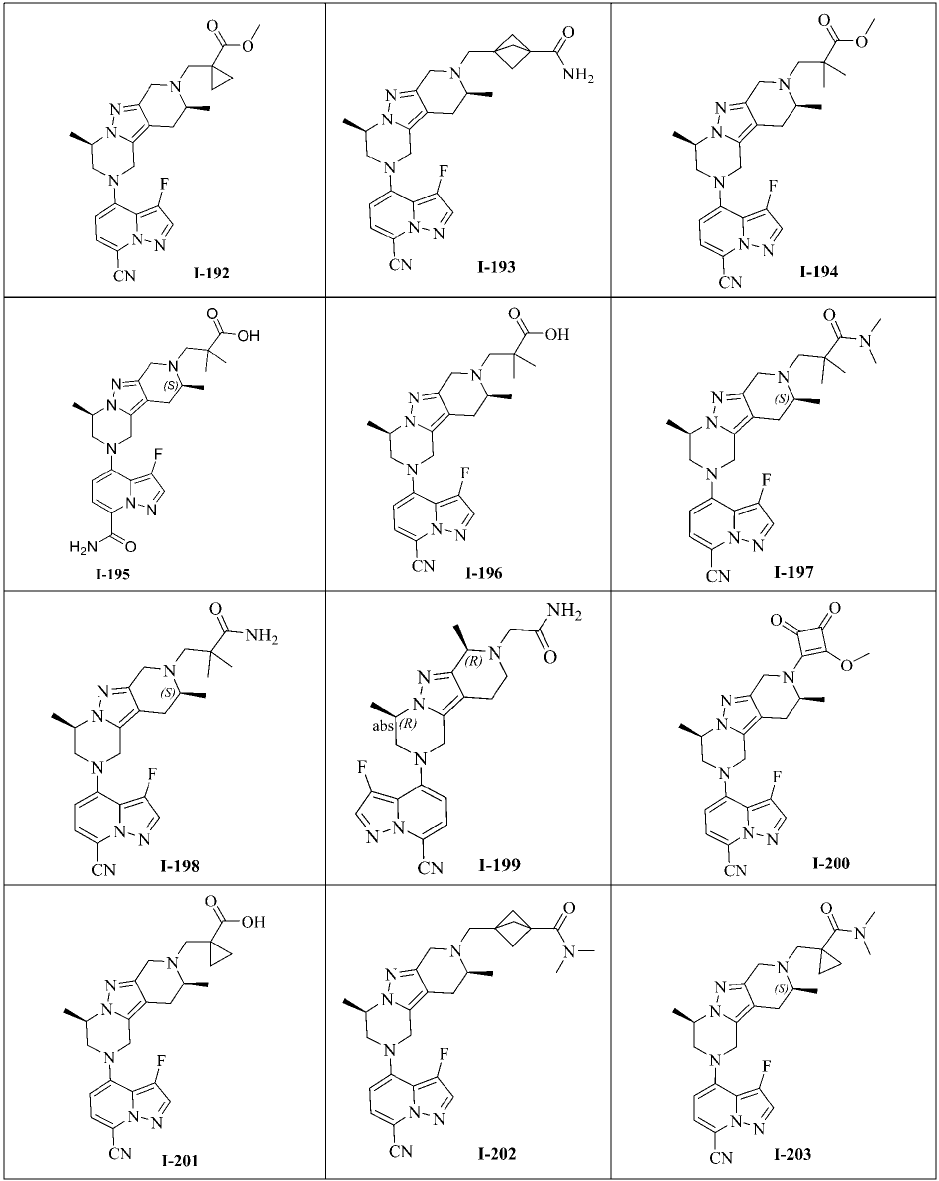

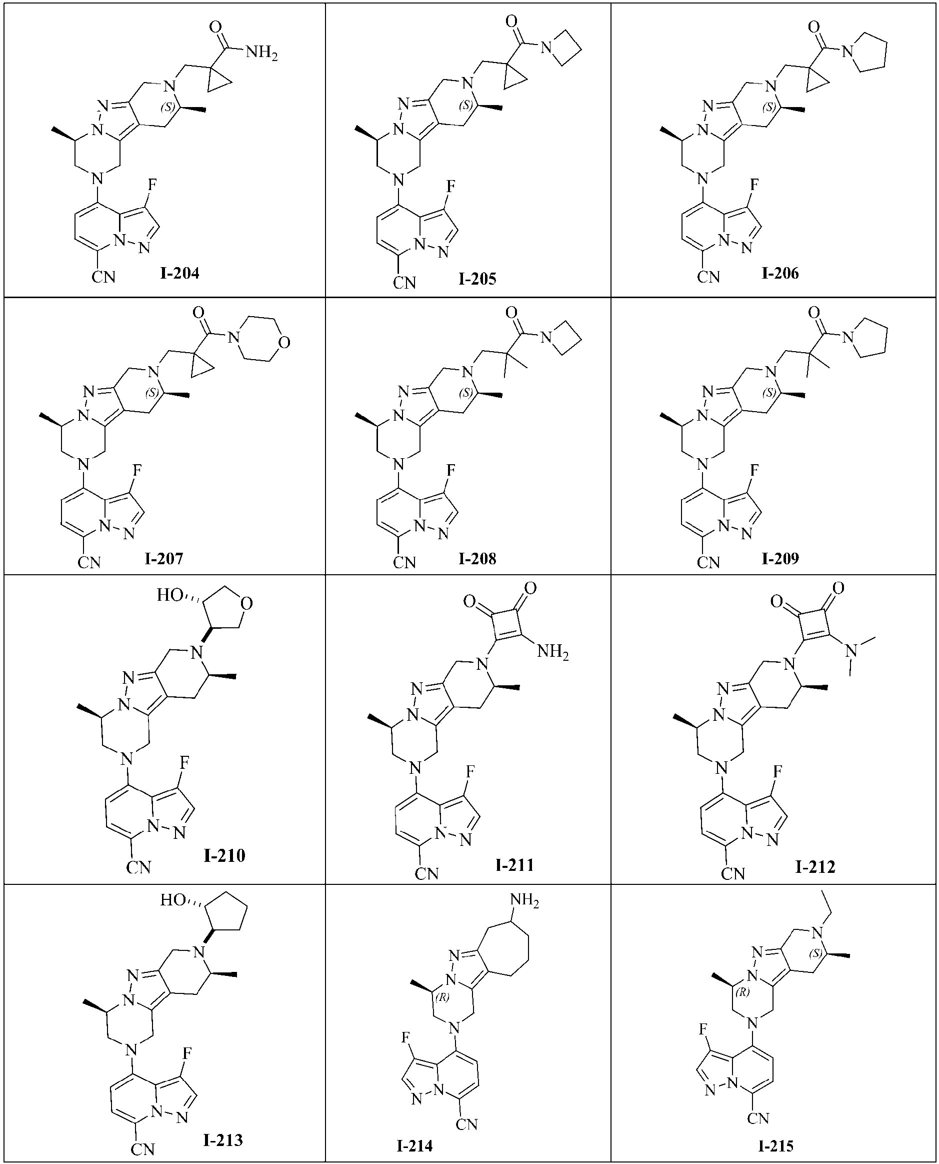

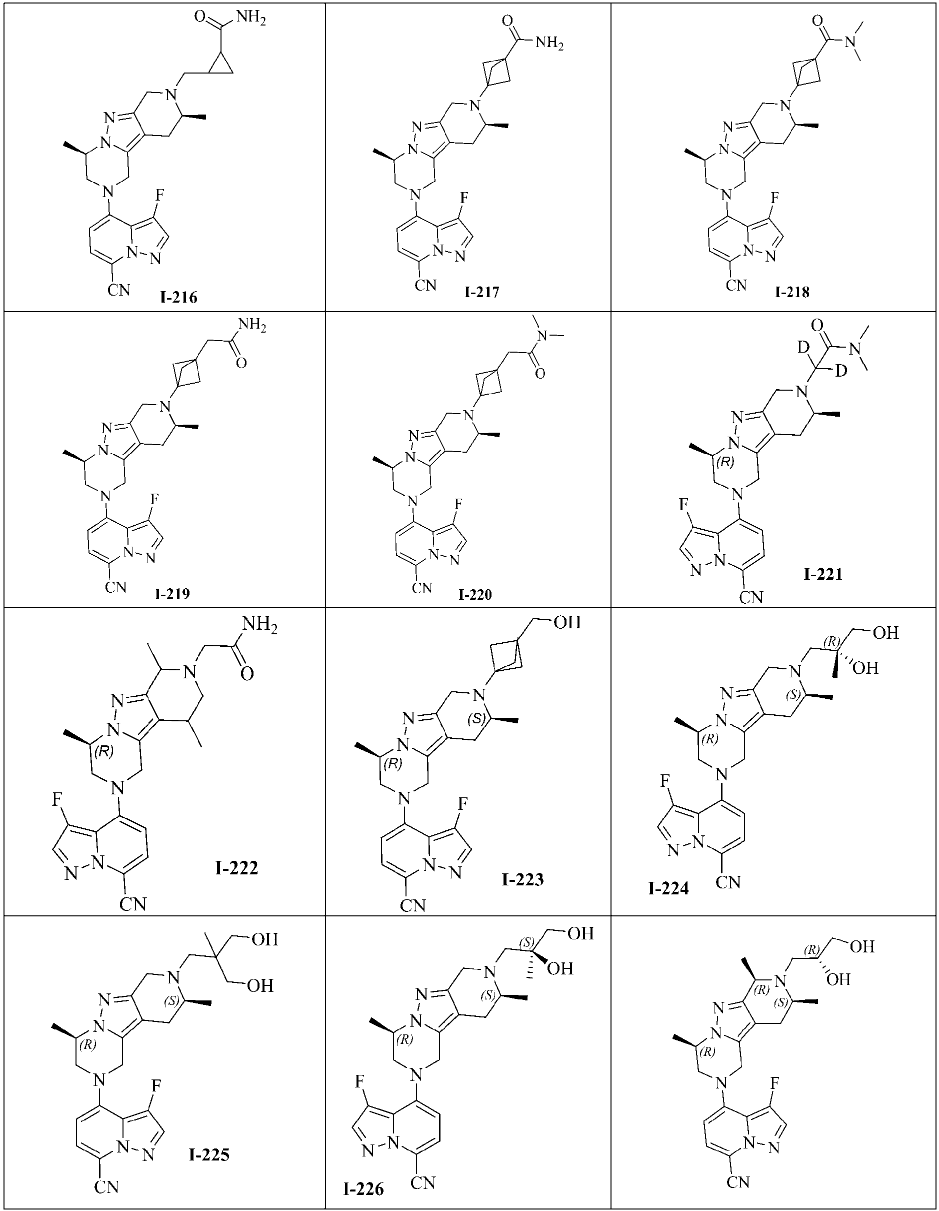

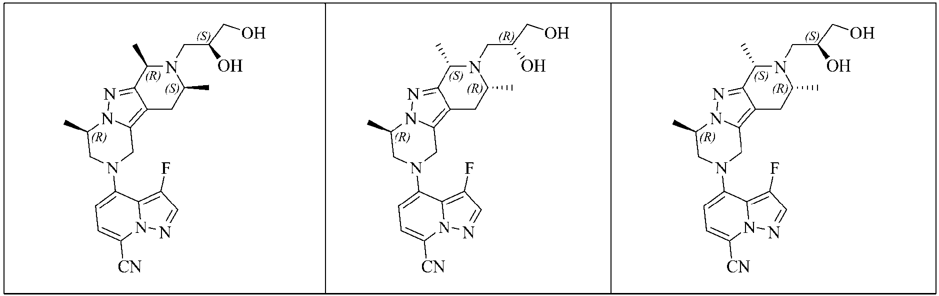

10. A pharmaceutical composition, wherein, the pharmaceutical composition comprises: one or more of the compound of formula I according to claim 1, the pharmaceutically acceptable salt, the racemate, the R-isomer, the S-isomer, ormixtures thereof, and one or more pharmaceutically acceptable carriers, excipients, adjuvants, excipients, and/or diluents.

11. A use of the compound of formula I according to claim 1, the pharmaceutically acceptable salt, the racemate, the R-isomer, the S-isomer, or mixtures thereof in the preparation of a pharmaceutical composition for the treatment or prevention of autoimmune diseases or chronic inflammatory diseases.

12. The use according to claim 11, wherein, the disease is selected from the group consisting of: Sjogren's syndrome, systemic lupus erythematosus, multiple sclerosis, rheumatoid arthritis, systemic sclerosis, psoriasis, systemic lupus erythematosus, and lupus nephritis.

13. An intermediate shown in formula B: 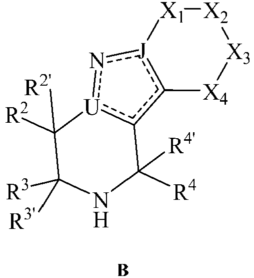
wherein, the definitions of each group are as described in claim 1;
preferably, the intermediate is selected from the group consisting of:
